# EUROPEAN PATENT APPLICATION

(11) **EP 4 725 942 A1**
(43) Date of publication of application: **15.04.2026**
(21) Application number: 24205638.0
(22) Date of filing: 09.10.2024
(51) Int. Cl.: C07D 213/74, C07D 213/81, C07D 239/48, C07D 401/04, C07D 401/06, C07D 401/14, C07D 403/12, C07D 405/14, C07D 413/04, C07D 417/14, C07D 471/04, C07D 495/08, C07D 498/08, C07D 513/08, A61P 35/00, A61K 31/4439, A61K 31/444, A61K 31/506

(54) **COMPOUNDS ACTIVE ON CYCLIN D1 PATHWAY AND THEIR USE**

(71) Applicant: Sibylla Biotech S.p.A., 20091 Bresso (IT)
(72) Inventor: SPAGNOLLI, Giovanni, 20091 Bresso (IT); MASSIGNAN, Tania, 20091 Bresso (IT); BONIFATI, Serena, 20091 Bresso (IT); PATSILINAKOS, Alexandros, 20091 Bresso (IT); TERRUZZI, Luca, 20091 Bresso (IT); BOLDRINI, Alberto, 20091 Bresso (IT); COLASURDO, Enrica, 20091 Bresso (IT); ASTOLFI, Andrea, 20091 Bresso (IT); IVANOVA BENCHEVA, Leda, 20091 Bresso (IT)
(74) Representative: Merli, Silvia

(57) **Abstract**

The present invention relates to compounds of general formula (I), compositions thereof and their use in methods for treating or preventing cancer.

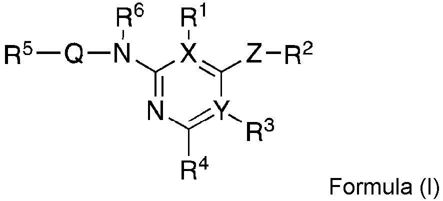

## Description

### Background

Cyclins are a group of related proteins involved in controlling cell cycle progression. They exert their main biological function by activating cyclin-dependent kinases (CDKs), a class of enzymes responsible for the phosphorylation of downstream targets involved in regulating cell proliferation (D. G. Johnson, and C. L. Walker, Annu Rev Pharmacol Toxicol, 1999; 39: 295-312).

Different cyclins display distinct expression and degradation patterns required for the temporal coordination of mitotic events. In particular, Cyclin D1 binds CDK4 and CDK6 and functions as their regulatory subunit. As a consequence of this interaction, CDK4/6 are activated. Such an event triggers a signaling pathway that results in the expression of genes for cell cycle progression from the G1 to the S phase (Fassl et al., Science, 2022; 375: eabc1495).

Cyclin D1 also exhibits distinctive functions that are not dependent on CDKs, such as augmenting or repressing gene expression, chromosomal stability, interacting with the DNA double-strand break (DSB) repair machinery, modulating cell migration and adhesion.

Cyclin D1 binds and directly controls the transcription of hormone receptors such as Estrogen receptors (ER) or Androgen receptor (AR) in breast cancer and liver cancer respectively. By binding to transcription factors as the Signal Transducer and Activator of Transcription 3 (STAT3) or the Dentin Matrix acidic Phosphoprotein 1 (DMP1), Cyclin D1 is involved in cell transformation in breast cancers (Inoue K, Fry EA. Sign Transduct Insights. 2015;4:1-13).

Cyclin D1 is able to control the DNA Damage Response (DDR) pathway as a downstream effector (Jirawatnotai S, Hu Y, Livingston DM, et al. Cancer Res. 2012;72:4289-4293). Furthermore, Cyclin D1 binds in a CDK4-independent manner to protein directly involved in the DDR machinery such as the RAD51/BRCA2 where it facilitates the Homologus Recombination (HR)-mediated DNA repair machinery of several tumours (Chalermrujinanant C, et al. Oncogene. 2016;35:2815-2823). Epithelial cells depleted of Cyclin D1 show an increased adhesion and reduced migration.

From a structural standpoint, Cyclin D1 is a globular protein made of 12 α-helices, and it is composed of five main motives and subdomains:
- The retinoblastoma protein binding motif.
- The cyclin box subdomain for CDK binding.
- The LxxLL binding motif for co-activator recruitment.
- The repressor subdomain.
- The PEST sequence: when phosphorylated, it results in Cyclin D1 degradation.

In cancer, the Cyclin D1 gene (CCND1) represents the second most amplified locus across all types of human tumours (Hydbring P. et al. Nat Rev Mol Cell Biol. 2016 May;17(5):280-92). Furthermore, the overexpression of Cyclin D1 correlates with tumor progression and angiogenesis, as well as increased resistance to chemotherapy and protection from apoptosis. This protein is found to be overexpressed in different types of tumours, including head and neck squamous cell carcinoma (Michalides R. et al. Cancer Res. 1995;55(5):975-8), nasopharyngeal carcinoma (NPC) (Zhou L. et al. Mol Ther Nucleic Acids 2020;22:1025-1039), small cell lung cancer (George J. et al. Nature. 2015;524:47-53), non-small-cell lung cancer (NSCLC) (Gautschi O. et al. Lung Cancer. 2007;55(1):1-14), endometrial cancer (Yildirim HT. et al. Indian J Pathol Microbiol. 2020;63(3):412-417), melanoma (Donigan JM, et al. Appl Immunohistochem Mol Morphol. 2017;25(2):91-94), pancreatic cancer (Zhang Y. et al. Mol Med Rep. 2017;16(6):9256-9262.; Kolsi LE et al. Sci Rep. 2018;8(1):15923; Capurso G. et al. J Mol Endocrinol. 2012;49:R37-R50), gastric cancer (Yuan C. et al. J Exp Clin Cancer Res. 2016,35:15), breast cancer (Abdalla AN. et al. Molecules. 2020,25(20):4606; Augello MA, et al. J Clin Invest. 2012;123:493-508), colorectal cancer (Albasri A. et al. Asian Pac J Cancer Prev. 2014;15(7):3133-7; Slattery ML, Herrick JS, Mullany LE, et al. Genes Chromosomes Cancer. 2017;56:769-787), mantle cell lymphoma (Navarro A. et al. Hematol Oncol Clin North Am. 2020;34(5):795-807), multiple myeloma (Sewify EM et al. Clin Lymphoma Myeloma Leuk. 2014;14(3):215-22), prostate cancer (Casimiro MC et al. Cancer Res. 2016;76(2):329-38), and papillary thyroid carcinoma (PTC) (Cai W. et al. Front Oncol. 2023; 13:1145082).

Given the pivotal role of Cyclin D1 in tumor growth and proliferation, exploiting a small molecule approach aimed at inducing the depletion of this target represents a valuable strategy for cancer treatment. Selective drug-induced cyclin D1 depletion may provide a useful treatment strategy for cancer.

### Description

The present disclosure provides substituted 2-amino pyridine, 2,4-disubstituted pyrimidine and 4,6-disubstituted pyrimidine that induce cyclin D1 depletion. The here identified compounds provide a new and useful treatment strategy for cancer.

### Drawings description

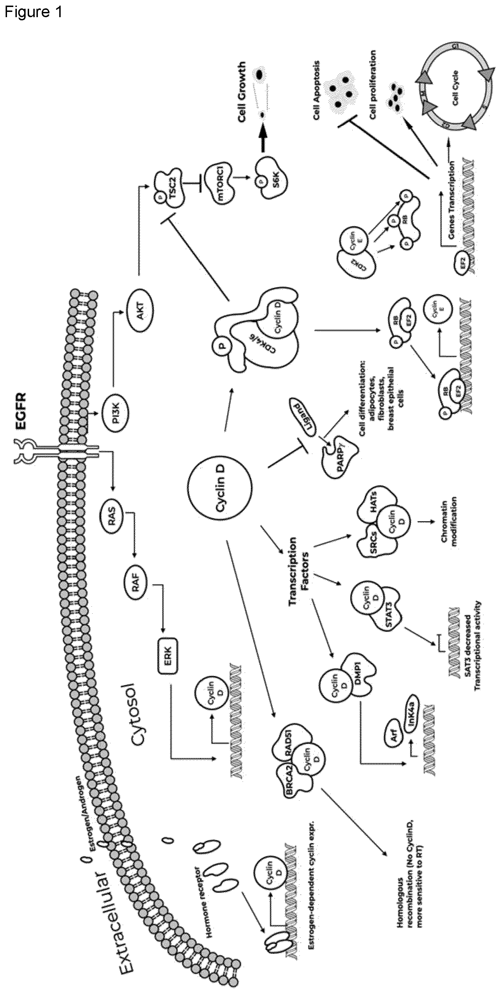

**Figure 1****:** Schematic representation of Cyclin D1 canonical pathway alongside a comprehensive summary of non-canonical and CDK-independent functions of Cyclin D1. Once Cyclin D1 is induced, its main function is to bind either CDK4 or CDK6 and drive cell cycle progression. Among the non-canonical functions of Cyclin D1 are its role in modulating transcription factors, influencing chromatin modifications, and participating in the double-strand break (DSB) DNA repair pathways.

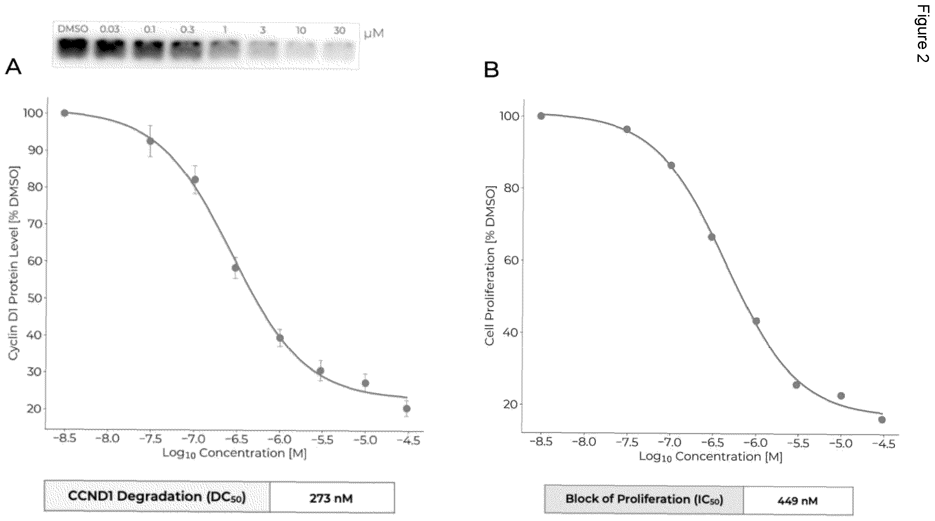

**Figure 2****:** Dose-response curve for **E6.** (A) representative image of a western blot experiment in which Cyclin D1 protein level was quantified after the treatment with E6 at the indicated concentrations in Huh7 cells. In the graph below, each point corresponds to the mean ± standard error of three western blot quantifications, normalized on the total protein content, resulting from independent biological experiments. Fitting the concentration-response curve with a four-parameter logistic (4PL) regression resulted in a half-maximal degradation concentration (DC₅₀) of 273 nM. (B) The graph shows the concentration-response effect of the compound in Huh7 cell proliferation measured using cell imaging. Each point corresponds to the average of two independent replicates. Fitting the concentration-response curve with a 4PL regression resulted in a half-maximal inhibitory concentration (IC₅₀) of 449 nM.

### Detailed description

A first object of the invention is represented by the compounds within the general formulas of claim 1, and dependent claims, capable of inducing the degradation of the Cyclin D1 protein, and which are identified using the PPI-FIT methodology.

The method for identifying target protein folding intermediates suitable to be tested as targets for drug discovery procedures disclosed in WO2020/021493 is applied in the present invention to the design and selection of compounds against the most kinetically and thermodynamically relevant folding intermediate of the Cyclin D1 protein, in order to stabilize such intermediate and inhibit its transition to the native form. In a cellular environment, said stabilized folding intermediate is recognized by the protein folding quality control machinery as an improperly folded polypeptide and sent to degradation.

The PPI-FIT methodology (WO2021191883) was applied to Cyclin D1, obtaining the all-atom simulation of the entire sequence of events underlying the Cyclin D1 folding path.

The data obtained revealed the existence of a folding intermediate showing potentially druggable pockets, not present in the native conformation. A virtual screening campaign was then conducted aimed at these pockets, with the aim of identifying molecules capable of decreasing the expression of Cyclin D1.

Embodiments disclosed hereinafter may be combined with each other in any possible way that would give rise to a stable compound. All such combinations are within the scope of the present invention.

It forms a first object of the present invention a compound of formula (I): 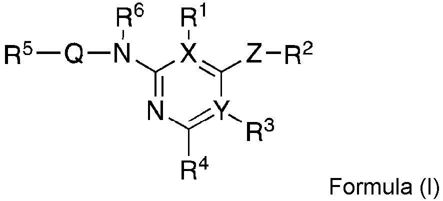 wherein, independently for each occurrence:
- **X** and **Y are** selected from C or N atom
   - **Z** is a direct bond, -C(=O)-, -C(=S)-, or -(CH₂)ₙ- , wherein n represents an integer from 1 to 2;
- **R¹** is selected from the group consisting of -H, halogen, and -CH₃ with the proviso that if X is a nitrogen atom, then R¹ is null (i.e. the nitrogen X has 3 chemical bonds and a lone pair in this case)
- **R²** is selected from the group consisting of:
   a) -NHR⁷
   b) -NR⁷R⁸
   c) -NHC(=O)R⁷
   d) -NHC(=O)NR⁷R⁸
   e) -C(=O)H
   f) -OR⁷
   g) 5-membered heteroaromatic ring
- **R³** is selected from the group consisting of -H, -F, -CH₃, -CF₃, and -CHF₂ with the proviso that if Y is a nitrogen atom, then R³ is null (i.e. the nitrogen Y has 3 chemical bonds and a lone pair in this case).
- **R⁴** is selected from the group consisting of -H, -CH₃, -NH₂, halogen, hydroxy, methoxy, secondary and tertiary amine, cycloalkylamine;
- **Q** is a direct bond, C₁₋₄ alkanediyl, substituted C₁₋₄ alkanediyl, cycloalkanediyl, substituted cycloalkanediyl, azetidine, piperidine, -CH₂C(=O)-, -NHC(=O)-, -OCH₂C(=O)- ;
- **R⁵** is selected from the group consisting of -(C₁₋₆)-alkyl, -(C₁₋₆)-alkyloxy, a monocyclic or bicyclic 5- to 14-membered aryl, wherein aryl is unsubstituted or mono-, di- or tri-substituted halogen, alkylhalogen, of -(C₁₋₆)-alkyl, -(C₁₋₆)-alkyloxy, -(C₁₋₆)-haloalkyloxy, aryl or heteroaryl;
- **R⁶** is selected from the group consisting of -H, -CH₃
- **R⁷** and **R⁸** can be the same or different and independently represent a hydrogen atom, -(C₁₋₆)-alkyl, -(C₁₋₆)- cycloalkyl, phenyl or benzyl, wherein those can be unsubstituted or mono-, di or trisubstituted independently of one another by **R⁹**
- or **R⁷** and **R⁸** can form together with the nitrogen atom to which they are attached a 4- to 8-term ring, substituted or unsubstituted condensed bicycles or spiro cycles,
- or **R⁷** and **R⁸** can form together with the nitrogen atom to which they are attached a substituted or unsubstituted cyclic amine, a substituted or unsubstituted cyclic urea, substituted or unsubstituted cyclic carbamate, substituted or unsubstituted lactam.
- **R⁹** is selected from the group consisting of halogen, -OH, -CN, -OCH_{3,}-OCF₃.

Preferably, R² is -NHR⁷. Preferably, R² is -NR⁷R⁸. Preferably, R² is -NHC(=O)R'. Preferably, R² is -NHC(=O)NR⁷R⁸. Preferably, R² is -C(=O)-. Preferably, R² is -OR⁷. Preferably, R² is a 5-membered heteroaromatic ring. Preferably, R⁹ is halogen. Preferably, R⁹ is -OH. Preferably, R⁹ is -CN. Preferably, R⁹ is -OCH₃. Preferably, R⁹ is -OCF₃.

In one embodiment, the invention relates to compounds of Formula (I-A): 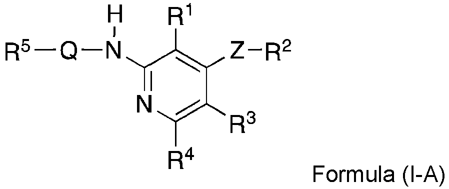
or pharmaceutically acceptable salts, tautomers, solvates or stereoisomers thereof. In particular, in Formula I-A, **R¹**= H, **R³**= H and **R⁴**= H, Qis selected from the group consisting of is -(CH₂)ₙ-, wherein n represents an integer from 1 to 2; 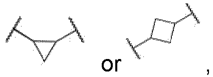
and the remaining substituents are as defined above in Formula (I);
   or 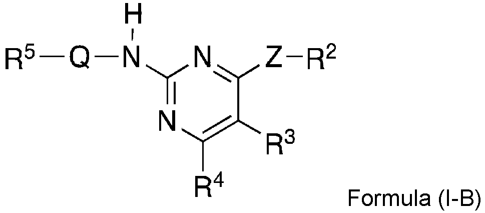
or pharmaceutically acceptable salts, tautomers, solvates or stereoisomers thereof. In particular, in Formula I-B, R³= H, R⁴ is -H or -CH_{3;}
Q is -(CH₂)ₙ-, wherein n represents an integer from 1 to 2; Z= direct bond or - C(=O)-, and the remaining substituents are as defined above in Formula (I);
   or 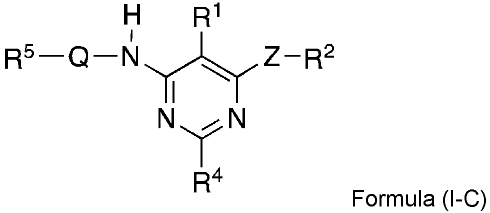
or pharmaceutically acceptable salts, tautomers, solvates or stereoisomers thereof. In particular, in Formula I-C, R¹ is H, R⁴ is -H or -CH₃,
Q is -(CH₂)ₙ-, wherein n represents an integer from 1 to 2; Z= direct bond or - C(=O)-, R⁶= H; and the remaining substituents are as defined above in Formula (I);

In a preferred embodiment, compounds of the invention have Formula II-A: 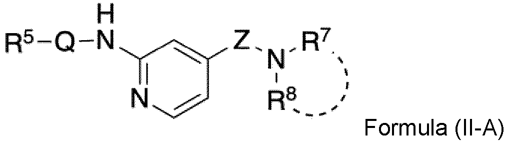 wherein:
- **Z** is:
   a. -C(=O)-
   b. -C(=S)-;
- **R⁷** and **R⁸** form, together with the nitrogen atom to which they are attached, a cyclic amine, a cyclic urea, a cyclic carbamate, a lactam, preferably selected
   in the group consisting of: 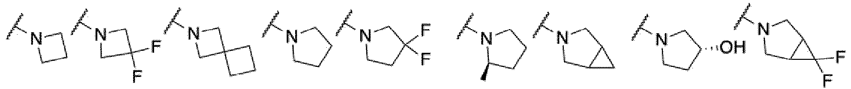 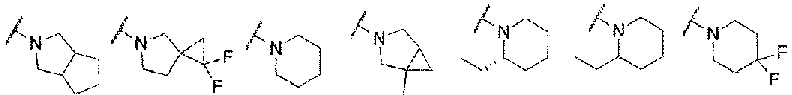 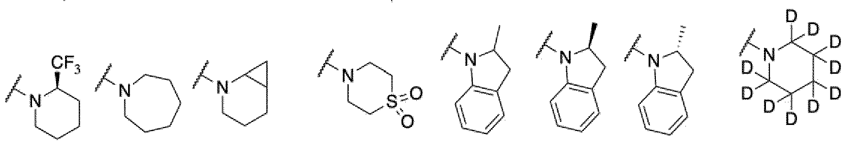 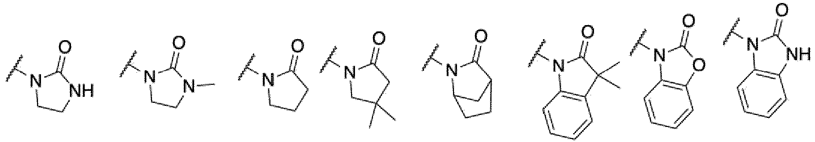 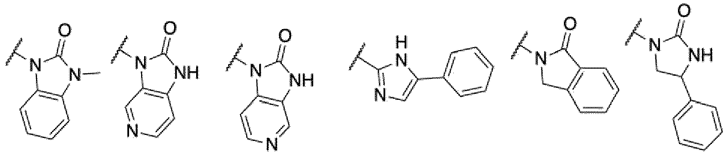
- Q is selected from the group consisting of -CH₂-, -CH₂CH₂-, 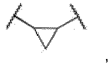 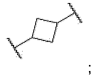
- **R⁵** is selected from the group consisting of 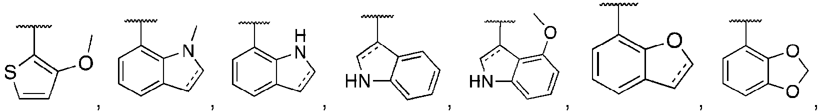 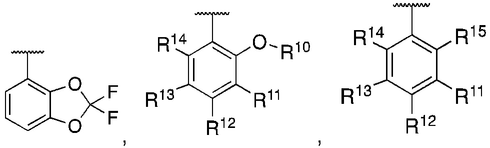 wherein
- **R¹⁰** is independently selected from the group consisting of hydrogen, methyl, ethyl, cyclopropyl, cyclobutyl, cyclopenthyl, -CF₃, CHF₂, -CH₂F_{;}
- **R¹¹- R¹⁴** is independently selected from the group consisting of -H, -OCH₃, -OH, -CH₃, -F, -Cl, -Br, -OCH₃, -OCH₂OCH₃, -OCF₃, -OCHF₂, -CF₃, -OCH₂, aryl or heteroaryl;
- **R¹⁵** is independently selected from the group consisting of -H, -CH₃, CF₃, -F, -Cl, -Br;

In a preferred embodiment, compounds of the invention have Formula (II-B): 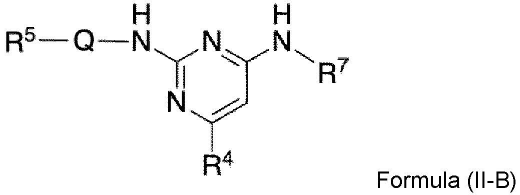
- **R⁴** is -H or -CH_{3;}
- **Qis** selected from the group consisting of -CH₂-, -CH₂CH₂-;
- **R⁵** is selected from the group consisting of 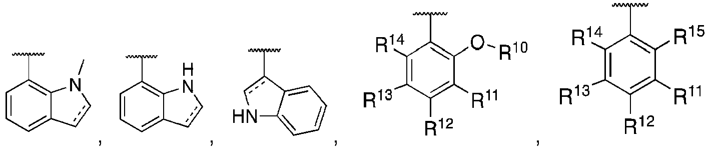 wherein
- **R¹⁰** is independently selected from the group consisting of hydrogen, methyl, ethyl, cyclopropyl, cyclobutyl, cyclopenthyl, -CF₃, CHF₂, -CH₂F_{;}
- **R¹¹- R¹⁴** is independently selected from the group consisting of -H, -OCH₃, -OH, -CH₃, -F, -Cl, -Br, -OCH₃, -OCH₂OCH₃, -OCF₃, -OCHF₂, -CF₃, -OCH₂, aryl or heteroaryl;
- **R¹⁵** is independently selected from the group consisting of -H, -CH₃, CF₃, -F, -Cl, -Br;
- **R⁷** is

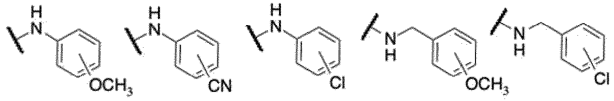

In a preferred embodiment, compounds of the invention have Formula (II-C): 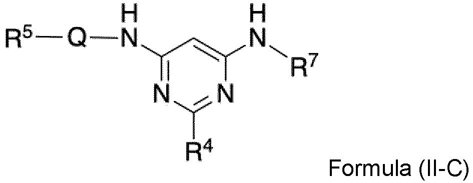
- **R⁴** is -H or -CH_{3;}
- Qis selected from the group consisting of -CH₂-, -CH₂CH₂-;
- **R⁵** is selected from the group consisting of 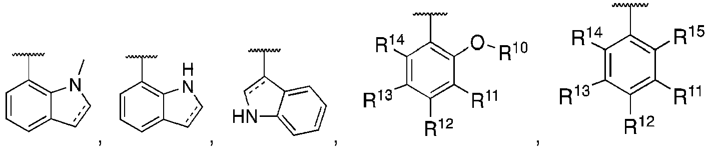 wherein
- **R¹⁰** is independently selected from the group consisting of hydrogen, methyl, ethyl, cyclopropyl, cyclobutyl, cyclopenthyl, -CF₃, CHF₂, -CH₂F_{;}
- **R¹¹- R¹⁴** is independently selected from the group consisting of -H, -OCH₃, -OH, -CH₃, -F, -Cl, -Br, -OCH₃, -OCH₂OCH₃, -OCF₃, -OCHF₂, -CF₃, -OCH₂, aryl or heteroaryl;
- **R¹⁵** is independently selected from the group consisting of -H, -CH₃, CF₃, -F, -Cl, -Br;
- **R⁷** is selected from

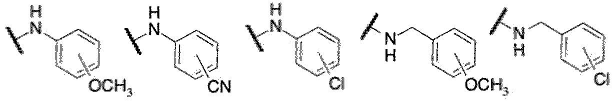

It is a further object of the invention a pharmaceutical composition comprising an effective amount of one or more compounds as defined above or a pharmaceutically acceptable prodrug thereof, alone or in combination with other active compounds, and at least one pharmaceutically acceptable excipient.

The present invention includes within its scope prodrugs of the compounds of the invention which are readily convertible in vivo into the required compound of formula (I) as indicated above. Conventional procedures for the selection and preparation of suitable prodrug derivatives are described, for example, in "Design of Prodrugs", ed. H. Bundgaard, Elsevier, 1985.

A prodrug may be a pharmacologically inactive derivative of a biologically active substance (the "parent drug" or "parent molecule") that requires transformation within the body in order to release the active drug, and that has improved delivery properties over the parent drug molecule. The transformation in vivo may be, for example, as the result of some metabolic process, such as chemical or enzymatic hydrolysis of a carboxylic, phosphoric or sulphate ester, or reduction or oxidation of a susceptible functionality.

The invention also includes all suitable isotopic variations of a compound of the disclosure. Examples of isotopes that can be incorporated into compounds of the disclosure include isotopes such as ²H, ³H, ¹³C, ¹⁴C, ¹⁵N, ¹⁸F and ³⁶Cl, respectively. Certain isotopic variations of the disclosure, for example, those in which a radioactive isotope such as ³H or ¹⁴C is incorporated, are useful in drug and/or substrate tissue distribution studies. Further, substitution with isotopes, such as deuterium ²H, may afford certain therapeutic advantages resulting from greater metabolic stability. Isotopic variations of the compounds of the disclosure can generally be prepared by conventional procedures such as by the illustrative methods or by the preparations described in the examples hereafter using appropriate isotopic variations of suitable reagents. The present invention includes within its scope solvates of the compounds of the invention or of the relative salts, for example, hydrates.

In addition, the compounds disclosed herein may exist as tautomers and all tautomeric forms are intended to be encompassed by the scope of the invention, even though only one tautomeric structure is depicted. In addition, the compounds disclosed herein may exist as tautomers and all tautomeric forms are intended to be encompassed by the scope of the invention, even though only one tautomeric structure is depicted.

The compounds may exist in different isomeric forms, all of which are encompassed by the present invention.

The compounds of the present invention may have asymmetric centers, chiral axes, and chiral planes (as described in: E.L. Eliel and S.H. Wilen, Stereochemistry of Carbon Compounds, John Wiley &Sons, New York, 1994, pages 1119-1190), and occur as racemates, racemic mixtures, and as individual diastereomers, with all possible isomers and mixtures thereof, including optical isomers, all such stereoisomers being included in the present invention.

Pure stereoisomeric forms of the compounds and intermediates of this invention may be obtained by the application of art-known procedures and are intended to be encompassed by the scope of the invention. In particular, "pure stereoisomeric form" or "stereoisomerically pure" indicates a compound having a stereoisomeric excess of at least 80%, preferably of at least 85%. For instance, enantiomers may be separated from each other by the selective crystallization of their diastereomeric salts or by chromatographic techniques using chiral stationary phases. Pure stereochemically isomeric forms may also be derived from the corresponding pure stereochemically isomeric forms of the appropriate starting materials, provided that the reaction occurs stereospecifically. The term "enantiomerically pure" shall be interpreted in a similar way, having regard to the enantiomeric ratio.

When any variable (e.g. R', and R², n and m, etc.) occurs more than one time in any constituent, its definition of each occurrence is independent of every other occurrence. Also, combinations of substituents and variables are permissible only if such combinations result in stable compounds. Lines drawn into the ring systems from substituents represent that the indicated bond may be attached to any of the substitutable ring atoms. If the ring system is polycyclic, it is intended that the bond be attached to any of the suitable atoms on the proximal ring only.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" should be taken to be equivalent to the phrase "unsubstituted or substituted with one or more substituents" and in such cases the preferred embodiment will have from zero to three substituents. More particularly, there are zero to two substituents.

As used herein, "alkyl" is intended to include both branched and straight-chain saturated aliphatic hydrocarbon groups having the specified number of carbon atoms.

As used herein, the term "C₁₋₆ alkanediyl" as group or part of a group defines bivalent straight or branched chained saturated hydrocarbon radicals having from 1 to 6 carbon atoms. C₁₋₆ alkanediyl group, is preferably a C₁₋₄ alkanediyl group, a C₁₋₃ alkanediyl or more preferably a C₁₋₂ alkanediyl. Examples include, but are not limited ot methanediyl, ethanediyl, propanediyl, butanenediyl, pentanediyl and hexanediyl. Preferred are methanediyl, ethanediyl and propanediyl.

As used herein, "alkoxy" represents an alkyl group of indicated number of carbon atoms attached through an oxygen bridge. "Alkoxy" therefore encompasses the definitions of alkyl above. C₁₋₆ alkoxy group is preferably a linear or branched C₁₋₄ alkoxy group, more preferably a C₁₋₄ alkoxy group, still more preferably a C₁₋₂ alkoxy group. Examples of suitable alkoxy groups include, but are not limited to methoxy, ethoxy, *n*-propoxy, *i*-propoxy, *n*-butoxy, *s*-butoxy or *t*-butoxy; preferred alkoxy groups include methoxy, ethoxy and t-butoxy.

As used herein, the terms "haloC₁₋₆ alkyl" and "haloC₁₋₆alkoxy" mean a C₁₋₆alkyl or C₁₋₆alkoxy group in which one or more (in particular, 1 to 3) hydrogen atoms have been replaced by halogen atoms, especially fluorine or chlorine atoms. HaloC₁₋₆alkoxy group is preferably a linear or branched haloC₁₋₄alkoxy group, more preferably a haloC₁₋₃alkoxy group, still more preferably a haloC₁₋₂alkoxy group, for example OCF₃, OCHF₂, OCHF, OCH₂CH₂F, OCHCHF₂ or OCHCF₃, and most especially OCF₃ or OCHF₂. HaloC₁₋₆alkyl group is preferably a linear or branched haloC₁₋₃alkyl group, more preferably a haloC₁₋₂alkyl group for example, CF₃, CHF₂, CH₂F, CH₂CH₂F, CH₂CHF₂, CH₂CF3 or CH(CH₃)CF₃.

As used herein, the term "hydroxy C₁₋₆ alkyl" means a C₁₋₆ alkyl group in which one or more (in particular, 1 to 3) hydrogen atoms have been replaced by hydroxy groups. Similarly, the term "hydroxy C₁₋₄ alkyl" means a C₁₋₄ alkyl group in which one or more (in particular, 1 to 2) hydrogen atoms have been replaced by hydroxy groups. Illustrative examples include, but are not limited to CH₂OH, CH₂CH₂OH, CH(CH₃)OH and CHOHCH₂OH.

As used herein, the term "aryl" means a monocyclic or polycyclic aromatic ring comprising carbon atoms and hydrogen atoms. If indicated, such aromatic ring may include one or more heteroatoms, then also referred to as "heteroaryl", preferably, 1 to 3 heteroatoms, independently selected from nitrogen, oxygen, and sulfur, preferably nitrogen.

A substituent on a saturated, partially saturated or unsaturated heterocycle can be attached at any substitutable position.

As used herein, the term "halogen" refers to fluorine, chlorine, bromine and iodine, of which fluorine, chlorine and bromine are preferred.

The term "heteroatom" refers to an atom other than carbon or hydrogen in a ring structure or a saturated backbone as defined herein. Typical heteroatoms include N(H), O, S.

As used herein, the term "C-s Cycloalkyl" means saturated cyclic hydrocarbon (cycloalkyl) with 3 or 4, 5, 6, 7 or 8 carbon atoms and is generic to cyclopropyl, cyclobutyl, cyclopentyl, cyclohexyl, cycloheptyl or cyclooctyl.

Included in the instant invention is the free base of compounds of the invention as well as the pharmaceutically acceptable salts and stereoisomers thereof. Some of the specific compounds exemplified herein are the protonated salts of amine compounds. Compounds of the invention containing one or more N atoms may be protonated on anyone, some or all of the N atoms. The term "free base" refers to the amine compounds in non-salt form. The encompassed pharmaceutically acceptable salts not only include the salts exemplified for the specific compounds described herein, but also all the typical pharmaceutically acceptable salts of the free form of compounds of the disclosure. The free form of the specific salt compounds described may be isolated using techniques known in the art. For example, the free form may be regenerated by treating the salt with a suitable dilute aqueous base solution such as dilute aqueous NaOH, potassium carbonate, ammonia and sodium bicarbonate. The free forms may differ from their respective salt forms somewhat in certain physical properties, such as solubility in polar solvents, but the acid and base salts are otherwise pharmaceutically equivalent to their respective free forms for purposes of the invention.

The pharmaceutically acceptable salts of the instant compounds can be synthesized from the compounds of this invention which contains a basic or acidic moiety by conventional chemical methods. Generally, the salts of the basic compounds are prepared either by ion exchange chromatography or by reacting the free base with stoichiometric amounts or with an excess of the desired salt-forming inorganic or organic acid in a suitable solvent or various combinations of solvents. Similarly, the salts of the acidic compounds are formed by reactions with the appropriate inorganic or organic base. In a preferred embodiment, the compounds of the invention have at least one acidic proton and the corresponding sodium or potassium salt can be formed, for example, by reaction with the appropriate base.

Thus, pharmaceutically acceptable salts of the compounds of this invention include the conventional non-toxic salts of the compounds of this invention as formed by reacting a basic instant compound with an inorganic or organic acid or an acid compound with an inorganic or organic base. For example, conventional non-toxic salts include those derived from inorganic acids such as hydrochloric, hydrobromic, sulfuric, sulfamic, phosphoric, nitric and the like, as well as salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, oxalic, isethionic, trifluoroacetic and the like. Convential non- toxic salts further include those derived from an inorganic base, such as potassium, sodium hydroxide, magnesium or calcium hydroxide, as well as salts prepared from organic bases, such as ethylene diamine, lysine, tromethamine, meglumine and the like. Preferably, a pharmaceutically acceptable salt of this invention contains one equivalent of a compound of formula (1) and 1, 2 or 3 equivalent of an inorganic or organic acid or base. More particularly, pharmaceutically acceptable salts of this invention are the tartrate, trifluoroacetate or the chloride salts.

The preparation of the pharmaceutically acceptable salts described above and other typical pharmaceutically acceptable salts is more fully described by Berg et al., "Pharmaceutical Salts," J. Pharm. Sci., 1977:66:1-19.

The compounds of the present invention find use in a variety of applications for human and animal health. The compounds of the present invention are typically Cyclin D1 degraders and useful for cancer treatment.

The compounds of this invention may be administered to mammals, preferably humans, either alone or in combination with pharmaceutically acceptable carriers, excipients or diluents, in a pharmaceutical composition, according to standard pharmaceutical practice. In one embodiment, the compounds of this invention may be administered to animals. The compounds can be administered orally or parenterally, including the intravenous, intramuscular, intraperitoneal, subcutaneous, rectal and topical routes of administration.

When a compound according to this invention is administered into a human subject, the daily dosage will normally be determined by the prescribing physician with the dosage generally varying according to the age, weight, sex and response of the individual patient, as well as the severity of the patient's symptoms.

The instant compounds are also useful in combination with known therapeutic agents for simultaneous, separate or sequential administration.

The term "administration" and variants thereof (e.g., "administering" a compound) in reference to a compound of the invention means introducing the compound or a prodrug of the compound into the system of the animal in need of treatment. When a compound of the invention or prodrug thereof is provided in combination with one or more other active agents (e.g., a cytotoxic agent, etc.), "administration" and its variants are each understood to include concurrent and sequential introduction of the compound or prodrug thereof and other agents.

In a preferred embodiment, it forms an object of the present invention a compound selected from the group consisting of: E1, E2, E3, E4, E6, E10, E15, E16, E18, E21, E22, E23, E24, E25, E27, E29, E31, E32, E37, E38, E40, E41, E42, E44, E45, E46, E47, E48, E50, E51, E52, E54, E56, E57, E58, E59, E60, E61, E62, E64, E65, E66, E67, E68, E69, E70, E75, E77, E78, E80, E84, E99, E98, E100, E102, E104, E107, E115, E116, E119, E125, E128, E129, E130, E132, E135, E136, E137, E148, E149, E150, E153, E158, E160, E161, E162, E163, E164, E165, E166, E167, E168, E171, E172, E174, E175, E176, E177, E178 E179, E180, E181, E182, E183, E184, E185, E186, E187, E189, E194, E195, E196, E199, E237, E238, E228, E234, E235, wherein the references have the meaning reported in Table 3 that follows.

The present invention further relates to the compound according to the invention for use in the treatment or prevention of cancer.

In a preferred embodiment, the cancer refers to or includes head and neck squamous cell carcinoma, nasopharyngeal carcinoma (NPC), small cell lung cancer, non-small-cell lung cancer (NSCLC), endometrial cancer, melanoma, pancreatic cancer, gastric cancer, breast cancer, colorectal cancer, mantle cell lymphoma, multiple myeloma, prostate cancer, and papillary thyroid carcinoma (PTC), preferably wherein the cancer is breast cancer or liver cancer.

In a preferred embodiment, the cancer is a cyclin D1 dependent cancer. Preferably the term "cyclin D1 dependent cancer" refers to a cancer, in which cyclin D1 in which Cyclin D1 plays a crucial role in cancer cell growth, proliferation, and/or survival.

The present invention further relates to a pharmaceutical composition comprising at least one compound according to the invention or its salt or solvate and one or more pharmaceutically acceptable excipients.

In a preferred embodiment, the pharmaceutical composition is present in a form selected from the group consisting of tablets, sublingual tablets, melting tablets, dragees, capsules, soft capsules, hard capsules, gastroresistant capsules, granules, powders and printlets, semi-solid dosage forms such as ointments, creams, pastes and gels, liquid dosage forms such as suspensions, crystal suspensions, emulsions, solutions, drops, nasal drops, eye drops, ear drops, syrups, juices, injection solutions, injection dispersions, injection solutions and injection solution concentrates, and further dosage forms such as suppositories, lozenges, effervescent tablets, effervescent powders, inhalation powders, plasters, metered dose inhalers, foams, sprays, sublingual sprays, shampoos, mouthwashes.

The present invention further relates to the pharmaceutical composition according to the invention for use in the treatment or prevention of cancer.

In a preferred embodiment, the cancer refers to or includes head and neck squamous cell carcinoma, nasopharyngeal carcinoma (NPC), small cell lung cancer, non-small-cell lung cancer (NSCLC), endometrial cancer, melanoma, pancreatic cancer, gastric cancer, breast cancer, colorectal cancer, mantle cell lymphoma, multiple myeloma, prostate cancer, and papillary thyroid carcinoma (PTC), preferably wherein the cancer is breast cancer or liver cancer.

In a preferred embodiment, the cancer is a cyclin D1 dependent cancer. Preferably the term "cyclin D1 dependent cancer" refers to a cancer, in which Cyclin D1 plays a crucial role in cancer cell growth, proliferation, and/or survival.

The experiments that follow have the pure scope of illustrating the invention, they are not to be intended limitative of the same, whose scope of protection is defined by the claims that follow.

### Experimental Section

### MATERIALS and METHODS

Unless otherwise indicated, commercially available reagents and solvents (HPLC grade) were used without further purification.

Specifically, the following abbreviations may have been used in the descriptions of the experimental methods:
LC-MS: Liquid Chromatography Mass Chromatography Spectrum;
H or H' : proton;
h or hr: hour(s);
HPLC: High Performance Liquid Chromatography;
Hz: Hertz;
mg: milligram(s);
g: gram(s);
anh: anhydrous;
aq: aqueous;
Boc: tert-butyloxycarbonyl protecting group;
CDI: 1,1-Carbonyldiimidazole
DCE: dichloroethane;
DCM: dichloromethane;
DEE: diethyl ether
DMF: dimethylformamide;
DMA: dimethylacetamide;
DIPEA: N,N-disopropylethylamine;
DMSO: dimethysulfoxide;
eq.: equivalent(s);
EtOH: ethanol;
EtOAc: ethyl acetate;
HATU: 1-[Bis(dimethylamino)methylene]-1H-1,2,3-triazolo[4,5-b]pyridinium 3-oxid hexafluorophosphate;
µL: microliter(s);
MeOH: methanol;
MeCN: Acetonitrile;
min: minute(s);
mL: millilitre(s);
mmol: millimole(s);
nm: nanometer(s);
M: micromolar;
M: molarity or molar concentration;
MHz: Megahertz;
MW: microwave;
NMP: N-methyl-2-pyrrolidone
NMR: Nuclear Magnetic Resonance;
NP: Normal Phase,
ON: overnight;
RBF: round bottom flask
RP: Reversed Phase;
Rt: retention time in minutes;
RT: room temperature;
s: second(s);
ss: saturated solution;
SNAr: nucleophilic aromatic substitution
STAB: Sodium triacetoxyborohydride;
T3P : Propylphosphonic anhydride;
TCFH: Chloro-N,N,N',N'-tetramethylformamidinium hexafluorophosphate;
TEA: triethylamine;
THF: tetrahydrofuran;
TBTU: 2-(1H-Benzotriazole-1-yl)-1, 1,3,3-tetramethylaminium tetrafluoroborate.

Except where indicated otherwise, all temperatures are expressed in °C (degrees centigrade).

NMR spectra were measured at 298 K, unless indicated otherwise, and were referenced relative to the solvent resonance. The chemical shifts are reported in parts per million (δ ppm).

¹H-NMR spectra were performed on a 400 Agilent INOVA, 500 Agilent VNMRS, 600 Agilent INOVA spectrometers operating at 400, 500, 600 MHZ (proton frequency), or on a a Bruker Avance III HD spectrometer equipped with Bruker BBO probe, operating at 400.17 MHz (1H), or Bruker Fourier HD spectrometer, equipped with Bruker C-H DUL probe, operating at 300.17 MHz (1H). Chemical shifts are reported as 6 values in ppm using residual solvent peak as an internal reference. Coupling

constants (J values) are given in hertz (Hz) and multiplicities are reported using the following abbreviation (s=singlet, d=doublet, t=triplet, q=quartet, p= quintet, h= heptet, m=multiplet, br. s=broad singlet, nd=not determined, dd=double-doublet, ddd=double-double-doublet, quin= quintuplet, td= triple doublet, tt= triple triplet, qd= quadrupole doublet).

### LC/UV/MS Analytical Methods

LC/MS retention times are estimated to be affected by an experimental error of +0.5 min. LC/MS may be recorded under the following conditions: diode array DAD chromatographic traces, mass chromatograms and mass spectra may be taken on UPLC/PDA/MS AcquityTM system coupled with Micromass ZQTM or Waters SQD single quadrupole mass spectrometer operated in positive and/or negative electrospray ES ionization mode, or Shimadzu LCMS-2020 system coupled with Single Quadrupole Liquid Chromatograph Mass Spectrometer or Dionex UHPLC Ultimate 3000 with DAD detector coupled with ISQ EC Single Quadrupole Mass Spectrometer and/or Fractionlynx system used in analytical mode coupled with ZQTM single quadrupole operated in positive and/or negative ES ionization mode. Quality Control methods used operated under low pH conditions or under high pH conditions:
**Method 1** (2 min): low pH conditions column: Acquity CSH C18 2.1x50mm 1.7um, the column temperature was 40 °C; mobile phase solvent A was milliQ water+0.1% HCOOH, mobile phase solvent B MeCN+0.1% HCOOH. The flow rate was 0.9 mL/min.

The gradient table was t=0 min 97% A 3% B, t=1.4 min 0.1% A 99.9% B, t=1.9 min 0.1% A 99.9% B and t=2 min 97% A 3% B. The UV detection range was 210-350 nm and ES+/ES- range was 100 to 1000 AMU.

**Method 2** (4 min): low pH conditions column: Acquity CSH C18 2.1x50mm 1.7um, the column temperature was 40 °C; mobile phase solvent A was milliQ water+0.1% HCOOH, mobile phase solvent B MeCN+0.1% HCOOH. The flow rate was 0.9 mL/min.

The gradient table was t=0 min 97% A 3% B, t=3.4 min 0.1% A 99.9% B, t=3 min 0.1% A 99.9% B and t=4 min 97% A 3% B. The UV detection range was 210-350 nm and ES+/ES- range was 100 to 1000 AMU.

**Method 3** (3 min): low pH conditions column: Acquity UPLC BEH - Waters, 1.7 µm C18 (2.1 x 100 mm), 130 Å, the column temperature was 25/20 °C; mobile phase solvent A was milliQ water+0.1% HCOOH, mobile phase solvent B MeCN+0.1% HCOOH. The flow rate was 0.5 mL/min. The gradient table was t=0.1 min 80% A 20% B, t=2 min 5% A 95% B, t=2.70 min 5% A 95% B, t=2.80 min 80% A 20% B, t=3.00 min 80% A 20% B. The UV at 254 and 280 nm, MS by alternate ionization.

**Method 4** (6 min): low pH conditions column: Kinetex^{®} 2.6 µm XB-C18 (4.6 x 50 mm), 110 Å, column no. 00B-4496-E0, the column temperature was 25/20 °C; mobile phase solvent A was milliQ water+0.1% HCOOH, mobile phase solvent B MeCN+0.1% HCOOH. The flow rate was 1 mL/min.

The gradient table was t=0 min 90% A 10% B, t=3.90 min 5% A 95% B, t=4.75 min 5% A 95% B, t=5 min 90% A 10% B, t=6 min 90% A 10% B. The wavelength range was 190 - 350 nm +/- 4 nm, MS by alternate ionization.

**Method 5** (6 min): low pH conditions column: Kinetex^{®} 2.6 µm XB-C18 (4.6 x 50 mm), 110 Å, column no. 00B-4496-E0, the column temperature was 25/20 °C; mobile phase solvent A was milliQ water+0.1% HCOOH, mobile phase solvent B MeCN+0.1% HCOOH. The flow rate was 1 mL/min.

The gradient table was t=0 min 90% A 10% B, t=3.35 min 30% A 70% B, t=3.75 min 30% A 70% B, t=3.90 min 5% A 95% B, t=4.75 min 5% A 95% B, t=5 min 90% A 10% B, t=6 min 90% A 10% B. The wavelength range was 190 - 350 nm +/- 4 nm, MS by alternate ionization.

**Method 6** (9 min): medium/high pH conditions column: Gemini-NX 3 µm (4.6x50mm), 110A, column no. 00B-4453-EO, the column temperature was 25/20 °C; mobile phase solvent C was 0.05% milliQ water solution of ammonium hydroxide (28.0-30.0% NH₃basis*),* mobile phase solvent D was MeCN. The flow rate was 1 mL/min. The gradient table was t=0 min 90% C 10% D, t=6.35 min 30% C 70% D, t=6.75 min 30% C 70% D, t=6.90 min 5% C 95% D, t=7.75 min 5% C 95% D, t=8 min 90% C 10% D, t=9 min 90% C 10% D. The wavelength range was 190 - 350 nm +/-4 nm, MS by alternate ionization.

**Method 7** (9 min): medium/high pH conditions column: Gemini-NX 3 µm (4.6x50mm), 110A, column no. 00B-4453-EO, the column temperature was 25/20 °C; mobile phase solvent C was 0.05% milliQ water solution of ammonium hydroxide (28.0-30.0% NH₃basis*),* mobile phase solvent D was MeCN. The flow rate was 1 mL/min.

The gradient table was t=0 min 70% C 30% D, t=6.35 min 20% C 80% D, t=6.75 min 20% C 80% D, t=6.90 min 5% C 95% D, t=7.75 min 5% C 95% D, t=8 min 70% C 30% D, t=9 min 70% C 30% D. The wavelength range was 190 - 350 nm +/- 4 nm, MS by alternate ionization.

**Method 8** (6 min): low pH conditions column: Kinetex^{®} 2.6 µm XB-C18 (4.6 x 50 mm), 110 Å, column no. 00B-4496-E0, the column temperature was 25/20 °C; mobile phase solvent A was milliQ water+0.1% HCOOH, mobile phase solvent B MeCN+0.1% HCOOH. The flow rate was 1 mL/min. The gradient table was t=0 min 80% A 20% B, t=3.35 min 20% A 80% B, t=3.75 min 20% A 80% B, t=3.90 min 5% A 95% B, t=4.75 min 5% A 95% B, t=5 min 80% A 20% B, t=6 min 80% A 20% B. The wavelength range was 190 - 350 nm +/- 4 nm, MS by alternate ionization.

**Method 9** (7 min): low pH conditions column: Kinetex^{®} 2.6 µm XB-C18 (4.6 x 50 mm), 110 Å, column no. 00B-4496-E0, the column temperature was 25/20 °C; mobile phase solvent A was milliQ water+0.1% HCOOH, mobile phase solvent B MeCN+0.1% HCOOH. The flow rate was 1 mL/min.

The gradient table was t=0 min 95% A 5% B, t=1 min 95% A 5% B, t=4.75 min 20% A 80% B, t=5.25 min 20% A 80% B, t=6 min 95% A 5% B, t=7 min 95% A 5% B. The wavelength range was 190 - 350 nm +/- 4 nm, MS by alternate ionization.

**Method 10** (2 min): low pH conditions column: Agilent Poroshell 2.7 µm 120 SB-C18 (4.6 x 30 mm), the column temperature was 60 °C; mobile phase solvent A was milliQ water+0.1% HCOOH, mobile phase solvent B MeCN+0.1% HCOOH. The flow rate was 3 mL/min.

The gradient table was t=0 min 99% A 1% B, t=0,01 min 99% A 1% B, t=1,5 min 0% A 100% B, t=2 min 0% A 100% B. The wavelengths used for UV detection were 215 nm, 254 nm and 280 nm, MS by alternate ionization.

### Synthesis

The following schemes are examples of synthetic schemes that may be used to synthesize the compounds of the invention. In the following schemes, reactive groups can be protected with protecting groups and deprotected according to well-established techniques.

The compounds of formula (**IIA**), (**IIB**) or (**IIC**) can be prepared by well-known literature methods, by the methods given below, by the methods given in the experimental part below or by analogous methods. Optimum reaction conditions may vary depending from reactants or solvents used, the conditions can be determined by a person skilled in the art by routine optimisation procedures. In some cases, the order of carrying out the following reaction schemes, and/or reaction steps, may be varied to facilitate the reaction or to avoid unwanted reaction products. The use of protecting groups is well known in the art (see for example "Protective Groups in Organic Synthesis", T.W. Greene, P.G.M. Wuts, Wiley-Interscience, 1999). For the purposes of this discussion, it will be assumed that such protecting groups as necessary are in place. In some cases, the final product may be further modified, for example, by manipulation of substituents to give a new final product. These manipulations may include, but are not limited to, reduction, oxidation, alkylation, acylation, hydrolysis and transition-metal catalysed cross-coupling reactions which are commonly known to those skilled in the art. The compounds obtained may also be converted into salts, especially pharmaceutically acceptable salts, in a manner known *per sé.*

A general synthetic route allowing the preparation of compounds of Formula **(IIA-**1) is presented in *Scheme 1.*

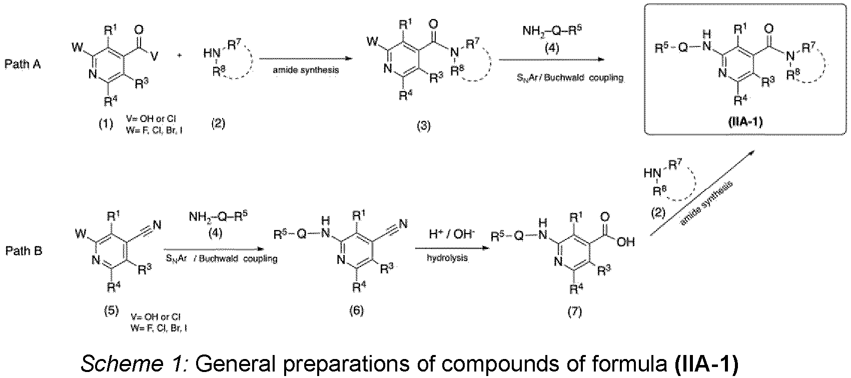

Compounds of generic Formula (IIA-1) can be prepared following synthetic Path A or B.

Path A requires two steps: amide synthesis and S_{N}Ar or Buchwald coupling. Thus, precursors **(3)** can be prepared after amide formation between commercially available 2-halo-isonicotinic acid **(1),** wherein V= OH and W = F, Cl, Br or I and primary or secondary amines HNR⁷R⁸ (2) *(Scheme 1, Path A),* in presence of a coupling agent such as CDI, HATU or TBTU; base such as, DIPEA or TEA in a solvent such as DMF or DCM at low temperatures, or at RT or at elevated temperatures. In alternative, the amide formation can be conducted in presence of *n*-propanephosphonic acid anhydride (T3P) and base such as TEA in a solvent such as DCM; alternatively the amide can be prepared using the combination of *N,N,N',N'-*tetramethylchloroformamidinium hexafluorophosphate (TCFH) and *N-*methylimidazole (NMI) in a solvent as MeCN. In alternative, precursors **(3)** can be prepared by 2-halo-isonicotinic acyl chloride **(1),** wherein V= CI and W = F, Cl, Br or I and primary or secondary amines HNR⁷R⁸ in presence of base TEA or DIPEA in a solvent as DCM at low temperature. Compounds of formula **(IIA-1)** can be next produced via nucleophilic aromatic substitutions (S_{N}Ar) between substituted 2-F-isonicotin amide **(3)** and primary amines H₂N-Q-R⁵ **(4),** (*Scheme 1, Path A*)*,* in the presence of a base such as TEA, DIPEA or K₂CO₃, in a solvent such as isopropanol, n-butanol, DMF or THF, at RT or at elevated temperatures. In a variant, compounds of formula **(IIA-1)** can be prepared via metal-catalyzed cross-coupling reactions of the substituted 2-halo-isonicotin amide **(3),** wherein **W** is a chlorine, a bromine or an iodine with primary amines H₂N-Q-R⁵ **(4),** for example Buchwald couplings.

Alternatively according to *Path B,* compounds of formula **(IIA-1)** can be synthesized by 3 reactions synthetic route: reacting a compound 2-F-isonicotinitrile with formula **(5)** with H₂N-Q-R⁵ **(4)** via nucleophilic aromatic substitutions (*Scheme 1, Path B*) or coupling reaction, wherein **W** is a chlorine, a bromine or an iodine, followed by nitrile hydrolysis under acidic or basic conditions to obtain carboxylic derive **(7)** and last amide formation between intermediate **(7)** and primary or secondary amine HNR⁷R⁸ **(2)** (*Scheme 1, Path B*) in presence of condensing agent as CDI, HATU, TBTU, T3P and base as DIPEA or TEA in a solvent as DMF or DCM at low temperatures, or at RT or at elevated temperatures.

The starting H₂N-Q-R⁵ **(4)** can be obtained from commercial sources, or synthesized by methods described in the literature or by methods known by a person skilled in the art. When H₂N-Q-R⁵ are β-aminoethyl aromatics can be prepared starting from aldehyde precursors **(8)** via Henry reaction (in mixture of nitromethane/acetic acid/NH₄OAc optional/ reflux) to give aryl or heteroaryl β-nitroalkene compounds **(9)** and subsequent reduction via LiAlH₄/THF, or NaBH₄/BF₃Et₂O/THF or Pd black catalysed hydrogenation.

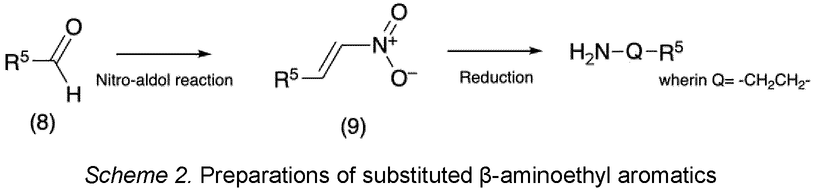

A general synthetic route allowing the preparation of compounds of Formula **(IIA-2)** and **(IIA-3)** is presented in *Scheme 3.*

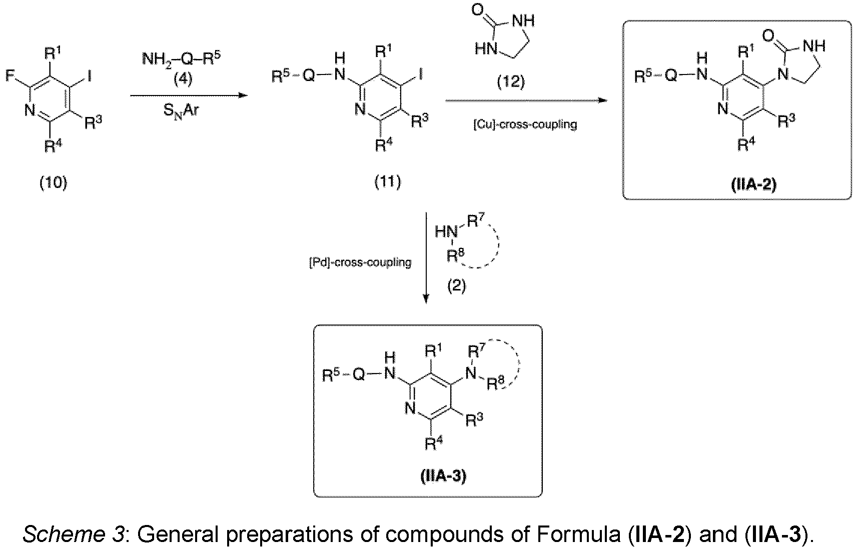

Thus, precursors **(11)** can be produced via nucleophilic aromatic substitutions (SNAr) between primary amines **(4)** and substituted 2-fluoro-4-iodopyridine **(10),** (*Scheme 3*), in the presence of a base such as TEA, DIPEA or K₂CO₃, in a solvent such as isopropanol, n-butanol, DMF or THF, at RT or at elevated temperatures. Compounds of formula **(IIA-2)** can be next synthesized starting from precursors **(11)** by copper catalysed cross-coupling, in presence of trans-N,N'-Dimethylcyclohexane-1,2-diamine and base as Potassium phosphate tribasic in a solvent as DMF. The described pathway is rapresentative and can be utilized to synthesize substituted or unsubstituted cyclic ureas, substituted or unsubstituted cyclic carbamates, and substituted or unsubstituted lactams.

Compounds of Formula **(IIA-3)** can be next synthesized starting from precursors **(11)** by Pd-catalyzed cross-coupling in presence of Pd sources as [1,1'-Bis(diphenylphosphino)ferrocene]dichloropalladium(II) (Pd(dppf)Cl₂) or Palladium (II) Acetate, tris(dibenyzlideneacetone)dipalladium (Pd₂(dba)₃) in presence of phosphine as 2,2'-Bis(diphenylphosphino)-1,1'-binaphthalene (BINAP) and base as KtOBu or Na*t*OBu in a solvent as DME, DMF, toluene, 1,4-dioxane or tBuOH.

A general synthetic route allowing the preparation of 2,4-disubstituted pyrimidine and 4,6-disubstituted pyrimidine compounds of formula (**IIB**) and (**IIC**) are presented in *Scheme 4.*

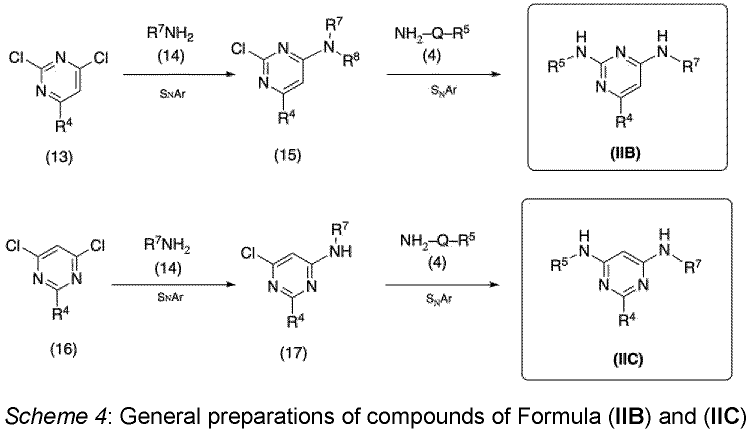

The compounds with formula **(IIB)** and (**IIC**) can be synthesized by S_{N}Ar reactions between commercially available 2,4-dichloropyrimidine **(13)** or 4,6-dichloropyrimidine **(16)** and primary amines H₂NR⁷ **(14)** (*Scheme 4*)*,* in the presence of a base such as TEA, DIPEA or K₂CO₃, in a solvent such as isopropanol, n-butanol, DMF or THF, at RT or at elevated temperatures. In case of 2,4-disubstituted pyrimidine analogues, the desired regioisomer **(15)** can be isolated by Flash Chromatography. The intermediate **(15)** and **(17)** can be converted into the compounds of formula (**IIB**) and (**IIC**), respectively, by a second S_{N}Ar with H₂N-Q-R⁵ **(4)** in the same conditions as described above.

In the following paragraphs, the Descriptions 1 (D1) to 104 (D104) illustrate the preparation of intermediates used to make compounds of the invention and salts thereof. The Examples 1 (E1) to 238 (E238) illustrate the preparation of the compounds of the invention and salts thereof. Where the compounds have more than one chiral center, it is understood that they might exist as mixtures of diastereomers or as single isomers. Both racemic and chiral compounds are within the scope of the present invention. The indicated procedures are provided merely for assistance to the skilled chemist. The starting material may not necessarily have been prepared from the batch of the Description or the Example referred to.

Where not otherwise indicated, starting materials and/or intermediates were obtained from commercial sources or can be obtained through synthetic procedures known in the chemistry literature. The indication of the commercial source of certain compounds in the description of the experimental procedure, when provided, is only for easy reference to skilled chemist and should not be interpreted as the indication to use only that particular commercial compound.

The compounds described in the here reported synthesis are summarised in Table 1.

### Description D1: 2-((2-methoxyphenethyl)amino)isonicotinonitrile (D1)

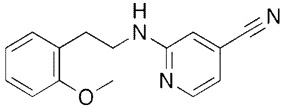

A mixture of 2-(2-methoxyphenyl)ethanamine (4.84 mL, 33.07 mmol) and 2-fluoro-4-pyridinecarbonitrile (4.04 g, 33.07 mmol) in DIPEA (8.64 mL, 49.6 mmol) was heated at 100 °C in a closed vial for 4.5 h. The crude product was diluted with water and extracted with DCM (2x). The organics were combined and evaporated under reduced pressure. The residue was taken up with DCM and the resulting suspension was filtered to give **D1** ( 6.48 g, 25.58 mmol, yield= 77.37%) as a white solid.

Method 1: R*t*= 1.62 min, MS (ESI) m/z = 254.19 [M+H]⁺.

¹H NMR (400 MHz, DMSO-*d*₆) δ ppm 8.17 (dd, J = 5.1, 0.9 Hz, 1H), 7.21 (ddd, J = 8.2, 7.4, 1.8 Hz, 1H), 7.17 -7.10 (m, 2H), 6.97 (dd, J = 8.2, 1.1 Hz, 1H), 6.87 (td, J = 7.4, 1.1 Hz, 1H), 6.82 - 6.74 (m, 2H), 3.80 (s, 3H), 3.48 - 3.38 (m, 2H), 2.81 (dd, J = 8.3, 6.5 Hz, 2H).

### Description D2: 2-((2-methoxyphenethyl)amino)isonicotinic acid (D2)

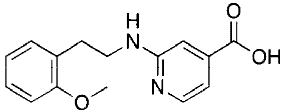

### Procedure 1 (basic conditions):

A suspension of **D1** (6.48 g, 25.58 mmol) in EtOH (256 mL) and NaOH (10.23 g, 255.82 mmol) was heated at 100 °C for 16 h. Then the solvent was removed partially under reduced pressure. The residue was treated with 2M HCl until product precipitation was observed. The resulting suspension was filtered to give **D2** (6.31 g, 23.17 mmol, yield = 90.58%) as beige solid.

### Procedure 2 (acidic conditions):

A suspension of **D1** (1.94 g, 7.66 mmol) in Hydrogen chloride (37%, 14 mL, 84.25 mmol) and heated in a microwave oven to 120 °C for 3h. The solid was filtered and washed with water (3 x 5 mL), dried under vacuum to afford D2 (1.55 g, 5.678 mmol, yield= 74.13%) as with-off solid.

Method 1: R*t*= 0.56 min, MS (ESI) m/z = 273.25 [M+H]⁺.

¹H NMR (400 MHz, DMSO-*d*₆) δ ppm 13.72 (br s, 1H), 8.05 (d, J = 5.9 Hz, 1H), 8.10 (br s, 1H), 7.30 - 7.15 (m, 3H), 6.97 (d, J = 7.8 Hz, 2H), 6.88 (td, J = 7.4, 0.8 Hz, 1H), 3.78 (s, 3H), 3.49 (br s, 2H), 2.85 (br t, J = 7.3 Hz, 2H)

### Description D3: (2-ethylpiperidin-1-yl)(2-fluoropyridin-4-yl)methanone (D3)

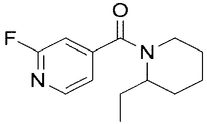

A solution of 2-fluoropyridine-4-carbonyl chloride (1.07 mL, 9.4 mmol) in DCM (10 mL) was dropped over 2 minutes in a previously prepared solution of 2-ethylpiperidine hydrochloride (1.4 g, 9.4 mmol) and TEA (4.2 mL, 30.08 mmol) in DCM (30 mL) at -25°C. The reaction was stirred at 0° for 5min and then at RT, ON. The reaction mixture was diluted with ss NaHCOs (15 mL). The organic layer was additionally washed with water and brine; then dried over anh Na₂SO₄, filtered, and concentrated under reduced pressure to afford D3 (2.24 g, 9.48 mmol, yield= 100.83%) as a yellow oil.

Method 1: R*t*= 1.53 min, MS (ESI) m/z = 237.2 [M+H]⁺.

¹H NMR (400 MHz, DMSO-*d*₆) δ ppm 8.28 (dt, J = 5.0, 0.8 Hz, 1H), 7.13 (ddd, J = 5.0, 2.0, 1.2 Hz, 1H), 6.89 (ddd, J = 2.7, 1.3, 0.7 Hz, 1H), 4.90 - 4.49 (m, 1H), 3.55 - 3.26 (m, 1H), 3.18 - 2.69 (m, 1H), 1.87 - 1.56 (m, 8H), 0.87 (d, J = 75.1 Hz, 3H).

### Description D4: 2-[2-(3-chlorophenyl)ethylamino]pyridine-4-carbonitrile (D4)

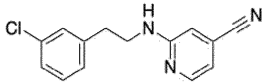

Prepared similarly as described for compound D1 starting from commercially available 2-(3-chlorophenyl)ethanamine (894 µL, 6.43 mmol), and 2-fluoro-4-pyridinecarbonitrile (784.6 mg, 6.43 mmol) in DIPEA (1.78 mL, 9.64 mmol) to yield title compound **D4** (1.53 g, 5.916 mmol, yield= 92.07%) as a yellow oil. Method 1: Rt= 1.91 min, MS (m/z) = 258.3 [M+H]⁺.

¹H NMR (400 MHz, DMSO-*d*₆) δ ppm 8.21 (d, J = 5.1 Hz, 1H), 7.24 - 7.19 (m, 3H), 7.15 - 7.04 (m, 1H), 6.74 (dd, J = 5.1, 1.4 Hz, 1H), 6.58 - 6.49 (m, 1H), 4.74 (s, 1H), 3.60 (q, J = 6.6 Hz, 2H), 2.91 (t, J = 6.9 Hz, 2H).

### Description D5: 2-[2-(3-chlorophenyl)ethylamino]pyridine-4-carboxylic acid, hydrochloride (D5)

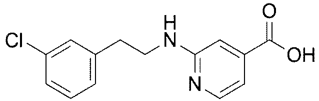

Prepared similarly as described for compound **D2** (procedure 2) starting from **D4** (1.52 g, 5.92 mmol) to afford **D5** (1.58 g, 5.045 mmol, yield= 85.27%) as a yellow solid.

¹H NMR (500 MHz, DMSO-*d*₆) δ ppm 12.22 - 15.64 (m, 2 H), 8.66 - 9.72 (m, 1 H), 7.99 (d, J=6.4 Hz, 1 H), 7.51 (br s, 1 H), 7.46 (s, 1 H), 7.27 - 7.38 (m, 3 H), 7.08 (dd, J=6.5, 1.4 Hz, 1 H), 3.69 (br t, J=7.1 Hz, 2 H), 2.94 (t, J=7.3Hz, 2 H)

### Description D6: (2-fluoropyridin-4-yl)(2-methylindolin-1-yl)methanone (D6)

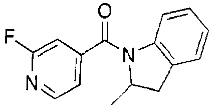

To a stirred solution of TEA (1.64 mL, 11.34 mmol) in DCM (2 mL), T3P (50% solution in EtOAc, 2.53 mL, 4.25 mmol) and 2-fluoro-4-pyridinecarboxylic acid (0.97 mL, 3.54 mmol) were added; then 2-methyl-2,3-dihydro-1H-indole (0.46 mL, 3.54 mmol) was added dropwise and stirred at RT for 3h. The reaction mixture was quenched with Na₂CO₃ (3M), and extracted with EtOAc (30 mL). The organic layer was washed with water (2x15 mL) and brine. The organic layer was dried over anh Na₂SO₄ and concentrated under a high vacuum to afford **D6** (0.64 g, 2.492 mmol, yield= 70.33%) as dark solid.

Method 1: R*t*= 0.99 min, MS (ESI) m/z = 257.2 [M+H]⁺.

### Description D7: (S)-(2-ethylpiperidin-1-yl)(2-fluoropyridin-4-yl)methanone (D7)

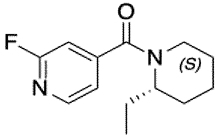

Prepared similarly as described for compound **D6** starting from (2*S*)-2-ethylpiperidine hydrochloride (992 mg, 6.63 mmol) and 2-fluoro-4-pyridinecarboxylic acid (935 mg, 6.63 mmol). After work-up was obtained **D7** (1.28 g, 5.417 mmol, yield= 81.73%) as a yellowish oil.

### Method 1: Rt= 1.50 min, MS (ESI) m/z = 237.2 [M+H]⁺.

### Description D8: (6,6-difluoro-3-azabicyclo[3.1.0]hexan-3-yl)(2-fluoropyridin-4-yl)methanone (D8)

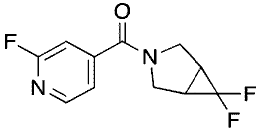

Prepared similarly as described for compound D3 starting from 2-fluoropyridine-4-carbonyl chloride (250 mg, 1.57 mmol) and 6,6-difluoro-3-azabicyclo[3.1.0]hexane;hydrochloride (0.28 g, 1.79 mmol). After work-up was obtained D8 (261 mg, 1.078 mmol, yield= 68.77%) as a yellowish oil.

### Method 1: Rt= 1.06 min, MS (ESI) m/z = 243.2[M+H]⁺.

¹H NMR (400 MHz, DMSO-*d*₆) δ ppm 8.36 (d, J = 5.1 Hz, 1H), 7.40 (ddd, J = 5.1, 2.1, 1.3 Hz, 1H), 7.29 (dd, J = 2.3, 1.2 Hz, 1H), 4.07 (d, J = 12.8 Hz, 1H), 3.90 (dt, J = 11.3, 4.7 Hz, 1H), 3.73 (ddd, J = 12.9, 5.4, 4.0 Hz, 1H), 3.54 (d, J = 11.3 Hz, 1H), 2.61 (dddd, J = 25.6, 12.4, 9.9, 5.3 Hz, 2H).

### Description 9: (S)-(2-fluoropyridin-4-yl)(3-hydroxypyrrolidin-1-yl)methanone (D9)

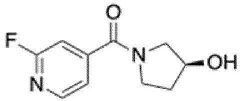

To a solution of (S)-3-Hydroxypyrrolidine (371 mg, 4.252 mmol, 1.2 eq), 2-fluoropyridine-4-carboxylic acid (500 mg, 3.5436 mmol, 1.0 eq) and DIPEA (1.4 mL, 10.631 mmol, 3.0 eq) in anh DMF (16 mL, 0.22 M), HATU (1.5 g, 3.898 mmol, 1.1 eq) was added in one portion. The reaction mixture was stirred at the RT for 1h. The reaction mixture was concentrated under reduced pressure to the -10% of the initial volume, and diluted with EtOAc (-180 mL). The mixture was transferred and washed with brine (3x 50 mL), citric acid (10% aq solution, 2x30 mL), water (2x30 mL), brine (3x50 mL). The water phase was additionally extracted with mixture DCM/MeOH (20%) (5x30 mL). The combined organic extracts were washed with brine (1x30 mL) dried over anh Na₂SO₄, filtered and evaporated to give **D9** (520 mg, 2.48mmol, yield= 64%) as crude which was used in the next step without additional purification.

Method 3: R*t*= 0.9 min, MS (ESI) m/z = 211.2 [M+H]⁺.

### Description 10: (R)-(2-fluoropyridin-4-yl)(3-hydroxypyrrolidin-1-yl) methanone (D10)

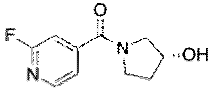

Prepared similarly as described for **D9,** starting from (*R*)-3-Hydroxypyrrolidine (310 mg, 3.544 mmol, 1.0 eq) and 2-Fluoropyridine-4-carboxylic acid (500 g, 3.544 mmol, 1.0 eq). After work-up, the crude was purified by NP Flash Chromatography (silica gel column, 0-50% MeOH in DCM) to afforded D10 (110 mg, 0.508 mmol, yield = 14%).

Method 3: R*t*= 0.9 min, MS (ESI) m/z = 210.85 [M+H]⁺.

¹H NMR (400 MHz, DMSO-*d*₆) δ ppm 8.37 - 8.30 (m, 1H), 7.48 - 7.40 (m, 1H), 7.33 - 7.25 (m, 1H), 5.02 (dd, J = 24.9, 3.5 Hz, 1H), 4.40 - 4.18 (m, 1H), 3.61 - 3.45 (m, 2H), 3.46 - 3.31 (m, 1H), 3.12 (dt, J = 10.9, 1.8 Hz, 1H), 2.05 - 1.72 (m, 2H).

### Description 11 : 2-((2-(2-methoxyphenyl)propyl)amino)isonicotinonitrile (D11)

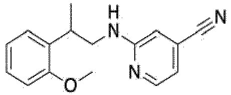

A mixture of 2-(2-methoxyphenyl)propan-1-amine;hydrochloride (99.1 mg, 0.490 mmol) and DIPEA (317.5 mg, 2.46mmol) in DMSO (540µL) was stirred at RT for 30 min; then 2-fluoro-4-pyridinecarbonitrile (60 mg, 0.490 mmol) was added. The reaction mixture was stirred at 100°C. After 4h the mixture was cooled down to RT, diluted with water, and extracted with EtOAc (2x 2mL). The combined organic layers were washed with brine, dried over Na₂SO₄, filtered and concentrated under reduced pressure to give **D11** (0.155 g, 0.580 mmol, yield = 118%) as solid. The latter was used in the next step without further purification.

Method 1: Rt= 1.79 min, MS (ESI) m/z = 268.2 [M+H]⁺.

### Description 12: 2-((2-(2-methoxyphenyl)propyl)amino)isonicotinic acid (D12)

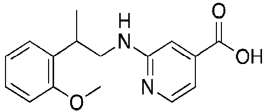

Prepared as described for **D2** (procedure 2), starting from **D11** (155 mg, 0.580 mmol) to give a solid **D12** (70 mg, 0.244 mmol, yield= 42.2%) that was used in the next step without further purification. Method 1: Rt= 0.94 min, MS (ESI) m/z = 287.2 [M+H]⁺. ¹H NMR (400 MHz, DMSO-*d*₆ ) δ ppm 12.99 - 13.30 (m, 1 H), 8.07 (d, J=5.2 Hz, 1 H), 7.13 - 7.24 (m, 2 H), 7.00 (s, 1 H), 6.95 (d, J=8.0 Hz, 1 H), 6.90 (t, J=7.3 Hz, 1 H), 6.78 - 6.86 (m, 2 H), 3.77 (s, 3 H), 3.33 - 3.47 (m, 3 H), 1.13 - 1.22 (m, 3 H)

### Description 13: (E)-7-(2-nitrovinyl)benzofuran (D13)

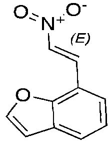

To a solution of 1-benzofuran-7-carbaldehyde (300 mg, 0.6166 mmol, 1.0 eq) in glacial acetic acid (16 mL, 0.12 M), nitromethane (3.3 mL, 61.58 mmol, 30 eq) and ammonium acetate (633 mg, 8.211 mmol, 4 eq) were added in one portion. The reaction mixture was stirred at 105°C for 4 hours. The reaction mixture was concentrated under reduced pressure to -5% of the initial volume and diluted with ~100 mL of EtOAc. The organic layer was washed with distilled water and brine, dried over anh Na₂SO₄, filtered and evaporated to give a crude product. Purification using NP Flash Chromatography (silica gel column, gradient elution: 0% - 45% EtOAc in Cyclohexane) afforded **D13** (0.36 g, 1.75 mmol, yield= 85%) as a yellow solid.

Method 3: Rt= 1.62 min, MS (ESI) m/z = not detected [M+H]⁺.

¹H NMR (300 MHz, DMSO-*d*₆) 8.37 - 8.25 (m, 2H), 8.20 (d, J= 2.2 Hz,1H), 7.90 (dd, J= 7.7, 1.2 Hz, 1H), 7.82 (dd, J= 7.6, 1.2 Hz,1H), 7.40 (t, J=7.6 Hz, 1H), 7.12 (d, J= 2.2 Hz, 1H).

### Description 14: 2-(benzofuran-7-yl)ethan-1-amine (D14)

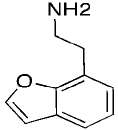

A solution of **D13** (360 mg, 1.93 mmol, 1.0 eq) in anh THF (5 mL, 0.12 M) was cooled to 0°C, and anh solution of LiAlH₄ (2M in THF, 0.92 mL, 1.84 mmol, 3.0 eq) was then added dropwise in 10 min. The reaction mixture was warmed to RT and stirred for 18 hours (progress was monitored by UPLC). The reaction mixture was cooled to 0°C and quenched with a saturated solution of Rochelle salt and diluted with ~100 mL of EtOAc. The organic layer was washed with distilled water, and brine, dried over anh Na₂SO₄, filtered and evaporated to give a crude product of **D14** (95 mg, 0.47 mmol, yield= 24%) which was used in the next step without additional purification.

Method 3: Rt= 1.38 min, MS (ESI) m/z = 162.1 [M+H]⁺.

### Description 15: (E)-7-(2-nitrovinyl)-2,3-dihydrobenzofuran (D15)

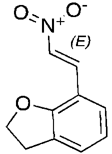

Prepared similarly as described for compound **D13** starting from 2,3-dihydro-1-benzofuran-7-carbaldehyde (300 g, 2.025 mmol, 1.0 eq). After a work-up, the crude was purified using NP Flash Chromatography (silica gel column, 0-15% EtOAc in Cyclohexane) and afforded **D15** (180 mg, 0.928 mmol, yield= 46%) as a yellow crystal.

Method 3: Rt= 2.37 min, MS (ESI) m/z = not detected [M+H]⁺.

¹H NMR (300 MHz, DMSO-*d*₆) δ ppm 8.07 (s, 1H), 7.57 - 7.38 (m, 2H), 6.95 (t, J = 7.5 Hz, 1H), 4.74 (t, J = 8.8 Hz, 2H), 3.30 (t, 3H).

### Description 16: 2-(2,3-dihydrobenzofuran-7-yl)ethan-1-amine (D16)

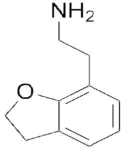

Prepared similarly as described for compound **D14** starting from **D15** (180 g, 0.928 mmol, 1.0 eq). After a work-up, the crude product was isolated and purified by NP Flash Chromatography (eluent mixture: DCM / MeOH / TEA from 99.5 / 0 / 0.5 to 90.25 / 9.25 / 0.5) to give **D16** (409 mg, 0.285 mmol, yield= 31%) as a sticky pale yellow solid.

### Method 3: Rt= 1.03 min, MS (ESI) m/z = 164.1 [M+H]⁺.

¹H NMR (300 MHz, DMSO-*d*₆) δ ppm 7.11 -7.02 (t, 1H), 6.92 (d, J = 7.4 Hz, 1H), 6.74 (t, J = 7.4 Hz, 1H), 4.49 (t, J = 8.7 Hz, 2H), 3.16 (t, J = 8.7 Hz, 2H), 2.72 (dd, J = 8.5, 6.1 Hz, 2H), 2.65 - 2.52 (m, 2H).

### Description 17: 2-cyclopropoxybenzaldehyde (D17)

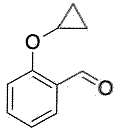

To a solution of salicylaldehyde (5 g, 40.94 mmol, 1.0 eq), cyclopropyl bromide (14.9 g, 122.83 mmol, 3.0 eq) and NaI (1.84 g, 12.28 mmol, 0.3 eq) in anh DMA (50 mL, 0.82 M), Cs₂CO₃ (26.7 g, 81.89 mmol, 2.0 eq) was added in one portion. The pressure vial was flushed with Ar and stirred at 150 °C for 18h. The reaction mixture was diluted with -150 mL and filtered off. The solid was washed with DCM (4x50 mL). The combined organic layers were concentrated under reduced pressure to -5% of the initial volume. The resulting oil was dissolved in -150 mL of EtOAc and washed with brine (2x30 mL), water (4x50 mL), brine (2x30 mL), dried over anh Na₂SO₄, filtered and evaporated. The crude was purified using NP Flash Chromatography (silica gel column, 0-25% EtOAc in Cyclohexane) to afford D17 (0.726 g, yield= 5%, purity: 50%) as a colorless oil.

Method 3: R*t*= 2.26 min, MS (ESI) m/z = 163.05 [M+H]⁺.

¹H NMR (300 MHz, CDCl₃) δ ppm 10.42 (s, 1H), 7.84 (dd, J = 7.7, 1.8 Hz, 1H), 7.59 - 7.55 (m, 1H), 7.39 (dd, J = 8.4, 1.0 Hz, 1H), 7.06 - 7.02 (m, 1H), 3.93 - 3.81 (m, 1H), 0.93 - 0.85 (m, 4H).

### Description 18: (E)-1-cyclopropoxy-2-(2-nitrovinyl)benzene (D18)

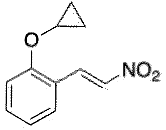

Prepared similarly as described for compound **D13** starting from **D17** (200 mg, 0.6166 mmol, 1.0 eq) in glacial acetic acid (5 mL, 0.12 M), nitromethane (1.0 mL, 18.50 mmol, 30 eq) and ammonium acetate (190 mg, 2.47 mmol, 4 eq). The crude product obtained after work-up, was purified using NP Flash Chromatography (silica gel column, 0-45% EtOAc in Cyclohexane) to afford **D18** (140 mg, 0.62 mmol, yield= 99%) as yellow solid.

Method 3: R*t*= 2.49 min, MS (ESI) m/z = not detected [M+H]⁺.

¹H NMR (300 MHz, CDCl₃) δ ppm 8.12 (d, J = 13.6 Hz, 1H), 7.83 (d, J = 13.6 Hz, 1H), 7.50 - 7.42 (m, 2H), 7.39 (dd, J = 8.3, 1.2 Hz, 1H), 7.06 (dd, J = 7.5, 1.2 Hz, 1H), 3.93 - 3.81 (m, 1H), 0.95 - 0.87 (m, 4H).

### Description 19: 2-(2-cyclopropoxyphenyl)ethan-1-amine (D19)

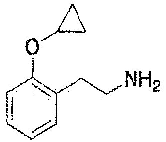

Prepared similarly as described for compound **D14** starting from **D18** (140 mg, 0.614 mmol, 1.0 eq). The crude product **D19** (120 mg, 0.61 mmol, yield= 99%) was obtained as yellowish oil and used in the next step without additional purification.

Method 3: R*t*= 1.53 min, MS (ESI) m/z = 178.2 [M+H]⁺.

¹H NMR (300 MHz, CDCl₃) δ ppm 7.27 - 7.19 (m, 2H), 7.15 (dd, J = 7.7, 6.2 Hz, 1H), 6.97 - 6.89 (m, 1H), 3.80 - 3.72 (m, 1H), 2.97 (t, J = 6.9 Hz, 2H), 2.79 (t, J = 6.8 Hz, 2H), 0.84 - 0.72 (m, 4H).

### Description 20: 2-((3-methoxyphenethyl)amino)isonicotinic acid (D20)

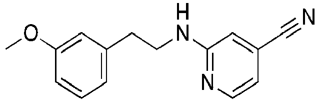

Prepared similarly as described for **D1** starting from 2-(3-methoxyphenyl)ethanamine (1.25 mL, 8.6 mmol), 2-fluoro-4-pyridinecarbonitrile (1.05 g, 8.6 mmol) in DIPEA (2.38 mL, 12.9 mmol) to afford title compound **D20** (2.15 g, 8.492 mmol, yield= 98.77%) as a yellow oil.

Method 1: R*t*= 1.60 min, MS (m/z) 254.2 [M+H]⁺.

¹H NMR (400 MHz, CDCl₃) δ ppm 8.19 (dd, J = 5.1, 0.9 Hz, 1H), 7.25 - 7.20 (m, 1H), 6.83 - 6.77 (m, 2H), 6.75 (t, J = 2.1 Hz, 1H), 6.72 (dd, J = 5.1, 1.3 Hz, 1H), 6.52 (t, J = 1.1 Hz, 1H), 4.77 (s, 1H), 3.80 (s, 3H), 3.58 (td, J = 6.8, 5.8 Hz, 2H), 2.90 (t, J = 6.9 Hz, 2H).

### Description 21: 2-((3-methoxyphenethyl)amino)isonicotinic acid (D21)

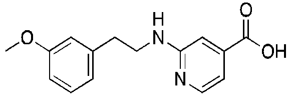

Prepared similarly as described for **D2** (procedure 2) starting from **D20** (1.51 g, 5.96 mmol) to afford **D21** (273 mg, 1.003 mmol, yield= 16.82%) as grey solid.

Method 1: Rt= 0.79 min, MS (m/z) 273.2 [M+H]⁺.

¹H NMR (500 MHz, DMSO-*d*₆) δ ppm 13.22 (br s, 1 H), 8.11 (d, J=5.2 Hz, 1 H), 7.20 (t, J=8.0 Hz, 1 H), 6.97 (s, 1 H), 6.94 (br s, 1 H), 6.85 (dd, J=5.3, 1.3 Hz, 1 H), 6.79 - 6.83 (m, 2 H), 6.73 - 6.78 (m, 1 H), 3.73 (s, 3 H), 3.46 - 3.52 (m, 2 H), 2.80 (t, J=7.3 Hz, 2 H)

### Description 22: (2-chloro-6-methylpyridin-4-yl)(2-ethylpiperidin-1-yl) methanone (D22)

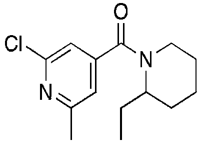

Prepared similarly as described for D3 starting from 2-chloro-6-methyl-pyridine-4-carbonyl chloride (370 µL, 2.63 mmol) and 2-ethylpiperidine hydrochloride (393.8 mg, 2.63 mmol). After work-up, the crude was purified by RP Flash Chromatography (Sfar C18, 12 g, eluent CH₃CN (+0.1% HCOOH) in H₂O (+0.1% HCOOH)) to give **D22** (542 mg, 2.032 mmol, yield= 77.22%) as a yellow oil.

Method 1: R*t*= 1.76 min, MS (ESI) m/z = 267.1 [M+H]⁺.

### Description 23: (2,6-dichloropyridin-4-yl)(2-ethylpiperidin-1-yl) methanone (D23)

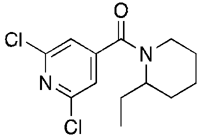

Prepared similarly as described for **D3** starting from 2,6-dichloropyridine-4-carbonyl chloride (0.65 mL, 4.75 mmol) and 2-ethylpiperidine hydrochloride (711.1 mg, 4.75 mmol). After a work-up, **D23** ( 1.07 g, 3.726 mmol, yield= 78.41%) was obtained as a yellow solid. The crude was used as such in the next synthetic step.

Method 1: R*t*= 2.04 min, MS (ESI) m/z = 287.12 [M+H]⁺.

### Description 24: (2,6-dichloro-4-pyridyl)-(6,6-difluoro-3-azabicyclo[3.1.0] hexan-3-yl)methanone (D24)

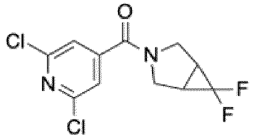

Prepared similarly as described for **D3** starting from 2,6-dichloropyridine-4-carbonyl chloride (70 µL, 0.480 mmol) and 6,6-difluoro-3-azabicyclo[3.1.0]hexane;hydrochloride (88.7 mg, 0.570 mmol). After work-up, the crude was purified by RP Flash Chromatography (Sfar C18 12 g, 2-98% CH₃CN (+0.1% HCOOH) in H₂O (+0.1% HCOOH)) to give **D24** (100 mg, 0.341 mmol, yield= 71.8%) as a yellowish oil.

Method 1: R*t*= 1.57 min, MS (ESI) m/z = 293.00 [M+H]⁺.

¹H NMR (400 MHz, DMSO-*d*₆) δ ppm 7.86 (s, 1H), 7.65 (s, 1H), 4.03 (d, J = 12.9 Hz, 1H), 3.90 (ddd, J = 11.3, 5.3, 3.9 Hz, 1H), 3.72 (ddd, J = 12.9, 5.4, 3.9 Hz, 1H), 3.59 (d, J = 11.2 Hz, 1H), 2.70 - 2.52 (m, 2H).

### Description 25: (2-chloro-6-(pyrrolidin-1-yl)pyridin-4-yl)(6,6-difluoro-3-azabicyclo[3.1.0]hexan-3-yl)methanone (D25)

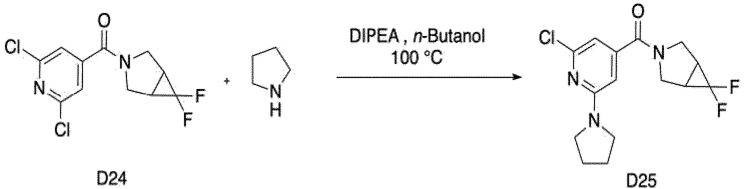

To a mixture of **D24** (50 mg, 0.170 mmol) and pyrrolidine (20 µL, 0.200 mmol) in 1-Butanol (4 mL), DIPEA (60 µL, 0.340 mmol) was added dropwise. The reaction mixture was stirred at 100 °C for 4h. Water (3 mL) was then added and the mixture was extracted with DCM (3 x 10 mL). The organic layer was separated by a phase separator, and was concentrated under high vacuum to give **D25** (20 mg, 0.06 mmol, yield= 36%) as a dark yellow oil.

Method 1: R*t*= 1.89 min, MS (ESI) m/z = 328.2 [M+H]⁺.

¹H NMR (400 MHz, DMSO-*d*₆) δ ppm 6.50 (d, J = 1.0 Hz, 1H), 6.34 (d, J = 1.0 Hz, 1H), 3.14 - 3.07 (m, 7H), 1.87 - 1.81 (m, 7H).

### Description 26: 1-(2-iodoisonicotinoyl)pyrrolidin-2-one (D26)

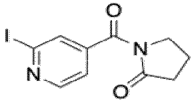

A mixture of 2-lodopyridine-4-carboxylic acid (300 mg, 1.2048 mmol, 1.0 eq) and anh Pyridine (1 mL, 13.253 mmol, 11 eq) in anh DCM (20 mL, 0.06 M), was cooled to ~0°C and oxalyl chloride (112 µL, 1.325 mmol, 1.1 eq) was added by dropwise and stirred at RT for 2h. Then the reaction mixture was cooled to ~0°C and mixture of DIPEA (1 mL, 6.024 mmol, 5.0 eq) and pyrrolidin-2-one (307 mg, 3.614 mmol, 3.0 eq) in 5 mL anh DCM was added. The reaction was warm to the RT and stirred ON. The reaction mixture is diluted with ~90 mL of DCM and 10 mL MeOH. The organic layer was washed with 10% citric acid, ss NaHCOs, brine, dried over anh Na₂SO₄, filtered and concentrated. The crude was purified by NP Flash Chromatography (silica gel column, 0-70% EtOAc in DCM) to afford **D26** (383 mg, 1.151 mmol, yield= 96%) as semi-solid compound.

Method 1: R*t*= 1.63 min, MS (ESI) m/z = 317.0 [M+H]⁺.

### Description 27: sodium; 2-((2-methoxyphenethyl)amino)-3-methylisonicotinate (D27)

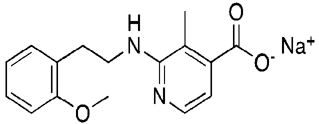

A suspension of Palladium (II) Acetate (1.12 mg, 0.010 mmol) and [1-(2-diphenylphosphino-1-naphthalenyl)-2-naphthalenyl]-diphenylphosphine (BINAP) (156 mg, 0.250 mmol) in 1,2-dimethoxyethane (DME) (5 mL) was stirred at RT for 10 min. Then sodium *tert*-butylate (96.28 mg, 1 mmol) was added. The obtained dark pink solution was treated with 2-chloro-3-methyl-pyridine-4-carboxylate (100 mg, 0.500 mmol) and 2-(2-methoxyphenyl)ethanamine (90.9 mg, 0.600 mmol). The reaction mixture was refluxed for 12h, quenched with water and extracted with DCM (x2). Then the water phase was concentrated under vacuum and stripped with toluene to obtain crude **D27** (260 g, 0.934 mmol).

Method 1: R*t*= 0.78 min, MS (ESI) m/z = 287.13 [M+H]⁺.

### Description 28: (2-(benzyloxy)-6-chloropyridin-4-yl)(2-ethylpiperidin-1-yl)methanone (D28)

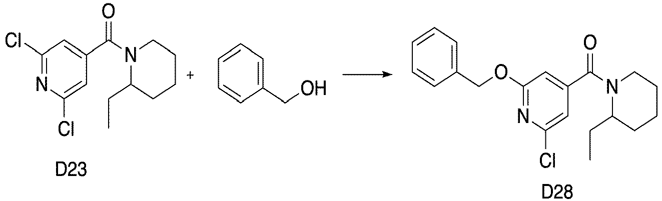

To a mixture of benzyl alcohol (0.1 mL, 0.970 mmol) in THF (10.55 mL), NaH 60 % dispersion in mineral oil (27.9 mg, 0.700 mmol) was added at 0°C and stirred for 1h. **D23** (100 mg, 0.350 mmol) was added portion-wise to the solution. The reaction mixture was stirred at RT for 1h and then at 40°C for 2h. The reaction was quenched with water (10 mL) and extracted with DCM (5mL). The organic layer was separated and concentrated under a high vacuum. The crude was purified by RP Flash Chromatography (Sfar C18, 12 g, eluent: CH₃CN (+0.1% HCOOH) in H₂O (+0.1% HCOOH)) to give **D28** (117 mg, 0.326 mmol, yield= 93.63%) as a light yellow oil.

Method 1: R*t*= 2.57 min, MS (ESI) m/z = 359.26 [M+H]⁺.

### Description 29: tert-butyl (1-(2-methoxyphenyl)azetidin-3-yl)carbamate (D29)

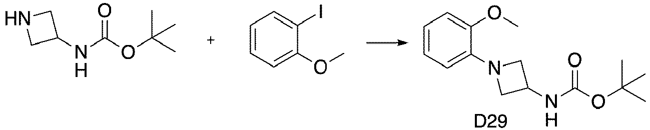

3-*N*-Boc-amino-azetidine (200 mg, 1.1 mmol, 1 eq), cesium carbonate (520 mg, 1.602 mmol, 1.5 eq), and 1-iodo-2-methoxybenzene (250 mg, 1.1 mmol, 1.0 eq) were suspended in dry toluene (0.11 M, 10 mL). Argon was bubbled through the suspension for 2 minutes, and then tris(dibenyzlideneacetone)dipalladium Pd₂(dba)₃ (78.3 mg, 0.085 mmol, 0.08 eq) and rac-2,2-bis(diphenylphosphino)-1,1'-binaphthyl (133 mg, 0.214 mmol, 0.2 eq) were added. The reaction vial was capped and heated to 110 °C with rapid stirring. The reaction mixture was diluted with ~90 mL of EtOAc. The organic layer was washed with a mixture of distilled water/brine (1:1), brine, dried over anh Na₂SO₄, filtered and concentrated. The crude was purified by NP Flash Chromatography (silica gel column, 0-70% EtOAc in Cyclohexane) to afford **D29** (0.25 g, 0.803 mmol, yield= 75%) as reddish solid. The crude was used in the next synthetic step without further purification..

Method 3: Rt= 2.73 min, MS (ESI) m/z = 279.2 [M+H]⁺.

¹H NMR (400 MHz, DMSO-*d*₆) δ ppm 7.44 (d, J = 7.5 Hz, 1H), 6.84 (dd, J = 7.9, 1.5 Hz, 1H), 6.79 (td, J = 7.6, 1.5 Hz, 1H), 6.71 (td, J = 7.6, 1.6 Hz, 1H), 6.37 (dd, J = 7.7, 1.6 Hz, 1H), 4.29 (p, J = 6.9 Hz, 1H), 4.06 (t, J = 7.7 Hz, 2H), 3.71 (s, 3H), 3.52 (t, J = 7.2 Hz, 2H), 1.39 (s, 9H).

### Description 30: 1-(2-methoxyphenyl)azetidin-3-amine; trifluoracetate (D30)

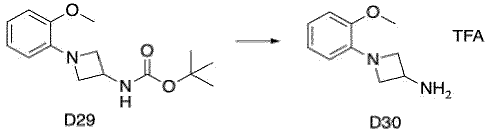

**D29** (249 mg, 0.805 mmol, 1.0 eq) was dissolved in a mixture DCM/TFA (10 mL, 1:1) and stirred for 30 min. DEE (100mL) was added to the reaction mixture and the solid was filtered, washed with DEE and dried on a filter. The crude D30 (240 mg, 0.59 mL, yield= 73%) was used in the next synthetic step without further purification.

Method 3: R*t*= 1.06 min, MS (ESI) m/z = 179.1 [M+H]⁺.

¹H NMR (400 MHz, DMSO-*d*₆) δ ppm 8.32 (s, 3H), 6.89 (dd, J = 7.8, 1.5 Hz, 1H), 6.83 (td, J = 7.5, 1.5 Hz, 1H), 6.77 (td, J = 7.6, 1.7 Hz, 1H), 6.46 (dd, J = 7.6, 1.7 Hz, 1H), 4.10 (dd, J = 8.3, 7.1 Hz, 2H), 3.79 (dd, J = 8.8, 4.4 Hz, 2H), 3.73 (s, 3H).

### Description 31: 2-((2-methoxybenzyl)amino)isonicotinonitrile (D31)

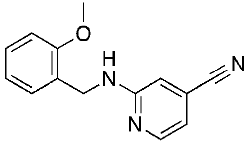

Prepared similarly as described for **D1** starting from 2-fluoro-4-pyridinecarbonitrile (2.22 g, 18.22 mmol) and (2-methoxyphenyl)methanamine (2.38 mL, 18.22 mmol) to give **D31** (2.48 g, 10.36 mmol, yield= 56.87%) as a white solid.

Method 1: R*t*= 1.58 min, MS (ESI) m/z = 240.17[M+H]⁺.

¹H NMR (400 MHz, DMSO-*d*₆) δ ppm 8.15 (dd, J = 5.2, 0.9 Hz, 1H), 7.41 (t, J = 5.9 Hz, 1H), 7.29 - 7.16 (m, 2H), 7.00 (dd, J = 8.3, 1.1 Hz, 1H), 6.93 - 6.84 (m, 2H), 6.79 (dd, J = 5.2, 1.4 Hz, 1H), 4.46 (d, J = 5.9 Hz, 2H), 3.83 (s, 3H).

### Description 32: 2-((2-methoxybenzyl)amino)isonicotinic acid (D32)

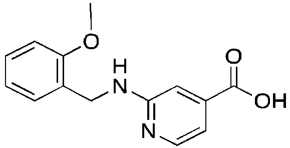

Prepared similarly as described for **D2** (procedure 2), starting from **D31** (2.51 g, 10.36 mmol) to give **D32** (2.11 g, 8.17 mmol, yield= 78.82%) as pale pink solid. The crude product was used as such in the next synthetic step. Method 1: Rt= 0.52 min, MS (ESI) m/z = 259.18[M+H]⁺.

¹H NMR (400 MHz, DMSO-*d*₆) δ ppm 13.24 (br s, 1H), 8.06 (d, J = 5.3 Hz, 1H), 7.25 - 7.15 (m, 3H), 7.05 (s, 1H), 6.98 (d, J = 8.0 Hz, 1H), 6.90 - 6.82 (m, 2H), 4.46 (d, J = 5.9 Hz, 2H), 3.82 (s, 3H)

### Description 33: 2-((3-methoxybenzyl)amino)isonicotinonitrile (D33)

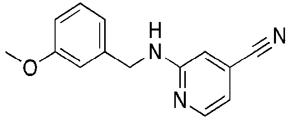

Prepared similarly as described for **D1** starting from 2-fluoro-4-pyridinecarbonitrile (2.23 g, 18.22 mmol) and (3-methoxyphenyl)methanamine (2.33 mL, 18.22 mmol) to give D33 (2.5 g, 10.45 mmol, yield= 57.33%) as a pale yellow solid. The crude was used as such in the next synthetic step.

Method 1: Rt= 1.57 min, MS (ESI) m/z =240.14 [M+H]⁺.

¹H NMR (400 MHz, DMSO-*d*₆) δ ppm 8.16 (dd, J = 5.2, 0.9 Hz, 1H), 7.61 (t, J = 6.0 Hz, 1H), 7.23 (t, J = 8.0 Hz, 1H), 6.93 - 6.85 (m, 3H), 6.84 - 6.76 (m, 2H), 4.47 (d, J = 6.0 Hz, 2H), 3.73 (s, 3H).

### Description 34: 2-((3-methoxybenzyl)amino)isonicotinic acid (D34)

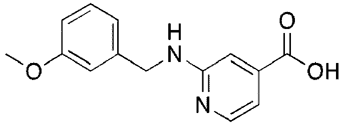

Prepared similarly as described for **D2** (procedure 2), starting from **D33** (2.13 g, 8.9 mmol) to obtain **D34** (2.27 g, 8.789 mmol, yield= 98.73%) as beige solid. The crude product was used as such in the next synthetic step.

¹H NMR (400 MHz, DMSO-*d*₆) δ ppm 13.24 (br s, 1H), 8.08 (d, J = 5.3 Hz, 1H), 7.41 (br s, 1H), 7.22 (t, J = 8.1 Hz, 1H), 7.04 (s, 1H), 6.88 (t, J = 4.5 Hz, 3H), 6.79 (dd, J = 8.1, 2.1Hz, 1H), 4.48 (d, J = 5.8 Hz, 2H), 3.72 (s, 3H).

### Description 35: 2-((2-(trifluoromethoxy)benzyl)amino)isonicotinonitrile (D35)

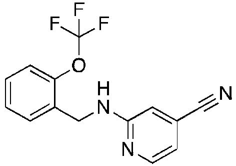

Prepared similarly as described for **D1** starting from 2-fluoro-4-pyridinecarbonitrile (500 mg, 4.1mmol) and [2-(trifluoromethoxy)phenyl]methanamine (780 mg, 4.1 mmol) to give D35 (390 mg, 1.337 mmol, yield=32.64%) as a white solid.

Method 1: Rt= 2.04 min, MS (ESI) m/z =294.10 [M+H]⁺.

¹H NMR (400 MHz, DMSO-*d*₆) δ ppm 8.16 (dd, J = 5.2, 0.9 Hz, 1H), 7.63 (t, J = 6.0 Hz, 1H), 7.47 - 7.31 (m, 4H), 6.92 (t, J = 1.1 Hz, 1H), 6.83 (dd, J = 5.2, 1.4 Hz, 1H), 4.59 (d, J = 5.9 Hz, 2H).

### Description 36: 2-((2-(trifluoromethoxy)benzyl)amino)isonicotinic acid (D36)

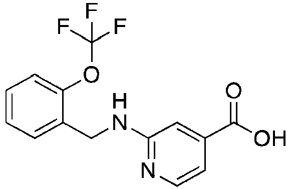

Prepared similarly as described for **D2** (procedure 2), starting from **D35** (392 mg, 1.34 mmol) to give **D36** (250 mg, 0.801 mmol, yield= 59.89%) as beige solid.

¹H NMR (400 MHz, DMSO-*d*6) δ ppm 13.37 (s, 1H), 8.08 (d, J = 5.4 Hz, 1H), 7.53 - 7.25 (m, 5H), 7.15 (s, 1H), 7.00 - 6.84 (m, 1H), 4.62 (d, J = 4.3 Hz, 2H).

### Description 37: methyl 2-(3-(2-methoxyphenyl)ureido)isonicotinate (D37)

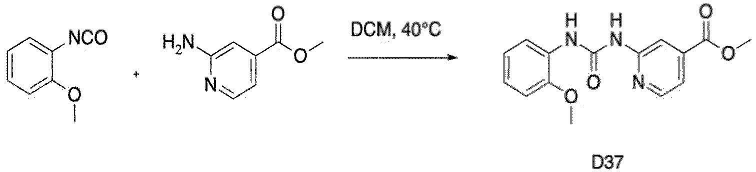

To a suspension of 2-amino-4-pyridinecarboxylic acid methyl ester (500 mg, 3.35 mmol) in DCM (5 mL), a solution of 1-isocyanato-2-methoxybenzene (510 mg, 3.35 mmol) in DCM (10 mL) was added at RT. The reaction was stirred at 40°C for 64 h. The solid was filtered, washed with DCM (5 mL) and dried under vacuum to afford **D37** (460 mg, 1.527 mmol, yield= 45.54%) as a white solid.

Method 1: Rt= 1.01 min, MS (ESI) m/z = 302.20 [M+H]⁺.

¹H NMR (400 MHz, DMSO-*d*₆) δ ppm 10.54 (br. s., 1 H), 10.08 (s, 1 H), 8.48 (dd, J=5.17, 0.77 Hz, 1 H), 8.20 (dd, J=7.92, 1.54 Hz, 1 H), 8.01 (s, 1 H), 7.43 (dd, J=5.28, 1.54 Hz, 1 H), 7.09 - 6.97 (m, 2 H), 6.95 - 6.88 (m, 1 H), 3.91 (d, J=2.64 Hz, 6 H).

### Description 38: 2-(3-(2-methoxyphenyl)ureido)isonicotinic acid (D38)

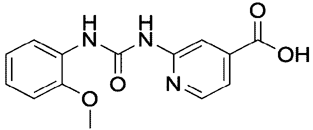

To a suspension of **D37** (460 mg, 1.51 mmol) in MeOH (10 mL), NaOH (2M)_{aq} (7.6 mL, 15.11 mmol) was added at RT. The reaction mixture was stirred at 40°C for 4 h. The solid formed was filtered and dried under vacuum to afford the first batch of **D38** (30 mg, 0.104 mmol,yield= 6.9%) as a white solid.

The mother liquor was partially concentrated under vacuum, pH was adjusted to 2 - 3 with HCl 37%. The precipitated product was filtered out, washed with water and dried under vacuum to afford a second batch of **D38** (410 g, 1.319 mmol, yield= 87.29%) as a white solid.

Method 1: R*t*= 0.90 min, MS (ESI) m/z = 288.1 [M+H] ⁺.

¹H NMR (400 MHz, DMSO-*d*₆) δ ppm 13.67 (br s, 1 H), 10.77 (br s, 1 H), 10.03 (s, 1 H), 8.44 (d, J=5.3 Hz, 1 H), 8.20 (dd, J=8.0, 1.2 Hz, 1 H), 7.91 (br s, 1 H), 7.40 (dd, J=5.2, 1.2 Hz, 1 H), 6.95 - 7.08 (m, 2 H), 6.88 - 6.95 (m, 1 H), 3.91 (s, 3 H).

### Description 39: methyl 2-(2-(2-methoxyphenyl)acetamido)isonicotinate (D39)

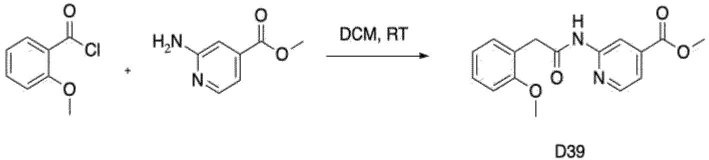

To a suspension of 2-amino-4-pyridinecarboxylic acid methyl ester (1.24 g, 8.12 mmol) in DCM (10 mL), a solution of 2-(2-methoxyphenyl)acetyl chloride (500 mg, 2.71 mmol) in DCM (20 mL) was added at 25 °C dropwise. The reaction was stirred at RT for 2h, then washed with ss NaHCOs (2 x 15 mL), then with brine (15 mL). The organic layer was dried over anh Na₂SO₄ and concentrated under vacuum to afford the impure product (225 mg, 0.749 mmol, yield= 27.66%) as a red solid.

The solid was suspended in MeCN (10 mL) filtered and washed additionally with small portions of MeCN. The solid was dried under vacuum to give **D39** (157 mg, 0.52 mmol, yield= 20%).

Method 1: R*t*= 1.70 min, MS (ESI) m/z = 301.2 [M+H] ⁺. ¹H NMR (400 MHz, DMSO-*d*₆) δ ppm 10.80 (s, 1 H) 8.57 (d, J=1.32 Hz, 1 H) 8.52 (dd, J=5.17, 0.77 Hz, 1 H) 7.53 (dd, J=5.06, 1.54 Hz, 1 H) 7.19 - 7.31 (m, 2 H) 6.95 - 7.02 (m, 1 H) 6.85 - 6.95 (m, 1 H) 3.88 (s, 3 H) 3.74 - 3.78 (m, 5 H)

### Description 40: 2-(2-(2-methoxyphenyl)acetamido)isonicotinic acid (D40)

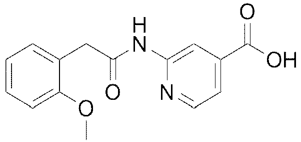

Prepared similarly as described for **D38,** starting from **D39** (224.3 mg, 0.750 mmol) to afford **D40** (191 mg, 0.667 mmol, yield= 89.34%) as a white solid. Method 1: R*t*= 1.35 min, MS (ESI) m/z = 287.2 [M+H] ⁺. ¹H NMR (400 MHz, DMSO-*d*₆) δ ppm 13.55 (br s, 1 H), 10.74 (s, 1 H), 8.54 (s, 1 H), 8.48 (dd, J=5.1, 0.7 Hz, 1 H), 7.50 (dd, J=5.0, 1.4 Hz, 1 H), 7.19 - 7.28 (m, 2 H), 6.98 (d, J=7.8 Hz, 1 H), 6.90 (td, J=7.4, 0.9 Hz, 1 H), 3.75 (s, 3 H), 3.74 (s, 2 H).

### Description 41: 4-iodo-N-(2-methoxyphenethyl)pyridin-2-amine (D41)

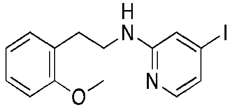

A mixture of 2-(2-methoxyphenyl)ethanamine (330 µL, 2.24 mmol), 2-fluoro-4-iodopyridine (500 mg, 2.24 mmol) and DIPEA (980 µL, 5.61 mmol) in DMSO (2mL) was stirred for 18 hours at 65°C. The mixture was then allowed to cool to RT, water was added and the mixture was extracted with EtOAc (2x). The combined organic layers were washed with brine, dried over Na₂SO₄, filtered and evaporated under reduced pressure. The residue was purified by NP Flash Chromatography (silica gel column Sfar D, 25 g, 0-60% EtOAc in Cyclohexane) to give **D41** (435 mg, 1.228 mmol, yield= 54.77%) as an off-white solid.

Method 1: Rt= 1.17 min, MS (ESI) m/z = 355.1 [M+H]⁺.

¹H NMR (400 MHz, DMSO-*d*₆) δ ppm 7.68 (d, J = 5.23 Hz, 1H), 7.19 (ddd, J = 8.15, 7.38, 1.77 Hz, 1H), 7.13 (dd, J = 7.45, 1.76 Hz, 1H), 6.96 (dd, J = 8.25, 1.10 Hz, 1H), 6.92 - 6.82 (m, 2H), 6.80 (dd, J = 5.30, 1.45 Hz, 1H), 6.70 (t, J = 5.70 Hz, 1H), 3.80 (s, 3H), 3.36 (ddd, J = 8.66, 7.43, 5.88 Hz, 2H), 2.91 - 2.69 (m, 2H).

### Description 42: 2-fluoro-4-iodopyridine (D42)

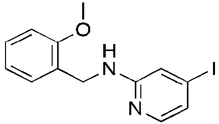

Prepared similarly as described for compound **D41,** starting from (2-methoxyphenyl)methanamine (300 µL, 2.24 mmol) and 2-fluoro-4-iodopyridine (500 mg, 2.24 mmol) to afford **D42** (389 mg, 1.144 mmol, yield= 51%) as an off-white solid.

Method 1: R*t*= 1.11 min, MS (ESI) m/z = 341.1 [M+H] ⁺.

¹H NMR (400 MHz, DMSO-*d*₆) δ ppm 7.65 (d, J = 5.30 Hz, 1H), 7.38 - 7.10 (m, 2H), 7.08 - 6.94 (m, 3H), 6.87 (td, J = 7.40, 1.08 Hz, 1H), 6.81 (dd, J = 5.34, 1.46 Hz, 1H), 4.39 (d, J = 5.94 Hz, 2H), 3.81 (s, 3H).

### Description 43: 2-((2-chloropyrimidin-4-yl)amino)benzonitrile (D43)

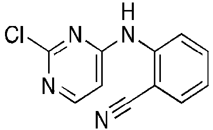

To a solution of H₂O (70 mL) and 37% HCl (0.7 mL, 8.4 mmol), 2-aminobenzonitrile (555 mg, 4.7 mmol) and 2,4-dichloropyrimidine (700 mg, 4.7 mmol) were added. The reaction mixture was stirred at 50 °C for 29 hours. The white solid formed was filtered over a Buchner funnel and then dried under vacuum for 2 days to give D43 (661 mg, 2.866 mmol, yield= 61%) as a white solid. Method 1: Rt= 1.32 min, MS (ESI) m/z = 231.2 [M+H]⁺.

¹H NMR (400 MHz, DMSO-*d*₆) δ ppm 10.26 (s, 1 H), 8.24 (d, J=5.9 Hz, 1 H), 7.88 (dd, J=7.8, 1.2 Hz, 1 H), 7.71 - 7.78 (m, 1 H), 7.64 (dd, J=8.2, 0.7 Hz, 1 H), 7.41 (td, J=7.7, 1.2 Hz, 1 H), 6.80 (d, J=5.8 Hz, 1 H).

### Description 44: 2-chloro-N-(2-chlorobenzyl)pyrimidin-4-amine (D44)

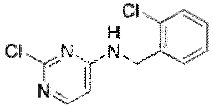

To a solution of 2,4-dichloropyrimidine (400 mg, 2.68 mmol) and TEA (560 µL, 4.03 mmol) in ethanol (12 mL) at 0 °C, (2-chlorophenyl)methanamine (320 µL, 2.68 mmol) was added. The mixture was warmed to RT and stirred for 5 hours. The solvent was evaporated under vacuum and the crude was purified by NP Flash Chromatography (silica gel column Sfar D, 25 g, 0-25% EtOAc in Cyclohexane) to give **D44** (438 mg, 1.724 mmol, yield= 64.2%) as a white solid.

Method 1: Rt= 1.00 min, MS (ESI) m/z = 254.1 [M+H]⁺.

¹H NMR (400 MHz, DMSO-*d*₆) 8.38 (br s, 1H), 7.96 (br d, J = 5.5 Hz, 1H), 7.52 - 7.43 (m, 1H), 7.43 - 7.26 (m, 3H), 6.70 - 6.31 (m, 1H), 4.56 (br d, J = 4.5 Hz, 2H).

### Description 45 and 46: 2-chloro-N-(2-methoxybenzyl)pyrimidin-4-amine (D45) and 4-chloro-N-(2-methoxybenzyl)pyrimidin-2-amine (D46)

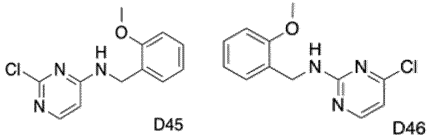

Prepared similarly as described for **D44,** starting from 2,4-dichloropyrimidine (500 mg, 3.36 mmol) and 2-methoxyphenyl)methanamine (450 µL, 3.36 mmol). The crude was purified by NP Flash Chromatography (silica gel column Sfar D, 25 g, 0-25% EtOAc in Cyclohexane) to give **D45** (518 mg, 2.074 mmol, yield= 61.8%) and **D46** (107 mg, 0.429 mmol, yield= 12.8%) as white solids.

**D45:** Method 1: Rt= 0.94 min, MS (ESI) m/z = 250.2 [M+H]⁺.

¹H NMR (400 MHz, DMSO-*d*₆ δ ppm 8.09 - 8.36 (m, 1 H), 7.79 - 8.06 (m, 1 H), 7.24 - 7.33 (m, 1 H), 7.20 (br d, J=7.0 Hz, 1 H), 7.01 (d, J=8.0 Hz, 1 H), 6.91 (t, J=7.3 Hz, 1 H), 6.28 - 6.62 (m, 1 H), 4.21 - 4.56 (m, 2 H), 3.82 (s, 3 H).

D46: Method 1: R*t*= 1.09 min, MS (ESI) m/z = 250.2 [M+H]⁺.

¹H NMR (400 MHz, DMSO-*d*₆) δ ppm 8.22 (d, J = 5.20 Hz, 1H), 7.97 (t, J = 6.27 Hz, 1H), 7.30 - 7.18 (m, 1H), 7.15 (d, J = 7.21 Hz, 1H), 6.97 (d, J = 8.08 Hz, 1H), 6.87 (t, J = 7.37 Hz, 1H), 6.67 (d, J = 5.14 Hz, 1H), 4.45 (d, J = 5.11 Hz, 2H), 3.81 (s, 3H).

### Description 47: 6-chloro-N-(2-chlorobenzyl)pyrimidin-4-amine (D47)

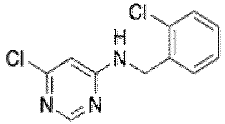

Prepared similarly as described for **D44,** starting from 4,6-dichloropyrimidine (400 mg, 2.68 mmol) and (2-chlorophenyl)methanamine (320 µL, 2.68 mmol). The crude was purified by NP Flash Chromatography (silica gel column Sfar D, 25 g, 0-30% EtOAc in Cyclohexane) to afford **D47** (517 mg, 2.034 mmol, yield= 75.77%) as a white solid.

Method 1: Rt= 1.02 min, MS (ESI) m/z = 254.1 [M+H]⁺.

¹H NMR (400 MHz, DMSO-*d*₆) δ ppm 8.28 (s, 1H), 8.21 (br s, 1H), 7.52 - 7.42 (m, 1H), 7.40 - 7.23 (m, 3H), 6.66 (br s, 1H), 4.61 (br s, 2H).

### Description 48: 2-methoxy-6-(methoxymethoxy)benzaldehyde (D48)

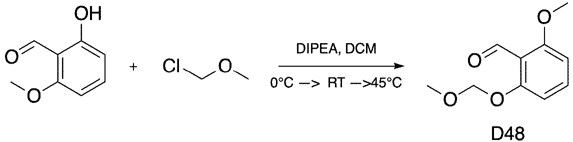

To a solution of 2-hydroxy-6-methoxybenzaldehyde (1 g, 6.57 mmol, 1 eq.) and DIPEA (3.43 mL, 19.72 mmol, 3 eq.) in DCM at 0°C, chloro(methoxy)methane (600 µL, 7.89 mmol, 1.2 eq.) was dropwise added in 1 min. After 6h, DIPEA (1.13 mL, 6.50 mmol, 1 eq.) and chloro(methoxy)methane (300 µl, 0.6 eq.) were added. The reaction was heated to 45°C and stirred for 6 h, then was diluted with water. The organic layer was separated and washed with ss NaHCO₃, dried over anh Na₂SO₄ and concentrated under vacuum to afford **D48** (1.2 g, 6.116 mmol, yield= 93.06%) as red oil.

Method 1: Rt= 1.26 min, MS (ESI) m/z = not detected [M+H]⁺ .

¹H NMR (400 MHz, CDCl₃) δ ppm 10.55 (s, 1H), 7.40-7.50 (m, 1H), 6.81 (d, J=8.36 Hz, 1H), 6.65 (d, J=8.36 Hz, 1H), 5.29 (s, 2H), 3.89-3.99 (m, 3H), 3.53 (s, 3H).

### Description 49: (E)-1-methoxy-3-(methoxymethoxy)-2-(2-nitrovinyl) benzene (D49)

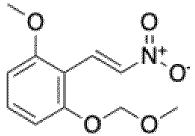

**D48** (1.2 g, 6.12 mmol) was dissolved in nitromethane (10 mL) and ammonium;acetate (613 mg, 7.95 mmol) was then added. The reaction was stirred at 100 °C for 2h. The solvent was removed and the residue was dissolved in DCM. The organic layer was washed with water (x3) and brine, then dried over MgSOa and concentrated under vacuum to yield **D49** as red solid (1.4 g, 5.85 mmol, yield= 95.69%).

Method 1: Rt= 1.94 min, MS (ESI) m/z = 240.1 [M+H]⁺

¹H NMR (400 MHz, CDCl₃) δ ppm 8.58 (d, J=13.65 Hz, 1H), 8.11 (d, J=13.65 Hz, 1H), 7.38 (t, J=8.47 Hz, 1H), 6.82-6.88 (m, 1H), 6.62-6.67 (m, 1H), 5.32 (s, 2H), 3.96 (s, 3H), 3.53 (s, 3H)

### Description 50: 2-(2-methoxy-6-(methoxymethoxy)phenyl)ethan-1-amine (D50)

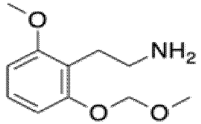

Prepared similarly as described for **D14,** starting from **D49** (1.4 g, 5.85 mmol). A work-up yielded **D50** (910 mg, 4.31 mmol, yield= 73.6%) as red oil. The product was used in the following step without further purification and analysis.

Method 1: Rt= r.t. 0.59 min, MS (ESI) m/z = 212.1 [M+H]⁺.

### Description 51: 2-((2-methoxy-6-(methoxymethoxy) phenethyl) amino) isonicotinonitrile (D51)

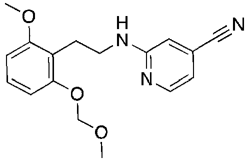

Prepared similarly as described for compound **D1** starting from 2-fluoro-4-pyridinecarbonitrile (347 mg, 2.84 mmol) and **D50** (600 mg, 2.84 mmol) in DIPEA (740 µL, 4.26 mmol). After a work-up, **D51** (412 mg, 1.315 mmol, yield= 46.3%) was obtained as a brown solid.

Method 1: Rt= 1.68 min, MS (ESI) m/z =314.18 [M+H]⁺.

### Description 52: 2-((2-methoxy-6-(methoxymethoxy)phenethyl)amino) isonicotinic acid (D52)

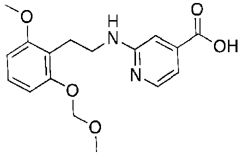

Prepared similarly as described for compound **D2** (Procedure1) starting from **D51** (412 mg, 1.31 mmol) to give **D52** (120 mg, 0.361 mmol, yield= 27.46%) as solid. The product was used in the next step without further purification.

### Description 53:1-(cyclopentyloxy)-2-[(1E)-2-nitroethenyl]benzene (D53)

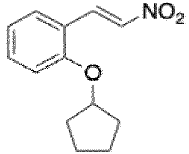

Prepared similarly as described for compound **D49** starting from 2-(cyclopentyloxy)benzaldehyde (500 mg, 2.628 mmol, 1.0 eq). After a work-up, the crude was purified by NP Flash Chromatography (silica gel, 0-20% EtOAc in Cyclohexane) to afford **D53** as yellow liquid (491 mg, 2.08 mmol, yield= 79%).

Method 3: Rt= 2.07min, MS (ESI) m/z = not detected [M+H]⁺.

### Description 54: 2-(2-(cyclopentyloxy)phenyl)ethan-1-amine (D54)

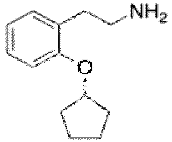

Prepared similarly as described for compound **D14** starting from **D53** (491 mg, 2.084 mmol, 1.0 eq). After work-up, crude **D54** (427 mg, 1.456 mmol, yield= 70%) was obtained as yellowish oil, which was used in the next step without additional purification.

Method 3: R*t*= 1.50 min, MS (ESI) m/z = 206.2 [M+H]⁺.

### Description 55:(2-fluoropyridin-4-yl)(pyrrolidin-1-yl)methanone (D55)

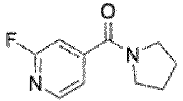

Prepared similarly as described for compound **D9** starting from Pyrrolidine (587 µL, 7.03 mmol, 1.0 eq), and 2-Fluoropyridine-4-carboxylic acid (1.091 g, 7.733 mmol, 1.1 eq). After a work-up, a crude **D55** (457 mg, 1.977 mmol, yield= 28%) was obtained as brown oil and used in the next step without any further purification.

Method 3: R*t*= 0.87 min, MS (ESI) m/z =194.9 [M+H]⁺.

¹H NMR (400 MHz, DMSO-*d*₆) δ ppm 8.34 (dd, J = 5.0, 0.9 Hz, 1H), 7.45 (ddd, J = 5.1, 2.2, 1.2 Hz, 1H), 7.31 (dt, J = 2.1, 1.0 Hz, 1H), 3.47 (t, J = 6.7 Hz, 2H), 3.34 (d, J = 6.6 Hz, 2H), 1.96 - 1.77 (m, 4H).

### Description 70a: (E)-4-bromo-1-methoxy-2-(2-nitrovinyl)benzene (D70a)

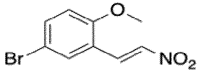

Prepared similarly as described for compound **D49** starting from 5-bromo-2-methoxybenzaldehyde (1.5 g, 6.98 mmol)) to yield **D70a** (0.3 g, 1.162 mmol, yield= 16.67%).

Method 1: R*t*= 2.14. min, MS (m/z) 258 [M+H]⁺

¹H NMR (400 MHz, CDCl₃) δ ppm 8.07 (d, J = 13.6 Hz, 1H), 7.84 (d, J = 13.6 Hz, 1H), 7.59 (d, J = 2.5 Hz, 1H), 7.56 (dd, J = 8.8, 2.5 Hz, 1H), 6.89 (d, J = 8.8 Hz, 1H), 3.97 (s, 3H).

### Description 70b: 2-(5-bromo-2-methoxyphenyl)ethan-1-amine (N1424-52) (D70b)

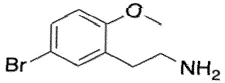

To an ice-water bath cooled suspension of sodium borohydride (220 mg, 5.81 mmol) in anh THF (7.2 mL), boron trifluoride diethyl etherate (860 µL, 6.97 mmol) was added dropwise. The reaction mixture was stirred at RT for 20 minutes and a solution of **D70a** (300 mg, 1.16 mmol) in anh THF (3 mL) was added. The resulting mixture was heated to reflux and stirred ON. After cooling again in the ice-water bath, 2N HCl in water was added (caution!). The resulting mixture was stirred at 80°C for 2h. After cooling, the reaction mixture was extracted with diethyl ether. The aqueous layer was neutralized with NaHCO₃ and extracted with EtOAc (x3). The combined organic layers were washed with brine (3 times), dried over Na₂SO₄, filtered and evaporated. The crude **D70b** (210 mg, 0.9 mmol, yield= 77.76%) was used for the next step without further purification.

Method 1: R*t*= 1.21 min, MS (ESI) m/z = 230.03 [M+H]⁺

¹H NMR (400 MHz, DMSO) δ ppm 7.76 (s, 2H), 7.44 (dd, J = 8.7, 2.6 Hz, 1H), 7.37 (d, J = 2.5 Hz, 1H), 6.99 (d, J = 8.8 Hz, 1H), 3.81 (s, 3H), 3.05 - 2.92 (m, 2H), 2.83 (dd, J = 8.8, 6.6 Hz, 2H).

### Description 70c: 2-((5-bromo-2-methoxyphenethyl) amino) isonicotinonitrile (D70c)

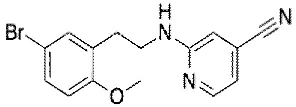

Prepared similarly as described for compound **D1** starting from **D70b** (208 mg, 0.90 mmol) and 2-fluoro-4-pyridinecarbonitrile (110 mg, 0.90 mmol) in DIPEA (160 µL, 0.90 mmol) to yield **D70c** (27 mg, 0.081mmol, yield= 9%).

### Description 70: 2-((5-bromo-2-methoxyphenethyl)amino)isonicotinic acid (D70)

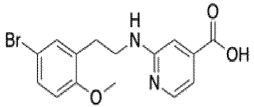

Prepared similarly as described for compound **D2** (procedure 1) starting from **D70c** (31 mg, 0.090 mmol) to yield **D70** (30 mg, 0.085mmol, yield= 91.5%). The crude product was used as such in the next synthetic step.

### Description 80: 2-methoxy-[1,1'-biphenyl]-3-carbaldehyde (D80)

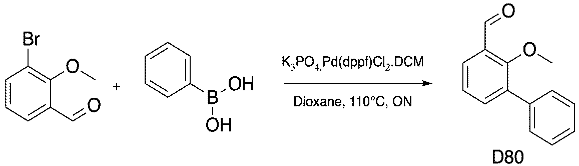

In vial, 3-bromo-2-methoxybenzaldehyde (1 g, 4.65 mmol, 1.0 eq.), phenyl boronic acid (0.68 g, 5.58 mmol, 1.2 eq.), and K₃PO₄ tribasic (2.96 g, 13.95 mmol, 3.0 eq.) were dissolved in anh Dioxane (18 mL). The mixture was flushed with N₂ for 10 min. Pd(dppf)Cl₂.DCM (0.4 g, 0.46 mmol, 0.1 eq.) was added. The vial was sealed and stirred at 110 °C, ON. The reaction mixture was cooled to RT, filtered through celite, washed with EtOAc, the organic phase was washed with brine, dried over anh Na₂SO₄, filtered, and concentrated in vacuum to give a crude as brown oil. The crude product was purified on NP Flash Chromatography (silica gel, eluent hexane/EtOAc = 9/1) to give pure **D80** as brown oil (550 mg, yield= 53%).

Method 3: R*t*= 3.73 min, MS (ESI) m/z =212.9 [M+H]⁺.

¹H NMR (400 MHz, DMSO-*d*₆) δ ppm 10.37 (d, J = 0.8 Hz, 1H), 7.77 (dd, J = 7.7, 1.8 Hz, 1H), 7.70 (dd, J = 7.6, 1.8 Hz, 1H), 7.61 - 7.56 (m, 2H), 7.50 (tq, J = 8.2, 1.6, 1.2 Hz, 2H), 7.46 - 7.36 (m, 2H), 3.49 (s, 3H).

### Description 84: trans-6,6-difluoro-N-((2-fluoropyridin-4-yl) methyl) bicyclo [3.1.0]hexane-3-carboxamide (D84)

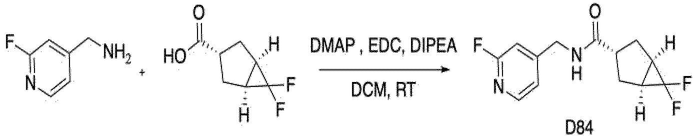

To a solution of (2-fluoropyridin-4-yl)methanamie hydrochloride (CAS n°1447942-40-6) (1 g, 6.17 mmol, 2 eq.) in anh DCM (20 mL), in RBF under N₂, DMAP (0.19 g, 1.54 mmol, 0.5 eq.), EDC (1.48 g, 7.70 mmol), and DIPEA (3.3 mL, 18.50 mmol, 6eq.) were added. Then, a solution of *trans*-6,6-difluorobicyclo[3.1.0]hexane-3-carboxylicl acid (0.5 g, 3.08 mmol, 1.0 eq.) in anh DCM (20 mL) was added to the mixture. The mixture was stirred at RT. After 1.5 h the mixture was quenched with ss NaHCO₃, extracted with DCM, washed with brine (2 x 20 mL), dried over anh Na₂SO₄, and concentrated in vacuum to give a brown solid (1.21 g, over yield). The crude was used as such in the next synthetic step.

Method 3: R*t*= 1.64 min, MS (ESI) m/z = 271.8 [M+H]⁺.

### Description 86: 2-(2-fluoropyridin-4-yl)-4-phenylthiazole (D86)

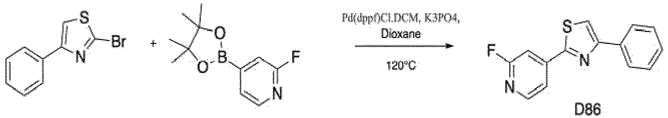

In vial, 2-bromo-4-phenylthiazole (CAS n° 57516-16-2) (150 mg, 0.6247 mmol, 1.0 eq.), 2-fluoropyridine-4-boronic acid pinacol ester (CAS n° 401815-98-3) (167 mg, 0.7496 mmol, 1.2 eq.), and K₃PO₄ tribasic (400 mg, 1.8741 mmol, 3.0 eq.) were dissolved in dioxane (2.5 mL). The mixture was flushed with N₂ for approx. 5 min followed by Pd(dppf)CIDCM (51 mg, 0.0625 mmol, 0.1 eq.) addition. The vial was sealed and stirred at 120°C, ON. The mixture was filtered through celite, washed with EtOAc (2 x 5 mL), brine, dried over anh Na₂SO₄, filtered, and concentrated in vacuum to give a crude as brown solid. The crude product was purified on NP Flash Chromatography (silica gel, eluent hexane/EtOAc = 9/1) to give a pure product as yellow solid (0.14 g, yield= 87%).

Method 3: Rt= 2.06 min, MS (ESI) m/z = 257.1 [M+H]⁺.

### Description 87: 2-fluoro-4-(5-phenyl-1H-imidazol-2-yl)pyridine (D87)

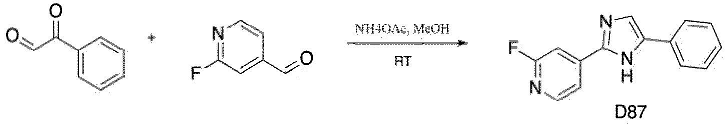

In RBF under N₂, a solution of phenylglyoxal (320 mg, 2.39 mmol, 1.0 eq.) in anh MeOH (10 mL) was added dropwise to a solution of 2-fluoro-pyridine-4-carbaldehyde (300 mg, 2.39 mmol, 1.0 eq.) and NH₄OAc (920 mg, 11.99 mmol, 5.0 eq.) in anh MeOH (10 mL). The reaction was stirred at RT, ON; then was concentrated under vacuum. The residue was partitioned between ss NaHCOs and DCM:MeOH (9:1). The organic phase was dried over Na₂SO₄, filtered, and concentrated under vacuum to give a crude D87 as yellow solid (600 mg, purity= 58%, yield= 61%). The crude product was used without further purification in the next synthetic step.

Method 3: R*t*= 1.64 min, MS (ESI) m/z =240.1 [M+H]⁺ .

¹H NMR (300 MHz, DMSO-*d*₆) δ ppm 8.32 (d, J = 5.3 Hz, 1H), 7.96 - 7.82 (m, 4H), 7.65 (s, 1H), 7.42 (t, J = 7.7 Hz, 2H), 7.30 - 7.24 (m, 1H).

### Description 94: 3-((2-chloropyridin-4-yl)methyl)-6,6-difluoro-3-azabicyclo [3.1.0]hexane (D94)

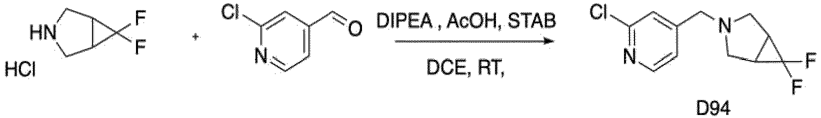

To suspension of 6,6-difluoro-3-azabicyclo[3.1.0]hexane HCl (200 mg, 1.2856 mmol, 1.0 eq) and 2-Chloroisonicotinaldehyde (200 mg, 1.414 mmol, 1.1 eq) in anh DCE (20 mL, 0.06 M), DIPEA (680 µL, 3.86 mmol, 3.0 eq) was added and reaction mixture was stirred at RT for 15 min. Then AcOH (150 µL, 2.57 mmol, 2.0 eq) was added and the reaction mixture was stirred at the same temperature for 1.5h. Then STAB (0.6 g, 2.83 mmol, 2.2 eq) was added and the reaction mixture was stirred at the same temperature for 2h. The reaction mixture was diluted with DCM (70 mL). Organic layer washed with ss NaHCO₃ (3x20 mL), brine (2x30 mL), dried over anh Na₂SO₄, filtered and evaporated to give a 350 mg colourless oil as crude product. Crude used in the next step without additional purification.

Method 3: Rt= 0.97 min, MS (ESI) m/z =245.08 [M+H]⁺.

¹H NMR (400 MHz, DMSO-*d*₆) δ ppm 8.35 (dd, J = 5.0, 0.7 Hz, 1H), 7.39 (d, J = 0.7 Hz, 1H), 7.33 - 7.27 (m, 1H), 3.69 (s, 2H), 3.01 (d, J = 3.0 Hz, 1H), 2.98 (d, J = 3.0 Hz, 1H), 2.87 - 2.76 (m, 2H), 2.35 (dt, J = 13.6, 2.3 Hz, 2H).

### Description 100: 4-chloro-6-(3-chlorophenoxy)pyrimidine (D100)

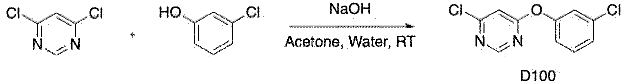

Sodium hydroxide (268.49 mg, 6.71 mmol) was added to a solution of 4,6-dichloropyrimidine (1 g, 6.71 mmol) and 3-chlorophenol (0.71 mL, 6.71 mmol) in Acetone (10 mL) and Water (10 mL) at 25 °C. After 6 h 0.5 M sodium hydroxide solution was added and the mixture was extracted with DCM. Organic phase was concentrated under vacuum. The crude was purified by RP Flash Chromatography (C-18 column 30 g, 10-100% CH₃CN (+0.1% HCOOH) in H₂O (+0.1% HCOOH)) to afford **D100** (786 mg, 3.26 mmol, yield= 48.57% yield) as colourless oil. UPLC and NMR were consistent with chemical structure.

Method 3: R*t*= 2.02 min, MS (ESI) m/z = 241.0 [M+H]⁺.

¹H NMR (400 MHz, DMSO-*d*₆) δ ppm 8.66-8.70 (m, 1H), 7.49-7.55 (m, 1H), 7.47 (d, J=0.66 Hz, 1H), 7.46 (t, J=2.09 Hz, 1H), 7.37-7.43 (m, 1H), 7.26 (ddd, J=0.99, 2.31, 8.14 Hz, 1H)

### Description 104: N-(2-iodopyridin-4-yl)pyrrolidine-1-carboxamide (D104)

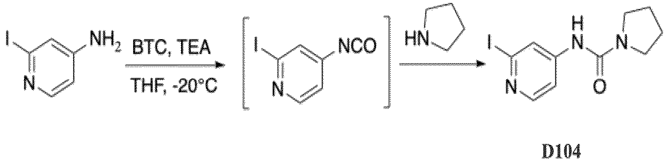

One equivalent of 2-iodopyridin-4-amine (500 mg, 2.2726 mmol, 1.0 eq) was dissolved in anh THF (23 mL, 0.1 M) and mixed with anh TEA (1.3 mL, 9.09 mmol, 2.0 eq). This mixture was slowly added dropwise to a solution of Triphosgene (BTC) (674 mg, 2.273 mmol, 0.5 eq) in anh THF (2.0 mL, 0.008 M) at -20 °C. The temperature of the reaction mixture was then raised to RT and stirred for 2 hours. The crude isocyanate solution was directly treated with Pyrrolidine (0.95 mL, 11.363 mmol, 5.0 eq) and stirred at RT ON. Reaction mixture was diluted with water and extracted with EtOAc. Then the extract was washed with 10% solution of citric acid (x3), ss NaHCO₃ (x3), brine, dried under anh Na₂SO₄, then filtered and concentrated to give crude product. The crude was purified by NP Flash Chromatography (silica gel column, 0-20% MeOH in DCM) to afford **D104** (422 mg, yield= 56%), light yellow solid.

Method 3: R*t*= 1.53 min, MS (ESI) m/z = 318.0 [M+H]⁺.

¹H NMR (400 MHz, DMSO-*d*₆) δ ppm 8.67 (s, 1H), 8.08 (d, J = 1.9 Hz, 1H), 8.06 (d, J = 5.7 Hz, 1H), 7.57 (dd, J = 5.7, 2.0 Hz, 1H), 3.45 - 3.35 (m, 4H), 1.92 - 1.78 (m, 4H).

**Table 1: summary of the described synthesis.**

| Descri ption | lupac Name | Structure | Repre sentati ve Proce dure | Starting Material | Ms (Esi) M/Z | Nmr |
|---|---|---|---|---|---|---|
| D56a | (E)-1-fluoro-3-methoxy-2-(2-nitrovinyl)benzene | 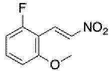 | D13 | 2-fluoro-6-methoxybenzaldeh yde (CAS n° 146137-74-8) | - | ¹H NMR (400 MHz, DMSO-*d*₆) δ ppm 8.15 (d, J = 13.7 Hz, 1H), 8.02 (d, J = 13.7 Hz, 1H), 7.60 (td, J = 8.5, 6.9 Hz, 1H), 7.17 - 6.91 (m, 2H), 3.99 (s, 3H). |
| D56b | 2-(2-fluoro-6-methoxyphenyl)etha n-1-amine | 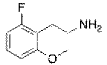 | D14 | D56a | - | ¹H NMR (400 MHz, DMSO-*d*₆) δ ppm 7.25 - 7.15 (m, 1H), 6.82 (d, J = 8.3 Hz, 1H), 6.75 (t, J = 8.9 Hz, 1H), 3.80 (d, J = 1.7 Hz, 3H), 2.90 - 2.68 (m, 2H), 2.63 (d, J = 2.8 Hz, 2H). |
| D56c | 2-((2-fluoro-6-methoxyphenethyl)a mino)isonicotinonitril e | 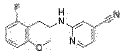 | D1 | D56b | - | - |
| D56 | 2-((2-fluoro-6-methoxyphenethyl)a mino)isonicotinic acid | 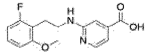 | D2 (Proce dure 2) | D56c | - | - |
| D57a | (*E*)-1-chloro-2-fluoro-4-methoxy-3-(2-nitrovinyl)benzene | 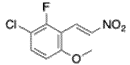 | D49 | 3-chloro-2-fluoro-6-methoxybenzaldeh yde (CAS n° 398456-81-0) | Method 1:R*t*= 2.16 min, MS (ESI) m/z = 232.0 [M+H]⁺ | - |
| D57b | 2-(3-chloro-2-fluoro-6-methoxyphenyl)etha n-1-amine | 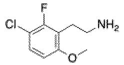 | D14 | D57a | Method 1:R*t*= 0.46 min, MS (ESI) m/z = 204.3 [M+H]⁺ | ¹H NMR (400 MHz, CDCl₃) δ ppm 7.17 (t, J = 8.7 Hz, 1H), 6.59 (dd, J = 8.9, 1.6 Hz, 1H), 3.80 (s, 3H), 3.11 - 2.49 (m, 6H). |
| D57c | 2-((3-chloro-2-fluoro-6-methoxyphenethyl)a mino)isonicotinonitril e | 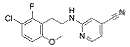 | D1 | D57b | Method 1:R*t*=1.14 min, MS (ESI) m/z = 306 [M+H]⁺ | - |
| D57 | 2-((3-chloro-2-fluoro-6-methoxyphenethyl)a mino)isonicotinic acid | 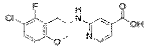 | D2 (proce dure 1) | D57c | - | - |
| D58a | (*E*)-1-methoxy-2-(2-nitrovinyl)-4-(trifluoromethoxy)be nzene | 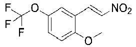 | D13 | 2-methoxy-5-(trifluoromethoxy)b enzaldehyde (CAS n°145742-65-0) | Method 1:R*t*= 2.21 min, MS (ESI) m/z = 264.0 [M+H]⁺ | ¹H NMR (400 MHz, CDCl₃) δ ppm 8.10 (d, J = 13.6 Hz, 1H), 7.87 (d, J = 13.6 Hz, 1H), 7.34 (ddd, J = 6.7, 2.5, 1.0 Hz, 2H), 7.04 - 6.98 (m, 1H), 4.00 (s, 3H). |
| D58b | 2-(2-methoxy-5-(trifluoromethoxy) ph enyl)ethan-1-amine | 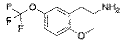 | D14 | D58a | Method 1:R*t*= 0.84 min, MS (ESI) m/z = 236.1 [M+H]⁺ | ¹H NMR (400 MHz, DMSO-*d*₆) δ ppm 7.22 - 7.12 (m, 2H), 7.03 (d, J = 8.8 Hz, 1H), 3.81 (s, 3H), 2.79 - 2.71 (m, 2H), 2.67 (ddd, J = 8.2, 6.6, 1.8 Hz, 2H). |
| D58c | 2-((2-methoxy-5-(trifluoromethoxy)ph enethyl)amino)isonic otinonitrile | 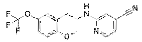 | D1 | D58b | Method 1:R*t*=1.17 min, MS (ESI) m/z = 338 [M+H]⁺ | ¹H NMR (400 MHz, DMSO-*d*₆) - 8.09 (dd, J = 5.3, 1.1 Hz, 1H), 7.19 (ddd, J = 8.9, 3.1, 1.1 Hz, 1H), 7.14 (d, J = 3.0 Hz, 1H), 7.04 (d, J = 8.9 Hz, 1H), 6.99 - 6.93 (m, 1H), 6.93 - 6.83 (m, 2H), 3.83 (s,3H), 3.51 - 3.41 (m, 2H), 2.84 (t, J = 7.2 Hz, 2H). |
| D58 | 2-((2-methoxy-5-(trifluoromethoxy)ph enethyl)amino)isonic otinic acid | 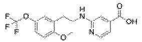 | D2 (proce dure 1) | D58c | - | - |
| D59a | 1,2-difluoro-4-methoxy-5-(2-nitroethyl)benzene | 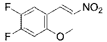 | D13 | 4,5-difluoro-2-methoxybenzaldeh yde (CAS n° 145742-34-3) | Method 1:R*t*=1.95 min, MS (ESI) m/z = 215.9 [M+H]⁺ | ¹H NMR (400 MHz, CDCl₃) δ ppm 8.06 (d, J = 13.6 Hz, 1H), 7.80 (dd, J = 13.7, 0.7 Hz, 1H), 7.31 (dd, J = 10.4, 8.8 Hz, 1H), 6.83 (dd, J = 11.7, 6.4 Hz, 1H), 3.96 (s, 3H). |
| | (*E*)-1,2-difluoro-4-methoxy-5-(2-nitrovinyl)benzene | | | | | |
| D59b | 2-(4,5-difluoro-2-methoxyphenyl)etha n-1-amine | 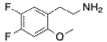 | D14 | D59a | Method 1:R*t*= 0.53 min, MS (ESI) m/z =188 [M+H]⁺ | ¹H NMR (400 MHz, DMSO-*d*₆) δ ppm 6.26 (dd, J = 11.1, 9.1 Hz, 1H), 6.09 (dd, J = 12.6, 6.8 Hz, 1H), 4.02 (s, 3H), 2.02 (ddd, J = 7.6, 6.5, 1.2 Hz, 2H), 1.97 - 1.87 (m, 2H). |
| D59c | 2-((4,5-difluoro-2-methoxyphenethyl)a mino)isonicotinonitril e | 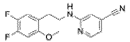 | D1 | D59b | Method 1:R*t*= 1.06. min, MS (ESI) m/z = 290.1 [M+H]⁺ | ¹H NMR (400 MHz, DMSO-*d*₆) δ ppm 13.24 (s, 1H), 8.10 (d, J = 5.2 Hz, 1H), 7.39 (t, J = 8.8 Hz, 1H), 6.98 (s, 1H), 6.88 (d, J = 7.2 Hz, 2H), 3.81 (s, 3H), 3.42 (q, J = 6.7 Hz, 2H), 2.88 (t, J = 7.1 Hz, 2H). |
| D59 | 2-((4,5-difluoro-2-methoxyphenethyl)a mino)isonicotinic acid | 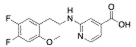 | D2 (proce dure 1) | D59c | - | - |
| D60 | (2-chloropyridin-4-yl)(6,6-difluoro-3-azabicyclo[3.1.0]hex an-3-yl)methanone | 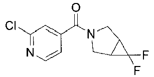 | D3 | 2-chloropyridine-4-carbonyl chloride (CAS N° 65287-34-5) and 6,6-difluoro-3-azabicyclo[3.1.0]he xane HCl (CAS n° 1215071-13-8) | Method 1:R*t*= 1.2 min, MS (ESI) m/z = 259 [M+H]⁺ | ¹H NMR (400 MHz, CDCl₃) δ ppm 8.48 (dd, J = 5.0, 0.8 Hz, 1H), 7.34 (dd, J = 1.3, 0.8 Hz, 1H), 7.23 (dd, J = 5.0, 1.4 Hz, 1H), 4.27 (d, J = 12.9 Hz, 1H), 3.78 (dddd, J = 24.6, 11.2, 5.4, 3.7 Hz, 2H), 3.67 (d, J = 11.1 Hz, 1H), 2.54 - 2.22 (m, 2H). |
| D61 | (S)-(2-iodopyridin-4-yl)(2-methylpyrrolidin-1-yl)methanone | 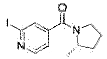 | D9 | (S)-2-Methylpyrrolidine hydrochloride (CAS n° 174500-74-4) and 2-lodopyridine-4-carboxylic acid (CAS n° 58481-10-0) | Method 3= R*t*=2.49min, MS (ESI) m/z = 316.7 [M+H]⁺ | - |
| D62a | (*E*)-3-methoxy-2-(2-nitrovinyl)thiophene | 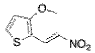 | D49 | 3-methoxythiophene-2-carbaldehyde (CAS n° 35134-07-7) | Method 1:R*t*=1.65 min, MS (ESI) m/z = 186.0 [M+H]⁺ | ¹H NMR (400 MHz, DMSO-*d*₆) δ ppm 8.14 (dd, J = 13.2, 0.5 Hz, 1H), 8.00 (d, J = 5.5 Hz, 1H), 7.79 (d, J = 13.2 Hz, 1H), 7.20 (d, J = 5.5 Hz, 1H), 4.01 (s, 3H). |
| D62 | 2-(3-methoxythiophen-2-yl)ethan-1-amine | 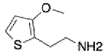 | D14 | D62a | Method 1: R*t*= 0.31 min, MS (ESI) m/z = 157.9 [M+H]⁺ | ¹H NMR (400 MHz, CDCl₃) δ ppm 7.05 (d, J = 5.5 Hz, 1H), 6.86 (d, J = 5.5 Hz, 1H), 3.84 (s, 3H), 3.02 - 2.91 (m, 2H), 2.85 (td, J = 6.5, 1.1 Hz, 2H), 1.72 (s, 2H). |
| D63a | (*E*)-4-fluoro-2-methoxy-1-(2-nitrovinyl)benzene | 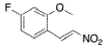 | D49 | 4-fluoro-2-methoxybenzaldeh yde CAS n° 450-83-9) | Method 1: R*t*=1.88 min, MS (ESI) m/z = 198.0 [M+H]⁺ | ¹H NMR (400 MHz, DMSO-*d*₆) δ ppm 8.20 - 8.05 (m, 2H), 7.90 (dd, J = 8.7, 6.9 Hz, 1H), 7.12 (dd, J = 11.4, 2.5 Hz, 1H), 6.93 (td, J = 8.5, 2.5 Hz, 1H), 3.96 (s, 3H). |
| D63 | 2-(4-fluoro-2-methoxyphenyl)etha n-1-amine | 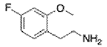 | D14 | 4-fluoro-2-methoxybenzaldeh yde CAS n° 450-83-9) | Method 1: R*t*=0.44 min, MS (ESI) m/z = 170.1 [M+H]⁺ | ¹H NMR (400 MHz, CDCl₃) δ ppm 7.10 (dd, J = 9.0, 6.7 Hz, 1H), 6.68 - 6.53 (m, 2H), 3.82 (s, 3H), 2.97 (t, J = 7.0 Hz, 2H), 2.80 (t, J = 6.8 Hz, 2H), 2.50 - 1.87 (m, 2H). |
| D64a | (*E*)-1-fluoro-3,4-dimethoxy-2-(2-nitrovinyl)benzene | 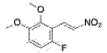 | D49 | 6-fluoro-2,3-dimethoxybenzalde hyde (CAS n° 457628-14-7) | Method 1: R*t*=1.94 min, MS (ESI) m/z = 228.08 [M+H]⁺ | ¹H NMR (400 MHz, DMSO-*d*₆) δ ppm 8.07 (d, J = 13.9 Hz, 1H), 7.93 (dd, J = 13.9, 0.7 Hz, 1H), 7.30 (dd, J = 9.2, 5.5 Hz, 1H), 7.12 (dd, J = 10.4, 9.2 Hz, 1H), 3.90 (s, 3H), 3.85 (s, 3H). |
| D64b | 2-(6-fluoro-2,3-dimethoxyphenyl)eth an-1-amine | 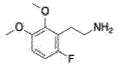 | D14 | D64a | Method 1: R*t*= 0.49 min, MS (ESI) m/z = 200.01 [M+H]⁺ | ¹H NMR (400 MHz, DMSO-*d*₆) δ ppm 6.90 (d, J = 61.5 Hz, 2H), 3.84 - 3.47 (m, 10H). |
| D64c | 2-((6-fluoro-2,3-dimethoxyphenethyl)amino)isonicotinonitri le | 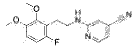 | D1 | D64b | Method 1: R*t*=1.78min, MS (ESI) m/z = 302.1 [M+H]⁺ | ¹H NMR (400 MHz, DMSO-*d*₆) δ ppm 8.17 (dd, J = 5.0, 1.0 Hz, 1H), 7.24 (t, J = 6.0 Hz, 1H), 7.01 - 6.84 (m, 2H), 6.78 (dd, J = 6.5, 1.3 Hz, 1H), 3.77 (s, 3H), 3.80 (s, 3H), 3.49 - 3.40 (m, 2H), 2.84 (ddd, J = 8.8, 6.0, 1.5 Hz, 2H). |
| D64 | 2-((6-fluoro-2,3-dimethoxyphenethyl) amino)isonicotinic acid | 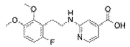 | D2 (proce dure 1) | D64c | Method 1: R*t*= 0.58 min, MS (ESI) m/z = 321.09 [M+H]⁺ | ¹H NMR (400 MHz, DMSO-*d*₆) δ ppm 8.09 (d, J = 5.6 Hz, 1H), 6.98 - 6.84 (m, 5H), 3.78 (s, 3H), 3.80 (s, 3H), 3.38 (d, J = 7.5 Hz, 2H), 2.88 (d, J = 7.5 Hz, 2H). |
| D65a | (*E*)-4-methoxy-1-(2-nitrovinyl)-2-(trifluoromethoxy)be nzene | 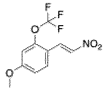 | D49 | 4-methoxy-2-(trifluoromethoxy)b enzaldehyde (CSA n°886503-52-2) | Method 1: R*t*=2.17 min, MS (ESI) m/z = 264.04 [M+H]⁺ | ¹H NMR (400 MHz, DMSO-*d*₆) δ ppm 8.20 (d, J = 13.6 Hz, 1H), 8.09 (d, J = 8.9 Hz, 1H), 8.04 (d, J = 13.6 Hz, 1H), 7.14 (dd, J = 8.9, 2.5 Hz, 1H), 7.08 (dt, J = 2.6, 1.4 Hz, 1H), 3.89 (s, 3H). |
| D65b | 2-(4-methoxy-2-(trifluoromethoxy)ph enyl)ethan-1-amine | 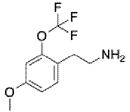 | D14 | D65a | Method 1: R*t*=0.75 min, MS (ESI) m/z = 236.1 [M+H]⁺ | ¹H NMR (400 MHz, DMSO-*d*₆) δ ppm 7.32 (s, 1H), 6.95 (dd, J = 8.5, 2.6 Hz, 1H), 6.86 (dq, J = 3.0, 1.6 Hz, 1H), 3.76 (s, 3H), 2.83 - 2.74 (m, 2H), 2.77 - 2.64 (m, 2H). |
| D65c | 2-((4-methoxy-2-(trifluoromethoxy)ph enethyl)amino)isonic otinonitrile | 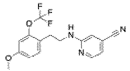 | D1 | D65b | Method 1: R*t*=2.12 min, MS (ESI) m/z = 338.1 [M+H]⁺ | ¹H NMR (400 MHz, DMSO-*d*₆) δ ppm 8.20 - 8.14 (m, 1H), 7.34 (d, J = 8.6 Hz, 1H), 6.95 (dd, J = 8.6, 2.6 Hz, 1H), 6.86 (dq, J = 3.0, 1.6 Hz, 1H), 6.81 - 6.75 (m, 2H), 3.77 (s, 3H), 3.45 (dt, J = 7.6, 6.0 Hz, 2H), 2.82 (dd, J = 8.1, 6.5 Hz, 2H). |
| D65 | 2-((4-methoxy-2-(trifluoromethoxy)ph enethyl)amino)isonic otinic acid | 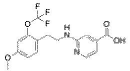 | D2 (Proce dure 1) | D65c | Method 1: R*t*= 1.19 min, MS (ESI) m/z = 357.01 [M+H]⁺ | ¹H NMR (400 MHz, DMSO-*d*₆) δ ppm 8.05 (d, J = 6.1 Hz, 1H), 7.44 (d, J = 8.6 Hz, 1H), 7.29 (s, 1H), 7.05 - 6.94 (m, 2H), 6.87 (dq, J = 3.1, 1.6 Hz, 1H), 3.78 (s, 3H), 2.60 (t, J = 7.3 Hz, 2H), 2.90 (t, J = 7.3 Hz, 2H). |
| D66a | 2-((2-(benzo[*d*][1,3]dioxol-4-yl)ethyl)amino)isonic otinonitrile | 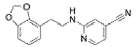 | D1 | 2-(1,3-dioxaindan-4-yl)ethan-1-amine (CAS n°33542-90-4) | Method 1: R*t*= 1.58 min, MS (ESI) m/z =268.1 [M+H]⁺ | - |
| D66 | 2-((2-(benzo[*d*][1,3]dioxol-4-yl)ethyl)amino)isonic otinic acid | 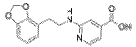 | D2 (proce dure 1) | D66a | Method 1: R*t*=0.45 min, MS (ESI) m/z =286.09 [M+H]⁺ | - |
| D67a | 2-((2,3-dimethoxyphenethyl) amino)isonicotinonitri le | 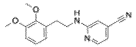 | D1 | 2-(2,3-dimethoxyphenyl)e than-1-amine (CAS n° 3213-29-4) | - | - |
| D67 | 2-((2,3-dimethoxyphenethyl) amino)isonicotinic acid | 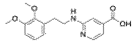 | D2 (proce dure 1) | D67a | - | - |
| D68a | (*E*)-4-chloro-1-methoxy-2-(2-nitrovinyl)benzene | 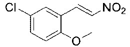 | D49 | 5-chloro-2-methoxybenzaldeh yde (CAS n° 7035-09-8) | Method 1: R*t*= 2.08 min, MS (ESI) m/z = 214.03 [M+H]⁺ | - |
| D68b | 2-(5-chloro-2-methoxyphenyl)etha n-1-amine | 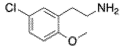 | D14 | D68a | - | - |
| D68c | 2-((5-chloro-2-methoxyphenethyl)a mino)isonicotinonitril e | 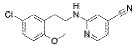 | D1 | D68b | - | - |
| D68 | 2-((5-chloro-2-methoxyphenethyl)a mino)isonicotinic acid | 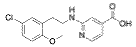 | D2 (proce dure 1) | D68c | - | - |
| D69a | (*E*)-1-chloro-3-methoxy-2-(2-nitrovinyl)benzene | 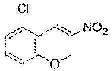 | D49 | 2-chloro-6-methoxybenzaldeh yde (CAS n° 29866-54-4) | Method 1: R*t*= 2.10 min, MS (ESI) m/z = 214.03 [M+H]⁺ | ¹H NMR (400 MHz, DMSO-*d*₆) δ ppm 8.34 (d, J = 13.5 Hz, 1H), 8.14 (d, J = 13.5 Hz, 1H), 7.55 (t, J = 8.3 Hz, 1H), 7.34 - 7.13 (m, 2H), 3.99 (s, 3H). |
| D69b | 2-(2-chloro-6-methoxyphenyl)etha n-1-amine | 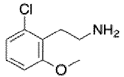 | D14 | D69a | - | - |
| D69c | 2-((2-chloro-6-methoxyphenethyl)a mino)isonicotinonitril e | 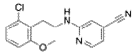 | D1 | D69b | - | - |
| D69 | 2-((2-chloro-6-methoxyphenethyl)amino)isonicotinic acid | 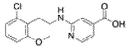 | D2 (procedure 1) | D69c | - | - |
| D71a | *rac*-2-{[(1R,2S)-2-(2-methoxyphenyl)cyclo propyl]amino}pyridin e-4-carbonitrile | 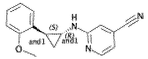 | D11 (in DMF at 80°C 4h) | *rac*-(1R,2S)-2-(2-methoxyphenyl)cyc lopropan-1-amine hydrochloride (CAS n° 131900-21-5) | Method 1:R*t*=1.77 min, MS (ESI) m/z = 266.1 [M+H]⁺ | - |
| D71 | *rac-*2-{[(1R,2S)-2-(2-methoxyphenyl)cyclo propyl]amino}pyridin e-4-carboxylic acid | 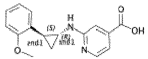 | D2 (proce dure 1) | D1 | Method 1:R*t=* 0.96 min, MS (ESI) m/z = 285.2 [M+H]⁺ | - |
| D72a | 2-((5-fluoro-2-methoxyphenethyl)a mino)isonicotinonitril e | 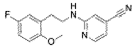 | D1 | 2-(5-fluoro-2-methoxyphenyl)eth an-1-amine (CAS n° 1000533-03-8) | Method 1:R*t*= 1.76 min, MS (ESI) m/z = 272.1 [M+H]⁺ | ¹H NMR (400 MHz, DMSO-*d*₆) δ ppm 8.16 (dd, J = 5.1, 0.9 Hz, 1H), 7.15 (t, J = 5.8 Hz, 1H), 7.02 - 6.94 (m, 3H), 6.80 (t, J = 1.2 Hz, 1H), 6.77 (dd, J = 5.2, 1.4 Hz, 1H), 3.76 (s, 3H), 3.44 (dt, J = 7.7, 6.0 Hz, 2H), 2.79 (d, J = 7.2 Hz, 2H). |
| D72 | 2-[2-(5-fluoro-2-methoxyphenyl)ethylamino]p yridine-4-carboxylic acid | 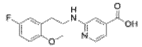 | D2 ( Proce dure 1) | D72a | Method 1:R*t*= 0.91 min, MS (ESI) m/z = 291.02 [M+H]⁺ | - |
| D73a | (*E*)-1,4-difluoro-2-methoxy-3-(2-nitrovinyl)benzene | 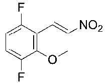 | D49 | 3,6-difluoro-2-methoxybenzaldeh yde(CAS n° 887267-04-1) | Method 1:R*t*= 2.01 min, MS (m/z) = 215.90 [M+H]⁺ | ¹H NMR (400 MHz, DMSO-*d*₆) δ ppm 8.03 (d, J = 13.8 Hz, 1H), 7.96 (dd, J = 13.8, 0.6 Hz, 1H), 7.57 (ddd, J = 11.6, 9.3, 5.4 Hz, 1H), 7.17 (ddd, J = 10.1, 9.3, 3.7 Hz, 1H), 4.05 (d, J = 3.0 Hz, 3H). |
| D73b | 2-(3,6-difluoro-2-methoxyphenyl)etha n-1-amine | 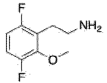 | D14 | D73a | Method 1:R*t*= 0.40 min, MS (m/z) = 188 [M+H]⁺ | ¹H NMR (400 MHz, CDCl₃) δ ppm 6.95 (dddd, J = 11.0, 9.4, 5.3, 4.2 Hz, 1H), 6.81 - 6.69 (m, 1H), 3.99 (d, J = 2.3 Hz, 3H), 3.08 - 2.99 (m, 2H), 2.94 (dd, J = 7.8, 6.2 Hz, 2H), 2.2 - 3.2 (br s, 2H, NH₂) |
| D73c | 2-((3,6-difluoro-2-methoxyphenethyl)a mino)isonicotinonitril e | 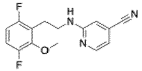 | D1 | D73b | - | ¹H NMR (400 MHz, DMSO-*d*₆) δ ppm 8.17 (dd, J = 5.0, 1.0 Hz, 1H), 7.25 (t, J = 6.0 Hz, 1H), 7.18 (ddd, J = 11.3, 9.1, 5.3 Hz, 1H), 6.94 (td, J = 9.1, 3.9 Hz, 1H), 6.82 - 6.69 (m, 2H), 3.87 (d, J = 1.9 Hz, 3H), 3.46 (dt, J = 7.6, 6.3 Hz, 2H), 2.87 (ddd, J = 8.3, 6.4, 1.6 Hz, 2H). |
| D73 | 2-[2-(3,6-difluoro-2-methoxyphenyl)ethylamino]p yridine-4-carboxylic acid | 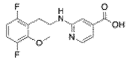 | D2 (proce dure 1) | D73c | Method 1:R*t*= 0.94 min, MS (m/z) = 309.07 [M+H]⁺ | ¹H NMR (400 MHz, DMSO-*d*₆) δ ppm 13.27 (s, 1H), 8.10 (dd, J = 5.3, 0.8 Hz, 1H), 7.18 (ddd, J = 11.3, 9.1, 5.3 Hz, 1H), 7.03 - 6.84 (m, 3H), 3.88 (d, J = 1.8 Hz, 3H), 3.46 (q, J = 6.7 Hz, 2H), 2.91 - 2.82 (m, 3H). |
| D74a | 2-methoxy-4-(methoxymethoxy)benzaldehyde | 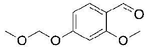 | D48 | 4-hydroxy-2-methoxybenzaldehyde (CAS n° 18278-34-7) | Method 1:R*t*= 1.40. min, MS (m/z) = 196.9 [M+H]⁺ | ¹H NMR (400 MHz, DMSO-*d*₆) δ ppm 10.20 (s,1H), 7.67 (d, J = 8.6 Hz, 1H), 6.78 (d, J = 2.2 Hz, 1H), 6.73 (dd, J = 8.6, 2.2 Hz, 1H), 5.32 (s, 2H), 3.90 (s, 3H), 3.41 (s, 3H). |
| D74b | (*E*)-2-methoxy-4-(methoxymethoxy)-1-(2-nitrovinyl)benzene | 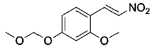 | D49 | D74a | - | ¹H NMR (400 MHz, DMSO-*d*₆) δ ppm 8.16 (d, J = 13.5 Hz, 1H), 8.04 (d, J = 13.5 Hz, 1H), 7.76 (d, J = 8.7 Hz, 1H), 6.78 (d, J = 2.3 Hz, 1H), 6.73 (dd, J = 8.6, 2.3 Hz, 1H), 5.31 (s, 2H), 3.93 (s, 3H), 3.41 (s,3H). |
| D74c | 2-(2-methoxy-4-(methoxymethoxy)ph enyl)ethan-1-amine | 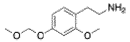 | D14 | D74b | - | ¹H NMR (400 MHz, DMSO-*d*₆) δ ppm 7.00 (d, J = 8.2 Hz, 1H), 6.59 (d, J = 2.4 Hz, 1H), 6.52 (dd, J = 8.2, 2.4 Hz, 1H), 5.15 (s, 2H), 3.74 (d, J = 1.8 Hz, 3H), 3.37 (s, 3H), 2.65 (ddd, J = 7.9, 6.5, 1.1 Hz, 2H), 2.57 - 2.50 (m, 2H). |
| D74d | 2-((2-methoxy-4-(methoxymethoxy)ph enethyl)amino)isonic otinonitrile | 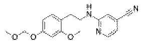 | D1 | D74c | - | ¹H NMR (400 MHz, DMSO-*d*₆) δ ppm 8.16 (dd, J = 5.1, 0.9 Hz, 1H), 7.11 (t, J = 5.7 Hz, 1H), 7.04 (d, J = 8.2 Hz, 1H), 6.84 - 6.72 (m, 2H), 6.63 (d, J = 2.4 Hz, 1H), 6.54 (dd, J = 8.2, 2.4 Hz, 1H), 5.16 (s, 2H), 3.77 (s, 3H), 3.43 - 3.33 (m, 5H), 2.73 (dd, J = 8.2, 6.4 Hz, 2H). |
| D74 | 2-[2-[2-methoxy-4-(methoxymethoxy)ph enyl]ethylamino]pyrid ine-4-carboxylic acid | 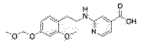 | D2 | D74d | Method1:R*t*=0.9 1 min, MS (m/z) = 333.08 [M+H]⁺ | ¹H NMR (400 MHz, DMSO-*d*₆) δ ppm 13.23 (s, 1H), 8.09 (d, J = 5.3 Hz, 1H), 7.04 (d, J = 8.3 Hz, 1H), 6.99 (s, 1H), 6.86 (dd, J = 5.2, 1.4 Hz, 1H), 6.63 (d, J = 2.3 Hz, 1H), 6.54 (dd, J = 8.2, 2.3 Hz, 1H), 5.16 (s, 2H), 3.78 (s, 3H), 3.38 (s, 5H), 2.74 (t, J = 7.4 Hz, 2H). |
| D75a | (*E*)-1,4-dimethoxy-2-(2-nitrovinyl)benzene | 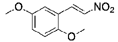 | D49 | 2,5-Dimethoxybenzald ehyde (CAS n°93-02-7) | Method1: R*t*= 1.94 min, MS (ESI) m/z = 209.94 [M+H]⁺ | ¹H NMR (400 MHz, DMSO-*d*₆) δ ppm 8.19 (s, 2H), 7.42 (dd, J = 2.7, 0.7 Hz, 1H), 7.17 - 7.05 (m, 2H), 3.87 (s, 3H), 3.76 (s, 3H). 1H NMR (400 MHz, CDCl3) - 8.14 (d, J = 13.6 Hz, 1H), 7.87 (d, J = 13.6 Hz, 1H), 7.04 (dd, J = 9.0, 3.1 Hz, 1H), 6.99 (d, J = 3.0 Hz, 1H), 6.93 (d, J = 9.0 Hz, 1H), 3.93 (s, 3H), 3.82 (s, 3H). |
| D75b | 2-(2,5-dimethoxyphenyl)eth an-1-amine | 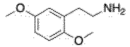 | D14 | D75a | Method1: R*t*= 0.49 min, MS (ESI) m/z = 182.03 [M+H]⁺ | ¹H NMR (400 MHz, DMSO-*d*₆) δ ppm 6.93 (d, J = 8.8 Hz, 1H), 6.82 (dd, J = 8.8, 3.1 Hz, 1H), 6.78 (d, J = 3.1 Hz, 1H), 3.76 (s, 3H), 3.71 (s, 3H), 3.18 (d, J = 4.5 Hz, 2H), 3.04 - 2.92 (m, 2H), 2.81 (dd, J = 9.1, 6.5 Hz, 2H) |
| D75c | 2-((2,5-dimethoxyphenethyl) amino)isonicotinonitri le | 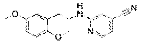 | D1 | D75b | - | - |
| D75 | 2-((2,5-dimethoxyphenethyl) amino)isonicotinic acid | 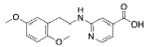 | D2 (proce dure1) | D75c | Method1: R*t*= 0.87 min, MS (m/z) 303.07 [M+H]⁺ | ¹H NMR (400 MHz, DMSO-*d*₆) δ ppm 13. 51 (s, 1H) 8.07 (d, J = 5.7 Hz, 1H), 7.16 (s, 1H), 6.93 (d, J = 5.7 Hz, 1H), 6.88 (d, J = 8.8 Hz, 1H), 6.81 (d, J = 3.0 Hz, 1H), 6.75 (dd, J = 8.8, 3.1 Hz, 1H), 4.85 - 4.81 (m, 3H), 3.74 (s, 3H), 3.68 (s, 3H) 2.75 - 2.70 (m, 2H). |
| D76a | (*E*)-1,3-dimethoxy-2-methyl-4-(2-nitrovinyl)benzene | 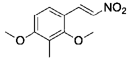 | D13 | 2,4-Dimethoxy-3-methylbenzaldehyd e (CAS n° 7149-92-0) | - | - |
| D76b | 2-(2,4-dimethoxy-3-methylphenyl)ethan-1-amine | 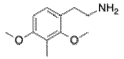 | D70b | D76a | Method1: R*t*= 0.66 min, MS (m/z) 196 [M+H]⁺ | ¹H NMR (400 MHz, DMSO-*d*₆) - 7.60 (s, 2H), 7.04 (d, J = 8.5 Hz, 1H), 6.73 (d, J = 8.4 Hz, 1H), 3.77 (s, 3H), 3.67 (s, 3H), 3.02 - 2.89 (m, 2H), 2.80 (dd, J = 9.5, 6.4 Hz, 2H), 2.07 (s, 3H). |
| D76c | 2-((2,4-dimethoxy-3-methylphenethyl)ami no)isonicotinonitrile | 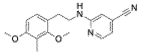 | D1 | D76b | Method1: R*t*= 1.84 min, MS (ESI) m/z = 298.19 [M+H]⁺ | - |
| D76 | 2-((2,4-dimethoxy-3-methylphenethyl)ami no)isonicotinic acid | 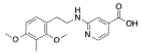 | D2 (proce dure 1) | D76c | Method1: R*t*= 1.01 min, MS (ESI) m/z = 317.1 [M+H]⁺ | - |
| D77a | 2-((2-(4-methoxy-1*H-*indol-3-yl)ethyl)amino)isonic otinonitrile | 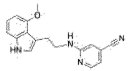 | D1 | 2-(4-methoxy-1H-indol-3-yl)ethanamine ( CAS n° 3610-35-3) | Method 1: R*t*=1.43 min, MS (ESI) m/z = 293.2 [M+H]⁺ | ¹H NMR (400 MHz, DMSO-*d*₆) δ ppm 8.17 (dd, J = 5.1, 0.8 Hz, 1H), 7.14 (t,J = 5.6 Hz, 1H), 7.04 - 6.89 (m, 3H), 6.81 (t,J = 1.2 Hz, 1H), 6.76 (dd,J = 5.1, 1.4 Hz, 1H), 6.45 (dd,J = 7.2, 1.3 Hz, 1H), 3.85 (s, 3H), 3.52 (q, J=6.8 Hz, 2H), 3.31 (s, 1H), 3.03 (t, J = 7.3 Hz, 2H). |
| D77 | 2-((2-(4-methoxy-1*H-*indol-3-yl)ethyl)amino)isonic otinic acid | 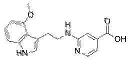 | D2 (proce dure 1) | D77a | Method 1: R*t*= 0.87 min, MS (ESI) m/z = 312.2 [M+H]⁺. | ¹H NMR (500 MHz, DMSO-*d*₆) δ ppm ppm 12.04 - 14.67 (m, 1 H), 10.79 (s, 1 H), 8.03 (d, J=5.1 Hz, 1 H), 6.99 (d, J=2.2 Hz, 1 H), 6.89 - 6.97 (m, 3 H), 6.83 (dd, J=5.2, 1.4 Hz, 1 H), 6.70 (br s, 1 H), 6.43 (dd, J=7.3, 1.0 Hz, 1 H), 3.85 (s, 3 H), 3.47 - 3.55 (m, 2 H), 3.03 (t, J=7.3 Hz, 2 H) |
| D78 | (2,3-dihydro-1*H-*pyrrolo[3,2-*b*]pyridin-1-yl)(2-fluoropyridin-4-yl)methanone | 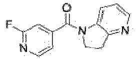 | D9 | 1H,2H,3H-pyrrolo[3,2-b]pyridine (CAS n°1211540-79-2) and 2-Fluoropyridine-4-carboxylic acid (CAS n° 402-65-3) | Method 3: R*t*= 2.49 min, MS (ESI) m/z = 243.95 [M+H]⁺. | - |
| D79 | 2-chloro-3-nitropyridine-4-carboxylic acid | 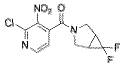 | D9 | 2-chloro-3-nitropyridine-4-carboxylic acid (CAS n° 353281-15-9) and 6,6-difluoro-3-azabicyclo[3.1.0]he xane HCl (CAS n° 1215071-13-8) | Method 3: R*t*= 1.70 min, MS (ESI) m/z = not detected [M+H]⁺. | ¹H NMR (400 MHz, DMSO-*d*₆) δ ppm 8.41 (d, J = 5.0 Hz, 1H), 7.71 (d, J = 5.0 Hz, 1H), 3.93 - 3.76 (m, 3H), 2.78 - 2.61 (m, 3H). |
| D81a | (*E*)-2-methoxy-3-(2-nitrovinyl)-1,1'-biphenyl | 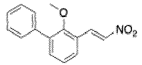 | D13 | 2-methoxy-[1,1'-biphenyl]-3-carbaldehyde **(D80)** | Method 3: R*t*= 2.75 min, MS (ESI) m/z = not detected [M+H]⁺. | ¹H NMR (400 MHz, DMSO-*d*₆) δ 8.24 (s, 2H), 7.93 (dd, J = 7.9, 1.7 Hz, 1H), 7.55 (td, J = 6.4, 3.4 Hz, 3H), 7.49 (t, J = 7.5 Hz, 2H), 7.45 - 7.38 (m, 1H), 7.33 (t, J = 7.7 Hz, 1H), 3.38 (s, 3H). |
| D81 | 2-(2-methoxy-[1,1'-biphenyl]-3-yl)ethan-1-amine | 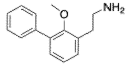 | D14 | D81a | Method 3: R*t*= 2.37 min, MS (ESI) m/z = 228 [M+H]⁺. | - |
| D82a | (*E*)-3-methoxy-4-(2-nitrovinyl)-1,1'-biphenyl | 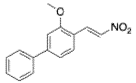 | D13 | 2-methoxy-4-phenylbenzaldehyd e (CAS n° 343603-82-7) | Method 3: R*t*= 2.75 min, MS (ESI) m/z = not detected [M+H]⁺. | ¹H NMR (400 MHz, DMSO-*d*₆) δ 8.28 - 8.13 (m, 2H), 7.90 (d, J = 8.1 Hz, 1H), 7.84 - 7.77 (m, 2H), 7.56 - 7.47 (m, 2H), 7.47 - 7.34 (m, 3H), 4.05 (s, 3H). |
| D82 | 2-(3-methoxy-[1,1'-biphenyl]-4-yl)ethan-1-amine | 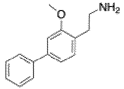 | D14 | D82a | Method 3: R*t*= 1.78 min, MS (ESI) m/z = 228.05 [M+H]⁺. | - |
| D83a | (*E*)-4-methoxy-3-(2-nitrovinyl)-1,1'-biphenyl | 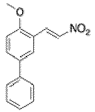 | D13 | 2-methoxy-5-phenylbenzaldehyd e (CAS n° 89536-71-0) | Method 3: R*t*= 2.74 min, MS (ESI) m/z = not detected [M+H]⁺ | - |
| D83 | 2-(4-methoxy-[1,1'-biphenyl]-3-yl)ethan-1-amine | 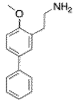 | D14 | D83a | Method 3: R*t*= 1.84 min, MS (ESI) m/z = 228.05 [M+H]⁺. | - |
| D85 | 1-(6,6-difluoro-3-azabicyclo[3.1.0]hex an-3-yl)-2-(2-fluoropyridin-4-yl)ethan-1-one | 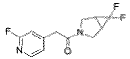 | D9 | 2-(2-Fluoropyridin-4-yl)acetic acid (CAS n° 1000518-05-7) and 6,6-difluoro-3-azabicyclo[3.1.0]he xane HCl (CAS n° 1215071-13-8) | Method 3: R*t*= 2.15 min, MS (ESI) m/z = 257.05 [M+H]⁺. | ¹H NMR (400 MHz, DMSO-*d*₆) δ ppm 8.15 (d, J = 5.1 Hz, 1H), 7.22 - 7.16 (m, 1H), 7.01 (s, 1H), 3.90 - 3.85 (m, 2H), 3.76 (s, 2H), 3.73 (d, J = 12.4 Hz, 1H), 3.64 - 3.55 (m, 1H), 2.68 - 2.52 (m, 2H). |
| D88 | (*R*)-(2-iodopyridin-4-yl)(2-methylpyrrolidin-1-yl)methanone | 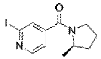 | D9 | (R)-2-Methylpyrrolidine hydrochloride (CAS n° 41720-98-3) and 2-lodopyridine-4-carboxylic acid (CAS n° 58481-10-0) | Method 3: R*t*=2.51min, MS (ESI) m/z = 316.7 [M+H]⁺ | - |
| D89 | (1,1-dioxidothiomorpholin o)(2-fluoropyridin-4-yl)methanone | 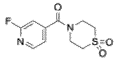 | D9 | Thiomorpholine 1,1-dioxide (CAS n° 39093-93-1) and 2-Fluoropyridine-4-carboxylic acid (CAS n° 402-65-3) | Method 3:R*t*= 1.36, MS (ESI) m/z = 258.8 [M+H]⁺ | ¹H NMR (300 MHz, DMSO-*d*₆) δ ppm 8.36 (dt, J = 5.0, 0.8 Hz, 1H), 7.47 (ddd, J = 5.1, 2.2, 1.2 Hz, 1H), 7.36 (dt, J = 2.0, 0.9 Hz, 1H), 4.02 (s, 2H), 3.62 (s, 2H), 3.23 (s, 4H). |
| D90 | ((1*S*,4*S*)-2,2-dioxido-2-thia-5-azabicyclo[2.2.1]hept an-5-yl)(2-fluoropyridin-4-yl)methanone | 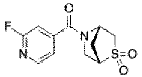 | D9 | (1S,4S)-2-Thia-5-azabicyclo[2.2.1]he ptane 2,2-dioxide hydrochloride ( CAS n° 1481613-21-1) and 2-Fluoropyridine-4-carboxylic acid (CAS n° 402-65-3) | Method 3: R*t*= 1.07, MS (ESI) m/z = 271.0 [M+H]⁺ | ¹H NMR (300 MHz, DMSO-*d*₆) δ ppm 8.36 (dt, J = 5.0, 0.8 Hz, 1H), 7.47 (ddd, J = 5.1, 2.2, 1.2 Hz, 1H),7.36 (dt, J = 2.0, 0.9 Hz, 1H), 4.02 (s, 2H), 3.62 (s, 2H), 3.23 (s, 4H). |
| D91 | (3,3-dioxido-3-thia-8-azabicyclo[3.2.1]octa n-8-yl)(2-fluoropyridin-4-yl)methanone | 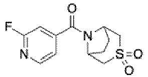 | D9 | 3λ⁶-thia-8-azabicyclo[3.2.1]oc tane 3,3-dioxide ( CAS n°1520084-41-6) and 2-Fluoropyridine-4-carboxylic acid (CAS n° 402-65-3) | Method 3: R*t*= 1.42, MS (ESI) m/z = 284.95 [M+H]⁺ | ¹H NMR (300 MHz, DMSO-*d*₆) δ ppm 8.36 (dt, J = 5.0, 0.8 Hz, 1H), 7.54 (ddd, J = 5.1, 2.2, 1.3 Hz, 1H), 7.44 (dt, J = 2.0, 0.9 Hz, 1H), 5.04 (s, 1H), 4.30 (s, 1H), 3.47 (s, 3H), 3.25 (d, J = 14.5 Hz, 1H), 2.37 - 2.00 (m, 4H). |
| D92 | (3,3-dioxido-3-thia-6-azabicyclo[3.1.1]hept an-6-yl)(2-fluoropyridin-4-yl)methanone | 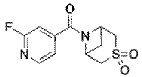 | D9 | 3λ⁶-thia-6-azabicyclo[3.1.1]he ptane 3,3-dioxide ( CAS n° 1520084-21-2) and 2-Fluoropyridine-4-carboxylic acid (CAS n° 402-65-3) | Method 3: R*t*= 1.52 min, MS (ESI) m/z = 270.8[M+H]⁺. | ¹H NMR (300 MHz, DMSO-*d*₆) δ ppm 8.41 (dt, J = 5.1, 0.8 Hz, 1H), 7.55 (ddd, J = 5.1, 2.1, 1.3 Hz, 1H), 7.46 - 7.14 (m, 1H), 4.82 (d, J = 52.6 Hz, 2H), 3.98 (s, 1H), 3.74 (dd, J = 26.9, 13.7 Hz, 2H), 3.52 (s, 1H), 1.33 - 0.97 (m, 2H). |
| D93 | ((1*R*,4*R*)-2,2-dioxido-2-thia-5-azabicyclo[2.2.1]hept an-5-yl)(2-fluoropyridin-4-yl)methanone | 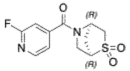 | D9 | (1R,4R)-2-Thia-5-azabicyclo[2.2.1]he ptane 2,2-dioxide hydrochloride ( CAS n° 2231666-40-1) and 2-Fluoropyridine-4-carboxylic acid (CAS n° 402-65-3) | Method 3: R*t*= 1.51min, MS (ESI) m/z = 270.8[M+H]⁺. | ¹H NMR (400 MHz, DMSO-*d*₆) δ ppm 8.39 (dd, J = 15.6, 5.1 Hz, 1H), 7.58-7.44 (m, 1H), 7.44 - 7.27 (m, 1H), 4.07 (d, J = 21.4 Hz, 1H), 3.90 - 3.74 (m, 2H), 3.69 - 3.47 (m, 1H), 2.74 (d, J = 0.7 Hz, 1H), 2.70 (s, 2H), 2.40 - 2.30 (m, 1H). |
| D95 | *trans*-6,6-difluoro-*N-*(2-fluoropyridin-4-yl)bicyclo[3.1.0]hexa ne-3-carboxamide | 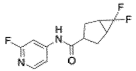 | D84 | 2-fluoropyridin-4-amine (CAS n° 18614-51-) and *trans*-6,6-difluorobicyclo[3.1. 0]hexane-3-carboxylic acid (CAS n°1447942-40-6) | Method 3= R*t*= 1.72, MS (ESI) m/z = 257.0 [M+H]⁺ | - |
| D96 | N-(2-(benzo[d][1,3]dioxol-4-yl)ethyl)-4-iodopyridin-2-amine | 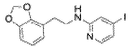 | D41 (110° C for 18h in NMP) | 2-(1,3-dioxaindan-4-yl)ethan-1-amine (CAS n°33542-90-4) and 2-Fluoro-4-iodopyridine (CAS n° 22282-70-8) | Method 3= R*t*= 1.64, MS (ESI) m/z = 369.1 [M+H]⁺ | ¹H NMR (300 MHz, DMSO-*d*₆) δ ppm 7.69 (d, J = 5.3 Hz, 1H), 6.92 - 6.86 (m, 1H), 6.85 - 6.66 (m, 5H), 5.98 (s, 2H), 3.51 - 3.37 (m, 2H), 2.77 (dd, J = 8.1, 6.6 Hz, 2H). |
| D97 | *N*-(2-(cyclopentyloxy)phen ethyl)-4-iodopyridin-2-amine | 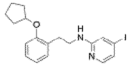 | D41 (110° C for 18h in NMP) | 2-(2-(cyclopentyloxy)ph enyl)ethan-1-amine (D54) and 2-Fluoro-4-iodopyridine (CAS n° 22282-70-8) | Method 3: R*t*= 2.00 min, MS (ESI) m/z = 409.2 [M+H]⁺. | ¹H NMR (400 MHz, DMSO-*d*₆) δ ppm 7.68 (d, J = 5.3 Hz, 1H), 7.19 - 7.09 (m, 2H), 6.96 - 6.90 (m, 1H), 6.88 - 6.77 (m, 3H), 6.71 (t, J = 5.6 Hz, 1H), 4.87 - 4.82 (m, 1H), 3.40 - 3.36 (m, 2H), 2.79 - 2.71 (m, 2H), 1.94 - 1.84 (m, 2H), 1.78 - 1.68 (m, 4H), 1.63 - 1.55 (m, 2H). |
| D98 | 4-iodo-*N*-((1r,3r)-3-(2-methoxyphenyl)cyclo butyl)pyridin-2-amine | 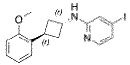 | D41 (110° C for 18h in NMP) | (1r,3r)-3-(2-methoxyphenyl)cyc lobutan-1-amine hydrochloride, trans and 2-Fluoro-4-iodopyridine (CAS n° 22282-70-8) | Method 3: R*t*= 2.88 min, MS (ESI) m/z = 380.95 [M+H]⁺. | ¹H NMR (400 MHz, DMSO-*d*₆) δ ppm 7.67 (d, J = 5.3 Hz, 1H), 7.34 (dt, J = 7.2, 1.4 Hz, 1H), 7.28 - 7.18 (m, 2H), 6.95 (t, J = 7.2 Hz, 2H), 6.88-6.79 (m, 2H), 4.24 (d, J = 6.8 Hz, 1H), 3.76 (s, 3H), 3.29 (s, 1H), 2.40 (dt, J = 12.0, 7.3 Hz, 2H), 2.32 - 2.20 (m, 2H). |
| D99 | 6-chloro-*N*-(2-methoxybenzyl)pyri midin-4-amine | 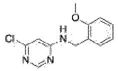 | D44 | 4,6-dichloropyrimidine (CAS no°1193-21-1 and (2-methoxyphenyl)me thanamine (CAS n° 6850-57-3) | Method 1: R*t*=0.76 min, MS (ESI) m/z = 355.3 [M+H]⁺. | ¹H NMR (500 MHz, DMSO-*d*₆) - ppm 7.89 (s, 1 H), 7.42 (d, J=6.7 Hz, 1 H), 7.23 - 7.32 (m, 3 H), 7.18 - 7.23 (m, 1 H), 7.09 - 7.17 (m, 2 H), 6.93 - 7.02 (m, 2 H), 6.86 (t, J = 7.5 Hz, 1 H), 5.25 - 5.54 (m, 1 H), 4.43 (br d, J = 4.9 Hz, 2 H), 4.32 (br s, 2 H), 3.80 (s, 3 H). |
| D101 | (6,6-difluoro-1-azaspiro[3.3]heptan-1-yl)(2-fluoropyridin-4-yl)methanone | 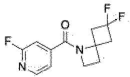 | D9 | 6,6-Difluoro-1-azaspiro[3.3]hepta ne oxalic acid (CAS n° 2173991-59-6) and 2-Fluoropyridine-4-carboxylic acid (CAS n° 402-65-3) | Method 3: R*t*= 2.08 min, MS (ESI) m/z = 256.8 [M+H]⁺. | |
| D102 | 2-(2-fluoropyridin-4-yl)-5-phenylthiazole | 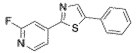 | D86 | 2-Fluoropyridine-4-boronic acid (CAS N° 401815-98-3) and 2-Bromo-5-phenylthiazole (CAS n° 133311-51-0) | | ¹H NMR (300 MHz, DMSO-*d*₆) δ 8.52 (s, 1H), 8.49 - 8.36 (m, 1H), 7.90 (dt, J = 5.3, 1.7 Hz, 1H), 7.79 (ddt, J = 8.8, 3.9, 2.1 Hz, 3H), 7.69 (d, J = 1.7 Hz, 1H), 7.45 - 7.39 (m, 1H). |
| D103 | 2-(2-fluoropyridin-4-yl)-5-phenyloxazole | 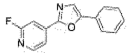 | D86 | 2-Fluoropyridine-4-boronic acid (CAS N° 401815-98-3) and 2-Bromo-5-phenyl-1,3-oxazole (CAS n° 129053-70-9) | | ¹H NMR (300 MHz, DMSO-*d*₆) δ 8.45 (dt, J = 5.2, 0.8 Hz, 1H), 8.03 (s, 1H), 7.99 - 7.88 (m, 3H), 7.81 (d, J = 1.9 Hz, 1H), 7.62 - 7.49 (m, 2H), 7.49 - 7.40 (m, 1H). |

### Example 1:

(4,4-difluoropiperidin-1-yl)(2-((2-methoxyphenethyl)amino)pyridin-4-yl)methanone; hydrochloride (E1)

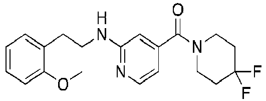

### STEP A:

To a stirred solution of **D2** (100 mg, 0.370 mmol) in DCM (2 mL) (0.17 mL, 1.18 mmol) and T3P (50% solution in EtOAc, 0.13 mL, 0.440 mmol)were added TEA. Then 4,4-difluoropiperidine (0.04 mL, 0.370 mmol) was added dropwise, and the mixture was stirred at RT for 3h. The mixture was quenched with Na₂CO₃ ss (3mL) and diluted with DCM (3mL). The mixture was separated by a phase separator and the organic layer was concentrated under reduced pressure to obtain (4,4-difluoropiperidin-1-yl)(2-((2-methoxyphenethyl)amino)pyridin-4-yl)methanone (80 mg, 0.213 mmol, yield= 58%) as a light yellow oil.

Method 1: Rt= 0.69 min, MS (ESI) m/z = 376.44 [M+H]⁺.

¹H NMR (400 MHz, DMSO-*d*₆) δ ppm ppm 8.03 (d, J=5.2 Hz, 1 H), 7.17 - 7.23 (m, 1 H), 7.15 (dd, J=7.3, 1.4 Hz, 1 H), 6.96 (d, J=8.1 Hz, 1 H), 6.87 (td, J=7.3, 0.8 Hz, 1 H), 6.75 (t, J=5.6 Hz, 1 H), 6.47 (dd, J=5.1, 1.2 Hz, 1 H), 6.43 (s, 1 H), 3.78 (s, 3 H), 3.61 - 3.75 (m, 2 H), 3.35 - 3.49 (m, 4 H), 2.81 (t, J=7.5 Hz, 2 H), 1.89 - 2.18 (m, 4 H)

### STEP B:

To a stirred solution of (4,4-difluoropiperidin-1-yl)(2-((2-methoxyphenethyl)amino)pyridin-4-yl)methanone (80 mg, 0.213 mmol) in MeOH (3.066 mL), HCl (1M in DEE, 0.23 mL, 0.280 mmol) was dropwise added. The mixture was stirred at RT for 2h. Then the mixture was concentrated under vacuum, stripped three times with water and then freeze-dried to obtain **E1** (60 mg, 0.146 mmol, yield= 66.92%) as a yellow solid.

Method 2: Rt= 1.14 min, MS (ESI) m/z = 376.4 [M+H]⁺.

¹H NMR (400 MHz, DMSO-*d*₆) δ ppm ppm 13.48 (br s, 1 H), 8.00 (br d, J=5.9 Hz, 1 H), 8.65 (br s, 1 H), 7.17 - 7.28 (m, 2 H), 6.98 (d, J=8.4 Hz, 1 H), 6.86 - 6.95 (m, 2 H), 6.74 - 6.85 (m, 1 H), 3.77 (s, 3 H), 3.64 - 3.74 (m, 2 H), 3.42 - 3.60 (m, 4 H), 2.88 (br t, J=7.1 Hz, 2 H), 1.94 - 2.19 (m, 4 H)

### Example 2: (R)-(2-((2-methoxyphenethyl)amino)pyridin-4-yl)(2-(trifluoromethyl)piperidin-1-yl)methanone; hydrochloride (E2)

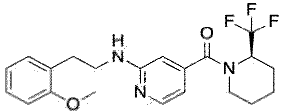

### STEP A:

To a solution of 1-methylimidazole (40.4 mL, 0.510 mmol) and D2 (60 mg, 0.220 mmol) in anh MeCN (1.84 mL) at 0°C, TCFH (CAS n°: 207915-99-9)(86.55 mg, 0.310 mmol) was added. The reaction mixture was stirred at 0°C for 10 min and (2R)-2-(trifluoromethyl)piperidine (37.12 mg, 0.240 mmol) was added. The reaction was stirred at RT for 3h. Then the reaction was concentrated, diluted with DCM (25 mL), and washed with ss NaHCO₃ (2x 20 mL) and brine. The organic layer was dried over anh Na₂SO₄ and concentrated under reduced pressure. The crude was purified by NP Flash Chromatography (silica gel column, 5-50% EtOAc in cyclohexane) to afford (R)-(2-((2-methoxyphenethyl)amino)pyridin-4-yl)(2-(trifluoromethyl)piperidin-1-yl)methanone (42 mg, 0.103 mmol, yield= 46.78%) as oil.

Method 2: Rt= 1.3 min, MS (ESI) m/z = 408.3 [M+H]⁺

### STEP B:

to a solution of (R)-(2-((2-methoxyphenethyl)amino)pyridin-4-yl)(2-(trifluoromethyl)piperidin-1-yl)methanone (41 mg, 0.100 mmol) in MeOH (0.600 mL) Hydrogen chloride (1M in DEE, 130 mL, 0.130 mmol) was added at RT. After 4h stirring, the mixture was concentrated under reduced pressure, stripped three times with water and freeze-dried to obtain **E2** (42.1 mg, 0.095 mmol, yield= 94.25%) as a white powder.

Method 2: Rt= 1.41 min, MS (ESI) m/z = 408.3 [M+H]⁺.

¹H NMR (400 MHz, DMSO-*d*₆) δ ppm 13.54 (s, 1 H), 8.60 (br s, 1 H), 8.00 (br d, J=6.0 Hz, 1 H), 7.17 - 7.30 (m, 2 H), 6.98 (d, J=8.3 Hz, 1 H), 6.89 (t, J=7.4 Hz, 1 H), 6.85 (br s, 1 H), 6.71 (br d, J=4.6 Hz, 1 H), 4.29 - 5.34 (m, 1 H), 3.77 (s, 3 H), 3.52 (br s, 2 H), 3.07 - 4.51 (m, 2 H), 2.88 (br t, J=7.3 Hz, 2 H), 1.35 - 2.08 (m, 6 H)

### Example 3: (3,3-difluoropyrrolidin-1-yl)(2-((2-methoxyphenethyl)amino)pyridin-4-yl)methanone (E3)

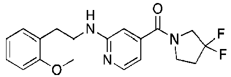

A solution of **D2** (100 mg, 0.370 mmol), HATU (167.56 mg, 0.440 mmol), 3,3-difluoropyrrolidine (47.2 mg, 0.440 mmol) and TEA (0,15mL, 1.1 mmol) in DMSO (2mL) was stirred at RT, ON. Water was added, and the mixture was extracted with DCM and concentrated under vacuum. The crude was purified by RP Flash Chromatography (Sfar C18, 12 g, eluent: CH₃CN (+0.1% HCOOH) in H₂O (+0.1% HCOOH)) to afford **E3** (84 mg, 0.232 mmol, yield= 63.29%) as a colourless oil.

Method 2: R*t*= 1.00 min, MS (ESI) m/z = 362.2 [M+H]⁺. ¹H NMR (400 MHz, DMSO-*d*₆) δ ppm 8.04 (d, J=5.2 Hz, 1 H), 7.17 - 7.23 (m, 1 H), 7.15 (dd, J=7.2, 1.1 Hz, 1 H), 6.96 (d, J=8.1 Hz, 1 H), 6.87 (t, J=7.3 Hz, 1 H), 6.77 (br t, J=5.3 Hz, 1 H), 6.44 - 6.59 (m, 2 H), 3.79 - 3.92 (m, 2 H), 3.79 (s, 3 H), 3.56 - 3.72 (m, 2 H), 3.37 - 3.46 (m, 2 H), 2.81 (t, J=7.5 Hz, 2 H), 2.37 - 2.48 (m, 2 H)

### Example 4: azepan-1-yl(2-((2-methoxyphenethyl)amino)pyridin-4-yl)methanone (E4)

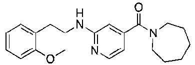

Prepared similarly as described for compound **E1** (Step A) starting from **D2** (50 mg, 0.170 mmol) and Homopiperidine (0.02 mL, 0.170 mmol) to afford **E4** (40.1 mg, 0.113 mmol, yield= 65.73%) as light yellow oil.

Method 1: R*t*= 0.69 min, MS (ESI) m/z = 354.4 [M+H]⁺.

¹H NMR (400 MHz, DMSO-*d*₆) δ ppm 8.00 (d, J=5.4 Hz, 1 H), 7.19 (td, J=7.8, 1.6 Hz, 1 H), 7.14 (dd, J=7.3, 1.4 Hz, 1 H), 6.96 (d, J=7.9 Hz, 1 H), 6.86 (td, J=7.4, 0.8 Hz, 1 H), 6.72 (t, J=5.7 Hz, 1 H), 6.32 - 6.41 (m, 2 H), 3.78 (s, 3 H), 3.47 - 3.56 (m, 2 H), 3.36 - 3.45 (m, 2 H), 3.27 (br t, J=5.8 Hz, 2 H), 2.80 (t, J=7.5 Hz, 2 H), 1.64 - 1.74 (m, 2 H), 1.44 - 1.61 (m, 6 H).

### Example 5: (6,6-dimethyl-3-azabicyclo[3.1.0]hexan-3-yl)(2-((2-methoxyphenethyl)amino)pyridin-4-yl)methanone; hydrochloride (E5)

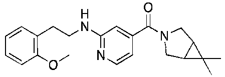

### Step A:

Prepared similarly as described for compound **E1** starting from **D2** (100 mg, 0.370 mmol) and 6,6-dimethyl-3-azabicyclo[3.1.0]hexane (0.04 mL, 0.370 mmol). The crude was purified by RP Flash Chromatography (Sfar C18, 12 g, eluent: CH₃CN (+0.1% HCOOH) in H₂O (+0.1% HCOOH)) to give (6,6-dimethyl-3-azabicyclo[3.1.0]hexan-3-yl)(2-((2-methoxyphenethyl)amino)pyridin-4-yl)methanone (46 mg, 0.126 mmol, yield= 34.27%) as light yellow oil.

### Step B:

To a stirred solution of (6,6-dimethyl-3-azabicyclo[3.1.0]hexan-3-yl)-[2-[2-(2-methoxyphenyl)ethylamino]-4-pyridyl]methanone (46 mg, 0.130 mmol) in MeOH (5.327 mL), HCl (1M in DEE, 0.25 mL, 0.250 mmol) ) was dropwise added. The mixture was stirred for 3h. Then concentrated under a vacuum, stripped three times with water and then freeze-dried to afford **E5** (50 mg, 0.124 mmol, yield= 98.84%) as a white solid.

Method 2: R*t*= 1.28 min, MS (ESI) m/z = 366.4 [M+H]⁺.

¹H NMR (400 MHz, DMSO-*d*₆) δ ppm 12.13 - 14.38 (m, 1 H), 7.98 (br d, J=6.0 Hz, 1 H), 7.43 - 9.33 (m, 1 H), 7.14 - 7.31 (m, 2 H), 6.98 (d, J=8.0 Hz, 1 H), 6.63 - 6.94 (m, 3 H), 3.76 (s, 3 H), 3.63 (dd, J=10.9, 4.8 Hz, 1 H), 3.54 - 3.58 (m, 2 H), 3.46 - 3.53 (m, 2 H), 3.15 (d, J=11.0 Hz, 1 H), 2.86 (br t, J=7.2 Hz, 2 H), 1.40 - 1.51 (m, 2 H), 1.01 (s, 3 H), 0.88 (s, 3 H)

### Example 6: (6,6-difluoro-3-azabicyclo[3.1.0]hexan-3-yl)(2-((2-methoxyphenethyl)amino)pyridin-4-yl)methanone; hydrochloride (E6)

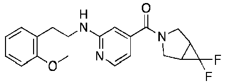

### Step A:

Prepared similarly as described for compound **E1** (Step A) starting from **D2** (100 mg, 0.370 mmol) and 6,6-difluoro-3-azabicyclo[3.1.0] hexane; hydrochloride (50 µL, 0.370 mmol). The crude was purified by RP Flash Chromatography (Sfar C18, 12 g, eluent: CH₃CN (+0.1% HCOOH) in H₂O (+0.1% HCOOH)) to give to afford (6,6-difluoro-3-azabicyclo[3.1.0]hexan-3-yl)(2-((2-methoxyphenethyl)amino)pyridin-4-yl)methanone (36 mg, 0.096 mmol, yield= 26.25%) as a light-yellow oil.

### Step B:

To a stirred solution of (6,6-difluoro-3-azabicyclo[3.1.0]hexan-3-yl)(2-((2-methoxyphenethyl)amino)pyridin-4-yl)methanone (36 mg, 0.100 mmol) in MeOH (4.169 mL), HCl (1M in DEE, 0.19 mL, 0.190 mmol) was dropwise added. The mixture was stirred for 3h. Then concentrated under a vacuum, stripped three times with water and was then freeze-dried to obtain **E6** (13 mg, 0.032 mmol, yield= 32.9%) as a yellow solid.

Method 2: Rt= 1.04 min, MS (ESI) m/z = 374.30 [M+H]⁺.

¹H NMR (400 MHz, DMSO-*d*₆) δ ppm ppm 13.45 (br s, 1 H), 8.49 (br s, 1 H), 7.99 (br d, J=6.1 Hz, 1 H), 7.17 - 7.29 (m, 2 H), 6.98 (d, J=8.2 Hz, 1 H), 6.86 - 6.94 (m, 2 H), 6.73 (br d, J=5.7 Hz, 1 H), 4.04 (d, J=12.7 Hz, 1 H), 3.78 - 3.87 (m, 1 H), 3.77 (s, 3 H), 3.70 (dt, J=12.7, 4.2 Hz, 1 H), 3.57 (br d, J=11.4 Hz, 1 H), 3.48 - 3.56 (m, 2 H), 2.83 - 2.94 (m, 2 H), 2.58 - 2.68 (m, 2 H).

### Example 7: (8-oxa-3-azabicyclo[3.2.1]octan-3-yl)(2-((2-methoxyphenethyl)amino)pyridin-4-yl)methanone; hydrochloride (E7)

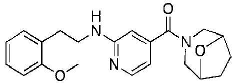

### Step A:

Prepared similarly as described for compound **E1** (Step A) starting from **D2** (50 mg, 0.180 mmol) and 8-Oxa-3-Azabicyclo[3.2.1]Octane hydrochloride (CAS° 54745-74-3, cat n° EN300-316696, Enamine). The crude was purified by NP Flash Chromatography (silica gel column, 10 g, 0-20% MeOH in DCM) to afford **E7** (10 mg, 0.027 mmol, yield= 14.81%) as a colourless oil. A second purification was performed by NP Flash Chromatography (NH₂-column, 5 g, 0-20% EtOAc in cyclohexane) to afford (8-oxa-3-azabicyclo[3.2.1]octan-3-yl)(2-((2-methoxyphenethyl)amino)pyridin-4-yl)methanon (13 mg, 0.035 mmol, yield= 19.25%).

Method 1: Rt= 0.58 min, MS (ESI) m/z = 368.33 [M+H]⁺.

### Step B:

A stirred solution of (8-oxa-3-azabicyclo[3.2.1]octan-3-yl)(2-((2-methoxyphenethyl)amino)pyridin-4-yl)methanone (12 mg, 0.030 mmol) in MeOH (1 mL) was treated with HCl (1M in DEE, 0,04mL, 0.040 mmol). The mixture was stirred for 3h, then concentrated under a vacuum, stripped three times with water and then freeze-dried to obtain **E7** (14 mg, 0.035 mmol, yield= 106.15%) as a yellow solid.

Method 2: R*t*= 0.89 min, MS (ESI) m/z = 368.4 [M+H]⁺. ¹H NMR (400 MHz, DMSO-*d*₆) δ ppm 12.49 - 14.23 (m, 1 H), 8.17 - 9.34 (m, 1 H), 7.98 (br d, J=6.1 Hz, 1 H), 7.17 - 7.31 (m, 2 H), 6.98 (d, J=8.0 Hz, 1 H), 6.85 - 6.95 (m, 2 H), 6.76 (br d, J=5.7 Hz, 1 H), 4.17 - 4.47 (m, 2 H), 3.77 (s, 3 H), 3.50 - 3.59 (m, 2 H), 3.09 - 4.14 (m, 2 H), 2.95 - 3.38 (m, 2 H), 2.88 (br t, J=7.3 Hz, 2 H), 1.52 - 1.90 (m, 4 H).

### Example 8: (1-(hydroxymethyl)-3-azabicyclo[3.1.0]hexan-3-yl)(2-((2-methoxyphenethyl)amino)pyridin-4-yl)methanone; hydrochloride (E8)

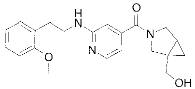

### Step A:

A suspension of **D2** (50 mg, 0.170 mmol) and TBTU (54 mg, 0.170 mmol) in DMF (1 mL) at 25 °C, was treated with DIPEA (120 µL, 0.670 mmol). After 5 minutes of stirring, a solution of 3-azabicyclo[3.1.0]hexan-1-ylmethanol;hydrochloride (25 mg, 0.170 mmol) in DMF (1 mL) was added. After 18 h, water (5 mL) was added, and the mixture was extracted with EtOAc (2 x 5 mL). The organic phase was concentrated under a vacuum. The crude was purified by RP Flash Chromatography (Sfar C18, 12 g, eluent: CH₃CN (+0.1% HCOOH) in H₂O (+0.1% HCOOH)) to give (1-(hydroxymethyl)-3-azabicyclo[3.1.0]hexan-3-yl)(2-((2-methoxyphenethyl)amino)pyridin-4-yl)methanone (28 mg, 0.076 mmol, yield= 45.6%) as an oil. Method 2: 0.81 min, MS (ESI) m/z = 368.4 [M+H]⁺. ¹H NMR (500 MHz, DMSO-*d*₆) δ ppm 7.94 - 8.05 (m, 1 H), 7.17 - 7.23 (m, 1 H), 7.15 (d, J=7.4 Hz, 1 H), 6.96 (d, J=8.1 Hz, 1 H), 6.87 (t, J=7.3 Hz, 1 H), 6.91 (br s, 1 H), 6.42 - 6.50 (m, 2 H), 4.55 - 4.82 (m, 1 H), 3.78 (s, 3 H), 3.35 - 3.53 (m, 4 H), 3.20 - 3.93 (m, 4 H), 2.76 - 2.87 (m, 2 H), 1.34 - 1.52 (m, 1 H), 0.66 - 0.80 (m, 1 H), 0.29 (t, J=4.4 Hz, 1 H)

### Step B:

To a solution of (1-(hydroxymethyl)-3-azabicyclo[3.1.0]hexan-3-yl)(2-((2-methoxyphenethyl)amino)pyridin-4-yl)methanone (18 mg, 0.050 mmol) in MeOH (2 mL), HCl (1M in DEE, 60 µL, 0.060 mmol) was added at 25 °C. After 15 min, the reaction mixture was concentrated under vacuum at 45°C to afford **E8** (19.5 mg, 0.048 mmol, yield= 98.55%) as a colorless oil. Method 2: 0.81 min, MS (ESI) m/z = 368.4 [M+H]⁺.

¹H NMR (400 MHz, DMSO-*d*₆) δ ppm 12.77 - 14.07 (m, 1 H), 8.16 - 9.13 (m, 1 H), 7.89 - 8.01 (m, 1 H), 7.19 - 7.28 (m, 2 H), 6.96 - 7.00 (m, 1 H), 6.86 - 6.92 (m, 1 H), 6.85 - 7.02 (m, 1 H), 6.79 (br d, J=3.9 Hz, 1 H), 3.77 (d, J=1.1 Hz, 3 H), 3.35 - 3.68 (m, 4 H), 3.19 - 3.92 (m, 4 H), 2.88 (br t, J=6.5 Hz, 2 H), 1.40 - 1.53 (m, 1 H), 0.71 - 0.79 (m, 1 H), 0.32 - 0.41 (m, 1 H)

### Example 9: (1-ethyl-3-azabicyclo[3.1.0]hexan-3-yl)(2-((2-methoxyphenethyl)amino)pyridin-4-yl)methanone; hydrochloride (E9)

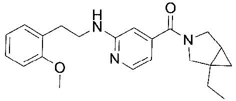

### Step A:

Prepared similarly as described for compound **E1** starting from **D2** (100 mg, 0.370 mmol) and 1-ethyl-3-azabicyclo[3.1.0]hexane;hydrochloride (54.2 mg, 0.370 mmol). The crude was purified by RP Flash Chromatography (Sfar C18, 12 g, eluent: CH₃CN (+0.1% HCOOH) in H₂O (+0.1% HCOOH)) to give (1-ethyl-3-azabicyclo[3.1.0]hexan-3-yl)(2-((2-methoxyphenethyl)amino)pyridin-4-yl)methanone (33.5 mg, 0.092 mmol, yield= 24.96%) as a colorless oil.

### Step B:

To a stirred solution of (1-ethyl-3-azabicyclo[3.1.0]hexan-3-yl)(2-((2-methoxy-phenethyl)amino)pyridin-4-yl)methanone (33.5 mg, 0.090 mmol) in MeOH (5.559 mL), HCl (1M in DEE, 0.18 mL, 0.180 mmol) was dropwise added. The mixture was stirred for 3h. Then concentrated under vacuum, strippeed three times with water and then freeze dried to give **E9** (36.7 mg, 0.091 mmol, yield= 99.61%) as a colorless oil.

Method 2: R*t*= 1.28 min, MS (ESI) m/z = 366.36 [M+H]⁺.

¹H NMR (400 MHz, DMSO-*d*₆) δ ppm 7.97 (br d, J=6.1 Hz, 1 H), 7.16 - 7.29 (m, 2 H), 6.98 (d, J=7.9 Hz, 1 H), 6.87 - 6.93 (m, 1 H), 6.58 - 6.94 (m, 2 H), 3.77 (s, 3 H), 3.12 - 3.92 (m, 6 H), 2.87 (br s, 2 H), 1.29 - 1.76 (m, 3 H), 0.76 - 0.98 (m, 3 H), 0.63 (dd, J=7.2, 5.3 Hz, 1 H), 0.29 (br d, J=1.1 Hz, 1 H).

### Example 10: (2-((2-methoxyphenethyl)amino)pyridin-4-yl)(piperidin-1-yl)methanone;hydrochloride (E10)

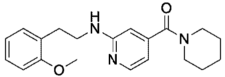

### Step A:

Prepared similarly as described for compound **E1** starting from **D2** (100 mg, 0.370 mmol) and piperidine (40 µL, 0.370 mmol). The crude was purified by RP Flash Chromatography (Sfar C18, 12 g, eluent: CH₃CN (+0.1% HCOOH) in H₂O (+0.1% HCOOH)) to give (2-((2-methoxyphenethyl)amino)pyridin-4-yl)(piperidin-1-yl)methanone (36 mg, 0.106 mmol, yield= 28.88%) as colorless oil.

### Step B:

To a stirred solution of (2-((2-methoxyphenethyl)amino)pyridin-4-yl)(piperidin-1-yl)methanone (36 mg, 0.110 mmol) in MeOH (5.559 mL), HCl (1M in DEE, 0,21mL, 0.210 mmol) was dropwise added. The mixture was stirred for 3h, then concentrated under vacuum, stripped three times with DCM and then freeze-dried to obtain E10 ( 27 mg, 0.072 mmol, yield= 67.73%) as a white solid.

Method 2: Rt= 1.07 min, MS (ESI) m/z = 340.32 [M+H]⁺.

¹H NMR (400 MHz, DMSO-*d*₆) δ ppm 12.24 - 14.73 (m, 1 H), 8.14 - 9.66 (m, 1 H), 7.97 (br d, J=6.1 Hz, 1 H), 7.15 - 7.29 (m, 2 H), 6.98 (d, J=8.4 Hz, 1 H), 6.83 - 6.94 (m, 2 H), 6.74 (br d, J=6.0 Hz, 1 H), 3.76 (s, 3 H), 3.48 - 3.64 (m, 2 H), 3.10 - 3.62 (m, 4 H), 2.88 (br t, J=7.3 Hz, 2 H), 1.37 - 1.72 (m, 6 H)

### Example 11: 2-((2-methoxyphenethyl)amino)-N,N-dimethylisonicotinamide; hydrochloride (E11)

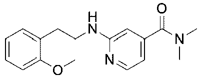

### Step A:

Prepared similarly as described for compound **E1** starting from **D2** (100 mg, 0.370 mmol) and N-methylmethanamine;hydrochloride (29.94 mg, 0.370 mmol). The crude was purified by RP Flash Chromatography (Sfar C18, 12 g, eluent: CH₃CN (+0.1% HCOOH) in H₂O (+0.1% HCOOH)) to give 2-((2-methoxyphenethyl)amino)-*N,N-*dimethylisonicotinamide (20 mg, 0.067 mmol, yield= 18.19%) as colourless oil.

### Step B:

To a stirred solution of 2-((2-methoxyphenethyl)amino)-*N,N-*dimethylisonicotinamide (20 mg, 0.070 mmol) in MeOH (3.088 mL), HCl (1M in DEE, 0.13 mL, 0.130 mmol) was dropwise added. The mixture was stirred for 3h, then concentrated under vacuum, stripped three times with DCM and then freeze-dried to give **E11** (11 mg, 0.033 mmol, yield= 49.03%) as white solid.

Method 2: Rt= 0.78 min, MS (ESI) m/z = 300.29 [M+H]⁺.

¹H NMR (400 MHz, DMSO-*d*₆) δ ppm 13.44 (br s, 1 H), 8.63 (br s, 1 H), 7.97 (brd, J=6.2 Hz, 1 H), 7.19 - 7.27 (m, 2 H), 6.98 (d, J=8.6 Hz, 1 H), 6.86 - 6.93 (m, 2 H), 6.76 (br d, J=6.0 Hz, 1 H), 3.76 (s, 3 H), 3.49 - 3.59 (m, 2 H), 2.98 (s, 3 H), 2.84 - 2.91 (m, 5 H)

### Example 12: (S)-(2-((2-methoxyphenethyl)amino)pyridin-4-yl)(2-(trifluoromethyl)piperidin-1-yl)methanone; hydrochloride (E12)

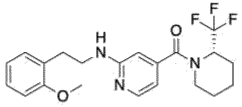

### Step A:

Prepared similarly as described for compound **E2** starting from **D2** (50 mg, 0.180 mmol) and (2S)-2-(trifluoromethyl)piperidine (30.93 mg, 0.200 mmol). The crude was purified by NP Flash Chromatography (silica gel column, Sfar D 1 g, 5-45% EtOAc in cyclohexane) affording (*S*)-(2-((2-methoxyphenethyl)amino)pyridin-4-yl)(2-(trifluoromethyl)piperidin-1-yl)methanone (49.5 mg, 0.121 mmol, yield= 66.17%).

### Step B:

To a solution of (*S*)-(2-((2-methoxyphenethyl)amino)pyridin-4-yl)(2-(trifluoromethyl)piperidin-1-yl)methanone (49 mg, 0.120 mmol) in MeOH (1 mL), HCl (1M in DEE, 156.4 mL, 0.160 mmol) was added at 25 °C. The mixture was stirred for 3h, then concentrated under vacuum, stripped three times with DCM and then freeze-dried to afford **E12** ( 48.7 mg, 0.110 mmol, yield= 91.22%) as a white powder.

Method 2: *Rt=* 1.41 min, MS (ESI) m/z = 408.3 [M+H]⁺.

¹H NMR (400 MHz, DMSO-*d*₆) δ ppm 12.97 - 14.65 (m, 1 H), 8.26 - 9.44 (m, 1 H), 8.00 (br d, J=6.0 Hz, 1 H), 7.16 - 7.30 (m, 2 H), 6.98 (d, J=8.2 Hz, 1 H), 6.84 - 6.94 (m, 2 H), 6.70 - 6.79 (m, 1 H), 4.32 - 5.32 (m, 1 H), 3.77 (s, 3 H), 3.46 - 4.56 (m, 1 H), 3.27 - 3.65 (m, 2 H), 2.80 - 3.21 (m, 1 H), 2.89 (br t, J=7.3 Hz, 2 H), 1.33 - 2.14 (m, 6 H)

### Example 13: (2-((2-methoxyphenethyl)amino)pyridin-4-yl)(4-azaspiro[2.5]octan-4-yl)methanone (E13)

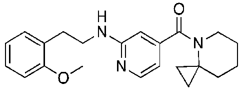

Prepared similarly as described for compound **E1** (Step A) starting from **D2** (46.11 mg, 0.170 mmol) and 4-azaspiro[2.5]octane;hydrochloride (25 mg, 0.170 mmol). The crude was purified by RP Flash Chromatography (Sfar C18, 12 g, eluent: CH₃CN (+0.1% HCOOH) in H₂O (+0.1% HCOOH)) to give **E13** (38 mg, 0.104 mmol, yield= 61.41%) as colorless oil. Method 2: Rt= 1.21 min, MS (ESI) m/z = 366.3 [M+H]⁺.

¹H NMR (500 MHz, DMSO-*d*₆) δ ppm 7.99 (d, J=5.2 Hz, 1 H), 7.16 - 7.23 (m, 1 H), 7.11 - 7.16 (m, 1 H), 6.96 (d, J=8.1 Hz, 1 H), 6.86 (t, J=7.3 Hz, 1 H), 6.66 - 6.75 (m, 1 H), 6.40 (br s, 2 H), 3.78 (s, 3 H), 3.35 - 3.47 (m, 2 H), 3.35 - 3.90 (m, 2 H), 2.80 (br t, J=7.4 Hz, 2 H), 1.26 - 1.89 (m, 6 H), 0.36 - 0.99 (m, 4 H)

### Example 14: rac-((3aR,6aS)-4,4-difluorohexahydrocyclopenta[c]pyrrol-2(1H)-yl)(2-((2-methoxyphenethyl)amino)pyridin-4-yl) methanone; hydrochloride (E14)

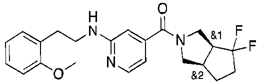

### Step A:

Prepared similarly as described for compound **E1** starting from **D2** (100 mg, 0.370 mmol) and *rac*-(3aR,6aS)-4,4-difluoro-2,3,3a,5,6,6a-hexahydro-1H-cyclopenta[c]pyrrole; hydrochloride (67 mg, 0.370 mmol). The crude was purified by RP Flash Chromatography (Sfar C18, 12 g, eluent: H₂O, CH₃CN 0.1% HCOOH) to give *rac*-((3a*R*,6a*S*)-4,4-difluorohexahydrocyclopenta[*c*]pyrrol-2(1*H*)-yl)(2-((2-methoxyphenethyl)amino)pyridin-4-yl)methanone (36 mg, 0.090 mmol, yield= 24.42%) as a yellow oil.

### Step B:

A solution of *rac*-((3a*R*,6a*S*)-4,4-difluorohexahydrocyclopenta[c]pyrrol-2(1*H*)-yl)(2-((2-methoxyphenethyl)amino)pyridin-4-yl)methanone (49 mg, 0.120 mmol) in MeOH (1 mL) was treated with HCl (1M in DEE, 180 mL, 0.180 mmol). The mixture was stirred for 3h, then concentrated under a vacuum, stripped three times with DCM and freeze-dried to give **E14** (37 mg, 0.084 mmol, yield= 94.22%) as a white solid.

Method 2: R*t*=1.22 min, MS (ESI) m/z = 402.45 [M+H]⁺.

¹H NMR (500 MHz, DMSO-*d*₆) δ ppm 13.49 (br s, 1 H), 7.99 (br s, 1 H), 7.53 - 9.64 (m, 1 H), 7.17 - 7.28 (m, 2 H), 6.98 (d, J=8.2 Hz, 1 H), 6.87 - 6.92 (m, 1 H), 6.93 (br s, 1 H), 6.77 (br s, 1 H), 3.76 (s, 3 H), 3.47 - 3.56 (m, 2 H), 3.13 - 3.82 (m, 4 H), 2.84

- 2.91 (m, 2 H), 2.83 - 3.06 (m, 2 H), 2.07 - 2.23 (m, 2 H), 1.89 - 2.07 (m, 1 H), 1.40 - 1.69 (m, 1 H)

### Example 15: (S)-(2-ethylpiperidin-1-yl)(2-((2-methoxyphenethyl)amino)pyridin-4-yl)methanone; hydrochloride (E15)

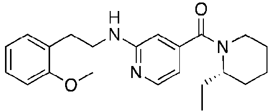

### Step A:

Prepared similarly as described for compound **E1** starting from **D2** (50 mg, 0.180 mmol) and (2S)-2-ethylpiperidine;hydrochloride (27.48 mg, 0.180 mmol). The crude was purified by RP Flash Chromatography (Sfar C18, 12 g, eluent: CH₃CN (+0.1% HCOOH) in H₂O (+0.1% HCOOH)) to give (S)-(2-ethylpiperidin-1-yl)(2-((2-methoxyphenethyl)amino)pyridin-4-yl)methanone (36.6 mg, 0.100 mmol, yield= 54.24%) as an off-white oil.

### Step B:

A solution of (S)-(2-ethylpiperidin-1-yl)(2-((2-methoxyphenethyl)amino)pyridin-4-yl)methanone (36.6 mg, 0.100 mmol) in MeOH (1.085 mL) was treated with 1.25M HCl in MeOH (180 mL, 0.180 mmol). The mixture was stirred for2h, then concentrated under vacuum, stripped three times with water and freeze-dried to afford **E15** (40.2 mg, 0.100 mmol, yield= 99.92%) as off-white solid.

Method 2: R*t*= 1.29 min, MS (ESI) m/z = 368.4 [M+H]⁺.

¹H NMR (400 MHz, DMSO-*d*₆) δ ppm 12.46 - 14.19 (m, 1 H), 7.97 (br d, J=6.1 Hz, 1 H), 7.69 - 9.86 (m, 1 H), 7.15 - 7.27 (m, 2 H), 6.98 (d, J=8.4 Hz, 1 H), 6.77 - 6.94 (m, 2 H), 6.64 - 6.76 (m, 1 H), 3.77 (s, 3 H), 3.48 - 3.57 (m, 2 H), 3.46 - 4.69 (m, 1 H), 3.16 - 4.42 (m, 1 H), 2.88 (br t, J=7.2 Hz, 2 H), 2.70 - 3.13 (m, 1 H), 1.26 - 1.88 (m, 8 H), 0.59 - 0.98 (m, 3 H)

### Example 16: (cis-hexahydrocyclopenta[c]pyrrol-2(1H)-yl)(2-((2-methoxyphenethyl)amino)pyridin-4-yl)methanone (E16)

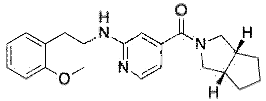

### Step A:

Prepared similarly as described for compound **E1** (Step A) starting from **D2** (200 mg, 0.730 mmol) and cis-Octahydrocyclopenta[c]pyrrole hydrochloride. The reaction was quenched with Na₂CO₃ ss (2x3mL). The crude was purified to afford **E16** (80 mg, 0.219 mmol, yield= 29.8%) as a light yellow solid.

Methodo 4: Rt= 1.23 min, MS (ESI) m/z = 366.4 [M+H]⁺ .

¹H NMR (400 MHz, DMSO-*d*₆) δ ppm 8.01 (d, J=5.7 Hz, 1 H), 7.17 - 7.24 (m, 1 H), 7.15 (dd, J=7.3, 1.2 Hz, 1 H), 6.96 (d, J=8.1 Hz, 1 H), 6.87 (t, J=7.3 Hz, 1 H), 6.72 (t, J=5.6 Hz, 1 H), 6.41 - 6.49 (m, 2 H), 3.78 (s, 3 H), 3.35 - 3.45 (m, 2 H), 3.23 - 3.70 (m, 2 H), 3.04 - 3.56 (m, 2 H), 2.80 (t, J=7.5 Hz, 2 H), 2.54 - 2.69 (m, 2 H), 1.25 - 1.87 (m, 6 H)

### Example 17: (R)-(2-ethylpiperidin-1-yl)(2-((2-methoxyphenethyl)amino)pyridin-4-yl)methanone; hydrochloride (E17)

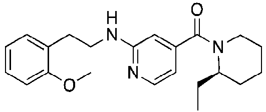

### Step A:

Prepared similarly as described for compound **E1** starting from **D2** (50 mg, 0.180 mmol)and (2R)-2-ethylpiperidine;hydrochloride (27.48 mg, 0.180 mmol). The crude was purified to afford (*R*)-(2-ethylpiperidin-1-yl)(2-((2-methoxyphenethyl)amino)pyridin-4-yl)methanone (34.7 mg, 0.094 mmol, yield= 51.42%) as a white oil.

Method 1: R*t*= 0.75 min, MS (ESI) m/z = 368.5 [M+H]⁺.

### Step B:

A stirred solution of (*R*)-(2-ethylpiperidin-1-yl)(2-((2-methoxyphenethyl)amino) pyridin-4-yl)methanone (34.7 mg, 0.090 mmol) in MeOH (1.028 mL), 1.25M HCl in MeOH (98.2 mL, 0.120 mmol) was dropwise added. The mixture was stirred for 2h, then concentrated under vacuum, stripped three times with water to afford **E17** (32.1 mg, 0.079 mmol, yield= 84.16%) as an off-white solid.

Method 2: R*t*= 1.30 min, MS (ESI) m/z = 368.4 [M+H]⁺.

¹H NMR (400 MHz, DMSO-*d*₆) δ ppm 13.43 (br s, 1 H), 7.98 (d, J=5.8 Hz, 1 H), 7.77 (br s, 1 H), 7.15 - 7.29 (m, 2 H), 6.97 (d, J=8.1 Hz, 1 H), 6.88 (t, J=7.4 Hz, 1 H), 6.49 - 6.74 (m, 2 H), 3.77 (s, 3 H), 3.45 - 4.67 (m, 1 H), 3.48 (br d, J=4.8 Hz, 2 H), 3.19 - 4.43 (m, 1 H), 2.85 (br t, J=7.3 Hz, 2 H), 2.67 - 3.14 (m, 1 H), 1.22 - 1.86 (m, 8 H), 0.57 - 0.97 (m, 3 H)

### Example 18: (2-azabicyclo[4.1.0]heptan-2-yl)(2-((2-methoxyphenethyl)amino)pyridin-4-yl)methanone; hydrochloride (E18)

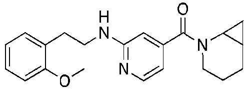

### Step A:

Prepared similarly as described for compound **E1** starting from **D2** (80 mg, 0.290 mmol) and 2-azabicyclo[4.1.0]heptane;hydrochloride (39.3 mg, 0.290 mmol) to afford (2-azabicyclo[4.1.0]heptan-2-yl)(2-((2-methoxyphenethyl)amino)pyridin-4-yl)methanone (88 mg, 0.250 mmol, yield= 85.14%) as an orange foam.

Method 1= 0.65 min, MS (ESI) m/z = 352.30 [M+H]⁺. ¹H NMR (400 MHz, DMSO-*d*₆) δ ppm 7.97 - 8.05 (m, 1 H), 7.17 - 7.24 (m, 1 H), 7.15 (dd, J=7.4, 1.5 Hz, 1 H), 6.96 (d, J=8.2 Hz, 1 H), 6.86 (t, J=7.4 Hz, 1 H), 6.71 (q, J=5.6 Hz, 1 H), 6.29 - 6.56 (m, 2 H), 3.79 (s, 3 H), 3.35 - 3.47 (m, 2 H), 3.14 - 4.10 (m, 1 H), 2.75 - 2.86 (m, 2 H), 2.54 - 3.05 (m, 1 H), 2.52 - 2.87 (m, 1 H), 1.72 - 1.92 (m, 2 H), 1.45 - 1.68 (m, 1 H), 1.19 - 1.39 (m, 2 H), 0.53 - 0.94 (m, 1 H), 0.25 - 0.48 (m, 1 H).

### Step B:

A solution of (2-azabicyclo[4.1.0]heptan-2-yl)(2-((2-methoxyphenethyl)amino) pyridin-4-yl)methanone (45 mg, 0.130 mmol) in MeOH (1 mL) was treated with HCl (1M in DEE). The mixture was stirred for 15 min, then concentrated under a vacuum, stripped three times with water and then freeze-dried to afford **E18** (49 mg, 0.126 mmol, yield= 99.65%) as a white foam.

Method 1: Rt= 0.67 min, MS (ESI) m/z = 352.3 [M+H]⁺. ¹H NMR (400 MHz, DMSO-*d*₆) δ ppm 13.63 (br s, 1 H), 8.94 (br s, 1 H), 7.91 - 8.04 (m, 1 H), 7.18 - 7.29 (m, 2 H), 6.71 - 7.13 (m, 4 H), 3.76 (s, 3 H), 3.48 - 3.63 (m, 3 H), 3.10 - 4.13 (m, 1 H), 2.81 - 2.96 (m, 1 H), 2.54 - 3.07 (m, 2 H), 1.75 - 1.93 (m, 2 H), 1.45 - 1.71 (m, 1 H), 1.17 - 1.45 (m, 2 H), 0.57 - 0.97 (m, 1 H), 0.38 - 0.57 (m, 1 H)

### Example 19: (4,4-difluoroazepan-1-yl)(2-((2-methoxyphenethyl)amino)pyridin-4-yl)methanone; hydrochloride (E19)

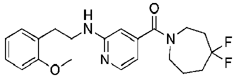

### Step A:

Prepared similarly as described for compound **E1** (Step A) starting from **D2** (50 mg, 0.180 mmol) and 4,4-difluoroazepane;hydrochloride (31.51 mg, 0.180 mmol). The mixture was quenched with Na₂CO₃ ss (3mL), then EtOAc was added (30 mL). The organic layer was washed with ss NaHCO₃ (2x15 mL), water and finally with brine. The organic layer was dried with Na₂SO₄ and concentrated under a high vacuum, affording (4,4-difluoroazepan-1-yl)(2-((2-methoxyphenethyl)amino)pyridin-4-yl)methanone (58 mg, 0.149 mmol, yield= 81.11%).

### Step B:

A solution of (4,4-difluoroazepan-1-yl)(2-((2-methoxyphenethyl)amino)pyridin-4-yl)methanone (58 mg, 0.149 mmol) in MeOH (1.719 mL) was treated with 1.25M HCl in MeOH (155 µL, 0.190 mmol). The mixture was stirred for 2h, then concentrated under vacuum, stripped three times with water to obtain **E19** ( 56.4 mg, 0.132 mmol, yield= 88.92%) as yellow solid.

Method 1: R*t*= 0.70 min, MS (ESI) m/z = 390.5 [M+H]⁺.

¹H NMR (400 MHz, DMSO-*d*₆) δ ppm 13.46 (s, 1 H), 8.00 (d, J=5.7 Hz, 1 H), 7.14 - 7.28 (m, 2 H), 6.97 (d, J=8.1 Hz, 1 H), 7.65 (br s, 1 H), 6.88 (t, J=7.3 Hz, 1 H), 6.63 (br d, J=18.9 Hz, 2 H), 3.77 (s, 3 H), 3.55 - 3.65 (m, 2 H), 3.42 - 3.51 (m, 2 H), 3.32 - 3.38 (m, 2 H), 2.85 (br t, J=7.3 Hz, 2 H), 1.62 - 2.38 (m, 6 H).

### Example 20: (hexahydrocyclopenta[c]pyrrol-2(1H)-yl)(2-((2-methoxyphenethyl)amino)pyridin-4-yl)methanone (E20)

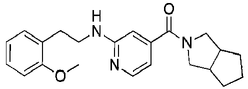

Prepared similarly as described for compound **E1** (Step A) starting from **D2** (41 mg, 0.150 mmol) and 1,2,3,3a,4,5,6,6a-octahydrocyclopenta[c]pyrrole; hydrochloride (22.26 mg, 0.150 mmol). After work-up the crude was purified to afford E20 (17 mg, 0.047 mmol, yield= 30.86%) as an oil. Method 1: Rt= 0.72 min, MS (ESI) m/z = 366.36 [M+H]⁺. ¹H NMR (400 MHz, DMSO-*d*₆) δ ppm 7.99 - 8.02 (m, 1 H), 7.17 - 7.23 (m, 1 H), 7.15 (dd, J=7.4, 1.5 Hz, 1 H), 6.96 (d, J=8.0 Hz, 1 H), 6.87 (td, J=7.3, 0.9 Hz, 1 H), 6.84 (br s, 1 H), 6.46 - 6.51 (m, 2 H), 3.78 (s, 3 H), 3.36 - 3.47 (m, 2 H), 3.25 - 3.72 (m, 2 H), 3.01 - 3.59 (m, 2 H), 2.81 (t, J=7.4 Hz, 2 H), 2.54 - 2.72 (m, 2 H), 1.17 - 1.89 (m, 6 H).

### Example 21: (3-azabicyclo[3.1.0]hexan-3-yl)(2-((2-methoxyphenethyl)amino)pyridin-4-yl)methanone (E21)

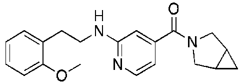

### Step A:

Prepared similarly as described for compound **E1** starting from **D2** (50 mg, 0.170 mmol) and 3-azabicyclo[3.1.0]hexane hydrochloride (20.64 mg, 0.170 mmol). The incomplete conversion was observed by UPLC-UV, therefore further 3-azabicyclo[3.1.0]hexane hydrochloride (16.51 mg, 0.140 mmol) was added. The mixture was left to stir for 3h. After an appropriate work-up, (3-azabicyclo[3.1.0]hexan-3-yl)(2-((2-methoxyphenethyl)amino)pyridin-4-yl)methanone (32 mg, 0.095 mmol, yield= 54.95%) was obtained. Method 1: Rt= 0.63 min, MS (ESI) m/z = 338.4 [M+H]⁺.

### Step B:

To a stirred solution of (3-azabicyclo[3.1.0]hexan-3-yl)(2-((2-methoxy-phenethyl)amino)pyridin-4-yl)methanone (32 mg, 0.090 mmol) in MeOH (0.948 mL), HCl (1.25M in MeOH) (98.63 mL, 0.120 mmol) was dropwise added. The mixture was stirred for 2h. Then concentrated under vacuum, stripped three times with water and freeze-dried to obtain E21 (31.2 mg, 0.083 mmol, yield= 87.99%) as a yellow solid.

Method 1: R*t*= 0.62 min, MS (ESI) m/z = 338.3 [M+H]⁺.

¹H NMR (400 MHz, DMSO-*d*₆) δ ppm 7.99 (d, J=5.7 Hz, 1 H), 7.12 - 7.32 (m, 2 H), 6.97 (d, J=8.0 Hz, 1 H), 6.88 (t, J=7.3 Hz, 1 H), 6.77 - 8.51 (m, 2 H), 6.49 - 6.76 (m, 2 H), 3.84 (d, J=11.9 Hz, 1 H), 3.77 (s, 3 H), 3.56 (dd, J=10.5, 3.8 Hz, 1 H), 3.42 - 3.50 (m, 2 H), 3.21 - 3.41 (m, 2 H), 2.84 (t, J=7.4 Hz, 2 H), 1.44 - 1.66 (m, 2 H), 0.58 - 0.74 (m, 1 H), 0.11 (q, J=4.1 Hz, 1 H).

### Example 22: (2-ethylpiperidin-1-yl)(2-((2-methoxyphenethyl)amino)pyridin-4-yl)methanone (E22)

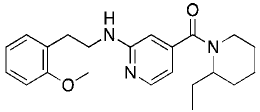

Prepared similarly as described for compound **E1** (Step A) starting from **D2** (96.78 mg, 0.330 mmol) and 2-ethylpiperdine HCl (50 mg, 0.330 mmol). The crude was purified by NP Flash Chromatography column (silica gel column, 0-20% MeOH in DCM) to afford **E22** ( 31 mg, 0.084 mmol, yield= 25.25%) as a colourless oil.

Method 1: Rt= 0.73 min, MS (ESI) m/z = 368.36[M+H]⁺.

¹H NMR (400 MHz, DMSO-*d*₆) δ ppm 8.00 (d, J=5.7 Hz, 1 H), 7.19 (td, J=7.8, 1.8 Hz, 1 H), 7.12 - 7.16 (m, 1 H), 6.96 (d, J=7.9 Hz, 1 H), 6.86 (td, J=7.4, 0.8 Hz, 1 H), 6.77 (br s, 1 H), 6.28 - 6.43 (m, 2 H), 3.78 (s, 3 H), 3.45 - 4.66 (m, 1 H), 3.36 - 3.45 (m, 2 H), 3.19 - 4.38 (m, 1 H), 2.80 (t, J=7.5 Hz, 2 H), 2.61 - 3.08 (m, 1 H), 1.68 - 1.81 (m, 2 H), 1.20 - 1.70 (m, 6 H), 0.45 - 1.00 (m, 3 H).

### Example 23: (3,3-difluoroazetidin-1-yl)(2-((2-methoxyphenethyl)amino)pyridin-4-yl)methanone (E23)

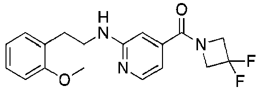

Prepared similarly as described for compound **E1** starting from **D2** (200 mg, 0.730 mmol) in DCM (2 mL) and 3,3-difluoroazetidin;hydrochloride (114.17 mg, 0.880 mmol). The reaction mixture was quenched with NaOH (1M, 2x5mL) and separated by a phase separator. The organic layer was concentrated under a high vacuum to afford **E23** (73 mg, 0.210 mmol, yield= 28.61%) as a light yellow solid.

Method 2: Rt= 1.00 min, MS (ESI) m/z = 348.23 [M+H]⁺ .

¹H NMR (400 MHz, DMSO-*d*₆) δ ppm 8.06 (d, J=5.3 Hz, 1 H), 7.17 - 7.25 (m, 1 H), 7.15 (dd, J=7.3, 1.5 Hz, 1 H), 6.96 (d, J=8.0 Hz, 1 H), 6.87 (td, J=7.4, 0.7 Hz, 1 H), 6.81 (t, J=5.6 Hz, 1 H), 6.69 (s, 1 H), 6.63 (dd, J=5.2, 1.3 Hz, 1 H), 4.32 - 4.93 (m, 4 H), 3.80 (s, 3 H), 3.38 - 3.52 (m, 2 H), 2.81 (t, J=7.4 Hz, 2 H)

### Example 24: (2-((2-methoxyphenethyl)amino)pyridin-4-yl)(1-methyl-3-azabicyclo[3.1.0]hexan-3-yl)methanone (SCU-465, N1315-31) (E24)

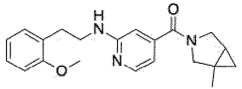

Prepared similarly as described for compound **E1** starting from **D2** (100 mg, 0.370 mmol) and 1-methyl-3-azabicyclo[3.1.0]hexane;hydrochloride (49.07 mg, 0.370 mmol). The reaction mixture was quenched with NaOH (1M, 2x5mL) and separated by a phase separator. The organic layer was concentrated under a high vacuum to afford **E24** (21 mg, 0.060 mmol, yield= 16.27%) as colourless oil.

Method 4: Rt= 1.15 min, MS (ESI) m/z = 352.38 [M+H]⁺

¹H NMR (500 MHz, DMSO-*d*₆) δ ppm 7.94 - 8.05 (m, 1 H), 7.17 - 7.24 (m, 1 H), 7.11 - 7.17 (m, 1 H), 6.96 (d, J=8.1 Hz, 1 H), 6.87 (t, J=7.3 Hz, 1 H), 6.66 - 6.79 (m, 1 H), 6.37 - 6.48 (m, 2 H), 3.78 (s, 3 H), 3.37 - 3.44 (m, 2 H), 3.12 - 3.92 (m, 4 H), 2.77 - 2.83 (m, 2 H), 1.24 - 1.34 (m, 1 H), 1.17 - 1.26 (m, 3 H), 0.58 (br t, J=6.0 Hz, 1 H), 0.14 - 0.34 (m, 1 H).

### Example 25: azetidin-1-yl(2-((2-methoxyphenethyl)amino)pyridin-4-yl)methanone (SCU-523, N1315-28-2, N1315-28-1:) (E25)

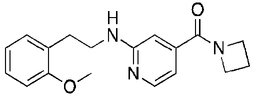

### Step A:

Prepared similarly as described for compound **E1** starting from **D2** (100 mg, 0.370 mmol) and azetidine;hydrochloride (34.36 mg, 0.370 mmol). The reaction mixture was quenched with Na₂CO₃ (1M, 2x5mL); separated by a phase separator, and the organic layer was concentrated under a high vacuum. The crude was purified by RP Flash Chromatography (Sfar C18, 12 g, eluent: CH₃CN (+0.1% HCOOH) in H₂O (+0.1% HCOOH)) to give azetidin-1-yl(2-((2-methoxyphenethyl)amino)pyridin-4-yl)methanone (27 mg, 0.087 mmol, yield= 23.61%) as a yellow oil.

Method 2: Rt= 0.83 min, MS (ESI) m/z = 312.3 [M+H]⁺.

### Step B:

A stirred solution of azetidin-1-yl-[2-[2-(2-methoxyphenyl)ethylamino]-4-pyridyl]methanone (27 mg, 0.090 mmol) in MeOH (4.169 mL) was treated with HCl (1M in DEE, 0.17 mL, 0.170 mmol). The mixture was stirred for 3h, then concentrated under vacuum, stripped three times with water and freeze-dried to obtain **E25** (34 mg, 0.098 mmol, yield= 112.73%) as a yellow solid. Further purification by RP Flash Chromatography (Sfar C18, 12 g, eluent: CH₃CN (+0.1% HCOOH) in H₂O (+0.1% HCOOH)) was conducted to give **E25** (20 mg, 0.064 mmol, yield= 74.07%).

Method 2: Rt= 0.92 min, MS (ESI) m/z = 312.3 [M+H]⁺.

¹H NMR (400 MHz, DMSO-*d*₆) δ ppm 11.88 - 14.70 (m, 1 H), 7.98 (br d, J=6.1 Hz, 1 H), 7.31 - 9.64 (m, 1 H), 7.16 - 7.30 (m, 2 H), 6.94 - 7.11 (m, 2 H), 6.89 (t, J=7.4 Hz, 1 H), 6.76 - 6.86 (m, 1 H), 3.77 (s, 3 H), 3.64 - 4.39 (m, 4 H), 3.51 (br s, 2 H), 2.78 - 2.93 (m, 2 H), 1.93 - 2.31 (m, 2 H)

### Example 26: (2-((2-methoxyphenethyl)amino)pyridin-4-yl)(2-azaspiro[3.3]heptan-2-yl)methanone (E26)

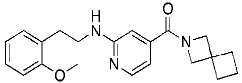

Prepared similarly as described for compound **E1** starting from **D2** (120 mg, 0.440 mmol) and 2-azaspiro[3.3]heptane;hydrochloride (58.89 mg, 0.440 mmol). The reaction mixture was quenched with Na₂CO₃ (1M, 2x5 mL) and separated by a phase separator. The organic layer was concentrated under a high vacuum and the crude was purified by RP Flash Chromatography (Sfar C18, 12 g, eluent: CH₃CN (+0.1% HCOOH) in H₂O (+0.1% HCOOH)) to give **E26** (14 mg, 0.040 mmol, yield= 40%).

Method 2: Rt= 1.20 min, MS (ESI) m/z = 352.31 [M+H]⁺.

¹H NMR (400 MHz, DMSO-*d*₆) δ ppm 8.02 (d, J=5.3 Hz, 1 H), 7.17-7.25 (m, 1 H), 7.12-7.16 (m, 1 H), 6.96 (d, J=8.2 Hz, 1 H), 6.87 (t, J=7.4 Hz, 1 H), 6.76 (t, J=5.6 Hz, 1 H), 6.62 (s, 1 H), 6.56 (dd, J=5.2, 1.0 Hz, 1 H), 4.20 (s, 2 H), 3.98 (s, 2 H), 3.80 (s, 3 H), 3.37-3.45 (m, 2 H), 2.80 (t, J=7.3 Hz, 2 H), 2.09-2.18 (m, 4 H), 1.71-1.81 (m, 2 H)

### Example 27: (R)-(2-((2-methoxyphenethyl)amino)pyridin-4-yl)(2-methylindolin-1-yl)methanone (E27)

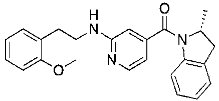

Prepared similarly as described for compound **E1** starting from **D2** (120 mg, 0.440 mmol) and (2R)-2-methylindoline;hydrochloride (74.76 mg, 0.440 mmol). The reaction mixture was quenched with Na₂CO₃ (1M, 2x5 mL), separated by a phase separator, and the organic layer was concentrated under a high vacuum. The crude was purified by RP Flash Chromatography (Sfar C18, 12 g, eluent: CH₃CN (+0.1% HCOOH) in H₂O (+0.1% HCOOH)) to afford **E27** (35 mg, 0.090 mmol, yield= 20.5%) as a yellow oil.

Method 1: Rt= r.t. 1.45 min, MS (ESI) m/z = 388.4 [M+H]⁺.

¹H NMR (500 MHz, CD₃OD) δ ppm 8.04 (d, J=5.2 Hz, 1 H), 7.28 (br d, J=7.5 Hz, 1 H), 7.17 (br t, J=7.8 Hz, 1 H), 7.13 (br d, J=6.6 Hz, 1 H), 6.92 (d, J=8.2 Hz, 1 H), 6.84 (t, J=7.3 Hz, 1 H), 6.50 - 6.79 (m, 2 H), 6.14 - 8.53 (m, 3 H), 4.67 (br s, 1 H), 3.80 (br s, 3 H), 3.37 - 3.56 (m, 3 H), 2.90 (br t, J=6.7 Hz, 2 H), 2.61 - 2.76 (m, 1 H), 1.25 (br s, 3 H).

### Example 28: (6,6-difluoro-2-azaspiro[3.3]heptan-2-yl)(2-((2-methoxyphenethyl)amino)pyridin-4-yl)methanone (E28)

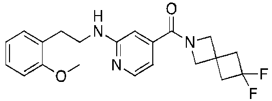

Prepared similarly as described for compound **E1** starting from **D2** (100 mg, 0.370 mmol) and 6,6-difluoro-2-azaspiro[3.3]heptane;hydrochloride (74.74 mg, 0.440 mmol). The reaction mixture was quenched with NaOH (1M, 2x5 mL), separated by a phase separator, and the organic layer was concentrated under a high vacuum to afford **E28** (11 mg, 0.028 mmol, yield= 7.73%) as colourless oil.

Method 2: Rt= r.t. 1.13 min, MS (ESI) m/z = 388.23 [M+H]⁺.

¹H NMR (400 MHz, DMSO-*d*₆) δ ppm 8.04 (d, J=5.3 Hz, 1 H), 7.20 (td, J=7.3, 1.4 Hz, 1 H), 7.14 (dd, J=7.3, 1.4 Hz, 1 H), 6.96 (d, J=8.0 Hz, 1 H), 6.87 (t, J=7.3 Hz, 1 H), 6.78 (t, J=5.6 Hz, 1 H), 6.63 (s, 1 H), 6.57 (dd, J=5.2, 1.2 Hz, 1 H), 4.36 (s, 2 H), 4.12 (s, 2 H), 3.80 (s, 3 H), 3.36 - 3.48 (m, 2 H), 2.74 - 2.97 (m, 6 H)

### Example 29: (2-((3-chlorophenethyl)amino)pyridin-4-yl)(2-ethylpiperidin-1-yl)methanone;hydrochloride (E29)

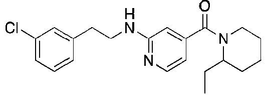

### Step A:

Prepared similarly as described for compound **E1** starting from **D5** (100 mg, 0.320 mmol) and 2-ethylpiperidine HCl (47.79 mg, 0.320 mmol). The reaction mixture was quenched with NaOH (1M). Organic phase was separated, then washed with brine solution, and concentrated under reduced pressure. The crude was purified by RP Flash Chromatography (Sfar C18, 12 g, eluent: CH₃CN (+0.1% HCOOH) in H₂O (+0.1% HCOOH)) to afford (2-((3-chlorophenethyl)amino)pyridin-4-yl)(2-ethylpiperidin-1-yl)methanone (48 mg, 0.129 mmol, yield= 40.42%) as a pale brown oil.

### Step B:

To a stirred solution of (2-((3-chlorophenethyl)amino)pyridin-4-yl)(2-ethylpiperidin-1-yl)methanone (48 mg, 0.130 mmol) in MeOH (1.076 mL), Hydrogen chloride (1M, 168 µL, 0.170 mmol) was dropwise added. The mixture was stirred for 2h. Then concentrated under vacuum, stripped three times with water and then freeze-dried to give **E29** (46.1 mg, 0.113 mmol, yield= 87.47%) as a pale yellow solid.

Method 2: R*t*= 1.41 min, MS (m/z) 372.3 [M+H]⁺.

¹H NMR (500 MHz, DMSO-*d*₆) δ ppm 12.33 - 14.30 (m, 1 H), 8.09 - 10.03 (m, 1 H), 7.97 (br d, J=6.2 Hz, 1 H), 7.43 (s, 1 H), 7.32 - 7.37 (m, 1 H), 7.25 - 7.31 (m, 2 H), 6.80 - 6.96 (m, 1 H), 6.73 (br s, 1 H), 3.58 - 3.69 (m, 2 H), 3.32 - 4.75 (m, 1 H), 3.18 - 4.35 (m, 1 H), 2.92 (t, J=7.3 Hz, 2 H), 2.70 - 3.14 (m, 1 H), 1.26 - 1.86 (m, 8 H), 0.58 - 0.93 (m, 3 H)

### Example 30: (2-ethylpiperidin-1-yl)(2-((2-(pyridin-2-yl)ethyl)amino)pyridin-4-yl)methanone; hydrochloride (SCU-475; N1344-16) (E30)

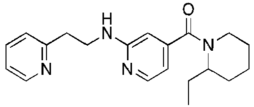

### Step A:

A mixture of 2-(2-pyridinyl)ethanamine (51µL, 0.420 mmol), dicesium; carbonate (275.78 mg, 0.850 mmol) and **D3** (100 mg, 0.420 mmol) was heated to 100 °C for 3h. At reaction completion, NaOH (1M, 10 mL) was added, and the mixture was extracted with DCM (4 x 10 mL). The mixture was separated by a phase separator, and the organic layer was concentrated under high vacuum. The crude was purified by RP Flash Chromatography (Sfar C18, 12 g, eluent: CH₃CN (+0.1% HCOOH) in H₂O (+0.1% HCOOH)) to afford (2-ethylpiperidin-1-yl)(2-((2-(pyridin-2-yl)ethyl)amino)pyridin-4-yl)methanone (36 mg, 0.106 mmol, yield= 25.13%) as a dark red oil.

### Step B:

A stirred solution of (2-ethylpiperidin-1-yl)(2-((2-(pyridin-2-yl)ethyl)amino) pyridin-4-yl)methanone (36 mg, 0.110 mmol) in MeOH (1.064 mL) was treated with HCl (1.25M in MeOH) (138 µL, 0.140 mmol). The mixture was stirred for 2h, then concentrated under vacuum, stripped three times with water and then freeze dried, obtaining **E30** (38.7 mg, 0.103 mmol, yield= 97%) as a brown solid.

Method 2: R*t*= 0.67 min, MS (m/z) 339.3 [M+H]⁺.

¹H NMR (400 MHz, DMSO-*d*₆) δ ppm 8.61 - 8.77 (m, 1 H), 8.07 - 8.25 (m, 1 H), 7.94 - 8.02 (m, 1 H), 7.67 - 7.79 (m, 1 H), 7.53 - 7.66 (m, 1 H), 7.14 - 9.71 (m, 2 H), 6.77 - 6.91 (m, 1 H), 6.63 - 6.75 (m, 1 H), 3.76 - 3.84 (m, 2 H), 3.37 - 4.69 (m, 1 H), 3.18 - 4.36 (m, 1 H), 3.17 - 3.26 (m, 2 H), 2.68 - 3.11 (m, 1 H), 1.23 - 1.86 (m, 8 H), 0.60 - 0.94 (m, 3 H)

### Example 31: (2-((2-chlorophenethyl)amino)pyridin-4-yl)(2-ethylpiperidin-1-yl)methanone (E31)

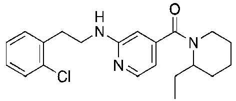

A mixture of 2-(2-chlorophenyl)ethanamine (65.86 mg, 0.420 mmol), DIPEA (753 µL, 4.23 mmol) and **D3** (100 mg, 0.420 mmol) was heated to 120 °C, ON; Ammonium chloride saturated solution (10 mL) was added, and the mixture was extracted with EtOAc (4 x 10 mL). The organic layer was washed with brine, dried over anh Na₂SO₄ and concentrated under a vacuum. The crude was purified by RP Flash Chromatography (Sfar C18, 12 g, eluent: CH₃CN (+0.1% HCOOH) in H₂O (+0.1% HCOOH)) to give **E31** (35.8 mg, 0.096 mmol, yield= 22.75%) as yellow oil.

### Method 2: Rt= 1.36 min, MS (m/z) 372.3 [M+H]⁺.

¹H NMR (500 MHz, DMSO-*d*₆) δ ppm 8.02 (d, J = 5.1 Hz, 1H), 7.42 (dd, J = 7.5, 1.6 Hz, 1H), 7.38 - 7.31 (m, 1H), 7.30 - 7.25 (m, 1H), 7.26 - 7.22 (m, 1H), 6.83 (br s, 1H), 6.36 (dd, J = 5.1, 1.1 Hz, 1H), 6.34 (s, 1H), 3.49 (q, J = 6.8 Hz, 2H), 4.01 (br s, 1H), 3.83 (s, 1H), 2.96 (t, J = 7.3 Hz, 2H), 3.08 - 2.65 (m, 1H), 1.84 - 1.17 (m, 8H), 0.93 - 0.58 (m, 3H)

### Example 32: (2-ethylpiperidin-1-yl)(2-((2-(trifluoromethyl)phenethyl)amino)-pyridin-4-yl)methanone (E32)

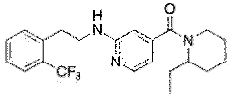

Prepared similarly as described for compound **E31** starting from **D3** (100 mg, 0.420 mmol) and 2-[2-(trifluoromethyl)phenyl]ethanamine (80.06 mg, 0.420 mmol). The crude was purified by RP Flash Chromatography (Sfar C18, 12 g, eluent: CH₃CN (+0.1% HCOOH) in H₂O (+0.1% HCOOH)) to afford **E32** (40.6 mg, 0.100 mmol, yield= 23.66%) as a yellow oil.

Method 2: R*t*= 1.48 min, MS (m/z) 406.3 [M+H]⁺.

1H NMR (500 MHz, DMSO-*d*₆) δ ppm 8.03 (d, J = 5.1 Hz, 1H), 7.69 (d, J = 7.8 Hz, 1H), 7.65 - 7.57 (m, 1H), 7.51 (d, J = 7.7 Hz, 1H), 7.43 (t, J = 7.6 Hz, 1H), 6.94 (br s, 1H), 6.38 (br d, J = 4.9 Hz, 1H), 6.35 (br s, 1H), 3.59 - 3.47 (m, 2H), 4.65 - 3.45 (m, 1H), 4.40 - 3.21 (m, 1H), 3.01 (br t, J = 7.3 Hz, 2H), 3.11 - 2.66 (m, 1H), 1.83 - 1.19 (m, 8H), 0.94 - 0.57 (m, 3H)

### Example 33: (2-ethylpiperidin-1-yl)(2-(((1-(2-methoxyphenyl)cyclopropyl)methyl)amino)pyridin-4-yl)methanone (E33)

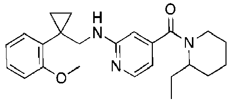

Prepared similarly as described for compound **E31** (step A) starting from **D3** (100 mg, 0.420 mmol) and [1-(2-methoxyphenyl)cyclopropyl]methanamine (75.01 mg, 0.420 mmol). The crude was purified by SCX to give the title compound **E33** (41.5 mg, 0.105 mmol, yield= 24.92%) as a yellow oil.

Method 2: R*t*= 1.39 min, MS (m/z) 394.4 [M+H]⁺.

¹H NMR (400 MHz, DMSO-*d*₆) δ ppm 7.90 (d, J=5.0 Hz, 1 H), 7.22 (dd, J=7.5, 1.3 Hz, 1 H), 7.14 - 7.21 (m, 1 H), 6.93 (d, J=8.1 Hz, 1 H), 6.82 (t, J=7.3 Hz, 1 H), 6.45 (br s, 1 H), 6.31 - 6.39 (m, 1 H), 6.28 (br d, J=4.8 Hz, 1 H), 3.83 (s, 3 H), 3.42 (d, J=5.7 Hz, 2 H), 3.38 - 4.63 (m, 1 H), 3.19 - 4.42 (m, 1 H), 2.62 - 3.04 (m, 1 H), 1.18 - 1.82 (m, 8 H), 0.51 - 0.94 (m, 7 H)

### Example 34: (2-ethylpiperidin-1-yl)(2-((2-(pyridin-4-yl)ethyl)amino)pyridin-4-yl)methanone; hydrochloride (E34)

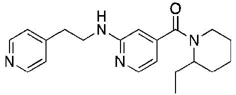

### Step A:

Prepared similarly as described for compound **E30** starting from **D3** (100 mg, 0.420 mmol) and 2-pyridin-4-ylethanamine (0.05 mL, 0.420 mmol). The crude was purified by RP Flash Chromatography (Sfar C18, 12 g, 0-50% CH₃CN in H₂O (+ 0.1% NH₄HCO₃, pH = 11)) to afford (2-ethylpiperidin-1-yl)(2-((2-(pyridin-4-yl)ethyl)amino)pyridin-4-yl)methanone (15.4 mg, 0.046 mmol, yield= 10.75%) as a colourless oil.

### Step B:

A stirred solution of (2-ethylpiperidin-1-yl)(2-((2-(pyridin-4-yl)ethyl)amino) pyridin-4-yl)methanone (14.4 mg, 0.040 mmol) in MeOH (851 µL), was treated with HCl (1.25M in MeOH) (64 µL, 0.060 mmol). The mixture was stirred for 2h, then concentrated under vacuum, stripped three times with water and freeze-dried to afford **E34** (15.4 mg, 0.041 mmol, yield= 96.54%) as an eloquent yellow solid.

Method 2: Rt= 0.54 min, MS (m/z) 339.3 [M+H]⁺.

¹H NMR (400 MHz, DMSO-*d*₆) δ ppm 8.77 (d, J = 6.0 Hz, 2H), 8.00 (d, J = 6.0 Hz, 1H), 7.87 (br d, J = 4.5 Hz, 2H), 9.05 - 7.42 (m, 2H), 6.77 (br s, 1H), 6.67 (br s, 1H), 3.73 (br s, 2H), 4.63 - 3.19 (m, 2H), 3.14 (br t, J = 6.9 Hz, 2H), 3.10 - 2.69 (m, 1H), 1.85 - 1.25 (m, 8H), 0.95 - 0.61 (m, 3H)

### Example 35: (2-ethylpiperidin-1-yl)(2-((2-(pyridin-3-yl)ethyl)amino)pyridin-4-yl)methanone; hydrochloride (E35)

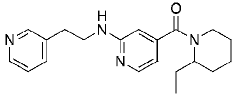

### Step A:

Prepared similarly as described for compound **E30** starting from **D3** (100 mg, 0.420 mmol) and 2-(3-pyridyl)ethanamine (50 µL, 0.420 mmol). The crude was purified by RP Flash Chromatography (Sfar C18, 12 g, eluent: CH₃CN (+0.1% HCOOH) in H₂O (+0.1% HCOOH)) to afford (2-ethylpiperidin-1-yl)(2-((2-(pyridin-3-yl)ethyl)amino)pyridin-4-yl)methanone (28.2 mg, 0.083 mmol, yield= 19.69%) as a dark red oil.

### Step B:

A stirred solution of (2-ethylpiperidin-1-yl)(2-((2-(pyridin-3-yl)ethyl)amino) pyridin-4-yl)methanone (28.2 mg, 0.080 mmol) in MeOH (0.833 mL) was treated with HCl (1.25M in MeOH) (108 µL, 0.110 mmol). The mixture was stirred for 2h, then concentrated under vacuum, stripped three times with water and freeze-dried to give **E35** (29.3 mg, 0.078 mmol, yield= 93.8%) as a brown solid. Method 2: Rt= 0.56 min, MS (m/z) 339.3 [M+H]⁺.

¹H NMR (400 MHz, DMSO-*d*₆) δ ppm 8.82 - 8.91 (m, 1 H), 8.65 - 8.76 (m, 1 H), 8.30 - 8.44 (m, 1 H), 7.94 - 8.05 (m, 1 H), 7.74 - 7.91 (m, 1 H), 7.54 - 9.18 (m, 1 H), 6.82 - 6.97 (m, 1 H), 6.63 - 6.77 (m, 1 H), 3.72 (br s, 2 H), 3.34 - 4.76 (m, 1 H), 3.19 - 4.37 (m, 1 H), 3.04 - 3.14 (m, 2 H), 2.69 - 3.16 (m, 1 H), 1.18 - 1.87 (m, 8 H), 0.58 - 1.00 (m, 3 H)

### Example 36: (2-((2-methoxyphenethyl)amino)pyridin-4-yl)(2-phenylazetidin-1-yl)methanone (E36)

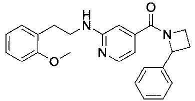

Prepared similarly as described for compound **E3** starting from **D2** (200 mg, 0.730 mmol) and 2-phenylazetidine (117 mg, 0.880 mmol). The reaction was stirred at RT, ON. After work-up the crude was purified twice by RP Flash Chromatography (Sfar C18, 12 g, 2-98% CH₃CN (+0.1 % HCOOH) in H₂O (+0.1 % HCOOH)) to give **E36** (37 mg, 0.095 mmol, yield= 13%) as a white foam.

### Method 2: Rt= 1.23 min, MS (m/z) 388.3 [M+H]⁺.

¹H NMR (400 MHz, DMSO-*d*₆) δ ppm 8.11 - 7.71 (m, 1H), 7.46 - 7.21 (m, 5H), 7.22 - 7.17 (m, 1H), 7.16 - 7.04 (m, 1H), 6.96 (d, J = 8.0 Hz, 1H), 6.87 (td, J = 7.3, 0.8 Hz, 1H), 6.84 - 6.54 (m, 1H), 6.76 - 6.24 (m, 2H), 5.60 - 5.33 (m, 1H), 4.52 - 4.03 (m, 2H), 3.79 (s, 3H), 3.51 - 3.14 (m, 2H), 2.98 - 2.63 (m, 3H), 2.19 - 1.99 (m, 1H).

### Example 37: (2-((2-(3H-indol-7-yl)ethyl)amino)pyridin-4-yl)(2-ethylpiperidin-1-yl)methanone (E37)

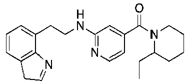

Prepared similarly as described for compound **E31** (step A) starting from **D3** (100 mg, 0.420 mmol) and 2-(1H-indol-7-yl)ethanamine (67.81 mg, 0.420 mmol). The crude was purified by RP Flash Chromatography (Sfar C18, 12 g, eluent: CH₃CN (+0.1 % HCOOH) in H₂O (+0.1 % HCOOH)) to afford the title compound **E37** (41 mg, 0.109 mmol, yield= 25.73%) as yellow oil.

Method 2: R*t*= 1.34 min, MS (m/z) 377.3 [M+H]⁺.

¹H NMR (500 MHz, DMSO-*d*₆) δ ppm 11.28 (br s, 1 H), 8.09 (d, J=5.8 Hz, 1 H), 7.38 - 7.41 (m, 1 H), 7.35 - 7.38 (m, 1 H), 6.88 - 6.96 (m, 2 H), 6.84 (br s, 1 H), 6.42 - 6.45 (m, 1 H), 6.34 - 6.41 (m, 2 H), 3.53 - 3.63 (m, 2 H), 3.47 - 4.67 (m, 1 H), 3.19 - 4.38 (m, 1 H), 3.09 (t, J=7.4 Hz, 2 H), 2.65 - 3.05 (m, 1 H), 1.20 - 1.82 (m, 8 H), 0.61 - 0.94 (m, 3 H).

### Example 38: (2-((2-(difluoromethoxy)phenethyl)amino)pyridin-4-yl)(2-ethylpiperidin-1-yl)methanone (E38)

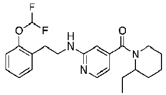

Prepared similarly as described for compound **E31** (step A) starting from **D3** (100 mg, 0.420 mmol) and 2-[2-(difluoromethoxy)phenyl]ethanamine (79.22 mg, 0.420 mmol). The crude was purified by SCX to give the title compound **E38** (N1344-20-1: 20.7 mg, 0.051 mmol, yield= 12.12%) as a yellow oil. Method 2: R*t*= 1.34 min, MS (m/z) 404.4 [M+H]⁺.

¹H NMR (500 MHz, DMSO-*d*₆) δ ppm 8.01 (d, J = 5.1 Hz, 1H), 7.32 (br d, J = 7.4 Hz, 1H), 7.31 - 7.26 (m, 1H), 7.18 - 7.14 (m, 2H), 7.19 (t, J = 74.4 Hz, 1H), 6.80 (br s, 1H), 6.35 (dd, J = 5.1, 1.0 Hz, 1H), 6.33 (s, 1H), 4.69 - 3.49 (m, 1H), 3.49 - 3.40 (m, 2H), 4.41 - 3.24 (m, 1H), 2.86 (t, J = 7.4 Hz, 2H), 3.09 - 2.65 (m, 1H), 1.83 - 1.45 (m, 2H), 1.71 - 1.15 (m, 6H), 0.96 - 0.56 (m, 3H)

### Example 39: (2-ethylpiperidin-1-yl)(2-((2-(piperidin-1-yl)ethyl)amino)pyridin-4-yl)methanone (E39)

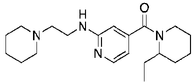

Prepared similarly as described for compound **E30** starting from **D3** (step A) (50 mg, 0.210 mmol) and 2-(1-piperidinyl)ethanamine (35.27 mg, 0.280 mmol). The crude was purified by RP Flash Chromatography (Sfar C18, 12 g, eluent: CH₃CN (+0.1% HCOOH) in H₂O (+0.1% HCOOH)) to afford the title compound E39 (36 mg, 0.105 mmol, yield= 49.39%) as orange oil. Method 2: Rt= 0.75 min, MS (ESI) m/z = 345.4 [M+H]⁺.

¹H NMR (500 MHz, DMSO-*d*₆) δ ppm 7.99 (d, J=4.9 Hz, 1 H), 6.54 (br s, 1 H), 6.37 (s, 1 H), 6.35 (d, J=5.2 Hz, 1 H), 3.46 - 4.74 (m, 1 H), 3.31 - 3.41 (m, 2 H), 3.11 - 4.37 (m, 1 H), 2.65 - 3.06 (m, 1 H), 2.46 (t, J=6.7 Hz, 2 H), 2.41 (br s, 4 H), 1.18 - 1.83 (m, 14 H), 0.61 - 0.92 (m, 3 H).

### Example 40: (2-((2-methoxyphenethyl)amino)pyridin-4-yl)(2-methylindolin-1 - yl)methanone; hydrochloride (E40)

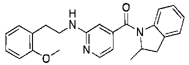

### Step A:

Prepared similarly as described for compound **E31** starting from **D6** (50 mg, 0.200 mmol) and 2-(2-methoxyphenyl)ethanamine (0.03 mL, 0.200 mmol). The reaction was stirred at 115 °C for 48 h, then was cooled to RT and NaOH (1M, 10 mL) was added. The mixture was extracted with DCM (4 x 10 mL). The organic layer was separated and concentrated under a high vacuum. The crude was purified by RP Flash Chromatography (Sfar C18, 12 g, eluent: CH₃CN (+0.1% HCOOH) in H₂O (+0.1% HCOOH)) to afford the title compound [(2-((2-methoxyphenethyl)amino)pyridin-4-yl)(2-methylindolin-1-yl)methanone (20 mg, 0.052 mmol, yield= 26.46%) as a colourless oil. The reaction was repeated, and the two batches were combined.

### Step B:

To a stirred solution of [(2-((2-methoxyphenethyl)amino)pyridin-4-yl)(2-methylindolin-1-yl)methanone (30 mg, 0.080 mmol) in MeOH (4.633 mL), HCl (1M in DEE, 0.15 mL, 0.150 mmol) was added dropwise. The mixture was stirred for 3h, then concentrated under vacuum, stripped three times with water and freeze-dried to give **E40** (27 mg, 0.064 mmol, yield= 82.26%) as red solid.

Method 2: R*t*= 1.44 min, MS (ESI) m/z = 388.33 [M+H]⁺.

¹H NMR (400 MHz, DMSO-*d*₆) δ ppm 8.06 (br d, J=5.8 Hz, 1 H), 8.09 (br s, 1 H), 7.33 (br d, J=7.5 Hz, 1 H), 6.98 (d, J=8.0 Hz, 1 H), 6.89 (br t, J=7.4 Hz, 2 H), 6.75 - 7.27 (m, 7 H), 4.47 (br s, 1 H), 3.76 (s, 3 H), 3.38 - 3.64 (m, 3 H), 2.87 (br t, J=6.9 Hz, 2 H), 2.60 - 2.73 (m, 1 H), 0.90 - 1.34 (m, 3 H).

### Example 41: (S)-(2-((2-methoxyphenethyl)amino)pyridin-4-yl)(2-methylindolin-1-yl)methanone (E41)

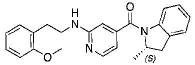

Prepared similarly as described for compound **E1** (step A) starting from **D2** (92.48 mg, 0.340 mmol) and (2S)-2-methyl-2,3-dihydro-1H-indole (45.26 mg, 0.340 mmol). The crude was purified by RP Flash Chromatography (Sfar C18, 12 g, eluent: CH₃CN (+0.1% HCOOH) in H₂O (+0.1% HCOOH)) to obtain **E41** (12 mg, 0.031 mmol, yield= 9.114%) as orange solid.

Method 2: R*t*= 1.43 min, MS (ESI) m/z = 388.33 [M+H]⁺.

¹H NMR (500 MHz, DMSO-*d*₆) δ ppm 8.08 (d, J=5.1 Hz, 1 H), 8.03 (br s, 1 H), 7.30 (br d, J=7.3 Hz, 1 H), 7.17 - 7.22 (m, 1 H), 7.14 (br d, J=6.7 Hz, 1 H), 7.01 - 7.22 (m, 2 H), 6.96 (d, J=8.1 Hz, 1 H), 6.86 (t, J=7.3 Hz, 1 H), 6.81 (t, J=5.6 Hz, 1 H), 6.49 - 6.63 (m, 2 H), 4.54 (br s, 1 H), 3.77 (s, 3 H), 3.38 - 3.46 (m, 3 H), 2.81 (br t, J=7.4 Hz, 2 H), 2.60 - 2.67 (m, 1 H), 1.09 (br s, 3 H).

### Example 42: (S)-(2-ethylpiperidin-1-yl)(2-((2-(trifluoromethoxy)phenethyl)-amino)pyridin-4-yl)methanone (E42)

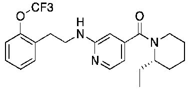

A mixture of 2-[2-(trifluoromethoxy)phenyl]ethanamine (130.25 mg, 0.630 mmol), **D7** (150 mg, 0.630 mmol) and HCl (2M in DEE, 0.32 mL, 0.630 mmol) was heated to 130 °C in DMSO (2.1mL). After 1h, DIPEA (0.11 mL, 0.630 mmol) was added, and the solution was heated at the same temperature ON. Ammonium chloride saturated solution (5 mL) was added, and the mixture was extracted with DCM (4 x 10 mL). The mixture was separated by a phase separator, and the organic layer was concentrated under a high vacuum. The crude was purified by RP Flash Chromatography (Sfar C18, 12 g, eluent: CH₃CN (+0.1% HCOOH) in H₂O (+0.1% HCOOH)) to obtain **E42** (254 mg, 0.603 mmol, yield= 94.94%) as yellow oil.

Method 2: R*t*= 1.50 min, MS (ESI) m/z = 422.22 [M+H]⁺.

¹H NMR (500 MHz, DMSO-*d*₆) δ ppm 8.01 (d, J=5.1 Hz, 1 H), 7.43 (dd, J=6.9, 2.1 Hz, 1 H), 7.29 - 7.38 (m, 3 H), 6.84 (br t, J=5.0 Hz, 1 H), 6.36 (dd, J=5.2, 1.1 Hz, 1 H), 6.33 (s, 1 H), 3.45 - 3.52 (m, 2 H), 3.43 - 4.64 (m, 1 H), 3.23 - 4.37 (m, 1 H), 2.91 (t, J=7.3 Hz, 2 H), 2.60 - 3.06 (m, 1 H), 1.69 - 1.81 (m, 1 H), 1.20 - 1.68 (m, 7 H), 0.57 - 0.96 (m, 3 H).

### Example 43: (S)-(2-ethylpiperidin-1-yl)(2-((2-(4-methoxypyridin-3-yl)ethyl)amino)pyridin-4-yl)methanone (E43)

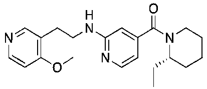

Prepared similarly as described for compound **E30** starting from **D7** (100 mg, 0.420 mmol) and 2-(4-methoxy-3-pyridyl)ethanamine;hydrochloride (79.84 mg, 0.420 mmol). The crude was purified by RP Flash Chromatography (Sfar C18, 12 g, eluent: CH₃CN (+0.1 % HCOOH) in H₂O (+0.1% HCOOH)) to obtain **E43** (8 mg, 0.022 mmol, yield= 5.13%) as a dark yellow oil.

Method 2: Rt*=* 0.46 min, MS (ESI) m/z = 369.28 [M+H]⁺.

¹H NMR (400 MHz, DMSO-*d*₆) δ ppm 8.32 (d, J = 5.7 Hz, 1H), 8.19 (s, 1H), 8.00 (d, J = 5.0 Hz, 1H), 7.00 (d, J = 5.7 Hz, 1H), 6.75 (br s, 1H), 6.39 - 6.30 (m, 2H), 3.85 (s, 3H), 4.66 - 3.47 (m, 1H), 3.43 (q, J = 6.6 Hz, 2H), 4.41 - 3.15 (m, 1H), 2.79 (t, J = 7.1 Hz, 2H), 3.09 - 2.60 (m, 1H), 1.83 - 1.19 (m, 8H), 0.96 - 0.57 (m, 3H).

### Example 44: (6,6-difluoro-3-azabicyclo[3.1.0]hexan-3-yl)(2-((2-(trifluoromethoxy)phenethyl)amino)pyridin-4-yl)methanone (E44)

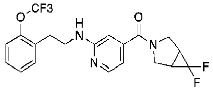

Prepared similarly as described for compound **E42** starting from **D8** (100 mg, 0.410 mmol) and 2-[2-(trifluoromethoxy)phenyl]ethanamine (84.72 mg, 0.410 mmol). The crude was purified by RP Flash Chromatography (Sfar C18, 12 g, eluent: CH₃CN (+0.1% HCOOH) in H₂O (+0.1% HCOOH)) to obtain E44 (32 mg, 0.075 mmol, yield= 18.14%).

¹H NMR (400 MHz, DMSO-*d*₆) δ ppm 8.04 (d, J=5.2 Hz, 1 H), 7.29 - 7.49 (m, 4 H), 6.89 (t, J=5.7 Hz, 1 H), 6.44 (dd, J=5.2, 1.2 Hz, 1 H), 6.40 (s, 1 H), 4.03 (d, J=12.6 Hz, 1 H), 3.78 (dt, J=11.2, 4.6 Hz, 1 H), 3.66 (dt, J=12.6, 4.6 Hz, 1 H), 3.55 (d, J=11.3 Hz, 1 H), 3.41 - 3.52 (m, 2 H), 2.90 (t, J=7.3 Hz, 2 H), 2.50 - 2.63 (m, 2 H).

### Example 45: (2-((2-(1H-indol-3-yl)ethyl)amino)pyridin-4-yl)(6,6-difluoro-3-azabicyclo[3.1.0]hexan-3-yl)methanone (E45)

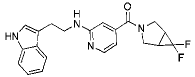

Prepared similarly as described for compound E42 starting from **D8** (93 mg, 0.380 mmol) and 2-(1H-indol-3-yl)ethanamine (61.52 mg, 0.380 mmol). The crude was purified by RP Flash Chromatography (Sfar C18, 12 g, eluent: CH₃CN (+0.1% HCOOH) in H₂O (+0.1% HCOOH)) to obtain **E45** (39 mg, 0.102 mmol, yield= 26.56%).

Method 2: Rt= 0.94 min, MS (ESI) m/z = 383.30 [M+H]⁺.

¹H NMR (400 MHz, DMSO-*d*₆) δ ppm 10.80 (br s, 1 H), 8.05 (d, J=5.9 Hz, 1 H), 7.55 (d, J=7.9 Hz, 1 H), 7.33 (d, J=8.1 Hz, 1 H), 7.17 (d, J=2.0 Hz, 1 H), 7.06 (t, J=7.2 Hz, 1 H), 6.93 - 7.01 (m, 1 H), 6.81 (t, J=5.6 Hz, 1 H), 6.36 - 6.48 (m, 2 H), 4.04 (br d, J=12.7 Hz, 1 H), 3.74 - 3.82 (m, 1 H), 3.66 (dt, J=12.6, 4.6 Hz, 1 H), 3.49 - 3.60 (m, 3 H), 2.94 (t, J=7.3 Hz, 2 H), 2.51 - 2.65 (m, 2 H)

### Example 46: (6,6-difluoro-3-azabicyclo[3.1.0]hexan-3-yl)(2-((2-methoxy-6-(methoxymethoxy)phenethyl)amino)pyridin-4-yl)methanone (E46)

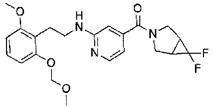

Prepared similarly as described for compound **E8** starting from **D52** (120 mg, 0.360 mmol) and 6,6-difluoro-3-azabicyclo[3.1.0]hexane;hydrochloride (67.41 mg, 0.430 mmol) in DMF (1 mL): The reaction was stirred at 60 °C for 12h. After work-up, the crude was purified by RP Flash Chromatography (Sfar C18, 12 g, eluent: CH₃CN (+0.1% HCOOH) in H₂O (+0.1% HCOOH)) to obtain **E46** (70 mg, 0.161 mmol, yield= 44.73%) as solid

Method 2: R*t*= 1.12 min, MS (ESI) m/z =434.2 [M+H]⁺.

¹H NMR (400 MHz, DMSO-*d*₆) δ ppm 8.01 (d, J=5.0 Hz, 1 H), 7.13 (t, J=8.3 Hz, 1 H), 6.76 (t, J=5.6 Hz, 1 H), 6.71 (d, J=8.3 Hz, 1 H), 6.67 (d, J=8.3 Hz, 1 H), 6.46 (s, 1 H), 6.43 (d, J=5.2 Hz, 1 H), 5.17 (s, 2 H), 3.76 (s, 3 H), 3.63 - 3.84 (m, 2 H), 3.52 - 4.09 (m, 2 H), 3.37 (s, 3 H), 3.24 - 3.30 (m, 2 H), 2.85 (br t, J=7.7 Hz, 2 H), 2.49 - 2.65 (m, 2 H)

### Example 47: (6,6-difluoro-3-azabicyclo[3.1.0]hexan-3-yl)(2-((2-hydroxy-6-methoxyphenethyl)amino)pyridin-4-yl)methanone (E47)

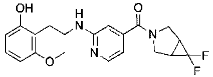

To a solution of **E46** (70 mg, 0.160 mmol) in MeOH (0.323 mL), HCl (1M, 16.36 mg, 0.160 mmol) was added. The reaction was stirred at RT. After 30 min, the mixture was basified with NaOH (1M) and extracted with DCM (3x). The crude was purified by RP Flash Chromatography (Sfar C18, 6 g, eluent: CH₃CN (+0.1% HCOOH) in H₂O (+0.1% HCOOH)) and freeze-dried to obtain **E47** (65 mg,0.162 mmol, yield= 67.92%) as a white solid. Method 2: R*t*= 0.92 min, MS (ESI) m/z =390.2 [M+H]⁺.

¹H NMR (400 MHz, DMSO-*d*₆) δ ppm 9.45 (br s, 1 H), 8.01 (d, J=5.0 Hz, 1 H), 6.97 (t, J=8.2 Hz, 1 H), 6.75 (t, J=5.5 Hz, 1 H), 6.37 - 6.50 (m, 4 H), 4.05 (d, J=12.7 Hz, 1 H), 3.76 - 3.87 (m, 1 H), 3.72 (s, 3 H), 3.67 (dt, J=12.6, 4.5 Hz, 1 H), 3.56 (d, J=11.4 Hz, 1 H), 3.18 - 3.28 (m, 2 H), 2.74 - 2.84 (m, 2 H), 2.51 - 2.66 (m, 2 H)

### Example 48: rac-(6,6-difluoro-3-azabicyclo[3.1.0]hexan-3-yl)(2-((2-(2-methoxyphenyl)propyl)amino)pyridin-4-yl)methanone (E48)

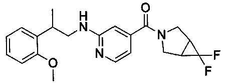

Prepared similarly as described for compound **E3** starting from **D12** (62 mg, 0.220 mmol) and 6,6-difluoro-3-azabicyclo[3.1.0]hexane (31 mg, 0.260 mmol). The crude was purified by RP Flash Chromatography (Sfar C18, 12 g, eluent: CH₃CN (+0.1% HCOOH) in H₂O (+0.1% HCOOH)) to afford **E48** (11 mg, 0.028 mmol, yield= 13.11%) as a yellow oil.

Method 2: R*t*= 1.12 min, MS (ESI) m/z = 388.2 [M+H]⁺. ¹H NMR (400 MHz, DMSO-*d*₆) δ ppm 8.00 (d, J=5.0 Hz, 1 H), 7.14 - 7.25 (m, 2 H), 6.95 (d, J=8.1 Hz, 1 H), 6.91 (t, J=7.5 Hz, 1 H), 6.67 - 6.75 (m, 1 H), 6.44 (s, 1 H), 6.41 (d, J=5.0 Hz, 1 H), 4.03 (d, J=12.8 Hz, 1 H), 3.76 - 3.85 (m, 1 H), 3.76 (s, 3 H), 3.66 (dt, J=12.7, 4.5 Hz, 1 H), 3.54 (d, J=11.2 Hz, 1 H), 3.34 - 3.45 (m, 3 H), 2.52 - 2.64 (m, 2 H), 1.18 (br d, J=6.1 Hz, 3 H).

### Example 49: (6,6-difluoro-3-azabicyclo[3.1.0]hexan-3-yl)(2-((3-methoxyphenethyl)amino)pyridin-4-yl)methanone; hydrochloride (E49)

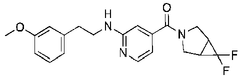

In pressure reactor, D8 (100 mg, 0.392 mmol, 1.0 eq), 2-(3-methoxyphenyl)ethylamine (119 mg, 0.784 mmol, 2.0 eq) and DIPEA (410 µL, 2.353 mmol, 6.0 eq) were dissolved in anh NMP (2.61 mL, 0.15 M). The reactor was sealed and purged with Argon for 10 minutes. Then it was placed in an oil bath for 18 hours at 180°C. The reaction mixture was diluted with water and EtOAc (x3). The organic phase was dried over anh MgSO₄, filtered and concentrated under vacuum. The crude was dissolved with methyl tert-butyl ether and washed with ice-cold water. The organic phase was dried over anh MgSO₄, filtered and concentrated under reduced pressure. The crude was purified by NP Flash chromatography (0-3% MeOH in DCM). The pure fraction was treated with HCl (2M in DEE) to obtain solid material. The latter was washed with diethyl ether (3x) to obtain E49 as a white fine powder (0.075 g, 0.196 mmol, yield= 50%).

Method 5: Rt= 2.08 min, MS (ESI) m/z = 374.2 [M+H]⁺.

¹H NMR (300 MHz, DMSO-*d*₆) δ ppm 8.68 (s, 1H), 7.98 (d, J = 6.2 Hz, 1H), 7.22 (t, J = 7.8 Hz, 1H), 6.98 - 6.84 (m, 3H), 6.79 (dd, J = 8.1, 2.6 Hz, 1H), 6.74 - 6.67 (m, 1H), 4.04 (d, J = 12.8 Hz, 1H), 3.82 (dt, J = 11.2, 4.2 Hz, 1H), 3.75 (s, 3H), 3.62 (q, J = 12.9, 11.5 Hz, 4H), 2.88 (t, J = 7.3 Hz, 2H), 2.63 (dt, J = 12.3, 4.1 Hz, 2H).

### Example 50: (2-((2-(benzofuran-7-yl)ethyl)amino)pyridin-4-yl)(6,6-difluoro-3-azabicyclo[3.1.0]hexan-3-yl)methanone; hydrochloride (E50)

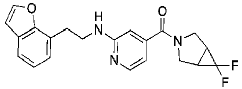

Prepared similarly as described for compound **E49** starting from **D8** (120 mg, 0.4707 mmol, 1.0 eq) and **D14** (95 mg, 0.4707 mmol, 1.0 eq). The crude material obtained after work-up, was purified by RP Flash Chromatography. The fractions containing the product were concentrated and added a few drops of conc HCl and freeze-dried to yield **E50** as a brown powder. (106 Mg, 2.52 mmol, yield= 57%).

Method 5: Rt= 2.26 min, MS (ESI) m/z = 384.2[M+H]⁺.

¹H NMR (400 MHz, DMSO-*d*₆) 8.96 (br. s,1 H), 8.05 - 7.96 (m, 2 H), 7.55 (dd, J = 7.7, 1.3 Hz, 1 H ), 7.29 (dd, J = 7.4, 1.4 Hz, 1 H,), 7.21 (t, J = 7.5 Hz, 1 H ), 6.97 (d, J = 2.2 Hz, 2 H,), 6.77 (dd, J = 6.4, 1.5 Hz, 1 H,), 4.04 (d, J = 12.8 Hz, 1 H), 3.84 - 3.68 (m, 4 H), 3.57 (d, J =11.2 Hz, 2 H), 3.22 (t, ,J = 7.2 Hz, 2 H), 2.68 - 2.59 (m, 2 H).

### Example 51: (6,6-difluoro-3-azabicyclo[3.1.0]hexan-3-yl)(2-((2-(2,3-dihydrobenzofuran-7-yl)ethyl)amino)pyridin-4-yl)methanone (E51)

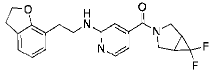

Prepared similarly as described for compound **E49** starting from **D8** (0.05 g, 0.196 mmol, 1.0 eq) and **D16** (0,05 g, 0.195 mmol, 1.5 eq). The crude material, obtained after work-up, was purified by RP Preparative HPLC (C18 column, eluent: MeCN (+0.05% NH₃) in H₂O (+0.05% NH₃), basic conditions) to yield **E51** (30 mg, 0.078 mmol, yield= 40%) was obtained as a white powder.

Method 5: R*t*= 2.14 min, MS (ESI) m/z = 386.42[M+H]⁺.

¹H NMR (300 MHz, DMSO-*d*₆) δ ppm 8.04 (dd, J = 5.1, 0.8 Hz, 1H), 7.08 (dd, J = 7.2, 1.3 Hz, 1H), 6.99 - 6.92 (m, 1H), 6.85 - 6.71 (m, 2H), 6.48 - 6.36 (m, 2H), 4.52 (t, J = 8.7 Hz, 2H), 4.04 (d, J = 12.7 Hz, 1H), 3.80 (dt, J = 11.4, 4.6 Hz, 1H), 3.67 (dt, J = 12.7, 4.6 Hz, 1H), 3.56 (d, J = 11.4 Hz, 1H), 3.44 (dt, J = 8.1, 6.0 Hz, 2H), 3.17 (t, J = 8.7 Hz, 2H), 2.75 (dd, J = 8.4, 6.3 Hz, 2H), 2.58 (dt, J = 9.1, 4.7 Hz, 2H).

### Example 52: (2-((2-cyclopropoxyphenethyl)amino)pyridin-4-yl)(6,6-difluoro-3-azabicyclo[3.1.0]hexan-3-yl)methanone hydrochloride (E52)

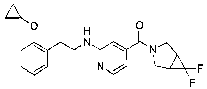

Prepared similarly as described for compound **E49** starting from **D8** (130 mg, 4.251 mmol, 1.0 eq) and **D19** (120 mg, 4.251 mmol, 1.19 eq). After the work-up, the crude was purified by RP preparative RP Preparative HPLC. The pure fractions containing the desired product were concentrated, acidified by HCl_{aq} and freeze-dried to yield **E52** hydrochloride salt (0.07 g, 0.175 mmol, yield= 34%).

Method 5: R*t*= 2.42 min, MS (ESI) m/z =400.2 [M+H]⁺.

¹H NMR (300 MHz, DMSO-*d*₆) δ ppm 8.96 (s, 1H), 7.99 (d, J = 6.5 Hz, 1H), 7.31-7.21 (m, 3H), 6.97 (s, 1H), 6.93 (td, J = 6.8, 2.2 Hz, 1H), 6.79 (dd, J = 6.4, 1.5 Hz, 1H), 4.05 (d, J = 12.8 Hz, 1H), 3.83 (tq, J = 6.1, 3.5, 2.8 Hz, 2H), 3.76-3.49 (m, 4H), 2.83 (t, J = 7.2 Hz, 2H), 2.64 (dd, J = 12.3, 4.4 Hz, 2H), 0.82-0.73 (m, 2H), 0.63-0.54 (m, 2H).

### Example 53: (2-((3-methoxyphenethyl)amino)pyridin-4-yl)(piperidin-1-yl)methanone (E53)

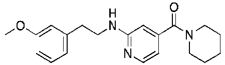

Prepared similarly as described for compound **E1** starting from **D21** (60 mg, 0.220 mmol) and piperidine (20 µL, 0.220 mmol) to obtain
**E53** (51.6 mg, 0.152 mmol, yield= 68.99%) as a pale brown oil.

Method 2: R*t*= 0.99 min, MS (m/z) 340.3 [M+H]⁺.

¹H NMR (500 MHz, CHLOROFORM-d) δ ppm 8.06 (d, J=5.2 Hz, 1 H), 7.21 - 7.26 (m, 1 H), 6.83 (d, J=7.5 Hz, 1 H), 6.73 - 6.81 (m, 2 H), 6.53 (dd, J=5.3, 0.9 Hz, 1 H), 6.37 (s, 1 H), 5.30 (br s, 1 H), 3.81 (s, 3 H), 3.65 - 3.74 (m, 2 H), 3.57 (q, J=6.7 Hz, 2 H), 3.25 - 3.34 (m, 2 H), 2.92 (t, J=6.9 Hz, 2 H), 1.42 - 1.74 (m, 6 H)

### Example 54: (2-ethylpiperidin-1-yl)(2-((3-methoxyphenethyl)amino)pyridin-4-yl)methanone; hydrochloride (E54)

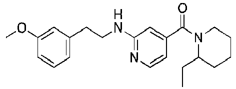

### Step A:

Prepared similarly as described for compound **E1** starting from **D21** (60 mg, 0.220 mmol) and 2-ethylpiperidine hydrochloride (32.98 mg, 0.220 mmol). The crude product was later purified by Sfar C18 5 g (5-50% MeCN 0.1%HCOOH in H₂O) to afford (2-ethylpiperidin-1-yl)(2-((3-methoxyphenethyl)amino)pyridin-4-yl)methanone (15.1 mg, 0.041 mmol, yield= 18.65%) as a colorless oil.

### Step B:

A stirred solution of (2-ethylpiperidin-1-yl)(2-((3-methoxyphenethyl)amino) pyridin-4-yl)methanone (15.1 mg, 0.040 mmol) in MeOH (0.996 mL), Hydrogen chloride (1.25 M in MeOH, 51 µL, 0.050 mmol) was dropwise added. The mixture was stirred for 2h, then concentrated under vacuum, stripped three times with water and then freeze-dried to give **E54** (15.7 mg, 0.039 mmol, yield= 99.56%) as a brown solid.

Method 2: R*t*=1.23 min, MS (m/z) 368.3 [M+H]⁺.

¹H NMR (400 MHz, DMSO-*d*₆) δ ppm 11.67 - 14.54 (m, 1 H), 7.88 - 8.01 (m, 1 H), 7.33 - 9.28 (m, 1 H), 7.16 - 7.26 (m, 1 H), 6.84 - 6.91 (m, 2 H), 6.75 - 6.84 (m, 2 H), 6.60 - 6.72 (m, 1 H), 3.74 (s, 3 H), 3.53 - 3.66 (m, 2 H), 3.41 - 4.61 (m, 1 H), 3.17 - 4.38 (m, 1 H), 2.87 (t, J=7.2 Hz, 2 H), 2.69 - 3.13 (m, 1 H), 1.27 - 1.86 (m, 8 H), 0.62 - 0.93 (m, 3 H)

### Example 55: (6,6-difluoro-3-azabicyclo[3.1.0]hexan-3-yl)(2-((2-(2-methylthiazol-5-yl)ethyl)amino)pyridin-4-yl)methanone (E55)

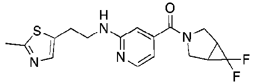

Prepared similarly as described for compound **E42** starting from **D8**(50 mg, 0.210 mmol) and 2-(2-methylthiazol-5-yl)ethanamine (32.3 mg, 0.230 mmol). After work-up, the crude was purified by RP Flash Chromatography (Sfar C18, 12 g, eluent: CH₃CN (+0.1% HCOOH) in H₂O (+0.1% HCOOH)) to give **E55** (25.7 mg, 0.071 mmol, yield= 34.16%) as a yellow oil. Method 2: R*t*= 0.65 min, MS (ESI) m/z = 365.21 [M+H]⁺.

¹H NMR (500 MHz, DMSO-*d*₆) δ ppm 8.04 (d, J=5.2 Hz, 1 H), 7.38 (s, 1 H), 6.90 (t, J=5.6 Hz, 1 H), 6.45 (dd, J=5.1, 1.1 Hz, 1 H), 6.43 (s, 1 H), 4.03 (d, J=12.8 Hz, 1 H), 3.79 (dt, J=11.2, 4.6 Hz, 1 H), 3.66 (dt, J=12.6, 4.6 Hz, 1 H), 3.55 (d, J=11.4 Hz, 1 H), 3.48 (q, J=6.6 Hz, 2 H), 3.01 (t, J=6.9 Hz, 2 H), 2.58 (s, 3 H), 2.51 - 2.62 (m, 2 H)

### Example 56: (6,6-difluoro-3-azabicyclo[3.1.0]hexan-3-yl)(2-((2-fluoro-6-methoxyphenethyl)amino)pyridin-4-yl)methanone (E56)

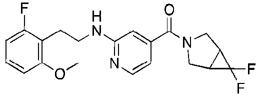

Prepared similarly as described for compound **E8** starting from **D56** (43 mg, 0.150 mmol) and 6,6-difluoro-3-azabicyclo[3.1.0]hexane;hydrochloride (25.42 mg, 0.150 mmol) at RT, ON. After work-up and purification in RP Flash Chromatography (silica C18 12 g, 1-50% CH₃CN (+0.1% HCOOH) in H₂O (+0.1% HCOOH)) appropriate fraction were collected and then evaporated in vacuum to give **E56** ( 15.3 mg, 0.039 mmol, yield= 26.39%) as pale yellow oil.

Method 2: R*t*= 1.10 min, MS (ESI) m/z = 392.17 [M+H]⁺.

¹H NMR (400 MHz, DMSO-*d*₆) δ ppm 8.02 (d, J=5.0 Hz, 1 H), 7.14 - 7.30 (m, 1 H), 6.79 - 6.88 (m, 2 H), 6.76 (t, J=8.8 Hz, 1 H), 6.43 (dd, J=5.2, 1.2 Hz, 1 H), 6.40 (s, 1 H), 4.04 (d, J=12.7 Hz, 1 H), 3.80 (s, 3 H), 3.75 - 3.82 (m, 1 H), 3.66 (dt, J=12.7, 4.6 Hz, 1 H), 3.55 (d, J=11.4 Hz, 1 H), 3.33 - 3.40 (m, 2 H), 2.83 (br t, J=7.3 Hz, 2 H), 2.52 - 2.64 (m, 2 H).

### Example 57: (2-((3-chloro-2-fluoro-6-methoxyphenethyl)amino)pyridin-4-yl)(6,6-difluoro-3-azabicyclo[3.1.0]hexan-3-yl)methanone (E57)

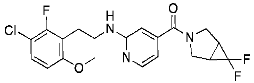

A mixture of **D57** (16.72 mg, 0.050 mmol) in anh DMSO (1 mL) was treated with CDI (9.18 mg, 0.060 mmol). The reaction mixture was heated to 60 °C and stirred for 45 minutes and 6,6-difluoro-3-azabicyclo[3.1.0]hexane; hydrochloride (8.81 mg, 0.060 mmol)was added. The reaction mixture was additionally stirred at 60 °C for further 1 h. After cooling, 1N NaOH (1 mL) was added and the reaction mixture was extracted with diethyl ether (3x). The combined organic layers were washed with brine (3x), dried over Na₂SO₄, filtered and evaporated. The residue was purified by NP Flash Chromatography (silica gel column, Sfar silica D, 5 g; 30-100% EtOAc in cyclohexane) to provide **E57** (10.1 mg, 0.024 mmol, yield= 46.06%) as white solid.

Method 2: R*t*= 1.26 min, MS (m/z)= 426.4 [M+H]⁺.

¹H NMR (400 MHz, DMSO-*d*₆) δ ppm 8.02 (d, J=5.2 Hz, 1 H), 7.38 (t, J=8.8 Hz, 1 H), 6.87 (dd, J=9.0, 1.1 Hz, 1 H), 6.83 (t, J=6.0 Hz, 1 H), 6.43 (dd, J=5.1, 1.3 Hz, 1 H), 6.39 (s, 1 H), 4.04 (d, J=12.7 Hz, 1 H), 3.79 (s, 3 H), 3.72 - 3.84 (m, 1 H), 3.66 (dt, J=12.6, 4.6 Hz, 1 H), 3.55 (d, J=11.4 Hz, 1 H), 3.36 - 3.45 (m, 2 H), 2.86 (br t, J=6.7 Hz, 2 H), 2.51 - 2.65 (m, 2 H)

### Example 58: (6,6-difluoro-3-azabicyclo[3.1.0]hexan-3-yl)(2-((2-methoxy-5-(trifluoromethoxy)phenethyl)amino)pyridin-4-yl)methanone (E58)

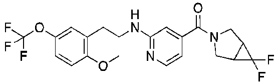

Prepared similarly as described for compound **E57** starting from **D58** and 6,6-difluoro-3-azabicyclo[3.1.0]hexane;hydrochloride (30 mg, 0.190 mmol). After work-up the crude residue was purified by NP Flash Chromatography (silica gel column, Sfar silica D, 5 g; 30-100% EtOAc in cyclohexane) to provide **E58** (56 mg, 0.122 mmol, yield= 69.84%) as white solid.

Method 2: R*t*= 1.40 min, MS (m/z)=458.5 [M+H]⁺.

¹H NMR (400 MHz, DMSO-*d*₆) δ ppm 8.02 (d, J=5.2 Hz, 1 H), 7.15 - 7.22 (m, 1 H), 7.13 (d, J=2.4 Hz, 1 H), 7.04 (d, J=9.0 Hz, 1 H), 6.78 (t, J=5.6 Hz, 1 H), 6.42 (dd, J=5.2, 1.2 Hz, 1 H), 6.40 (s, 1 H), 4.04 (d, J=12.7 Hz, 1 H), 3.81 (s, 3 H), 3.73 - 3.80 (m, 1 H), 3.62 - 3.70 (m, 1 H), 3.53 (d, J=11.3 Hz, 1 H), 3.37 - 3.49 (m, 2 H), 2.83 (t, J=7.2 Hz, 2 H), 2.50 - 2.66 (m, 2 H)

### Example 59: (2-((4,5-difluoro-2-methoxyphenethyl)amino)pyridin-4-yl)(6,6-difluoro-3-azabicyclo[3.1.0]hexan-3-yl)methanone (E59)

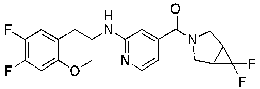

Prepared similarly as described for compound **E57** starting from **D59** (35.6 mg, 0.120 mmol) and 6,6-difluoro-3-azabicyclo[3.1.0]hexane;hydrochloride (19.8 mg, 0.130 mmol). After work-up, the crude residue was purified by NP Flash Chromatography (Sfar silica D, 6 g, 30-100% EtOAc in cyclohexane) to provide **E59** (35 mg, 0.085 mmol, yield= 74%) as white solid.

### Method 2: Rt= 1.10 min, MS (m/z)=410.4 [M+H]⁺.

¹H NMR (400 MHz, DMSO-*d*₆) δ ppm 8.02 (d, J=5.0 Hz, 1 H), 7.22 (dd, J=11.3, 9.4 Hz, 1 H), 7.09 (dd, J=12.9, 7.0 Hz, 1 H), 6.76 (t, J=5.6 Hz, 1 H), 6.43 (dd, J=5.2, 1.2 Hz, 1 H), 6.40 (s, 1 H), 4.04 (d, J=12.8 Hz, 1 H), 3.77 (s, 3 H), 3.75 - 3.84 (m, 1 H), 3.66 (dt, J=12.6, 4.6 Hz, 1 H), 3.54 (d, J=11.3 Hz, 1 H), 3.41 (q, J=6.5 Hz, 2 H), 2.76 (t, J=7.2 Hz, 2 H), 2.51 - 2.65 (m, 2 H)

### Example 60: (6,6-difluoro-3-azabicyclo[3.1.0]hexan-3-yl)(2-((2-(2,2-difluorobenzo[d][1,3]dioxol-4-yl)ethyl)amino)pyridin-4-yl)methanone (E60)

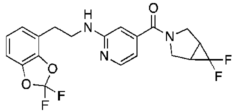

A suspension of Palladium (II); acetate (0.87 mg, 0 mmol) and [1-(2-diphenylphosphino-1-naphthalenyl)-2-naphthalenyl]-diphenylphosphine ( rac-BINAP) (120.4 mg, 0.190 mmol) in 1,2-dimethoxyethane (DME) (13.89 mL) was stirred at RT for 10 min. Then sodium tert-butylate (74.3 mg, 0.770 mmol) was added. The obtained dark pink solution was treated with **D60** (100 mg, 0.390 mmol) and 2-(2,2-difluoro-1,3-benzodioxol-4-yl)ethanamine (CAS n° 531508-47-1) (93.3 mg, 0.460 mmol). The reaction mixture was refluxed for 12h, quenched with water and extracted with DCM (x2). The organic layer was dried over Na₂SO₄, filtered and concentrated under high vacuum. The crude was purified by RP Flash Chromatography (silica C18 12 g, 1-40% CH₃CN (+0.1% HCOOH) in H₂O (+0.1% HCOOH)) to give **E60** (96 mg, 0.227 mmol, yield= 58.65%) as yellow oil.

Method 2: R*t*= 1.31 min, MS (m/z)= 424.16 [M+H]⁺.

¹H NMR (400 MHz, CDCl₃) δ ppm 8.13 (d, J = 5.1 Hz, 1H), 7.05-6.99 (m, 1H), 6.97-6.91 (m, 2H), 6.55 (dd, J = 5.1, 1.2 Hz, 1H), 6.38 (s, 1H), 4.88 (br s, 1H), 4.26 (d, J = 12.8 Hz, 1H), 3.84-3.76 (m, 1H), 3.75-3.69 (m, 2H), 3.68-3.60 (m, 2H), 3.00 (t, J = 6.9 Hz, 2H), 2.45-2.23 (m, 2H)

### Example 61: (6,6-difluoro-3-azabicyclo[3.1.0]hexan-3-yl)(2-((2-(3-methoxythiophen-2-yl)ethyl)amino)pyridin-4-yl)methanone (E61)

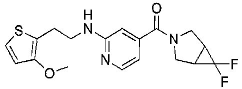

Prepared similarly as described for compound **E60** starting from **D60** (100 mg, 0.380 mmol) and **D62** (72.2 mg, 0.460 mmol). After work-up the crude was purified by RP Flash Chromatography (silica C18 12 g, 1-30% CH₃CN (+0.1% HCOOH) in H₂O (+0.1% HCOOH)) to give **E61** (20 mg, 0.053 mmol, yield= 13.77%) as yellow oil.

Method 2: R*t*= 0.97 min, MS (ESI) m/z = 380.19 [M+H]⁺.

¹H NMR (500 MHz, DMSO-*d*₆) δ ppm 8.03 (d, J=5.1 Hz, 1 H), 7.25 (d, J=5.5 Hz, 1 H), 6.97 (d, J=5.5 Hz, 1 H), 6.86 (t, J=5.7 Hz, 1 H), 6.43 (dd, J=5.1, 1.3 Hz, 1 H), 6.40 - 6.42 (m, 1 H), 3.74 - 3.76 (m, 3 H), 3.62 - 3.82 (m, 2 H), 3.50 - 4.07 (m, 2 H), 3.36 - 3.45 (m, 2 H), 2.86 (t, J=7.3 Hz, 2 H), 2.51 - 2.64 (m, 2 H)

### Example 62: (6,6-difluoro-3-azabicyclo[3.1.0]hexan-3-yl)(2-((4-fluoro-2-methoxyphenethyl)amino)pyridin-4-yl)methanone (E62)

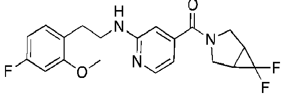

Prepared similarly as described for compound **E60** starting from **D60** (50 mg, 0.190 mmol) and **D63** (39.3 mg, 0.230 mmol). After work-up the crude was purified by RP Flash Chromatography (silica C18 12 g, 2-98% CH₃CN (+0.1% HCOOH) in H₂O (+0.1% HCOOH)) to give **E62**(5.2 mg, 0.013 mmol, yield= 6.873%) as colorless oil.

Method 2: R*t*= 1.13 min, MS (ESI) m/z = 392.2 [M+H]⁺.

¹H NMR (400 MHz, CDCl₃) δ ppm 8.05 (d, J = 5.3 Hz, 1H), 7.09 (t, J = 7.5 Hz, 1H), 6.65-6.56 (m, 2H), 6.52 (dd, J = 5.3, 1.2 Hz, 1H), 6.45 (s, 1H), 5.54 (br s, 1H), 4.27 (br d, J = 12.8 Hz, 1H), 3.84 (s, 3H), 3.82-3.63 (m, 3H), 3.52-3.42 (m, 2H), 2.90 (t, J = 6.9 Hz, 2H), 2.46-2.25 (m, 2H)

### Example 63: (6,6-difluoro-3-azabicyclo[3.1.0]hexan-3-yl)(2-((6-fluoro-2,3-dimethoxyphenethyl)amino)pyridin-4-yl)methanone (E63)

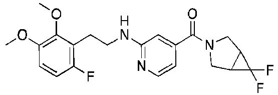

Prepared similarly as described for compound **E3** starting from **D64** (100 mg, 0.310 mmol) and (64.4 mg, 0.380 mmol). The reaction was stirred at 70°C for 12h. After work-up the residue was purified by RP Flash Chromatography (silica C18 12 g, 1-36% CH₃CN (+0.1% HCOOH) in H₂O (+0.1% HCOOH)) to give **E63** (20 mg, 0.047 mmol, yield= 15.19%) as yellow oil.

Method 2: R*t*= 1.13 min, MS (ESI) m/z = 422.21 [M+H]⁺.

¹H NMR (500 MHz, DMSO-*d*₆) δ ppm 8.03 (d, J=5.2 Hz, 1 H), 6.82 - 7.00 (m, 3 H), 6.43 (dd, J=5.2, 1.3 Hz, 1 H), 6.39 (s, 1 H), 3.78 (s, 3 H), 3.75 (s, 3 H), 3.50 - 4.09 (m, 4 H), 3.39 - 3.41 (m, 2 H), 2.83 (t, J=7.5 Hz, 2 H), 2.51 - 2.65 (m, 2 H)

### Example 64: (6,6-difluoro-3-azabicyclo[3.1.0]hexan-3-yl)(2-((4-methoxy-2-(trifluoromethoxy)phenethyl)amino)pyridin-4-yl)methanone (E64)

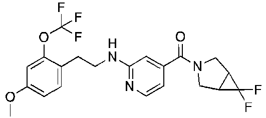

Prepared similarly as described for compound **E3** starting from **D65** (80 mg, 0.220 mmol) and 6,6-difluoro-3-azabicyclo[3.1.0]hexane;hydrochloride (46.2 mg, 0.270 mmol). The reaction was stirred at 70°C for 12h. After work-up, the residue was purified by RP Flash Chromatography (silica C18 12 g, 1- 43% CH₃CN (+0.1% HCOOH) in H₂O (+0.1% HCOOH)) to give **E64** (12 mg, 0.026 mmol, yield= 11.68%) as yellow oil.

Method 2: Rt*=* 1.33 min, MS (ESI) m/z = 458.51 [M+H]⁺.

¹H NMR (500 MHz, DMSO-*d*₆) δ ppm 8.03 (d, J=5.2 Hz, 1 H), 7.34 (d, J=8.6 Hz, 1 H), 6.95 (dd, J=8.6, 2.6 Hz, 1 H), 6.78 - 6.89 (m, 2 H), 6.43 (dd, J=5.2, 1.3 Hz, 1 H), 6.39 (s, 1 H), 3.77 (s, 3 H), 3.51 - 4.09 (m, 4 H), 3.40 - 3.49 (m, 2 H), 2.82 (t, J=7.3 Hz, 2 H), 2.52 - 2.62 (m, 2 H)

### Example 65: (2-((2-(benzo[d][1,3]dioxol-4-yl)ethyl)amino)pyridin-4-yl)(6,6-difluoro-3-azabicyclo[3.1.0]hexan-3-yl)methanone (E65)

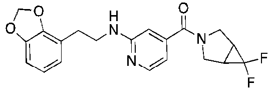

Prepared similarly as described for compound **E3** starting from **D66** (80 mg, 0.280 mmol) and 6,6-difluoro-3-azabicyclo[3.1.0]hexane;hydrochloride (47.96 mg, 0.280 mmol). The reaction was stirred at 70°C for 12h. After work-up the residue was purified by RP Flash Chromatography (silica C18 12 g, 1-70% CH₃CN (+0.1% HCOOH) in H₂O (+0.1% HCOOH)) to give **E65** (38 mg, 0.098 mmol, yield= 35.1 %) as solid.

Method 2: R*t*= 1.00 min, MS (ESI) m/z =388.2 [M+H]⁺ .

¹H NMR (400 MHz, DMSO-*d*₆) δ ppm 8.03 (d, J=5.0 Hz, 1 H), 6.81 (brt, J=5.6 Hz, 1 H), 6.69 - 6.79 (m, 3 H), 6.41 - 6.45 (m, 1 H), 6.40 (s, 1 H), 5.97 (s, 2 H), 4.03 (d, J=12.7 Hz, 1 H), 3.74 - 3.85 (m, 1 H), 3.66 (dt, J=12.6, 4.6 Hz, 1 H), 3.55 (d, J=11.4 Hz, 1 H), 3.45 - 3.52 (m, 2 H), 2.79 (t, J=7.3 Hz, 2 H), 2.51 - 2.65 (m, 2 H)

### Example 66: (6,6-difluoro-3-azabicyclo[3.1.0]hexan-3-yl)(2-((2,3-dimethoxyphenethyl)amino)pyridin-4-yl)methanone (E66)

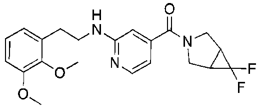

Prepared similarly as described for compound **E3** starting from 2-[2-(2,3-dimethoxyphenyl)ethylamino]pyridine-4-carboxylic acid (CAS n° 3213-29-4) (99 mg, 0.330 mmol) and 6,6-difluoro-3-azabicyclo[3.1.0]hexane; hydrochloride (67.44 mg, 0.390 mmol). The reaction was stirred at 70°C for 12h. After work-up, the residue was purified by RP Flash Chromatography (silica C18 12 g, 1-50% CH₃CN (+0.1% HCOOH) in H₂O (+0.1% HCOOH)) to give **E66** (84 mg, 0.208 mmol, yield= 63.59%) as solid.

Method 2: R*t*= 1.04 min, MS (ESI) m/z = 404.27 [M+H]⁺ .

¹H NMR (400 MHz, DMSO-*d*₆) δ ppm 8.04 (d, J=5.3 Hz, 1 H), 6.95 - 7.03 (m, 1 H), 6.87 - 6.94 (m, 1 H), 6.75 - 6.86 (m, 2 H), 6.43 (d, J=5.3 Hz, 1 H), 6.41 (s, 1 H), 3.79 (s, 3 H), 3.72 (s, 3 H), 3.50 - 4.10 (m, 4 H), 3.37 - 3.47 (m, 2 H), 2.75 - 2.86 (m, 2 H), 2.51 - 2.63 (m, 2 H)

### Example 67: (2-((5-chloro-2-methoxyphenethyl)amino)pyridin-4-yl)(6,6-difluoro-3-azabicyclo[3.1.0]hexan-3-yl)methanone (E67)

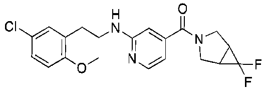

Prepared similarly as described for compound **E3** starting from **D68** (60 mg, 0.200 mmol) and 6,6-difluoro-3-azabicyclo[3.1.0]hexane;hydrochloride (40.3 mg, 0.230 mmol). The reaction was stirred at 65°C for 12h. After work-up the residue was purified by RP Flash Chromatography (silica C18 12 g, 1-50% CH₃CN (+0.1% HCOOH) in H₂O (+0.1% HCOOH)) to give **E67** (15 mg, 0.037 mmol, yield= 18.8%) as solid.

Method 2: R*t*= 1.27 min, MS (ESI) m/z = 408.21 [M+H]⁺ .

¹H NMR (400 MHz, DMSO-*d*₆) δ ppm 8.02 (d, J=5.4 Hz, 1 H), 7.24 (dd, J=8.5, 2.6 Hz, 1 H), 7.21 (d, J=2.6 Hz, 1 H), 6.99 (d, J=8.7 Hz, 1 H), 6.40 - 6.62 (m, 2 H), 4.04 (d, J=12.8 Hz, 1 H), 3.78 - 3.86 (m, 1 H), 3.77 (s, 3 H), 3.62 - 3.73 (m, 1 H), 3.56 (d, J=11.3 Hz, 1 H), 3.39 - 3.50 (m, 2 H), 2.81 (t, J=7.2 Hz, 2 H), 2.53 - 2.65 (m, 2 H)

### Example 68: (2-((2-chloro-6-methoxyphenethyl)amino)pyridin-4-yl)(6,6-difluoro-3-azabicyclo[3.1.0]hexan-3-yl)methanone (E68)

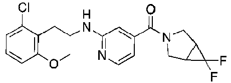

Prepared similarly as described for compound **E3** starting from **D69** (83 mg, 0.270 mmol) and 6,6-difluoro-3-azabicyclo[3.1.0]hexane;hydrochloride (55.7 mg, 0.320 mmol). The reaction was stirred at 70°C for 12h. After work-up, the residue was purified by RP Flash Chromatography (silica C18 12 g, 1-50% CH₃CN (+0.1% HCOOH) in H₂O (+0.1% HCOOH)) to give **E68** (55 mg, 0.135 mmol, yield= 49.84%) as solid.

Method 2: R*t*= 1.21 min, MS (ESI) m/z = 408.21 [M+H]⁺ .

¹H NMR (400 MHz, DMSO-*d*₆) δ ppm 8.02 (d, J=5.8 Hz, 1 H), 7.22 (t, J=8.3 Hz, 1 H), 7.01 (d, J=8.3 Hz, 1 H), 6.97 (d, J=8.3 Hz, 1 H), 6.83 (t, J=5.8 Hz, 1 H), 6.39 - 6.46 (m, 2 H), 4.05 (d, J=12.7 Hz, 1 H), 3.73 - 3.85 (m, 4 H), 3.67 (dt, J=12.7, 4.6 Hz, 1 H), 3.55 (d, J=11.4 Hz, 1 H), 3.31 - 3.42 (m, 2 H), 2.98 (t, J=7.6 Hz, 2 H), 2.51 - 2.65 (m, 2 H)

### Example 69: (2-((5-bromo-2-methoxyphenethyl)amino)pyridin-4-yl)(6,6-difluoro-3-azabicyclo[3.1.0]hexan-3-yl)methanone (E69)

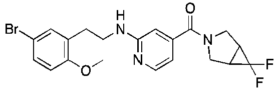

Prepared similarly as described for compound **E3** starting from **D70** (30 mg, 0.090 mmol) and 6,6-difluoro-3-azabicyclo[3.1.0]hexane;hydrochloride (17.6 mg, 0.100 mmol). The reaction was stirred at 70°C for 12h. After work-up, the residue was purified by RP Flash Chromatography (silica C18 12 g, 1-50% CH₃CN (+0.1% HCOOH) in H₂O (+0.1% HCOOH)) to give **E69** (4 mg, 0.009 mmol, yield= 10.35%) as solid.

Method 2: R*t*= 1.26 min, MS (ESI) m/z = 454.30 [M+H]⁺.

¹H NMR (400 MHz, DMSO-*d*₆) δ ppm 8.03 (d, J=5.2 Hz, 1 H), 7.35 (dd, J=8.7, 2.5 Hz, 1 H), 7.29 (d, J=2.5 Hz, 1 H), 6.93 (d, J=8.8 Hz, 1 H), 6.78 (t, J=5.6 Hz, 1 H), 6.42 (dd, J=5.3, 2.5 Hz, 1 H), 6.41 (s, 1 H), 4.04 (d, J=12.7 Hz, 1 H), 3.77 (s, 3 H), 3.76 - 3.84 (m, 1 H), 3.66 (dt, J=12.7, 4.6 Hz, 1 H), 3.55 (d, J=11.4 Hz, 1 H), 3.36 - 3.47 (m, 2 H), 2.79 (t, J=7.2 Hz, 2 H), 2.52 - 2.65 (m, 2 H)

### Example 70: (2-ethylpiperidin-1-yl)(2-((2-methoxyphenethyl)amino)-6-methylpyridin-4-yl)methanone (N1315-41-2, SCU-496) (E70)

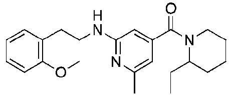

Prepared similarly as described for compound **E42** starting from 2-(2-methoxyphenyl)ethanamine (160 µL, 1.12 mmol) and **D22** (300 mg, 1.12 mmol) was heated to 100 °C for 3h; After work-up, the crude was purified by RP Flash Chromatography (Sfar C18, 12 g, eluent: CH₃CN (+0.1% HCOOH) in H₂O (+0.1% HCOOH)) to obtain **E70** (27.3 mg, 0.072 mmol, yield= 6.4%) as a yellow oil.

Method 2: R*t*= 1.35 min, MS (ESI) m/z = 382.33 [M+H]⁺.

¹H NMR (400 MHz, DMSO-*d*₆) δ ppm 7.17 - 7.23 (m, 1 H), 7.15 (br d, J=7.3 Hz, 1 H), 6.96 (d, J=8.1 Hz, 1 H), 6.86 (t, J=7.3 Hz, 1 H), 6.55 - 6.67 (m, 1 H), 6.22 (s, 1 H), 6.15 (br s, 1 H), 3.79 (s, 3 H), 3.53 - 4.68 (m, 1 H), 3.38 (br s, 2 H), 2.79 (t, J=7.5 Hz, 2 H), 2.54 - 4.41 (m, 2 H), 2.27 (s, 3 H), 1.18 - 1.86 (m, 8 H), 0.55 - 0.99 (m, 3 H)

### Example 71: (2-chloro-6-((2-methoxyphenethyl)amino)pyridin-4-yl)(2-ethylpiperidin-1-yl)methanone (E71)

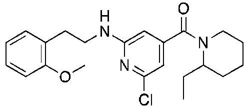

Prepared similarly as described for compound **E42** starting from 2-(2-methoxyphenyl)ethanamine (20 µL, 0.140 mmol) and **D23** (40 mg, 0.140 mmol). The reaction mixture was heated at 100°C, ON. Then DIPEA (0.01 mL, 0.070 mmol) was added and heated additionally for 1h. After work-up, the crude was purified by RP Flash Chromatography (Sfar C18, 12 g, eluent: CH₃CN (+0.1% HCOOH) in H₂O (+0.1% HCOOH)) to give **E71** (21 mg, 0.052 mmol, yield= 37.51%) as yellow oil.

Method 2: R*t*= 2.49 min, MS (ESI) m/z = 402.27 [M+H] ⁺.

¹H NMR (400 MHz, DMSO-*d*₆) δ ppm 7.17 - 7.26 (m, 2 H), 7.14 (br d, J=7.2 Hz, 1 H), 6.96 (d, J=8.1 Hz, 1 H), 6.86 (t, J=7.3 Hz, 1 H), 6.39 (s, 1 H), 6.30 (br s, 1 H), 3.79 (s, 3 H), 3.43 - 4.61 (m, 1 H), 3.32 - 3.42 (m, 2 H), 3.26 - 4.35 (m, 1 H), 2.79 (t, J=7.4 Hz, 2 H), 2.66 - 3.09 (m, 1 H), 1.19 - 1.84 (m, 8 H), 0.61 - 0.90 (m, 3 H)

### Example 72: (6,6-difluoro-3-azabicyclo[3.1.0]hexan-3-yl)(2-((2-methoxyphenethyl)amino)-6-(pyrrolidin-1-yl)pyridin-4-yl)methanone (E72)

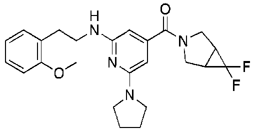

Prepared similarly as described for compound **E60** starting from **D25** (20 mg, 0.060 mmol) and 2-(2-methoxyphenyl)ethanamine (10 µL, 0.070 mmol). After work-up the crude was purified by RP Flash Chromatography (silica C18 12 g, eluent: CH₃CN (+0.1% HCOOH) in H₂O (+0.1% HCOOH)) to give the impure product (20 mg). The product was further purified to give **E72** (1.1 mg, 0.002 mmol, yield= 4.1%) as a light yellow oil.

Method 2: R*t*= 1.27 min, MS (ESI) m/z = 442.6 [M+H]⁺.

¹H NMR (500 MHz, CHLOROFORM-*d*) δ ppm 7.22 (td, J = 7.8, 1.6 Hz, 1H), 7.16 (dd, J = 7.3, 1.4 Hz, 1H), 6.93-6.85 (m, 2H), 5.64 (s, 1H), 5.61 (s, 1H), 5.07-4.40 (m, 1H), 3.85 (s, 3H), 4.26-3.69 (m, 4H), 3.46 (t, J = 7.2 Hz, 2H), 3.42 (br s, 4H), 2.97-2.89 (m, 2H), 2.39-2.21 (m, 2H), 1.96 (br t, J = 6.5 Hz, 4H)

### Example 73: (6,6-difluoro-3-azabicyclo[3.1.0]hexan-3-yl)(2-((2-methoxyphenethyl)amino)-3-methylpyridin-4-yl)methanone (E73)

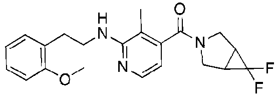

Prepared similarly as described for compound **E3** starting from **D27** (50 mg, 0.160 mmol) and 6,6-difluoro-3-azabicyclo[3.1.0]hexane;hydrochloride (33.4 mg, 0.190 mmol). The crude was purified by RP Flash Chromatography (Sfar C18, 12 g, eluent: CH₃CN (+0.1% HCOOH) in H₂O (+0.1% HCOOH)) to give **E73** (6 mg, 0.015 mmol, yield= 9.55%) as a light yellow solid.

Method 2: R*t*= 0.98 min, MS (ESI) m/z = 388.30 [M+H]⁺.

¹H NMR (400 MHz, DMSO-*d*₆) δ ppm 7.93 (d, J=5.2 Hz, 1 H), 7.16 - 7.22 (m, 1 H), 7.14 (d, J=6.1 Hz, 1 H), 6.96 (d, J=7.9 Hz, 1 H), 6.87 (t, J=7.0 Hz, 1 H), 6.27 (br d, J=4.9 Hz, 1 H), 6.09 (t, J=5.3 Hz, 1 H), 4.00 (d, J=12.5 Hz, 1 H), 3.81 - 3.89 (m, 1 H), 3.79 (s, 3 H), 3.63 - 3.75 (m, 2 H), 3.45 - 3.61 (m, 2 H), 2.82 - 2.90 (m, 2 H), 2.56 - 2.73 (m, 2 H), 1.85 (s, 3 H)

### Example 74: (2-ethylpiperidin-1-yl)(2-hydroxy-6-((2-methoxyphenethyl)amino)pyridin-4-yl)methanone (E74)

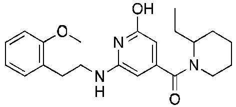

Step A: (2-(benzyloxy)-6-((2-methoxyphenethyl)amino)pyridin-4-yl)(2-ethylpiperidin-1-yl)methanone

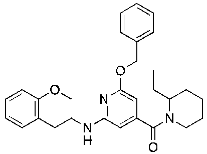

Prepared similarly as described for compound **E42** starting from 2-(2-methoxyphenyl)ethanamine (0.15 mL, 1.04 mmol) and **D28** (375 mg, 1.04 mmol). After work-up, the crude was purified by RP Flash Chromatography (Sfar C18, 12 g, eluent: CH₃CN (+0.1% HCOOH) in H₂O (+0.1% HCOOH)) to obtain (2-(benzyloxy)-6-((2-methoxyphenethyl)amino)pyridin-4-yl)(2-ethylpiperidin-1-yl)methanone (20 mg, 0.042 mmol, yield= 4.042%) as yellow oil. NMR and UPLC were consistent with chemical structure.

Method 2: Rt= 2.80 min, MS (ESI) m/z =474.28 [M+H]⁺.

### Step B:

(2-(benzyloxy)-6-((2-methoxyphenethyl)amino)pyridin-4-yl)(2-ethylpiperidin-1-yl)methanone (20 mg, 0.040 mmol) and Pd/C (4.49 mg) were stirred in MeOH (3 mL) under H₂ atmosphere (2.0 bar) for 2h. The obtained suspension was filtered over a celite pad (Attention pyrophoric!) and the organic layer was concentrated under high vacuum to give the product **E74** (10 mg, 0.026 mmol, yield= 61.75%) as a yellow oil.

Method 2: Rt= 1.73 min, MS (ESI) m/z = 384.28 [M+H]⁺.

¹H NMR (400 MHz, DMSO-*d*₆) δ ppm 10.48 (br s, 1H), 7.25-7.18 (m, 1H), 7.16 (d, J = 7.3 Hz, 1H), 6.97 (d, J = 8.0 Hz, 1H), 6.92-6.84 (m, 1H), 6.25 (br s, 1H), 5.48-5.20 (m, 2H), 3.78 (s, 3H), 4.63-3.57 (m, 1H), 4.39-3.37 (m, 1H), 3.30-3.22 (m, 2H), 2.78 (t, J = 7.4 Hz, 2H), 3.07-2.57 (m, 1H), 1.80-1.68 (m, 1H), 1.69-1.42 (m, 6H), 1.42-1.20 (m, 1H), 0.99-0.58 (m, 3H)

### Example 75: trans-(6,6-difluoro-3-azabicyclo[3.1.0]hexan-3-yl)(2-(((1r,3r)-3-(2-methoxyphenyl)cyclobutyl)amino)pyridin-4-yl)methanone (E75)

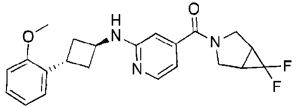

Prepared similarly as described for compound **E49** starting from **D8** (60 mg, 0.2353 mmol, 1.0 eq) and *trans-*(1r,3r)-3-(2-methoxyphenyl)cyclobutan-1-amine; hydrochloride (50 mg, 0.2353 mmol, 1.0 eq). After work-up, the crude was purified by RP preparative HPLC. The pure fractions containing the desired product were concentrated and freeze-dried to yield **E75** (4.,7 mg, 0.102 mmol, yield= 43%) as a white powder.

Method 6: Rt= 5.98 min, MS (ESI) m/z = 400.3 [M+H]⁺.

¹H NMR (400 MHz, DMSO-*d*₆) δ ppm 8.02 ( dd, J = 5.2, 0.8 Hz, 1 H), 7.39-7.30 (m, 1 H), 7.26-7.13 (m, 2 H), 7.01-6.88 ( m, 2 H), 6.44 (dd, J=5.1, 1.4 Hz, 1 H), 6.38 (s, 1 H), 4.34-4.22 (m, 1 H), 4.05 ( d, J=12.7 Hz, 1 H), 3.87-3.77 (m, 2 H), 3.76 (s, 3 H), 3.72-3.62 (m, 1 H), 3.57 (d, J= 11.3 Hz, 1 H), 2.66-2.53 (m, 2 H), 2.46-2.35 (m, 2 H), 2.35-2.23 (m, 2 H).

### Example 76: (6,6-difluoro-3-azabicyclo[3.1.0]hexan-3-yl)(2-((1-(2-methoxyphenyl)azetidin-3-yl)amino)pyridin-4-yl)methanone (E76)

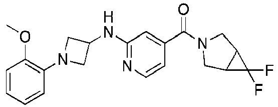

Prepared similarly as described for compound **E49** starting from **D30** (106 mg, 0.26 mmol, 1 eq) and **D8** (80 mg, 0.3303 mmol, 1.3 eq). The crude material obtained after the work-up was purified by RP Preparative HPLC. The pure fractions containing the desired product were concentrated and freeze-dried to yield **E76** (4.5 mg, 0.01 mmol, yield= 3%) as a white powder.

Method 7: Rt= 3.56 min, MS (ESI) m/z = 401.2 [M+H]⁺.

¹H NMR (400 MHz, DMSO-*d*₆) δ ppm 8.13 (dd, J= 5.2, 0.8 Hz, 1 H), 6.84 (dd, J= 8.0, 1.4 Hz, 1 H), 6.79 (td, J= 7.6, 1.4 Hz, 1 H), 6.63 (td, J = 7.7, 1.5 Hz, 1 H ), 6.58 ( dd, J= 5.1, 1.3 Hz, 1 H), 6.45 (dd, J= 7.8, 1.5 Hz, 1 H), 6.34 (d, J= 1.1Hz, 1 H), 5.49 ( d, J= 6.0Hz, 1 H ), 4.41-4.28 (m, 3 H), 4.04 (d, J= 12.7 Hz, 1 H), 3.90-3.84 (m, 2 H), 3.84-3.80 ( m, 1 H), 3.78 (s, 3 H), 3.73-3.63 (m, 1 H), 3.53 (d, J= 11.3Hz, 1 H), 2.67-2.55 (m, 2 H).

### Example 77: trans-(6,6-difluoro-3-azabicyclo[3.1.0]hexan-3-yl)(2-((2-(2-methoxyphenyl)cyclopropyl)amino)pyridin-4-yl)methanone (E77)

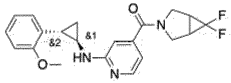

Prepared similarly as described for compound **E8** starting from **D71** (51 mg, 0.180 mmol) and 6,6-difluoro-3-azabicyclo[3.1.0]hexane;hydrochloride (33.5 mg, 0.220 mmol). The reaction was stirred at RT for 12h. After work-up, the residue was purified by RP Flash Chromatography (silica C18 12 g, 1-50% CH₃CN (+0.1% HCOOH) in H₂O (+0.1% HCOOH)) to give **E77** ( 3 mg, 0.008 mmol, yield= 4.339%) as an oil. r.t. 1.12 min, MS (ESI) m/z = 386.6 [M+H]+

Method 2: Rt= 1.12 min, MS (ESI) m/z = 386.6 [M+H]⁺.

¹H NMR (500 MHz, CHLOROFORM-d) δ ppm 8.12-8.04 (m, 1H), 7.24-7.18 (m, 1H), 6.94-6.85 (m, 3H), 6.81-6.76 (m, 1H), 6.66-6.61 (m, 1H), 6.34-6.10 (m, 1H), 4.21 (dd, J = 12.8, 4.0 Hz, 1H), 3.87-3.84 (m, 3H), 3.83-3.75 (m, 1H), 3.74-3.60 (m, 2H), 2.69-2.57 (m, 1H), 2.44-2.32 (m, 1H), 2.42-2.19 (m, 2H), 1.40-1.32 (m, 1H), 1.28-1.20 (m, 1H)

### Example 78: (S)-(2-((2-methoxybenzyl)amino)pyridin-4-yl)(2-methylindolin-1-yl)methanone (E78)

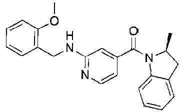

Prepared similarly as described for compound **E3** starting from **D32** (101.01 mg, 0.390 mmol) and (2S)-2-methyl-2,3-dihydro-1H-indole (0.05 mL, 0.390 mmol). The reaction mixture was stirred at 60 °C for 12h.. The crude was purified with FractionLynx (column: XTerra Prep Column, 5um, 30x75, Flow 30 mL/min, H₂O, CH₃CN 0.1% HCOOH) to obtain **E78** (108 mg, 0.289 mmol, yield= 74.7%) as a pale yellow solid.

Method 2: R*t*= 1.36 min, MS (ESI) m/z =374.20 [M+H]⁺.

¹H NMR (400 MHz, DMSO-*d*₆6) δ ppm 7.77 - 8.47 (m, 2 H), 6.54 - 7.45 (m, 9 H), 4.14 - 4.74 (m, 3 H), 3.82 (s, 3 H), 3.40 (br dd, J=15.9, 8.8 Hz, 1 H), 2.61 - 2.68 (m, 1 H), 0.81 - 1.29 (m, 3 H).

### Example 79: (S)-(2-((3-methoxybenzyl)amino)pyridin-4-yl)(2-methylindolin-1-yl)methanone E79

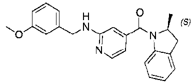

Prepared similarly as described for compound **E3** starting from **D34** (101 mg, 0.390 mmol) and (2S)-2-methyl-2,3-dihydro-1H-indole (50 µL, 0.390 mmol) in DMSO (0.646 mL) at 60 °C for 12h. The crude was purified with FractionLynx (column: XTerra Prep Column, 5um, 30x75, Flow 30 mL/min, H₂O, CH₃CN 0.1% HCOOH) to obtain E79 (17.3 mg, 0.046 mmol, yield= 11.96%) was as a pale yellow solid.

Method 2: *Rt=* 1.38 min, MS (ESI) m/z =374.2 [M+H]⁺.

¹H NMR (400 MHz, DMSO-*d*₆) δ ppm 8.08 (d, J=5.2 Hz, 1 H), 8.04 (br s, 1 H), 7.26 - 7.32 (m, 2 H), 7.22 (t, J=8.1 Hz, 1 H), 7.15 (br s, 1 H), 7.05 (br s, 1 H), 6.85 - 6.95 (m, 2 H), 6.74 - 6.83 (m, 1 H), 6.59 (br s, 1 H), 6.56 (br s, 1 H), 4.48 (d, J=5.9 Hz, 2 H), 4.45 (br s, 1 H), 3.72 (s, 3 H), 3.37 (dd, J=16.1, 8.9 Hz, 1 H), 2.61 (d, J=16.3 Hz, 1 H), 1.02 (br s, 3 H)

### Example 80: (6,6-difluoro-3-azabicyclo[3.1.0]hexan-3-yl)(2-((2-methoxybenzyl)amino)pyridin-4-yl)methanone (E80)

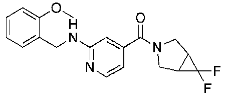

Prepared similarly as described for compound **E3** starting from **D32** (100 mg, 0.390 mmol) and 6,6-difluoro-3-azabicyclo[3.1.0]hexane;hydrochloride (66.5 mg, 0.39mmol). The crude was purified with FractionLynx (column: XTerra Prep Column, 5um, 30x75, Flow 30 mL/min, H₂O, CH₃CN 0.1% HCOOH). The fractions containing the desired product were lyophilized. A yellow solid containing acidic residue was obtained. The latter was, subsequently, washed with ss NaHCO₃ to give E80 (63.2 mg, 0.176 mmol, yield= 45.42%) as a white solid. Method 2: R*t*= 0.96 min, MS (ESI) m/z =360.2 [M+H]⁺.

¹H NMR (400 MHz, DMSO-*d*₆) δ ppm 8.56 (br s, 1H), 8.01 (d, J = 6.3 Hz, 1H), 7.33 (t, J = 7.8 Hz, 1H), 7.28 (d, J = 7.4 Hz, 1H), 7.06 (d, J = 8.2 Hz, 1H), 6.95 (t, J = 7.4 Hz, 1H), 6.91 (br s, 1H), 6.74 (br d, J = 4.8 Hz, 1H), 4.51 (br s, 2H), 3.82 (s, 3H), 4.08-3.55 (m, 4H), 2.67-2.55 (m, 2H)

### Example 81: (6,6-difluoro-3-azabicyclo[3.1.0]hexan-3-yl)(2-((3-methoxybenzyl)amino)pyridin-4-yl)methanone (E81)

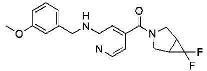

Prepared similarly as described for compound **E3** starting from **D34** (101 mg, 0.390 mmol) and 6,6-difluoro-3-azabicyclo[3.1.0]hexane;hydrochloride (66.5 mg, 0.390 mmol). The crude was purified with FractionLynx (column: XTerra Prep Column, 5um, 30x75, Flow 30 mL/min, H₂O, CH₃CN 0.1% HCOOH). The fractions containing the desired product were lyophilized. A yellow solid containing acidic residue was obtained. The latter was, subsequently, washed with ss NaHCO₃ to give **E81** ( 104 mg, 0.289 mmol, yield= 74.74%) as a white solid.

Method 2: R*t*= 0.96 min, MS (ESI) m/z =360.3 [M+H]⁺.

¹H NMR (400 MHz, DMSO-*d*₆) δ ppm 8.55 (br s, 1H), 8.03 (d, J = 6.0 Hz, 1H), 7.29 (t, J = 7.8 Hz, 1H), 6.98-6.92 (m, 2H), 6.91-6.86 (m, 1H), 6.85 (br s, 1H), 6.72 (br d, J = 5.1 Hz, 1H), 4.53 (br s, 2H), 3.75 (s, 3H), 4.08-3.51 (m, 4H), 2.68-2.54 (m, 2H)

### Example 82: (4,4-difluoropiperidin-1-yl)(2-((2-methoxybenzyl)amino)pyridin-4-yl)methanone (E82)

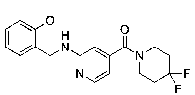

Prepared similarly as described for compound **E3** starting from **D32** (53 mg, 0.200 mmol) and 4,4-difluoropiperidine (30 mg, 0.250 mmol) in DMF. The reaction mixture was stirred at 60 °C for 12h. The crude was purified with FractionLynx (column: XTerra Prep Column, 5um, 30x75, Flow 30 mL/min, H₂O, CH₃CN 0.1% HCOOH). **E81** (66.1 mg, 0.183 mmol, yield= 89.35%) was obtained as a pale yellow solid.

Method 2: R*t*= 1.04 min, MS (ESI) m/z =362.3 [M+H]⁺.

¹H NMR (400 MHz, DMSO-*d*₆) δ ppm 8.01 (d, J=5.4 Hz, 1 H), 7.19 - 7.29 (m, 2 H), 7.01 (d, J=8.1 Hz, 1 H), 6.90 (t, J=7.3 Hz, 1 H), 6.84 - 7.76 (m, 1 H), 6.54 - 6.68 (m, 2 H), 4.46 (d, J=5.4 Hz, 2 H), 3.82 (s, 3 H), 3.27 - 3.75 (m, 4 H), 1.88 - 2.13 (m, 4 H)

### Example 83: (R)-(2-((2-methoxybenzyl)amino)pyridin-4-yl)(2-methylindolin-1-yl)methanone (E83)

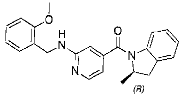

Prepared similarly as described for compound **E3** starting from **D32** (100 mg, 0.390 mmol) and (2*R*)-2-methyl-2,3-dihydro-1H-indole;hydrochloride (65.7 mg, 0.390 mmol) in DMF (1.291 mL). The reaction mixture was stirred at 60 °C for 12h. The crude was purified with FractionLynx (column: XTerra Prep Column, 5um, 30x75, Flow 30 mUmin, H₂O, CH₃CN 0.1% HCOOH). **E83** (108 mg, 0.289 mmol, yield= 74.69%) was obtained as a pale yellow solid.

Method 2: R*t*= 1.36 min, MS (ESI) m/z =374.2 [M+H]⁺.

¹H NMR (400 MHz, DMSO-*d*₆) δ ppm 8.07 (d, J=5.8 Hz, 1 H), 7.73 - 8.36 (m, 1 H), 7.23 - 7.33 (m, 1 H), 7.03 (d, J=8.1 Hz, 1 H), 6.92 (t, J=7.3 Hz, 1 H), 6.66 - 6.85 (m, 1 H), 6.62 - 7.38 (m, 5 H), 4.25 - 4.71 (m, 3 H), 3.82 (s, 3 H), 3.40 (br dd, J=16.0, 8.9 Hz, 1 H), 2.61 - 2.70 (m, 1 H), 0.75 - 1.34 (m, 3 H)

### Example 84: (6,6-difluoro-3-azabicyclo[3.1.0]hexan-3-yl)(2-((2-(trifluoromethoxy)benzyl)amino)pyridin-4-yl)methanone (E84)

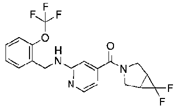

Prepared similarly as described for compound **E8** (step A) starting from **D36** (100 mg, 0.320 mmol) and 6,6-difluoro-3-azabicyclo[3.1.0]hexane;hydrochloride (49.8 mg, 0.320 mmol) in DMF (1 mL). The reaction mixture was at 65 °C for 12h. Then the crude mixture was precipitated with water, filtered and purified by RP Flash Chromatography (Sfar C18, 12 g, eluent: CH₃CN (+0.1% HCOOH) in H₂O (+0.1% HCOOH)). **E84** (72 mg, 0.174 mmol, yield= 54.39%) was obtained as a pale yellow oil.

Method 2: R*t*= 1.39 min, MS (ESI) m/z =414.1 [M+H]⁺.

¹H NMR (400 MHz, DMSO-*d*₆) δ ppm 8.01 (d, J=5.2 Hz, 1 H), 7.44 (br d, J=7.3 Hz, 1 H), 7.32 - 7.41 (m, 3 H), 7.28 (t, J=5.9 Hz, 1 H), 6.50 (s, 1 H), 6.48 (d, J=5.3 Hz, 1 H), 4.57 (d, J=5.7 Hz, 2 H), 3.60 - 3.83 (m, 2 H), 3.49 - 4.09 (m, 2 H), 2.52 - 2.64 (m, 2 H)

### Example 85: (6,6-difluoro-3-azabicyclo[3.1.0]hexan-3-yl)(2-(((2-methylthiazol-5-yl)methyl)amino)pyridin-4-yl)methanone (E85)

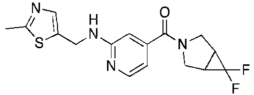

Prepared similarly as described for compound **E42** starting from **D8** (6,6-difluoro-3-azabicyclo[3.1.0]hexan-3-yl)-(2-fluoro-4-pyridyl)methanone (50 mg, 0.210 mmol) and (2-methylthiazol-5-yl)methanamine (26.5 mg, 0.210 mmol). The crude was purified by RP Flash Chromatography (Sfar C18, 12 g, eluent: CH₃CN (+0.1% HCOOH) in H₂O (+0.1% HCOOH)) to give **E85** (21 mg, 0.060 mmol, yield= 29.03%) as a yellow oil.

Method 2: R*t*= 0.75 min, MS (ESI) m/z = 351.21 [M+H]⁺.

¹H NMR (400 MHz, DMSO-*d*₆) δ ppm 8.08 (d, J=5.2 Hz, 1 H), 7.49 (s, 1 H), 7.31 (t, J=6.0 Hz, 1 H), 6.50 (dd, J=5.1, 1.2 Hz, 1 H), 6.44 (s, 1 H), 4.61 (d, J=6.0 Hz, 2 H), 4.03 (d, J=12.8 Hz, 1 H), 3.77 (dt, J=11.1, B854.6 Hz, 1 H), 3.66 (dt, J=12.6, 4.6 Hz, 1 H), 3.52 (d, J=11.4 Hz, 1 H), 2.56 (s, 3 H), 2.51 - 2.64 (m, 2 H)

### Example 86: N-(2-fluorobenzyl)-2-((2-methoxyphenethyl)amino)-isonicotinamide (E86)

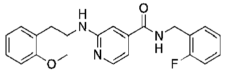

Prepared similarly as described for compound **E3** starting from **D2** (100 mg, 0.370 mmol) and (2-fluorophenyl)methanamine (50 µL, 0.440 mmol). The reaction mixture was stirred at RT for 12h. The crude was purified by RP Flash Chromatography (Sfar C18, 12 g, eluent: CH₃CN (+0.1% HCOOH) in H₂O (+0.1% HCOOH)) to give **E86** ( 34 mg, 0.090 mmol, yield= 24.4%) as light yellow solid.

Method 2: R*t*= MS (ESI) m/z = 380.24 [M+H]⁺.

¹H NMR (400 MHz, DMSO-*d*₆) δ ppm 9.04 (t, J=5.8 Hz, 1 H), 8.07 (d, J=5.2 Hz, 1 H), 7.28 - 7.37 (m, 2 H), 7.10 - 7.24 (m, 4 H), 6.96 (d, J=7.5 Hz, 1 H), 6.83 - 6.89 (m, 3 H), 6.79 (t, J=5.6 Hz, 1 H), 4.48 (d, J=5.9 Hz, 2 H), 3.79 (s, 3 H), 3.37 - 3.46 (m, 2 H), 2.75 - 2.88 (m, 2 H)

### Example 87: N-(3-fluorobenzyl)-2-((2-methoxyphenethyl)amino)-isonicotinamide(E87)

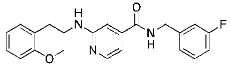

Prepared similarly as described for compound **E3** starting from **D2** (100 mg, 0.370 mmol) and (3-fluorophenyl)methanamine (50 µL, 0.440 mmol); the reaction mixture was stirred at RT for 4h. The crude was purified by RP Flash Chromatography (Sfar C18, 12 g , eluent: CH₃CN (+0.1% HCOOH) in H₂O (+0.1% HCOOH)) to give **E87** (24 mg, 0.063 mmol, yield= 17.22%) as light yellow oil.

Method 2: R*t*= 1.27 min, MS (ESI) m/z = 380.27 [M+H]⁺.

¹H NMR (400 MHz, DMSO-*d*₆) δ ppm 9.09 (t, J=6.1 Hz, 1 H), 8.07 (d, J=5.3 Hz, 1 H), 7.30 - 7.42 (m, 1 H), 7.17 - 7.22 (m, 1 H), 7.03 - 7.16 (m, 4 H), 6.96 (d, J=8.1 Hz, 1 H), 6.83 - 6.90 (m, 3 H), 6.75 - 6.82 (m, 1 H), 4.45 (d, J=6.0 Hz, 2 H), 3.79 (s, 3 H), 3.38 - 3.46 (m, 2 H), 2.81 (t, J=7.3 Hz, 2 H)

### Example 88: (S-N-(1-(4-fluorophenyl)-2-hydroxyethyl)-2-((2-methoxy-phenethyl)amino)isonicotinamide (E88)

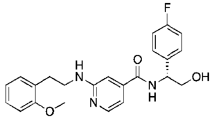

Prepared similarly as described for compound **E1** (stepA) starting from **D2** (100 mg, 0.370 mmol) and (2*S*)-2-amino-2-(4-fluorophenyl)ethanol (68.4 mg, 0.440 mmol). The mixtures were stirred at RT for 3h. The crude was purified by RP Flash Chromatography (Sfar C18, 12 g, eluent: CH₃CN (+0.1% HCOOH) in H₂O (+0.1% HCOOH)) to give **E88** ( 30 mg, 0.073 mmol, yield= 19.95%) as a colourless oil.

Method 2: R*t*= 1.13 min, MS (ESI) m/z = 410.25 [M+H]⁺.

¹H NMR (400 MHz, DMSO-*d*₆) δ ppm 8.72 (d, J=8.1 Hz, 1 H), 8.07 (d, J=5.2 Hz, 1 H), 7.35 - 7.47 (m, 2 H), 7.19 (td, J=7.8, 1.7 Hz, 1 H), 7.11 - 7.17 (m, 3 H), 6.96 (d, J=7.7 Hz, 1 H), 6.84 - 6.89 (m, 2 H), 6.83 (s, 1 H), 6.78 (br t, J=4.3 Hz, 1 H), 5.02 (td, J=7.7, 6.1 Hz, 1 H), 4.93 (t, J=5.7 Hz, 1 H), 3.79 (s, 3 H), 3.56 - 3.72 (m, 2 H), 3.38 - 3.47 (m, 2 H), 2.81 (t, J=7.4 Hz, 2 H)

### Example 89: N-(cuban-1-yl)-2-{[2-(2-methoxyphenyl)ethyl]amino}pyridine-4-carboxamide (E89)

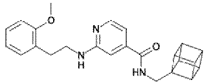

Prepared similarly as described for compound **E3** starting from **D2** (50 mg, 0.180 mmol) and cuban-1-ylmethanamine;hydrochloride (31.2 mg, 0.180 mmol) (stock solution 50% solution in EtOAc). The mixtures were stirred at RT for 3h. The crude was purified by RP Flash Chromatography (Sfar C18, 12 g, eluent: CH₃CN (+0.1% HCOOH) in H₂O (+0.1% HCOOH)) to give **E89** (26 mg, 0.067 mmol, yield= 36.54%) as a colourless oil.

Method 2: R*t*= 1.47 min, MS (ESI) m/z = 388.3 [M+H]⁺.

¹H NMR (400 MHz, DMSO-*d*₆) δ ppm 8.50 (t, J=5.8 Hz, 1 H), 8.05 (d, J=5.3 Hz, 1 H), 7.17 - 7.22 (m, 1 H), 7.15 (d, J=7.2 Hz, 1 H), 6.96 (d, J=8.2 Hz, 1 H), 6.87 (t, J=7.4 Hz, 1 H), 6.80 (s, 1 H), 6.72 - 6.79 (m, 2 H), 3.95 - 4.03 (m, 1 H), 3.85 (d, J=3.6 Hz, 6 H), 3.80 (s, 3 H), 3.46 (d, J=5.9 Hz, 2 H), 3.34 - 3.45 (m, 2 H), 2.81 (t, J=7.3 Hz, 2 H)

### Example 90: (S)-N-(1-(4-fluorophenyl)ethyl)-2-((2-methoxyphenethyl)amino)-isonicotinamide (E90)

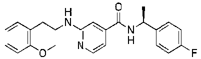

Prepared similarly as described for compound **E1** (stepA) starting from **D2** 2-[2-(2-methoxyphenyl)ethylamino]pyridine-4-carboxylic acid (120 mg, 0.440 mmol) and (1*S*)-1-(4-fluorophenyl)ethan-1-amine (70 µL, 0.530 mmol). The reaction mixture was stirred for 3h and then quenched with 5 mL of NaOH (1M). The organic layer was separated and concentrated under high vacuum to give **E90** (98 mg, 0.249 mmol, yield= 56.52%) as off-white solid.

Method 2: R*t*= 1.32 min, MS (ESI) m/z = 394.23 [M+H]⁺.

¹H NMR (400 MHz, DMSO-*d*₆) δ ppm 8.83 (d, J=8.0 Hz, 1 H), 8.06 (d, J=5.2 Hz, 1 H), 7.37 - 7.44 (m, 2 H), 7.09 - 7.21 (m, 4 H), 6.95 (d, J=8.0 Hz, 1 H), 6.79 - 6.89 (m, 3 H), 6.76 (br t, J=5.5 Hz, 1 H), 5.12 (quin, J=7.3 Hz, 1 H), 3.78 (s, 3 H), 3.42 (q, J=7.4 Hz, 2 H), 2.81 (t, J=7.4 Hz, 2 H), 1.44 (d, J=7.1 Hz, 3 H)

### Example 91: N-(1-(4-fluorophenyl)cyclopropyl)-2-((2-methoxyphenethyl)-amino)isonicotinamide (E91)

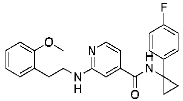

Prepared similarly as described for compound **E3** starting from **D2** (101 mg, 0.370 mmol) and 1-(4-fluorophenyl)cyclopropanamine (47 µL, 0.370 mmol) in DMSO (1.29 mL). The reaction mixture was stirred at 60 °C for 12h. The mixtures were stirred at RT for 3h. The crude was purified with FractionLynx (column: XTerra Prep Column, 5um, 30x75, Flow 30 mL/min, H₂O, CH₃CN 0.1% HCOOH) to yield **E91** N-[1-(4-fluorophenyl)cyclopropyl]-2-[2-(2-methoxyphenyl)ethylamino]pyridine-4-carboxamide (40.3 mg, 0.1 mmol, yield= 27.07%) Method 2: R*t*= 1.35 min, MS (ESI) m/z = 406.2 [M+H]⁺.

¹H NMR (400 MHz, DMSO-*d*₆) δ ppm 9.20 (s, 1 H), 8.06 (d, J=5.7 Hz, 1 H), 7.13 - 7.27 (m, 4 H), 7.05 - 7.13 (m, 2 H), 6.96 (d, J=8.1 Hz, 1 H), 6.81 - 6.90 (m, 3 H), 6.75 (t, J=5.6 Hz, 1 H), 3.79 (s, 3 H), 3.37 - 3.47 (m, 2 H), 2.81 (t, J=7.5 Hz, 2 H), 1.22 (s, 4 H)

### Example 92: N-(2-(4-fluorophenyl)propan-2-yl)-2-((2-methoxyphenethyl)-amino)isonicotinamide(E92)

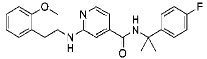

Prepared similarly as described for compound **E3** starting from **D2** (100 mg, 0.370 mmol) and2-(4-fluorophenyl)propan-2-amine (56.3 mg, 0.376 mmol)in DMSO (1.29 mL). The reaction mixture was stirred at 60°C for 12h. The crude was purified with FractionLynx (column: XTerra Prep Column, 5um, 30x75, Flow 30 mL/min) to give **E92** (14.6 mg, 0.036 mmol, yield= 9.76%) as a white solid.

Method 2: R*t*= 1.41 min, MS (ESI) m/z = 408.30 [M+H]⁺.

¹H NMR (400 MHz, DMSO-*d*₆) δ ppm 8.73 (br s, 1 H), 8.02 (d, J=6.0 Hz, 1 H), 7.38 (dd, J=8.6, 5.5 Hz, 2 H), 7.17 - 7.27 (m, 2 H), 7.11 (t, J=8.8 Hz, 2 H), 6.97 (d, J=8.2 Hz, 1 H), 6.93 - 7.15 (m, 2 H), 6.89 (t, J=7.4 Hz, 1 H), 3.77 (s, 3 H), 3.50 (br d, J=3.7 Hz, 2 H), 2.86 (br t, J=7.2 Hz, 2 H), 1.65 (s, 6 H)

### Example 93: (S)-N-(5-fluoro-2,3-dihydro-1H-inden-1-yl)-2-((2-methoxyphenethyl)amino)isonicotinamide (E93)

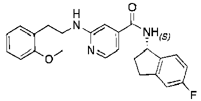

Prepared similarly as described for compound **E3** starting from **D2** (100 mg, 0.370 mmol) and (1*S*)-5-fluoroindan-1-amine;hydrochloride (68,9 mg, 0.367 mmol) in DMF (1.5 mL). The reaction mixture was stirred at 60°C for 12h. The crude was purified with FractionLynx (column: XTerra Prep Column, 5um, 30x75, Flow 30 mL/min) to obtain **E93** (43.5 mg, 0.107 mmol, yield= 29.21%) as white solid.

Method 2: R*t*= 1.40 min, MS (ESI) m/z =406.27 [M+H]⁺.

¹H NMR (400 MHz, DMSO-*d*₆) δ ppm 8.80 (br d, J=8.1 Hz, 1 H), 8.05 (d, J=5.2 Hz, 1 H), 7.17 - 7.24 (m, 2 H), 7.15 (dd, J=7.4, 1.4 Hz, 1 H), 7.10 (dd, J=9.2, 2.1 Hz, 1 H), 6.97 - 7.03 (m, 1 H), 6.96 (d, J=8.0 Hz, 1 H), 6.85 - 6.90 (m, 3 H), 6.79 (br s, 1 H), 5.47 (q, J=8.0 Hz, 1 H), 3.79 (s, 3 H), 3.38 - 3.47 (m, 2 H), 2.94 - 3.07 (m, 1 H), 2.83 - 2.89 (m, 1 H), 2.78 - 2.83 (m, 2 H), 2.40 - 2.49 (m, 1 H), 2.01 (dq, J=12.6, 8.5 Hz, 1 H)

### Example 94: N-(4-fluorobenzyl)-2-((2-methoxyphenethyl)amino)-isonicotinamide (E94)

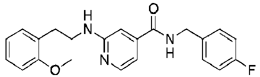

Prepared similarly as described for compound **E1** (stepA) starting from **D2** (120 mg, 0.440 mmol) and (4-fluorophenyl)methanamine (60 µL, 0.530 mmol). The reaction was stirred for 3h. The mixture was quenched with 5 mL of NaOH (1M), separated by a phase separator, and the organic layer was concentrated under high vacuum to give **E94** (78 mg, 0.206 mmol, yield= 46.65%) as colourless oil.

Method 2: R*t*= 1.25 min, MS (ESI) m/z = 380.21 [M+H]⁺.

¹H NMR (400 MHz, DMSO-*d*₆) δ ppm 9.05 (br t, J=5.9 Hz, 1 H), 8.06 (d, J=5.2 Hz, 1 H), 7.34 (dd, J=8.6, 5.6 Hz, 2 H), 7.10 - 7.23 (m, 4 H), 6.96 (d, J=8.0 Hz, 1 H), 6.81 - 6.90 (m, 3 H), 6.78 (br t, J=5.6 Hz, 1 H), 4.42 (d, J=5.9 Hz, 2 H), 3.79 (s, 3 H), 3.41 (q, J=7.0 Hz, 2 H), 2.81 (t, J=7.5 Hz, 2 H)

### Example 95: N-(4-fluorocuban-1-yl)-2-((2-methoxyphenethyl)-amino)isonicotinamide (E95)

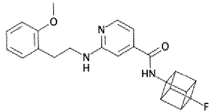

Prepared similarly as described for compound **E1** (stepA) starting from **D2** (58 mg, 0.210 mmol) and 4-fluorocuban-1-amine;hydrochloride (36.98 mg, 0.210 mmol). The reaction was stirred at RT for 3h. The mixture was quenched with aq Na₂CO₃ (10 mL), separated by a phase separator, and the organic layer was concentrated under high vacuum. The crude was purified by RP Flash Chromatography (Sfar C18, 12 g, eluent: CH₃CN (+0.1% HCOOH) in H₂O (+0.1% HCOOH)) to give an impure product. Additional work-up with ss NaHCO₃ (2 mL) and DCM extraction (2 x 4 mL) was conducted. Organics were concentrated under vacuum to afford **E95** (36 mg, 0.092 mmol, yield= 43.18%) as white solid.

Method 2: R*t*= 1.28 min, MS (ESI) m/z = 392.2 [M+H]⁺.

¹H NMR (400 MHz, DMSO-*d*₆) δ ppm 9.25 (s, 1H), 8.06 (d, J = 5.8 Hz, 1H), 7.23-7.17 (m, 1H), 7.14 (dd, J = 7.3, 1.4 Hz, 1H), 6.96 (d, J = 8.1 Hz, 1H), 6.86 (t, J = 7.4 Hz, 1H), 6.84-6.80 (m, 2H), 6.78 (t, J = 5.5 Hz, 1H), 4.28-4.18 (m, 3H), 4.02-3.94 (m, 3H), 3.79 (s, 3H), 3.45-3.38 (m, 2H), 2.81 (t, J = 7.4 Hz, 2H)

### Example 96: (R)-N-(1-(4-fluorophenyl)ethyl)-2-((2-methoxyphenethyl)amino)isonicotinamide (E96)

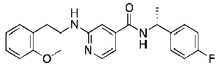

Prepared similarly as described for compound **E3** starting from **D2** (100 mg, 0.370 mmol) and (1R)-1-(4-fluorophenyl)ethanamine (61.33 mg, 0.440 mmol) in DMSO (1.3 mL). The reaction was stirred at 60 °C for 16h. Water (3 mL) was added, and the mixture was cooled to 5°C for 30 minutes. The solid was filtered, washed with water (2 x 2 mL) and dried under vacuum. Solid was suspended in water (2 mL) and heated to 65°C for 1 h. Solid was filtered, washed with water (2 x 2 mL) and dried under vacuum at 45° for 16 h to afford **E96** (67 mg, 0.170 mmol, yield= 46.37%) as cream solid.

Method 2: Rt= 1.33 min, MS (ESI) m/z = 394.3 [M+H]⁺.

¹H NMR (400 MHz, DMSO-*d*₆) δ ppm 8.83 (d, J=8.1 Hz, 1 H), 8.06 (d, J=5.3 Hz, 1 H), 7.40 (dd, J=8.6, 5.6 Hz, 2 H), 7.09 - 7.25 (m, 4 H), 6.95 (d, J=8.1 Hz, 1 H), 6.79 - 6.91 (m, 3 H), 6.76 (t, J=5.6 Hz, 1 H), 5.12 (quin, J=7.3 Hz, 1 H), 3.79 (s, 3 H), 3.37 - 3.46 (m, 2 H), 2.80 (t, J=7.4 Hz, 2 H), 1.44 (d, J=7.1 Hz, 3 H)

### Example 97: N-(cuban-1-yl)-2-{[2-(2-methoxyphenyl)ethyl]amino}pyridine-4-carboxamide (E97)

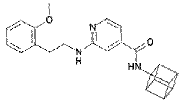

Prepared similarly as described for compound **E3** starting from **D2** (55 mg, 0.180 mmol) and cuban-1-amine (21.66 mg, 0.180 mmol). The reaction was stirred at RT for 16 h. Water was added, and the mixture was extracted with EtOAc (4 x 10 mL). Organic phase was washed with brine, dried over anh Na₂SO₄ and concentrated under a vacuum The crude was purified by RP Flash Chromatography (Sfar C18, 12 g, eluent: CH₃CN (+0.1% HCOOH) in H₂O (+0.1% HCOOH)) to give **E97** (13 mg, 0.035 mmol, yield= 19.15%) as white solid.

Method 2: R*t*= 1.38 min, MS (ESI) m/z = 374.3 [M+H]⁺.

¹H NMR (400 MHz, DMSO-*d*₆) δ ppm 9.13 (s, 1H), 8.09-8.02 (m, 1H), 7.23-7.17 (m, 1H), 7.15 (dd, J = 7.5, 1.4 Hz, 1H), 6.96 (d, J = 8.0 Hz, 1H), 6.89-6.85 (m, 1H), 6.86-6.82 (m, 2H), 6.88-6.70 (m, 1H), 4.16 (ddd, J = 5.9, 3.8, 2.3 Hz, 3H), 3.97-3.87 (m, 4H), 3.79 (s, 3H), 3.46-3.37 (m, 2H), 2.82 (t, J = 7.5 Hz, 2H)

### Example 98: (4-(benzo[d]thiazol-2-yl)piperidin-1-yl)(2-((2-methoxyphenethyl)amino)pyridin-4-yl)methanone (E98)

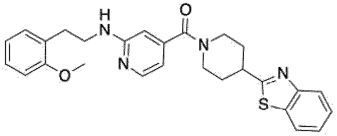

Prepared similarly as described for compound **E3** starting from **D2** (241 mg, 0.61 mmol, 1.2 eq.), 2-(4-Piperidinyl)-1,3-benzothiazole (111 mg, 0.5084 mmol, 1.0 eq.) and DIPEA (270 µL, 1.525 mmol, 3.0 eq.) in NMP (3.0 mL). After work-up, the crude was purified using RP Preparative HPLC (eluent: CH₃CN (+0.1% HCOOH) in H₂O (+0.1% HCOOH)). The fraction containing the product of interest was concentrated and freeze-dried. to yield **E98** (56.9 mg, 0.1173 mmol, yield= 23%) as a white powder.

Method 4: R*t*= 2.41 min, MS (ESI) m/z = 473.2 [M+H]⁺.

¹H NMR (400 MHz, DMSO-*d*₆) δ ppm 8.12-8.06 (m, 1H), 8.04 (dd, J = 5.0, 0.9 Hz, 1H), 8.00-7.93 (m, 1H), 7.51 (ddd, J = 8.4, 7.2, 1.3 Hz, 1H), 7.43 (ddd, J = 8.3, 7.2, 1.3 Hz, 1H), 7.23-7.13 (m, 2H), 6.96 (dd, J = 8.2, 1.1 Hz, 1H), 6.87 (td, J = 7.4, 1.1 Hz, 1H), 6.78 (t, J = 5.7 Hz, 1H), 6.48-6.42 (m, 2H), 4.53 (d, J = 13.0 Hz, 1H), 3.79 (s, 3H), 3.67 (d, J = 13.4 Hz, 1H), 3.55-3.38 (m, 3H), 3.24 (t, J = 12.8 Hz, 1H), 3.00 (t, J = 12.2 Hz, 1H), 2.82 (t, J = 7.4 Hz, 2H), 2.23 (d, J = 13.0 Hz, 1H), 2.11 (d, J = 13.3 Hz, 1H), 1.85-1.68 (m, 2H).

### Example 99: (6,6-difluoro-3-azabicyclo[3.1.0]hexan-3-yl)(2-((2-hydroxyphenethyl)amino)pyridin-4-yl)methanone (E99)

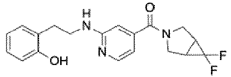

Prepared similarly as described for compound **E49** starting from **D8** (110 mg, 0.4542 mmol, 1.0 eq) and 2-(2-aminoethyl)phenol (156 mg, 1.135 mmol, 2.5 eq) in anh NMP (5 mL, 0.09 M). The reaction was stirred at 180°C for 6 hours. After work-up, the crude was purified using RP Preparative HPLC (eluent: CH₃CN (+0.1% HCOOH) in H₂O (+0.1% HCOOH)) to yield **E99** 2 (115 mg, 0.315 mmol, yield= 70%) as a white powder.

Method 4: R*t*= 1.89 min, MS (ESI) m/z = 360.2 [M+H]⁺.

¹H NMR (400 MHz, DMSO-*d*₆) δ ppm 9.47 (s, 1H), 8.03 (d, J = 5.7 Hz, 1H), 7.07 (dd, J = 7.4, 1.7 Hz, 1H), 7.02 (td, J = 7.6, 1.8 Hz, 1H), 6.84 (t, J = 5.6 Hz, 1H), 6.80 (dd, J = 8.1, 1.2 Hz, 1H), 6.72 (td, J = 7.4, 1.2 Hz, 1H), 6.46-6.40 (m, 2H), 4.05 (d, J = 12.7 Hz, 1H), 3.85-3.75 (m, 1H), 3.72-3.62 (m, 1H), 3.56 (d, J = 11.4 Hz, 1H), 3.46-3.36 (m, 2H), 2.77 (dd, J = 8.6, 6.3 Hz, 2H), 2.65-2.52 (m, 2H).

### Example 100: (2-((2-methoxyphenethyl)amino)pyridin-4-yl)(pyrrolidin-1-yl)methanone (E100)

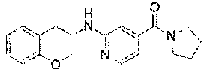

Prepared similarly as described for compound **E49** starting from **D55** (229 mg, 0.988 mmol, 1.0 eq) and 2-(2-methoxyphenyl)ethan-1-amine (224 mg, 1.482 mmol, 1.5 eq) in anh NMP (5.0 mL, 0.1975 M). The reaction was stirred at 180°C, ON. After work-up, the crude was purified using RP Preparative HPLC (eluent: CH₃CN (+0.1% HCOOH) in H₂O (+0.1% HCOOH)) to yield E100 (126 mg, 0.383 mmol, yield= 39%).

Method 4: R*t*= 1.80 min, MS (ESI) m/z = 326.2 [M+H]⁺.

¹H NMR (400 MHz, DMSO-*d*₆) δ ppm 8.04-7.99 (m, 1H), 7.23-7.12 (m, 2H), 6.96 (dd, J = 8.3, 1.1 Hz, 1H), 6.87 (td, J = 7.4, 1.1 Hz, 1H), 6.72 (t, J = 5.7 Hz, 1H), 6.49 (d, J = 4.7 Hz, 2H), 3.79 (s, 3H), 3.45-3.36 (m, 4H), 3.31 (t, J = 6.4 Hz, 2H), 2.80 (dd, J = 8.5, 6.4 Hz, 2H), 1.89-1.76 (m, 4H).

### Example 101: (S)-(3-hydroxypyrrolidin-1-yl)(2-((2-methoxyphenethyl)amino)pyridin-4-yl)methanone(E101)

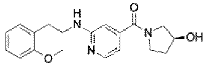

Prepared similarly as described for compound **E49** starting from **D9** (237 mg, 0.9245 mmol, 1.0 eq), 2-(2-methoxyphenyl)ethan-1-amine (210 mg, 1.3868 mmol, 1.5 eq) and DIPEA (320 µL, 1.817 mmol, 4.0 eq) in anh NMP (6 mL, 0.15 M). The reaction mixture was stirred at 180°C for 18 hours. After work-up, the crude was purified using preparative RP Preparative HPLC (eluent: CH₃CN (+0.1% HCOOH) in H₂O (+0.1% HCOOH)) to yield **E101** (31.5 mg, 0.092 mmol, yield= 10%) as a white solid.

Method 4: R*t*= 1.54 min, MS (ESI) m/z = 342.2 [M+H]⁺.

δ ppm H (300 MHz, DMSO-*d*₆) 8.07-7.99 (,m,1 H), 7.27-7.11 (m, 2 H), 6.97 (dd, J = 8.3, 1.1 Hz, 1 H), 6.88 (td,J =7.4, 1.0 Hz, 1 H), 6.80-6.70 (m, 1 H), 6.54-6.45 (m, 2 H), 4.98 (s, 1 H), 4.28 (d, J =25.9 Hz, 1 H), 3.80 (s, 3 H), 3.58-3.40 (m, 5 H), 3.23-3.06 (m, 1 H), 2.82 (dd, J =8.5, 6.3 Hz, 2 H), 2.02-1.87 (m, 1 H), 1.87-1.72 (m, 1 H).

### Example 102: (R)-(3-hydroxypyrrolidin-1-yl)(2-((2-methoxyphenethyl)amino)pyridin-4-yl)methanone (E102)

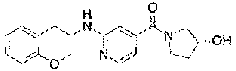

Prepared similarly as described for compound **E49** starting from **D10** (110 mg, 0.508 mmol, 1.0 eq), 2-(2-methoxyphenyl)ethan-1-amine (115 mg, 0.761 mmol, 1.5 eq) and DIPEA (354 µL, 2.03 mmol, 4.0 eq) in anh NMP (3.38 mL, 0.15 M). The reaction mixture was stirred for 5 hours at 180°C. After work-up, the crude product was purified on RP preparative HPLC (eluent: CH₃CN (+0.1% HCOOH) in H₂O (+0.1% HCOOH)) affording **E102** (0.05 g, 0.146 mmol, yield= 29%).

Method 4: R*t*= 1.54 min, MS (ESI) m/z = 342.2 [M+H]⁺.

¹H NMR (400 MHz, DMSO-*d*₆) δ ppm 8.05-7.99 (m, 1H), 7.23-7.11 (m, 2H), 6.96 (dd, J = 8.3, 1.1 Hz, 1H), 6.90-6.83 (m, 1H), 6.78-6.69 (m, 1H), 6.52-6.43 (m, 2H), 5.00 (d, 1H), 4.36-4.15 (m, 1H), 3.79 (s, 3H), 3.56-3.38 (m, 5H), 3.15 (d, J = 11.3 Hz, 1H), 2.86-2.74 (m, 2H), 1.99-1.71 (m, 2H).

### Example 103: (4-(benzo[d]thiazol-2-yl)piperidin-1-yl)(2-((2-methoxybenzyl)amino)pyridin-4-yl)methanone (E103)

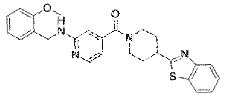

Prepared similarly as described for compound **E49** starting from **D51** (350 mg, 0.7894 mmol, 1.0 eq.), 2-methoxybenzylamine (310 µL, 2.3682 mmol, 3.0 eq.) and DIPEA (550 µL, 3.1576 mmol, 4.0 eq.) in anh NMP (7.5 mL). The vial was sealed and stirred at 180 °C, ON. After work-up, the crude product was purified on RP preparative HPLC (eluent: CH₃CN (+0.1% HCOOH) in H₂O (+0.1% HCOOH)) to yield **E103** (60 mg, 0,131mmol, yield= 17%).

Method 4: R*t*= 2.37 min, MS (ESI) m/z = 458.9 [M+H]⁺.

¹H NMR (400 MHz, DMSO-*d*₆) δ ppm 8.08 (ddd, J = 7.9, 1.4, 0.7 Hz, 1H), 8.01 (dd, J = 5.1, 0.8 Hz, 1H), 7.98-7.93 (m, 1H), 7.50 (ddd, J = 8.3, 7.2, 1.3 Hz, 1H), 7.42 (ddd, J = 8.3, 7.2, 1.3 Hz, 1H), 7.21 (t, J = 7.6 Hz, 2H), 7.05 (t, J = 5.9 Hz, 1H), 6.99-6.93 (m, 1H), 6.87 (td, J = 7.4, 1.1 Hz, 1H), 6.50-6.43 (m, 2H), 4.57-4.40 (m, 3H), 3.81 (s, 3H), 3.65 (d, J = 13.4 Hz, 1H), 3.46 (ddt, J = 11.2, 7.4, 3.9 Hz, 1H), 3.21 (t, J = 12.7 Hz, 1H), 2.98 (t, J = 12.3 Hz, 1H), 2.22 (d, J = 13.2 Hz, 1H), 2.07 (d, J = 12.8 Hz, 1H), 1.74 (t, J = 15.9 Hz, 2H).

### Example 104: (2-((2-(cyclopentyloxy)phenethyl)amino)pyridin-4-yl)(6,6-difluoro-3-azabicyclo[3.1.0]hexan-3-yl)methanone (E104)

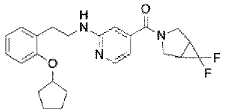

Prepared similarly as described for compound **E49** starting from **D8** (110 mg, 0.4542 mmol, 1.0 eq) and **D54** (200 mg, 0.681 mmol, 1.5 eq) and DIPEA (0.32 mL, 1.817 mmol, 4.0 eq) in anh NMP (5 mL, 0.09 M). The vial was sealed and stirred at 180 °C for 18h. After work-up, the crude product was purified on RP preparative HPLC (eluent: CH₃CN (+0.1% HCOOH) in H₂O (+0.1% HCOOH)) to yield **E104** (85.4 mg, 0.198 mmol, yield= 99.03%) as a off-white sticky powder.

Method 4: R*t*= 2.53 min, MS (ESI) m/z = 428.2 [M+H]⁺.

¹H NMR (400 MHz, DMSO-*d*₆) δ ppm 8.03 (dd, J =5.1, 0.8 Hz,1 H), 7.20-7.10 (m, 2 H), 6.97-6.90 (m, 1 H), 6.83 (td, J =7.4, 1.1Hz, 1 H), 6.77 (t, J =5.7Hz, 1 H), 6.45-6.37 (m, 2 H), 4.85 (ddd, J =7.2, 5.3, 1.7 Hz, 1 H), 4.04 ( d,J =12.7 Hz, 1 H), 3.79 (dt, J =11.2, 4.6 Hz,1 H), 3.67 (dt, J =12.7, 4.8Hz, 1 H), 3.56 (d, J =11.4 Hz, 1 H), 3.41 (q, J =6.6 Hz, 2 H), 2.77 (t, J =8.9 Hz, 2 H), 2.59 (qd, J =12.6, 11.3, 5.0 Hz, 2 H), 1.90 (d, J =13.1 Hz, , 2 H), 1.79-1.66 (m, 4 H), 1.65-1.52 (m, 2 H).

### Example 105: (2-ethylpiperidin-1-yl)(2-((2-methoxyphenethyl)amino)-6-morpholinopyridin-4-yl)methanone (E105)

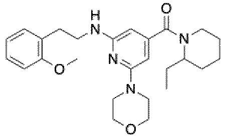

A mixture of Palladium (II) Acetate (390 mg) and [1-(2-diphenylphosphino-1-naphthalenyl)-2-naphthalenyl]-diphenylphosphine (rac-BINAP) (54.2 mg, 0.090 mmol) in 1,2-dimethoxyethane (5 mL) was stirred for 10 min at RT. Then sodium tert-butylate (33.5 mg, 0.350 mmol) was added giving dark pink solution. **E71** (70 mg, 0.170 mmol) and morpholine (0.02 mL, 0.210 mmol) were sequentially added and the solution was refluxed for 12h. The reaction mixture was diluted with DCM and water. The mixture was purified by RP Flash Chromatography (Sfar C18, 12 g, 2-98% CH₃CN (+0.1% HCOOH) in H₂O (+0.1% HCOOH)) to give **E105** (13.3 mg, 0.029 mmol, yield= 16.87%).

Method 2: R*t*= 1.98min, MS (ESI) m/z = 453.33 [M+H]⁺.

¹H NMR (500 MHz, CD₃OD) δ ppm 7.17 (td, J=7.8, 1.7 Hz, 1 H), 7.08 - 7.13 (m, 1 H), 6.92 (d, J=8.1 Hz, 1 H), 6.84 (td, J=7.4, 0.8 Hz, 1 H), 5.74 - 5.81 (m, 2 H), 3.82 (s, 3 H), 3.72 - 3.80 (m, 4 H), 3.70 - 4.72 (m, 1 H), 3.50 - 4.51 (m, 1 H), 3.42 - 3.49 (m, 6 H), 2.84 - 2.92 (m, 2 H), 2.77 - 3.14 (m, 1 H), 1.35 - 1.95 (m, 8 H), 0.76 - 1.00 (m, 3 H)

### Example 106: (2-(3-azabicyclo[3.1.0]hexan-3-yl)-6-((2-methoxyphenethyl) amino)pyridin-4-yl)(2-ethylpiperidin-1-yl)methanone (E106)

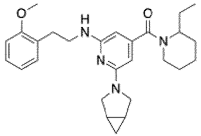

Prepared similarly as described for compound E105 starting from E71 (68 mg, 0.170 mmol) and 3-azabicyclo[3.1.0]hexane hydrochloride (20 mg, 0.170 mmol). After work-up the crude was purified by RP Flash Chromatography (Sfar C18, 12 g, 2-98% CH₃CN (+0.1% HCOOH) in H₂O (+0.1% HCOOH)) to yield **E106** (4 mg, 0.009 mmol, yield= 5.32%).

Method 2: R*t*= 1.65min, MS (ESI) m/z = 449.29[M+H]⁺.

¹H NMR (400 MHz, DMSO-*d*₆) δ ppm 7.19 (td, J=7.4, 1.6 Hz, 1 H), 7.14 (dd, J=7.2, 1.6 Hz, 1 H), 6.96 (d, J=8.0 Hz, 1 H), 6.87 (t, J=7.4 Hz, 1 H), 6.35 (br t, J=4.8 Hz, 1 H), 5.58 (br d, J=6.4 Hz, 1 H), 5.36 (s, 1 H), 3.77 (br s, 3 H), 3.53 - 4.66 (m, 1 H), 3.36 - 4.39 (m, 1 H), 3.21 - 3.70 (m, 4 H), 3.20 - 3.42 (m, 2 H), 2.74 - 2.86 (m, 2 H), 2.56 - 3.08 (m, 1 H), 1.16 - 2.05 (m, 10 H), 0.60 - 0.93 (m, 4 H), 0.11 - 0.23 (m, 1 H)

### Example 107: (6,6-difluoro-3-azabicyclo[3.1.0]hexan-3-yl)(2-((3-fluoro-2-methoxyphenethyl)amino)pyridin-4-yl)methanone (E107)

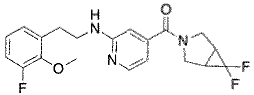

Prepared similarly as described for compound **E60** starting from **D60** (100 mg, 0.390 mmol) and 2-(3-fluoro-2-methoxy-phenyl)ethanamine;hydrochloride (95.4 mg, 0.460 mmol). After work-up and after RP Flash Chromatography (Sfar C18, 12 g, 2-37% CH₃CN (+0.1% HCOOH) in H₂O (+0.1% HCOOH)) was obtained **E107** (10 mg, 0.026 mmol, yield= 6.6%) as yellow oil.

Method 2: R*t*= 2.15min, MS (ESI) m/z = 414.07 [M+Na]⁺.

¹H NMR (500 MHz, CHLOROFORM-*d*) δ ppm 8.90 - 9.26 (m, 1 H), 7.73 - 7.87 (m, 1 H), 6.93 - 7.06 (m, 3 H), 6.84 (s, 1 H), 6.65 (br d, J=5.8 Hz, 1 H), 4.28 (br d, J=13.0 Hz, 1 H), 4.00 (d, J=2.2 Hz, 3 H), 3.79 - 3.89 (m, 1 H), 3.59 - 3.78 (m, 2 H), 3.44 - 3.58 (m, 2 H), 2.93 - 3.09 (m, 2 H), 2.31 - 2.52 (m, 2 H)

### Example 108: (6,6-difluoro-3-azabicyclo[3.1.0]hexan-3-yl)(2-((5-fluoro-2-methoxyphenethyl)amino)pyridin-4-yl)methanone (E108)

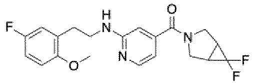

Prepared similarly as described for compound **E8** starting from **D72** 2-[2-(5-fluoro-2-methoxy-phenyl)ethylamino]pyridine-4-carboxylic acid (20 mg, 0.070 mmol) and 6,6-difluoro-3-azabicyclo[3.1.0]hexane;hydrochloride (12.86 mg, 0.080 mmol). The reaction mixture was stirred at RT for 12h. After work-up the residue was purified by RP Flash Chromatography (silica C18 12 g,1-50% CH₃CN (+0.1% HCOOH) in H₂O (+0.1% HCOOH)) to give **E108** ( 3 mg, 0.008 mmol, yield= 11.13%) as yellow oil.

Method 2: R*t*= 1.12min, MS (ESI) m/z =392.02 [M+H]⁺.

¹H NMR (400 MHz, CD₃OD) δ ppm 7.99 - 8.04 (m, 1 H), 6.86 - 6.96 (m, 3 H), 6.46 - 6.52 (m, 2 H), 3.81 - 3.83 (m, 3 H), 3.63 - 4.20 (m, 4 H), 3.46 - 3.53 (m, 2 H), 2.84 - 2.92 (m, 2 H), 2.36 - 2.55 (m, 2 H)

### Example 109: (2-((3,6-difluoro-2-methoxyphenethyl)amino)pyridin-4-yl)(6,6-difluoro-3-azabicyclo[3.1.0]hexan-3-yl)methanone (E109)

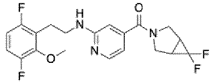

Prepared similarly as described for compound **E3** starting from **D73** (100 mg, 0.320 mmol) and 6,6-difluoro-3-azabicyclo[3.1.0]hexane;hydrochloride (66.8 mg, 0.390 mmol). The reaction was stirred at 70 °C for 12h. After work-up, the residue was purified by RP Flash Chromatography (silica C18 12 g, 1-50% CH₃CN (+0.1% HCOOH) in H₂O (+0.1% HCOOH)) to give **E109** ( 48.4 mg, 0.118 mmol, yield= 36.45%) as solid.

Method 2: Rt*=* 1.14min, MS (ESI) m/z =410.21 [M+H]⁺.

¹H NMR (400 MHz, DMSO-*d*₆) δ ppm 8.03 (d, J=5.1 Hz, 1 H), 7.18 (ddd, J=11.3, 9.2, 5.4 Hz, 1 H), 6.87 - 6.99 (m, 2 H), 6.43 (dd, J=5.1, 1.3 Hz, 1 H), 6.39 (s, 1 H), 4.04 (d, J=12.6 Hz, 1 H), 3.86 (d, J=1.8 Hz, 3 H), 3.78 (dt, J=11.3, 4.4 Hz, 1 H), 3.66 (dt, J=12.5, 4.7 Hz, 1 H), 3.54 (d, J=11.4 Hz, 1 H), 3.38 - 3.48 (m, 2 H), 2.86 (t, J=7.1 Hz, 2 H), 2.52 - 2.68 (m, 2 H)

### Example 110: (6,6-difluoro-3-azabicyclo[3.1.0]hexan-3-yl)(2-((2-methoxy-4-(methoxymethoxy)phenethyl)amino)pyridin-4-yl)methanone (E110)

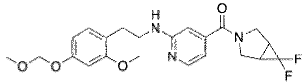

Prepared similarly as described for compound **E57** starting from **D74** (200 mg, 0.600 mmol) and 6,6-difluoro-3-azabicyclo[3.1.0]hexane;hydrochloride (103 mg, 0.660 mmol). After work-up, the crude was purified by RP Flash Chromatography (silica C18 12 g, 1-70% CH₃CN (+0.1% HCOOH) in H₂O (+0.1% HCOOH)) to give **E110** ( 220 mg, 0.508 mmol, yield= 84.34%) as a white solid.

Method 2: R*t*= 1.06min, MS (ESI) m/z =434.3[M+H]⁺.

¹H NMR (500 MHz, DMSO-*d*₆) δ ppm 8.02 (dd, J=4.9, 0.8 Hz, 1 H), 7.03 (d, J=8.2 Hz, 1 H), 6.75 (t, J=5.6 Hz, 1 H), 6.62 (d, J=2.2 Hz, 1 H), 6.54 (dd, J=8.1, 2.3 Hz, 1 H), 6.38 - 6.44 (m, 2 H), 5.16 (s, 2 H), 3.76 (s, 3 H), 3.51 - 4.10 (m, 4 H), 3.37 (s, 3 H), 3.33 - 3.40 (m, 2 H), 2.72 (t, J=7.4 Hz, 2 H), 2.51 - 2.62 (m, 2 H)

### Example 111: (6,6-difluoro-3-azabicyclo[3.1.0]hexan-3-yl)(2-((4-hydroxy-2-methoxyphenethyl)amino)pyridin-4-yl)methanone (E111)

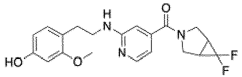

To a solution of compound **E110** (212 mg, 0.490 mmol) in MeOH (1.015 mL), Hydrogen chloride (0.04 mL, 0.490 mmol) was added and the reaction was stirred at RT, ON. Subsequently, the solvent was removed to give **E111** (120 mg, 0.308 mmol, yield= 62.96%) as solid.

Method 2: R*t*= 0.78min, MS (ESI) m/z =390.1 [M+H]⁺.

¹H NMR (400 MHz, DMSO-*d*₆) δ ppm 7.97 (br d, J=5.9 Hz, 1 H), 6.95 - 7.03 (m, 2 H), 6.77 (d, J=6.2 Hz, 1 H), 6.39 (d, J=2.2 Hz, 1 H), 6.30 (dd, J=8.1, 2.2 Hz, 1 H), 4.04 (br d, J=12.8 Hz, 1 H), 3.81 (dt, J=11.2, 4.2 Hz, 2 H), 3.69 (s, 3 H), 3.65 - 3.76 (m, 1 H), 3.58 (br d, J=10.9 Hz, 1 H), 3.44 - 3.53 (m, 2 H), 2.76 (br t, J=7.2 Hz, 2 H), 2.57 - 2.70 (m, 2 H).

### Example 112: (6,6-difluoro-3-azabicyclo[3.1.0]hexan-3-yl)(2-((2,4-dimethoxyphenethyl)amino)pyridin-4-yl)methanone (E112)

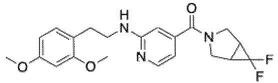

To a solution of **E111** (60 mg, 0.150 mmol) in DMF (308 µL), potassium carbonate (21.3 mg, 0.150 mmol) and iodomethane (10 µL, 0.150 mmol) were added and the reaction was stirred at RT for 3h. The reaction was poured in water and extracted several times with EtOAc. The organic phase was collected and evaporated in vacuum. The crude was purified by NP Flash Chromatography (Sfar D,125 g, 0-10% MeOH in DCM) to produce **E112** (8.1 mg, 0.020 mmol, yield= 13.03%) as oil.

Method 2: R*t*= 1.05min, MS (ESI) m/z =404.18 [M+H]⁺.

¹H NMR (400 MHz, DMSO-*d*₆) δ ppm 8.01 (d, J=5.4 Hz, 1 H), 7.06 (d, J=8.2 Hz, 1 H), 6.48 - 6.59 (m, 3 H), 6.45 (dd, J=8.3, 2.4 Hz, 1 H), 4.04 (d, J=12.7 Hz, 1 H), 3.76 (s, 3 H), 3.73 (s, 3 H), 3.63 - 3.83 (m, 2 H), 3.55 (br d, J=11.3 Hz, 1 H), 3.34 - 3.45 (m, 2 H), 2.70 - 2.79 (m, 2 H), 2.53 - 2.67 (m, 2 H)

### Example 113: (6,6-difluoro-3-azabicyclo[3.1.0]hexan-3-yl)(2-((2,5-dimethoxyphenethyl)amino)pyridin-4-yl)methanone (E113)

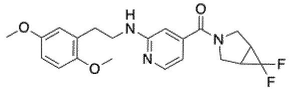

Prepared similarly as described for compound **E3** starting from **D75** (100 mg, 0.330 mmol) and 6,6-difluoro-3-azabicyclo[3.1.0]hexane;hydrochloride (68.12 mg, 0.400 mmol). After work-up, the residue was purified by RP Flash Chromatography (silica C18 12 g, 1-50% CH₃CN (+0.1% HCOOH) in H₂O (+0.1% HCOOH)) to give **E113** (27.1 mg, 0.067 mmol, yield= 20.31%) as solid.

Method 2: R*t*= 1.04min, MS (ESI) m/z = 404.28[M+H]⁺.

¹H NMR (400 MHz, DMSO-*d*₆) δ ppm 7.96 - 8.06 (m, 1 H), 6.83 - 6.92 (m, 1 H), 6.75 - 6.79 (m, 1 H), 6.72 - 6.75 (m, 2 H), 6.35 - 6.45 (m, 2 H), 3.71 - 3.76 (m, 3 H), 3.67 - 3.69 (m, 3 H), 3.50 - 4.07 (m, 4 H), 3.36 - 3.44 (m, 2 H), 2.72 - 2.81 (m, 2 H), 2.52 - 2.63 (m, 2 H)

### Example 114: (6,6-difluoro-3-azabicyclo[3.1.0]hexan-3-yl)(2-((2,4-dimethoxy-3-methylphenethyl)amino)pyridin-4-yl)methanone (E114)

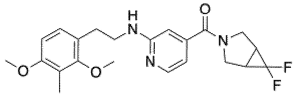

Prepared similarly as described for compound **E8** starting from **D76** (103.5 mg, 0.330 mmol) and 6,6-difluoro-3-azabicyclo[3.1.0]hexane;hydrochloride (61.1 mg, 0.390 mmol). The reaction was stirred at 70°C for 12h. After work-up, the residue was purified by RP Flash Chromatography (silica C18 12 g, 1-50% CH₃CN (+0.1% HCOOH) in H₂O (+0.1% HCOOH)) to give **E114** (40 mg, 0.096 mmol, yield= 29.28%) as solid.

Method 2: R*t*= 1.16min, MS (ESI) m/z = 418.29[M+H]⁺.

¹H NMR (400 MHz, DMSO-*d*₆) δ ppm 7.93 - 8.08 (m, 1 H), 6.98 - 7.06 (m, 1 H), 6.76 - 6.86 (m, 1 H), 6.64 - 6.74 (m, 1 H), 6.39 - 6.44 (m, 2 H), 3.73 - 3.76 (m, 3 H), 3.64 - 3.66 (m, 3 H), 3.52 - 4.09 (m, 4 H), 3.39 - 3.46 (m, 2 H), 2.72 - 2.81 (m, 2 H), 2.51 - 2.64 (m, 2 H), 2.00 - 2.11 (m, 3 H).

### Example 115: (6,6-difluoro-3-azabicyclo[3.1.0]hexan-3-yl)(2-((2-(4-methoxy-1H-indol-3-yl)ethyl)amino)pyridin-4-yl)methanone (E115)

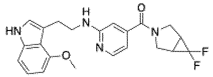

Prepared similarly as described for compound **E3** starting from **D77** (20 mg, 0.060 mmol) and 6,6-difluoro-3-azabicyclo[3.1.0]hexane (9.2 mg, 0.080 mmol). After work-up, the residue was purified by RP Flash Chromatography (C18 column 12 g, 2-98% CH₃CN (+0.1% HCOOH) in H₂O (+0.1% HCOOH)) to give **E115** (12 mg, 0.029 mmol, yield= 45.29%) as yellow oil.

Method 2: R*t*= 1.02min, MS (ESI) m/z = 413.3 [M+H]⁺.

¹H NMR (400 MHz, DMSO-*d*₆) δ ppm 10.78 (br s, 1 H), 8.02 (d, J=5.0 Hz, 1 H), 6.99 (d, J=1.5 Hz, 1 H), 6.89 - 6.98 (m, 2 H), 6.75 (br s, 1 H), 6.39 - 6.48 (m, 3 H), 4.04 (d, J=12.7 Hz, 1 H), 3.84 (s, 3 H), 3.73 - 3.82 (m, 1 H), 3.60 - 3.71 (m, 1 H), 3.45 - 3.59 (m, 3 H), 3.03 (t, J=7.3 Hz, 2 H), 2.53 - 2.68 (m, 2 H).

### Example 116: (1,1-difluoro-5-azaspiro[2.4]heptan-5-yl)(2-((2-methoxyphenethyl)amino)pyridin-4-yl)methanone (E116)

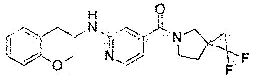

Prepared similarly as described for compound **E3** starting from **D2** (238 mg, 0.616 mmol, 1.2 eq.) and 1,1-difluoro-5-azaspiro[2.4]heptane hydrochloride (87 mg, 0.513 mmol, 1.0 eq) in NMP. The reaction was stirred at RT for 18h. After work-up, the crude was purified using RP Preparative HPLC (eluent: CH₃CN (+0.1% HCOOH) in H₂O (+0.1% HCOOH)). The appropriate fractions were concentrated and freeze-dried to yield **E116** (51.9 mg, 0.1326 mmol, yield= 26%) as a white powder.

Method 4: R*t*= 2.13 min, MS (ESI) m/z = 388.2 [M+H]⁺.

¹H NMR (400 MHz, DMSO-*d*₆) δ ppm 8.04 (d, J = 5.1 Hz, 1H), 7.25-7.17 (m, 1H), 7.16 (dt, J = 7.6, 1.9 Hz, 1H), 6.97 (d, J = 8.2 Hz, 1H), 6.87 (t, J = 7.4 Hz, 1H), 6.76 (q, J = 6.4, 6.0 Hz, 1H), 6.57-6.47 (m, 2H), 3.79 (d, J = 2.0 Hz, 3H), 3.73-3.46 (m, 4H), 3.41 (q, J = 6.7 Hz, 2H), 2.81 (t, J = 7.4 Hz, 2H), 2.15-1.96 (m, 2H), 1.76-1.54 (m, 2H).

### Example 117: (2-((2-methoxyphenethyl)amino)pyridin-4-yl)(3-(oxetan-3-yl)pyrrolidin-1-yl)methanone (E117)

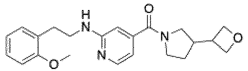

Prepared similarly as described for compound **E3** starting from **D2** (687 mg, 1.514 mmol, 1.1 eq) and 3-(Oxetan-3-yl)pyrrolidine (175 mg, 1.376 mmol, 1.0 eq) in NMP. The reaction was stirred at RT for 18h. After work-up, the crude was purified by RP Preparative HPLC (eluent: CH₃CN (+0.05% NH₃) in H₂O (+0.05% NH₃), basic conditions). The appropriate fractions were concentrated and freeze-dried to yield **E117** (17 mg, 0.044 mmol, yield= 3%).

Method 4: Rt= 2.42 min, MS (ESI) m/z = 388.2 [M+H]⁺.

¹H NMR (400 MHz, DMSO) δ ppm 8.02 (d, J = 5.6 Hz, 1H), 7.24-7.09 (m, 2H), 6.96 (d, J = 8.1 Hz, 1H), 6.87 (td, J = 7.3, 1.1 Hz, 1H), 6.58-6.44 (m, 2H), 6.36 (s, 1H), 4.65 (t, J = 7.1 Hz, 2H), 4.32 (t, J = 6.2 Hz, 2H), 3.81 (s, 3H), 3.53-3.31 (m, 4H), 3.02-2.88 (m, 4H), 2.89-2.78 (m, 2H), 2.01 (s, 1H), 1.62-1.42 (m, 1H).

### Example 118: (6,6-difluoro-3-azabicyclo[3.1.0]hexan-3-yl)(2-(phenethylamino)pyridin-4-yl)methanone) (E118)

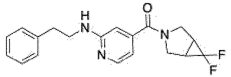

Prepared similarly as described for compound **E49** starting from **D8** (110 mg, 0.4542 mmol, 1.0 eq) and Phenethylamine (82.6 mg, 0.681 mmol, 1.5 eq). The reaction mixture was stirred at 180°C for 18 hours. After work-up, the residue was purified by RP Preparative HPLC (eluent: CH₃CN (+0.1% HCOOH) in H₂O (+0.1% HCOOH)). The appropriate fractions were concentrated and freeze-dried to yield **E118** (67.3 mg, 0.1935 mmol, yield= 43%) off-white solid.

Method 4: Rt*=* 1.93min, MS (ESI) m/z = 344.2 [M+H]⁺.

¹H NMR (400 MHz, DMSO) δ ppm 8.05 (dd, J =5.1, 0.9 Hz, 1 H), 7.34-7.16 (m, 5 H), 6.81 (t, J =5.6 Hz, 1 H), 6.46-6.39 (m, 2 H), 4.04 (d, ,J =12.7 Hz, 1 H), 3.84-3.74 (m, 1 H), 3.71-3.61 (m, 1 H), 3.55 (d, J =11.4 Hz, 1 H), 3.53-3.43 (m, 2 H), 2.83 ( t,J = 7.4 Hz, 2 H), 2.65-2.52 (m, 2 H).

### Example 119: (2-((2-methoxyphenethyl)amino)pyridin-4-yl)(piperidin-1-yl-d₁₀)methanone (E119)

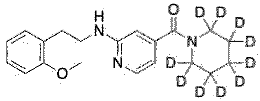

Prepared similarly as described for compound **E3** starting from **D2** (629 mg, 1.247 mmol, 1.2 eq) and Piperidine-d₁₁ (100 mg, 1.039 mmol, 1.0 eq). The reaction mixture was stirred for 24 hours at RT. After work-up, the residue was purified on RP Preparative HPLC (eluent: CH₃CN (+0.05% NH₃) in H₂O (+0.05% NH₃), basic conditions) obtaining **E119** (0.05 g, 0.143 mmol, yield= 14%).

Method 4: R*t*= 1.97 min, MS (ESI) m/z = 350.2 [M+H]⁺.

¹H NMR (400 MHz, DMSO) δ ppm 8.04-7.96 (m, 1H), 7.24-7.08 (m, 2H), 6.96 (dd, J = 8.2, 1.2 Hz, 1H), 6.87 (td, J = 7.3, 1.1 Hz, 1H), 6.74 (t, J = 5.6 Hz, 1H), 6.41-6.31 (m, 2H), 3.78 (s, 3H), 3.47-3.35 (m, 2H), 2.86-2.74 (m, 2H).

### Example 120: (6,6-difluoro-1-azaspiro[3.3]heptan-1-yl)(2-((2-methoxyphenethyl)amino)pyridin-4-yl)methanone (E120)

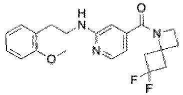

Prepared similarly as described for compound **E49** starting from **D101** (450 mg, 0.141 mmol, 1.0 eq) and 2-(2-methoxyphenyl)ethan-1-amine (64 mg, 0.422 mmol, 3.0 eq) in NMP. The reaction mixture was stirred at 110°C for 12h. After work-up, the crude was purified by RP preparative HPLC (eluent: CH₃CN (+0.05% NH₃) in H₂O (+0.05% NH₃), basic conditions) to give **E120** (9 mg, 0.023 mmol, yield= 16%).

Method 4: R*t*= 2.26 min, MS (ESI) m/z = 388.2 [M+H]⁺.

¹H NMR (400 MHz, DMSO-*d*₆) δ ppm 8.07-8.00 (m, 1H), 7.23-7.17 (m, 1H), 7.14 (dd, J = 7.4, 1.7 Hz, 1H), 6.96 (dd, J = 8.3, 1.1 Hz, 1H), 6.87 (td, J = 7.4, 1.1 Hz, 1H), 6.79 (t, J = 5.7 Hz, 1H), 6.61 (t, J = 1.1 Hz, 1H), 6.58 (dd, J = 5.2, 1.4 Hz, 1H), 4.17 (t, J = 7.5 Hz, 2H), 3.79 (s, 3H), 3.64 (ddd, J = 19.5, 15.0, 12.1 Hz, 2H), 3.40 (q, J = 6.8 Hz, 2H), 2.85-2.72 (m, 4H), 2.46 (s, 2H).

### Example 121: (2,3-dihydro-1H-pyrrolo[3,2-b]pyridin-1-yl)(2-((2-methoxyphenethyl)amino)pyridin-4-yl)methanone (E121)

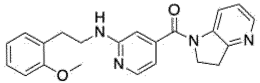

Prepared similarly as described for compound **E49** starting from **D78** (325 mg, 1.229 mmol, 1.0 eq) and 2-(2-methoxyphenyl)ethan-1-amine (279 mg, 1.844 mmol, 1.5 eq) in NMP. The reaction mixture was stirred at 180°C for 3h. After work-up, the residue was purified using RP Preparative HPLC (eluent: CH₃CN (+0.05% NH₃) in H₂O (+0.05% NH₃), basic conditions) to yield **E121** (20 mg, 0.053 mmol, yield= 4%), white powder.

Method 4: R*t*= 1.84 min, MS (ESI) m/z = 375.2 [M+H]⁺.

¹H NMR (400 MHz, DMSO-*d*₆) δ ppm 8.28 (s, 1H), 8.18 (s, 1H), 8.11 (d, J = 5.1 Hz, 1H), 7.21 (td, J = 7.8, 1.8 Hz, 1H), 7.16 (s, 1H), 6.97 (dd, J = 8.3, 1.1 Hz, 1H), 6.88 (td, J = 7.4, 1.1 Hz, 2H), 6.61 (d, J = 16.4 Hz, 2H), 4.02 (s, 2H), 3.79 (s, 3H), 3.44 (q, J = 6.8 Hz, 2H), 3.19 (t, J = 8.4 Hz, 2H), 2.83 (t, J = 7.4 Hz, 2H).

### Example 122: (6,6-difluoro-3-azabicyclo[3.1.0]hexan-3-yl)(2-((2-methoxyphenethyl)amino)-3-nitropyridin-4-yl)methanone (E122)

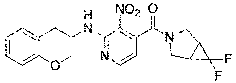

Prepared similarly as described for compound **E49** starting from **D79** (533 mg, 1.6675 mmol, 1.0 eq) and 2-(2-methoxyphenyl)ethan-1-amine (387 mg, 2.501 mmol, 1.5 eq) in anh NMP (7 mL, 0.23 M). The reaction mixture was stirred at 110°C for 2 hours (progress was monitored by UPLC). After work-up, the residue was purified using RP Preparative HPLC (eluent: CH₃CN (+0.05% NH₃) in H₂O (+0.05% NH₃), basic conditions) to yield **E122** (52.1 mg, 0.125mmol, yield= 7%) as yellow powder.

Method 4: R*t*= 3.62 min, MS (ESI) m/z = 419.1 [M+H]⁺.

¹H NMR (400 MHz, DMSO-*d*₆) δ ppm 8.47 (d, J = 4.6 Hz, 1H), 8.37 (t, J = 5.4 Hz, 1H), 7.25-7.15 (m, 2H), 6.96 (dd, J = 8.1, 1.1 Hz, 1H), 6.88 (td, J = 7.4, 1.1 Hz, 1H), 6.55 (d, J = 4.6 Hz, 1H), 3.90 (d, J = 12.5 Hz, 1H), 3.78 (s, 3H), 3.78-3.68 (m, 3H), 3.65-3.56 (m, 1H), 3.43 (d, J = 11.0 Hz, 1H), 2.92 (t, J = 6.9 Hz, 2H), 2.70-2.53 (m, 2H).

### Example 123: (3-amino-2-((2-methoxyphenethyl)amino)pyridin-4-yl)(6,6-difluoro-3-azabicyclo[3.1.0]hexan-3-yl)methanone (E123)

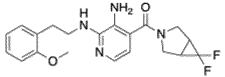

To a solution of **E122** (171.6 mg, 0.3691 mmol, 1 eq) in MeOH (0.01M, 50 mL) was added slurry of 10% Pd/C (18 mg in 10 mL of MeOH) under nitrogen atmosphere. The flask was flushed with H₂ (three cycles) and stirred at RT for ON. The reaction mixture was filtered through celite bed and concentrated under vacuum and purified with RP Preparative HPLC to afford **E123** (20.6 mg, 0.0518 mmol, yield= 14%) as a white powder.

Method 4: R*t*= 1.92min, MS (ESI) m/z = 389.2 [M+H]⁺.

¹H NMR (400 MHz, DMSO-*d*₆) δ ppm7.40 (d, J = 5.2 Hz, 1H), 7.23-7.13 (m, 2H), 6.97 (dd, J = 8.3, 1.1 Hz, 1H), 6.88 (td, J = 7.4, 1.1 Hz, 1H), 6.25 (d, J = 5.2 Hz, 1H), 5.99 (t, J = 5.3 Hz, 1H), 4.86 (s, 2H), 4.07 (d, J = 12.7 Hz, 1H), 3.79 (s, 3H), 3.78-3.64 (m, 2H), 3.50 (q, J = 6.2 Hz, 3H), 2.90-2.82 (m, 2H), 2.57 (d, J = 13.8 Hz, 2H).

### Example 124: N-(4-(6,6-difluoro-3-azabicyclo[3.1.0]hexane-3-carbonyl)-2-((2-methoxyphenethyl)amino)pyridin-3-yl)acetamide (E124)

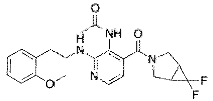

To solution of **E123** (70 mg, 0.1712 mmol, 1 eq) in dioxane (5 mL, 0.03M) were added DMAP (21 mg, 0.1712 mmol, 1 eq) and Ac₂O (16 µL, 0.1712 mmol, 1 eq) and stirred at the RT for 18 hours. Additional amounts of DMAP (21 mg, 0.1712 mmol, 1 eq) and Ac₂O (16 µL, 0.1712 mmol, 1 eq) were added and stirred for 18 h (repeated for 3 times). The reaction mixture was quenched with ss NaHCO₃ and DCM (3x15 mL). Organic layers were combined and washed with brine dried (Na₂SO₄), filtered and concentrated. The crude was purified by RP Preparative HPLC to provide **E124** (17.9 mg, 0.04mmol, yield= 24%) as a white solid after freeze-drying.

Method 4: R*t*= 2.02 min, MS (ESI) m/z = 431.0 [M+H]⁺.

¹H NMR (400 MHz, DMSO-*d*₆) δ ppm 9.13 (s, 1H), 7.99 (d, J = 5.0 Hz, 1H), 7.25-7.14 (m, 2H), 6.97 (dd, J = 8.3, 1.1 Hz, 1H), 6.89 (td, J = 7.4, 1.1 Hz, 1H), 6.33 (d, J = 5.0 Hz, 1H), 6.17 (t, J = 5.5 Hz, 1H), 3.86 (d, J = 12.5 Hz, 1H), 3.80 (s, 3H), 3.69-3.60 (m, 2H), 3.56 (d, J = 11.1 Hz, 1H), 3.53-3.44 (m, 2H), 2.83 (t, J = 7.6 Hz, 2H), 2.64-2.52 (m, 2H), 1.95 (s, 3H).

### Example 125: (2-(benzo[d]thiazol-2-ylamino)pyridin-4-yl)(6,6-difluoro-3-azabicyclo[3.1.0]hexan-3-yl)methanone (E125)

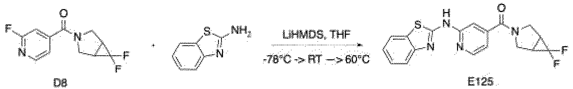

To solution of 2-aminobenzothiazole (78 mg, 0.518 mmol, 1.1 eq) in anh THF (4.7 mL, 0.1 M) was added LiHMDS (1M in THF, 460 µL, 0.461 mmol, 0.98 mmol) at-78°C and stirred for 30 min. Solution of **D8** (120 mg, 0.4707 mmol, 1.0 eq) was added and the reaction mixture was warmed to RT and then stirred at 60°C, ON. To complete the conversion the temperature was increased to 80°C and stirred for 18h and quenched with sat sol NH₄Cl. The reaction mixture was concentrated under reduced pressure to the -10% of the initial volume, and diluted with -80 mL of EtOAc. EtOAc solution washed with brine (3x50 mL), 10% solution of citric acid (2x30 mL), water (2x30 mL), brine (3x50 mL), dried over anh Na₂SO₄, filtered and evaporated. The crude was purified by NP Flash Chromatography (silica gel column, 0-10% MeOH in DCM) to afford **E125** (16.8 mg, 0.0423 mmol, yield= 9%) as off-white solid.

Method 4: R*t*= 3.24 min, MS (ESI) m/z = 373.1[M+H]⁺.

¹H NMR (400 MHz, DMSO-*d*₆) δ ppm 1.71 (1 H, s), 8.44 (1 H, dd, J 5.2, 0.8), 7.95-7.88 (1 H, m), 7.65 (1 H, d, J 8.1), 7.39 (1 H, ddd, J 8.3, 7.2, 1.3), 7.22 (1 H, ddd, J 8.2, 7.2, 1.2), 7.16 (1 H, s), 7.03 (1 H, dd, J 5.2, 1.3), 4.09 (1 H, d, J 12.8), 3.89-3.81 (1 H, m), 3.79-3.69 (1 H, m), 3.62 (1 H, d, J 11.4), 2.67-2.55 (2 H, m), 1.43-1.21 (3 H, m).

### Example 126: (2-((benzo[d]thiazol-2-ylmethyl)amino)pyridin-4-yl)(6,6-difluoro-3-azabicyclo[3.1.0]hexan-3-yl)methanone (E126)

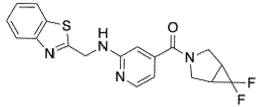

Prepared similarly as described for compound **E49** starting from **D8** (179 mg, 0.7021 mmol, 1.0 eq) and 1,3-benzothiazol-2-ylmethylamine (127 mg, 0.7723 mmol, 1.1 eq). The reaction mixture was stirred at 180°C for 18 hours. After work-up, the crude was purified using RP Preparative HPLC to yield **E126** (13 mg, 0.035 mmol, yield= 5%) as off-white powder.

Method 4: R*t*= 2.41 min, MS (ESI) m/z = 387.2[M+H]⁺.

¹H NMR (400 MHz, DMSO-*d*₆) δ ppm 8.05 (dd, J =5.2, 0.9 Hz, 1 H), 8.01 (d, J =7.9Hz, 1 H), 7.94 (d, J =7.9, 1 H), 7.79 (t, J =6.1 Hz, 1 H), 7.49 (ddd, J =8.3, 7.2, 1.3 Hz, 1 H), 7.39 ( ddd, J =8.3, 7.2, 1.2 Hz, 1 H), 6.60 (t, J =1.1 Hz, 1 H), 6.56 (dd, J=5.2, 1.4 Hz, 1 H), 4.90 (d, J =6.1 Hz, 2 H), 4.05 (d, J =12.7 Hz, 1 H), 3.83-3.75 (m, 1 H), 3.71-3.64 (m, 1 H), 3.54 (d, J =11.4 Hz, 1 H), 2.66-2.53 (m, 2 H).

### Example 127: (2-((2-(benzo[d]thiazol-2-yl)ethyl)amino)pyridin-4-yl)(6,6-difluoro-3-azabicyclo[3.1.0]hexan-3-yl)methanone (E127)

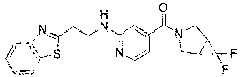

Prepared similarly as described for compound **E49** starting from **D8** (80 mg, 0.3303 mmol, 1.0 eq) and 2-(1,3-benzothiazol-2-yl)ethan-1-amine (65 mg, 0.3633 mmol, 1.1 eq). The reaction mixture was stirred at 180°C for 18 hours. After work-up, the crude was purified using RP Preparative HPLC to yield **E127** (22,8 mg, 0.0561 mmol, yield= 17%) as white powder.

Method 4: R*t*= 2.01 min, MS (ESI) m/z = 401.1[M+H]⁺.

¹H NMR (400 MHz, DMSO-*d*₆) δ ppm 8.10-8.02 (m, 2H), 7.99-7.92 (m, 1H), 7.54-7.45 (m, 1H), 7.45-7.37 (m, 1H), 7.01 (t, J = 5.8 Hz, 1H), 6.48 (dd, J = 5.2, 1.4 Hz, 1H), 6.46-6.42 (m, 1H), 4.04 (d, J = 12.7 Hz, 1H), 3.83-3.71 (m, 3H), 3.71-3.61 (m, 1H), 3.55 (d, J = 6.9 Hz, 1H), 3.38 (t, J = 6.8 Hz, 2H), 2.64-2.53 (m, 2H).

### Example 128: (6,6-difluoro-3-azabicyclo[3.1.0]hexan-3-yl)(2-((2-(2-methoxy-[1,1'-biphenyl]-3-yl)ethyl)amino)pyridin-4-yl)methanone (E128)

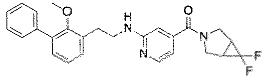

Prepared similarly as described for compound **E49** starting from **D8** (30 mg, 0.13 mmol, 1.0 eq.) and **D81** (40 mg, 0.13 mmol, 1.0 eq.). The reaction mixture was stirred at 160°C ON. After work-up, the crude product was purified on RP Preparative HPLC affording **E128** (12 mg, yield= 20%)

Method 4: R*t*= 2.49 min, MS (ESI) m/z = 450.2 [M+H]⁺.

¹H NMR (400 MHz, DMSO-*d*₆) δ ppm 8.04 (d, J = 5.3 Hz, 1H), 7.56-7.51 (m, 2H), 7.45 (dd, J = 8.4, 6.8 Hz, 2H), 7.41-7.32 (m, 1H), 7.27 (dd, J = 7.3, 2.0 Hz, 1H), 7.21 (dd, J = 7.6, 2.0 Hz, 1H), 7.16 (d, J = 7.4 Hz, 1H), 6.59-6.43 (m, 2H), 4.03 (d, J = 12.7 Hz, 1H), 3.79 (dt, J = 11.4, 4.6 Hz, 1H), 3.67 (dt, J = 12.7, 4.7 Hz, 1H), 3.60-3.48 (m, 3H), 3.29 (s, 3H), 2.91 (dd, J = 8.5, 6.4 Hz, 2H), 2.66-2.50 (m, 2H).

### Example 129: (6,6-difluoro-3-azabicyclo[3.1.0]hexan-3-yl)(2-((2-(3-methoxy-[1,1'-biphenyl]-4-yl)ethyl)amino)pyridin-4-yl)methanone (E129)

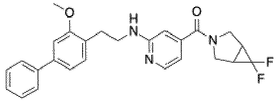

Prepared similarly as described for compound **E49** starting from **D8** (75 mg, 0.279 mmol, 1.0 eq) and **D82** (133 mg, 0.362 mmol, 1.3 eq). The reaction mixture was stirred for 6 hours at 160° C. After work-up, the crude was purified on RP preparative HPLC (eluent: CH₃CN (+0.05% NH₃) in H₂O (+0.05% NH₃), basic conditions) affording **E129** (11 mg, 0.023 mmol, yield= 8%).

Method 4: R*t*= 2.49 min, MS (ESI) m/z = 450.4[M+H]⁺.

¹H NMR (400 MHz, DMSO-*d*₆) δ ppm 8.04 (d, J = 5.7 Hz, 1H), 7.71-7.63 (m, 2H), 7.45 (t, J = 7.7 Hz, 2H), 7.40-7.32 (m, 1H), 7.28-7.18 (m, 2H), 7.16 (dd, J = 7.6, 1.7 Hz, 1H), 6.81 (t, 1H), 6.45-6.39 (m, 2H), 4.04 (d, J = 12.9 Hz, 1H), 3.89 (s, 3H), 3.83-3.74 (m, 1H), 3.72-3.61 (m, 1H), 3.55 (d, J = 11.4 Hz, 1H), 3.45 (q, J = 6.7 Hz, 2H), 2.84 (t, J = 7.3 Hz, 2H), 2.64-2.52 (m, 2H).

### Example 130: (6,6-difluoro-3-azabicyclo[3.1.0]hexan-3-yl)(2-((2-(4-methoxy-[1,1'-biphenyl]-3-yl)ethyl)amino)pyridin-4-yl)methanone (E130)

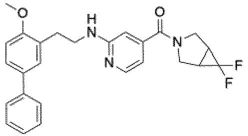

Prepared similarly as described for compound **E49** starting from **D8** (100 mg, 0.413 mmol, 1.0 eq) and (231 mg, 0.619 mmol, 1.5 eq). The reaction mixture was stirred for 6 hours at 160° C. After work-up, the crude was purified by RP Preparative HPLC (eluent: CH₃CN (+0.05% NH₃) in H₂O (+0.05% NH₃), basic conditions) to afford **E130** (61 mg, 0.132 mmol, yield= 32%).

Method 4: R*t*= 2.51 min, MS (ESI) m/z = 450.2 [M+H]⁺.

¹H NMR (400 MHz, DMSO-*d*₆) δ ppm 8.03 (dd, J = 5.0, 1.0 Hz, 1H), 7.61-7.54 (m, 2H), 7.49 (dd, J = 8.5, 2.4 Hz, 1H), 7.46-7.37 (m, 3H), 7.34-7.25 (m, 1H), 7.05 (d, J = 8.5 Hz, 1H), 6.81 (t, J = 5.7 Hz, 1H), 6.46-6.39 (m, 2H), 4.03 (d, J = 12.7 Hz, 1H), 3.83 (s, 3H), 3.76 (dt, J = 11.3, 4.7 Hz, 1H), 3.65 (dt, J = 12.7, 4.8 Hz, 1H), 3.55-3.43 (m, 3H), 2.88 (t, J = 7.2 Hz, 2H), 2.63-2.51 (m, 2H).

### Example 131: (2-(chroman-3-ylamino)pyridin-4-yl)(6,6-difluoro-3-azabicyclo[3.1.0]hexan-3-yl)methanone (E131)

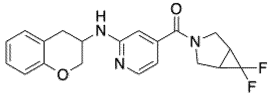

Prepared similarly as described for compound **E60** starting from **D60** (50 mg, 0.190 mmol) and chroman-3-amine;hydrochloride (43.1 mg, 0.230 mmol). The reaction was refluxed for 12h. After work-up, the crude purified by column chromatography (Sfar C18, 12 g; MeCN 0.1% HCOOH /H₂O 0.1% HCOOH) to obtain **E131** (5 mg, 0.013 mmol, yield= 6.97%) as yellow oil.

Method 2: R*t*= 1.15 min, MS (ESI) m/z = 372.21[M+H]⁺.

### Example 132: (6,6-difluoro-3-azabicyclo[3.1.0]hexan-3-yl)(2-((2-methoxyphenethyl)amino)pyridin-4-yl)methanethione (E132)

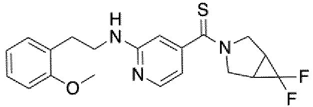

An oven-dried vial was equipped with a magnetic stir bar, backfilled with Ar and charged with **E6** (50 g, 0.08 mmol, 1.0 eq) and Lawesson reagent (16 mg, 0.04 mmol, 0.5 eq). Then, anh Toluene (1.61 mL, 0.05 M) was added via syringe and the mixture was purged with Argon for additional 10 minutes and finally the reaction mixture was stirred at 100°C, ON. The reaction mixture was evaporated under reduced pressure, filtered through celite, and then re-evaporated to give a crude product as a yellow oil. The crude material was purified by RP preparative HPLC (eluent: CH₃CN (+0.1% HCOOH) in H₂O (+0.1% HCOOH)) to yield **E132** (18 mg, 0.044 mmol, yield= 55%)

Method 8: R*t*= 1.99 min, MS (m/z) 390.1[M+H]⁺.

¹H NMR (400 MHz, DMSO-*d*₆) δ ppm 8.06 (d, J = 61.9 Hz, 1H), 7.79-7.52 (m, 1H), 7.32-6.77 (m, 6H), 4.35 (d, J = 14.4 Hz, 1H), 4.10-3.98 (m, 1H), 3.97-3.86 (m, 1H), 3.78 (s, 3H), 3.66 (d, J = 12.8 Hz, 1H), 3.43 (t, J = 7.6 Hz, 2H), 2.82 (t, J = 7.5 Hz, 2H), 2.79-2.62 (m, 2H).

### Example 133: N-(2-methoxyphenethyl)-4-(4-phenyloxazol-2-yl)pyridin-2-amine (E133)

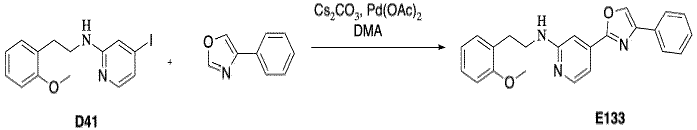

In vial, **D41** (100 mg, 0.2823 mmol, 1.0 eq.), 4-pheny-1,3-oxazole (80 µL, 0.5647 mmol, 2.0 eq.) and Cs₂CO₃ (270 mg, 0.8470 mmol, 3.0 eq.) were dissolved in DMA (1.2 mL). The mixture was flushed with N₂ for 10 min followed by addition of Pd(OAc)₂ (4.4 mg, 0.0141 mmol, 0.05 eq). The vial was sealed and stirred at 110 °C, ON. The mixture was cooled to RT, filtered through celite, washed with EtOH (3 x 5 mL), dried over Na₂SO₄, filtered, and concentrated in vacuum to give a crude as brown oil (700 mg). The crude was purified by RP preparative HPLC to give **E133** as white solid (40 mg, yield=38%).

Method 4: R*t*= 2.71 min, MS (ESI) m/z = 372.2 [M+H]⁺

¹H NMR (400 MHz, DMSO-*d*₆) δ ppm 8.78 (s, 1H), 8.15 (d, J = 5.3 Hz, 1H), 7.91-7.83 (m, 2H), 7.48 (dd, J = 8.4, 6.9 Hz, 2H), 7.43-7.33 (m, 1H), 7.25-7.13 (m, 2H), 7.13-7.08 (m, 1H), 7.04 (dd, J = 5.2, 1.5 Hz, 1H), 7.02-6.93 (m, 2H), 6.88 (td, J = 7.4, 1.1 Hz, 1H), 3.82 (s, 3H), 3.47 (q, J = 6.9 Hz, 2H), 2.84 (t, J = 7.4 Hz, 2H).

### Example 134: N-(2-methoxyphenethyl)-4-(4-phenylthiazol-2-yl)pyridin-2-amine (E134)

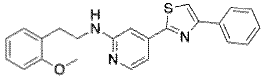

Prepared similarly as described for compound **E49** starting from **D86** (100 mg, 0.3902 mmol, 1.0 eq.) and 2-(2-methoxyphenyl)ethan-1-amine (180 mg, 1.1705 mmol, 3.0 eq.). The reaction was stirred at 180 °C, ON. After work-up, the crude was purified by RP preparative HPLC to yield **E134** as yellow solid (50 mg, yield= 33%).

Method 4: R*t*= 2.76 min, MS (ESI) m/z = 388.1[M+H]⁺

¹H NMR (400 MHz, DMSO-*d*₆) δ ppm 8.27 (s, 1H), 8.12 (d, J = 5.3 Hz, 1H), 8.07-8.00 (m, 2H), 7.49 (dd, J = 8.4, 7.0 Hz, 2H), 7.42-7.36 (m, 1H), 7.23-7.15 (m, 2H), 7.09 (d, J = 1.6 Hz, 1H), 7.02 (dd, J = 5.3, 1.5 Hz, 1H), 7.01-6.95 (m, 1H), 6.94-6.85 (m, 2H), 3.80 (s, 3H), 3.53-3.43 (m, 2H), 2.85 (dd, J = 8.4, 6.4 Hz, 2H).

### Example 135: N-(2-methoxyphenethyl)-4-(5-phenyl-1H-imidazol-2-yl)pyridin-2-amine (E135)

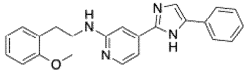

Prepared similarly as described for compound **E49** starting from **D87** (200 mg, 0.48 mmol, 1.0 eq.) and 2-(2-methoxyphenyl)ethan-1-amine (220 µL, 1.45 mmol, 3.0 eq.). The reaction was stirred at 180°C for 48h. After work-up, the crude was purified using RP preparative HPLC. Fractions containing the product of interest were concentrated and freeze-dried to yield **E135** as white solid (125 mg, yield= 67%).

Method 4: R*t*= 2.29 min, MS (ESI) m/z = 371.3 [M+H]⁺

¹H NMR (400 MHz, DMSO-*d*₆) δ ppm 12.81 (s, 1H), 8.05 (d, J = 5.3 Hz, 1H), 7.89-7.78 (m, 3H), 7.38 (t, J = 7.6 Hz, 2H), 7.25-7.17 (m, 3H), 7.08 (s, 1H), 7.03 (d, J = 5.3 Hz, 1H), 6.99-6.94 (m, 1H), 6.88 (td, J = 7.4, 1.1 Hz, 1H), 6.66 (s, 1H), 3.81 (s, 3H), 3.44 (dt, J = 7.9, 5.9 Hz, 2H), 2.85 (dd, J = 8.5, 6.4 Hz, 2H).

### Example 136: (S)-(2-((2-methoxyphenethyl)amino)pyridin-4-yl)(2-methylpyrrolidin-1-yl)methanone (E136)

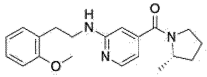

A pressure vial was charged with **D61** (146 mg, 0.429 mmol, 1.0 eq), 2-(2-methoxyphenyl)ethan-1-amine (75 µL, 0.515 mmol, 1.2 eq), cesium carbonate (700 mg, 2.147 mmol, 5.0 eq), 2,2'-Bis(diphenylphosphino)-1,1'-binaphthalene (rac-BINAP) (107 mg, 0.172 mmol, 0.4 eq) in anh Dioxane (4.13 mL, 0.104 M). The reaction solution was degassed with argon and Palladium (II) Acetate (19 mg, 0.086 mmol, 0.2 eq) was added. Finally, the sealed vial was purged argon and the reaction mixture was stirred at 110°C, ON . The mixture was filtered through celite and washed with water then extracted with EtOAc (x3) The organic phase was evaporated by reduced pressure. The crude material was purified by using RP preparative HPLC (eluent: CH₃CN (+0.05% NH₃) in H₂O (+0.05% NH₃), basic conditions) to yield **E136** (47 mg, 0.128 mmol, yield= 30%), yellow-orange oil.

Method 4: Rt= 1.96 min, MS (ESI) m/z = 340.3 [M+H]⁺

¹H NMR (400 MHz, 353.2K, DMSO-*d*₆) δ ppm 8.19-7.86 (m, 1H), 7.19 (ddd, J = 15.2, 7.5, 1.8 Hz, 2H), 6.97 (d, J = 8.1 Hz, 1H), 6.88 (td, J = 7.4, 1.2 Hz, 1H), 6.52-6.40 (m, 2H), 6.36 (s, 1H), 4.04 (d, J = 39.5 Hz, 1H), 3.82 (s, 3H), 3.59 (s, 2H), 3.51-3.44 (m, 2H), 2.85 (t, J = 7.4 Hz, 2H), 2.06 (dd, J = 12.3, 7.4 Hz, 1H), 1.91 (p, J = 6.9 Hz, 1H), 1.79 (s, 1H), 1.58 (tt, J = 11.7, 5.2 Hz, 1H), 1.18 (s, 3H).

### Example 137: (R)-(2-((2-methoxyphenethyl)amino)pyridin-4-yl)(2-methylpyrrolidin-1-yl)methanone (E137)

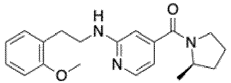

Prepared similarly as described for compound **E136** starting from **D88** (150 mg, 0.455 mmol) and 2-(2-methoxyphenyl)ethan-1-amine (80 µL, 0.547 mmol). After work-up, the crude material was purified by using RP preparative HPLC (eluent: CH₃CN (+0.05% NH₃) in H₂O (+0.05% NH₃), basic conditions) to yield E137 (11 mg, 0.03 mmol, yield= 5%), yellow oil.

Method 4: R*t*= 1.96 min, MS (ESI) m/z = 340.2 [M+H]⁺.

¹H NMR (400 MHz, DMSO-*d*₆) δ ppm 8.02 (d, J = 4.9 Hz, 1H), 7.26-7.08 (m, 2H), 6.97 (dd, J = 8.2, 1.1 Hz, 1H), 6.87 (td, J = 7.3, 1.1 Hz, 1H), 6.71 (t, J = 5.7 Hz, 1H), 6.56-6.37 (m, 2H), 4.11 (d, J = 6.3 Hz, 1H), 3.80 (s, 3H), 3.49-3.38 (m, 3H), 3.30-3.20 (m, 1H), 2.81 (t, J = 7.5 Hz, 2H), 2.14-1.97 (m, 1H), 1.88 (dq, J = 12.7, 6.8, 6.4 Hz, 1H), 1.73 (ddt, J = 19.2, 12.8, 6.6 Hz, 1H), 1.54 (dq, J = 12.3, 6.6 Hz, 1H), 1.31-1.16 (m, 3H).

### Example 138: 1-(2-((2-methoxyphenethyl)amino)isonicotinoyl)pyrrolidin-2-one (E138)

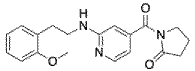

Prepared similarly as described for compound **E136** starting from **D26** (333 mg, 1.008 mmol, 1.0 eq) and 2-(2-methoxyphenyl)ethan-1-amine (197 mg, 1.301 mmol, 1.3 eq) in toluene instead of dioxane. The reaction was refluxed at 110°C for 30 min. After work-up, the crude material was purified by using RP preparative HPLC to yield **E138** (8 mg, 0.02mmol, yield= 2%) as a white solid after freeze drying.

Method 4: R*t*= 2.85 min, MS (ESI) m/z = 340.2[M+H]⁺.

¹H NMR (400 MHz, DMSO-*d*₆) δ ppm 8.78 (t, J = 5.5 Hz, 1H), 8.65 (s, 1H), 8.48 (dd, J = 5.2, 0.8 Hz, 1H), 7.44 (dd, J = 5.2, 1.5 Hz, 1H), 7.21 (td, J = 7.8, 1.7 Hz, 1H), 7.16 (dd, J = 7.4, 1.7 Hz, 1H), 6.97 (dd, J = 8.2, 1.1 Hz, 1H), 6.88 (td, J = 7.4, 1.1 Hz, 1H), 4.05-3.97 (m, 2H), 3.80 (s, 3H), 3.46 (dt, J = 7.9, 6.1 Hz, 2H), 2.84 (t, J = 7.4 Hz, 2H), 2.61 (t, J = 8.0 Hz, 2H), 2.07 (p, J = 7.7 Hz, 2H).

### Example 139: (1,1-dioxidothiomorpholino)(2-((2-methoxyphenethyl)amino)pyridin-4-yl)methanone (E139)

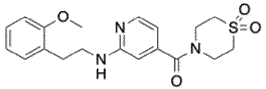

Prepared similarly as described for compound **E49** starting from **D89** (100 mg, 0.31 mmol, 1.0 eq.) and 2-(2-methoxyphenyl)ethan-1-amine (140 mg, 0.92 mmol, 3.0 eq.).After work-up, the crude material was purified by RP preparative HPLC. The fractions containing the product of interest were freeze-dried to yield E139 (42.3 mg, 0.109mmol, yield= 35%).

Method 4: R*t*= 2.27min, MS (ESI) m/z = 368.2[M+H]⁺

¹H NMR (400 MHz, DMSO-*d*₆) δ ppm 8.00 (d, J = 5.7 Hz, 1H), 7.19 (td, J = 7.8, 1.8 Hz, 1H), 7.14 (dd, J = 7.5, 1.7 Hz, 1H), 6.96 (dd, J = 8.3, 1.1 Hz, 1H), 6.86 (td, J = 7.4, 1.1 Hz, 1H), 6.74 (s, 1H), 6.35 (d, J = 4.5 Hz, 2H), 4.56 (s, 1H), 3.78 (s, 3H), 3.52 (s, 1H), 3.40 (q, J = 6.8 Hz, 2H), 2.83-2.76 (m, 2H), 1.75 (ddt, J = 16.7, 14.9, 7.6 Hz, 1H), 1.32 (s, 1H), 0.84 (s, 2H), 0.69 (s, 2H).

### Example 140: ((1S,4S)-2,2-dioxido-2-thia-5-azabicyclo[2.2.1]heptan-5-yl)(2-((2-methoxyphenethyl)amino)pyridin-4-yl)methanone (E140)

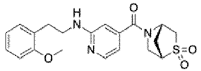

Prepared similarly as described for compound **E49** starting from **D90** (0.13 g, 0.26 mmol, 1.0 eq.) and 2-(2-methoxyphenyl)ethan-1-amine (120 µL, 0.78 mmol, 3.0 eq.). After work-up, the crude was purified by RP preparative HPLC. The fractions containing the product of interest were freeze-dried to yield **E140** (32.4 mg, 0.08mmol, yield= 31%).

Method 4: R*t*= 1.74min, MS (ESI) m/z = 402.2[M+H]⁺

¹H NMR (400 MHz, DMSO-*d*₆) δ ppm 8.06 (d, J = 5.1 Hz, 1H), 7.23-7.14 (m, 2H), 6.97 (d, J = 8.1 Hz, 1H), 6.87 (td, J = 7.4, 1.2 Hz, 1H), 6.52 (dd, J = 6.4, 1.3 Hz, 2H), 6.47 (s, 1H), 3.96 (s, 1H), 3.81 (s, 3H), 3.81-3.68 (m, 2H), 3.47 (dt, J = 8.4, 6.3 Hz, 2H), 3.24 (d, J = 7.0 Hz, 2H), 2.85 (t, J = 7.4 Hz, 2H), 2.48-2.43 (m, 3H).

### Example 141: (3,3-dioxido-3-thia-8-azabicyclo[3.2.1]octan-8-yl)(2-((2-methoxyphenethyl)amino)pyridin-4-yl)methanone (E141)

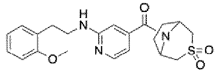

Prepared similarly as described for compound **E49** starting from **D91** (64 mg, 0.191 mmol, 1.0 eq) and 2-(2-methoxyphenyl)ethan-1-amine (87 mg, 0.574 mmol, 3.0 eq.). After work-up, the crude was purified by RP preparative HPLC (eluent: CH₃CN (+0.05% NH₃) in H₂O (+0.05% NH₃), basic conditions). The fractions containing the product of interest were freeze-dried to yield **E141** (35 mg, 0.084 mmol, yield= 44%).

Method 4: R*t*= 1.84min, MS (ESI) m/z = 416.1 [M+H]⁺

¹H NMR (400 MHz, DMSO-*d*₆) δ ppm 8.08-8.03 (m, 1H), 7.24-7.12 (m, 2H), 6.96 (dd, J = 8.3, 1.1 Hz, 1H), 6.87 (td, J = 7.4, 1.1 Hz, 1H), 6.80 (t, J = 5.6 Hz, 1H), 6.60-6.52 (m, 2H), 5.00 (s, 1H), 4.37 (s, 1H), 3.78 (s, 3H), 3.51-3.37 (m, 4H), 2.81 (t, J = 7.5 Hz, 2H), 2.51 (s, 2H), 2.31-2.14 (m, 2H), 2.12-1.95 (m, 2H).

### Example 142: (3,3-dioxido-3-thia-6-azabicyclo[3.1.1]heptan-6-yl)(2-((2-methoxyphenethyl)amino)pyridin-4-yl)methanone (E142)

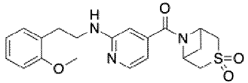

Prepared similarly as described for compound **E49** starting from **D92** (100 mg, 0.411 mmol, 1.0 eq) and 2-(2-methoxyphenyl)ethan-1-amine (77 mg, 0.507 mmol, 1.5 eq). The reaction mixture was stirred at 110°C, ON. After work-up, the crude was purified by RP preparative HPLC (eluent: CH₃CN (+0.05% NH₃) in H₂O (+0.05% NH₃), basic conditions). The fractions containing the product of interest were freeze-dried to yield **E142** (18 mg, 0.043 mmol, yield= 13%) as a transparent sticky solid

Method 4: R*t*= 1.76min, MS (ESI) m/z =402.1[M+H]⁺

¹H NMR (400 MHz, DMSO-*d*₆) δ ppm 8.08 (d, J = 5.2 Hz, 1H), 7.29-7.11 (m, 2H), 6.97 (d, J = 8.1 Hz, 1H), 6.93-6.81 (m, 2H), 6.71-6.52 (m, 2H), 4.74 (d, J = 45.5 Hz, 2H), 4.00 (d, J = 13.4 Hz, 1H), 3.80 (s, 3H), 3.73 (d, J = 12.1 Hz, 2H), 3.53-3.40 (m, 3H), 2.96 (q, J = 7.9 Hz, 1H), 2.82 (t, J = 7.4 Hz, 2H), 2.07 (d, J = 10.6 Hz, 1H).

### Example 143: ((1R,4R)-2,2-dioxido-2-thia-5-azabicyclo[2.2.1]heptan-5-yl)(2-((2-methoxyphenethyl)amino)pyridin-4-yl)methanone (E143)

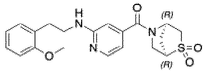

Prepared similarly as described for compound **E49** starting from **D93** (98 mg, 0.276 mmol, 1.0 eq) and 2-(2-methoxyphenyl)ethan-1-amine(63 mg, 0.413 mmol, 1.5 eq). The reaction mixture was stirred at 110°C, ON. After work-up, the crude was purified by RP preparative HPLC (eluent: CH₃CN (+0.05% NH₃) in H₂O (+0.05% NH₃), basic conditions). The fractions containing the product of interest were freeze-dried to yield E143 (10 mg, 0.043 mmol, 0.025mmol, yield= 9%)

Method 4: Rt= 1.74min, MS (ESI) m/z = 402.1[M+H]⁺.

¹H NMR (400 MHz,353.15K, DMSO-*d*₆) δ ppm 8.11-8.04 (m, 1H), 7.19 (ddd, J = 14.4, 7.2, 1.8 Hz, 2H), 7.00-6.94 (m, 1H), 6.88 (td, J = 7.4, 1.2 Hz, 1H), 6.52 (dd, J = 6.2, 1.2 Hz, 2H), 6.48 (s, 1H), 3.97 (s, 1H), 3.82 (s, 3H), 3.73 (dd, J = 11.4, 3.7 Hz, 2H), 3.48 (dt, J = 8.3, 6.3 Hz, 2H), 3.24 (s, 2H), 2.86 (t, J = 7.4 Hz, 2H), 2.47 (s, 2H).

### Example 144: 1-(2-methoxyphenyl)-3-(4-(piperidine-1-carbonyl)pyridin-2-yl)urea (SCU-489, N1301-30-1) (E144)

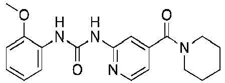

Prepared similarly as described for compound **E8** starting from **D38** (70 mg, 0.240 mmol) and piperidine (30 µL, 0.320 mmol) in DMF (2 mL) at 25 °C. The reaction mixture was stirred at RT for 16 h. After work-up, the crude was purified by RP Flash Chromatography (Sfar C18, 12 g, eluent: CH₃CN (+0.1% HCOOH) in H₂O (+0.1% HCOOH)). The fraction containing the product of interest was concentrated until a precipitate was formed. The solid was collected by filtration, washed with water (6 mL) and dried at 45°C under vacuum for 16 h to afford **E144** (21 mg, 0.059 mmol, yield= 24.32%) as a white solid. Method 2: R*t*= 1.76 min, MS (ESI) m/z = 355.3 [M+H] ⁺. ¹H NMR (400 MHz, DMSO-*d*₆) δ ppm 10.78 (br s, 1 H), 9.95 (s, 1 H), 8.36 (d, J=5.1 Hz, 1 H), 8.18 (dd, J=8.0, 1.4 Hz, 1 H), 7.32 (br s, 1 H), 7.02 - 7.06 (m, 1 H), 6.99 (td, J=7.8, 1.6 Hz, 1 H), 6.96 (dd, J=5.1, 1.2 Hz, 1 H), 6.91 (td, J=7.6, 1.3 Hz, 1H), 3.91 (s, 3 H), 3.20 - 3.64 (m, 4 H), 1.41 - 1.67 (m, 6 H)

### Example 145: 1-(4-(azetidine-1-carbonyl)pyridin-2-yl)-3-(2-methoxyphenyl)urea (E145)

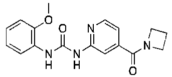

Prepared similarly as described for compound **E8** starting from **D38** (65 mg, 0.230 mmol) and and azetidine;hydrochloride (27.5 mg, 0.290 mmol). The reaction mixture was stirred at RT for 16h. After work-up, the crude was purified by RP Flash Chromatography (Sfar C18, 12 g, eluent: CH₃CN (+0.1% HCOOH) in H₂O (+0.1% HCOOH)) to afford **E145** (7 mg, 0.021 mmol, yield= 9.48%) as white solid. Method 2: R*t*= 1.50 min, MS (ESI) m/z = 327.3 [M+H] ⁺.

¹H NMR (400 MHz, DMSO-*d*₆) δ ppm 10.79 (br s, 1 H), 9.96 (s, 1 H), 8.38 (d, J=5.3 Hz, 1 H), 8.14-8.25 (m, 1 H), 7.57 (br s, 1 H), 7.14 (dd, J=5.3, 1.1 Hz, 1 H), 6.95-7.07 (m, 2 H), 6.85-6.95 (m, 1 H), 4.00-4.36 (m, 4 H), 3.91 (s, 3 H), 2.28 (quin, J=7.7 Hz, 2 H)

### Example 146: 1-(4-(2-ethylpiperidine-1-carbonyl)pyridin-2-yl)-3-(2-methoxyphenyl)urea (E146)

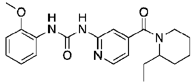

Prepared similarly as described for compound **E8** starting from **D38** (28 mg, 0.100 mmol) and 2-ethypiperidin hydrochloride (19 mg, 0.130 mmol). The reaction mixture was stirred at RT for 5h. After work-up, the crude was purified by RP Flash Chromatography (Sfar C18, 12 g, eluent: CH₃CN (+0.1% HCOOH) in H₂O (+0.1% HCOOH)) to afford **E146** (8 mg, 0.021 mmol, yield= 21.46%) as colourless oil.

Method 2: R*t*= 2.00 min, MS (ESI) m/z = 383.3 [M+H] ⁺. ¹H NMR (400 MHz, DMSO-*d*₆) δ ppm 10.23 - 11.16 (m, 1 H), 9.92 (s, 1 H), 8.35 (d, J=5.0 Hz, 1 H), 8.18 (dd, J=7.9, 1.5 Hz, 1 H), 7.32 (br s, 1 H), 6.96 - 7.07 (m, 2 H), 6.86 - 6.96 (m, 2 H), 3.91 (s, 3 H), 3.34 - 4.70 (m, 1 H), 3.16 - 4.42 (m, 1 H), 2.69 - 3.14 (m, 1 H), 1.25 - 1.89 (m, 8 H), 0.59 - 0.97 (m, 3 H)

### Example 147: N-(4-(2-ethylpiperidine-1-carbonyl)pyridin-2-yl)-2-(2-methoxyphenyl)acetamide (E147)

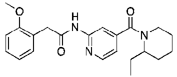

Prepared similarly as described for compound **E8** starting from **D40** (60 mg, 0.210 mmol) and 2-ethypiperidin hydrochloride in DMF. The reaction mixture was stirred at RT for 16h. After work-up, the crude was purified by RP Flash Chromatography (Sfar C18, 12 g, eluent: CH₃CN (+0.1% HCOOH) in H₂O (+0.1% HCOOH)) to afford **E147** ( 28mg, 0.073mmol, yield= 35%).

Method 2: R*t*= 1.90 min, MS (ESI) m/z = 382.3 [M+H] ⁺. ¹H NMR (400 MHz, DMSO-*d*₆) δ ppm 10.68 (s, 1 H), 8.38 (d, J=5.0 Hz, 1 H), 7.97 (s, 1 H), 7.22 - 7.28 (m, 1 H), 7.20 (dd, J=7.3, 1.4 Hz, 1 H), 7.02 (dd, J=4.9, 1.4 Hz, 1 H), 6.98 (d, J=8.1 Hz, 1 H), 6.90 (td, J=7.4, 0.8 Hz, 1 H), 3.75 (s, 3 H), 3.72 (s, 2 H), 3.32 - 4.68 (m, 1 H), 3.14 - 4.42 (m, 1 H), 2.69 - 3.10 (m, 1 H), 1.19 - 1.84 (m, 8 H), 0.55 - 0.94 (m, 3 H).

### Example 148: N⁴-(3-chlorophenyl)-N²-(2-methoxyphenethyl)pyridine-2,4-diamine; formate (E148)

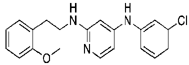

A mixture of **D41** (170 mg, 0.480 mmol), 3-chloroaniline (60 µL, 0.580 mmol) and potassium *tert*-butoxide (107.7 mg, 0.960 mmol) in DMF (5 mL) was degassed through N₂-vacuum cycles (3x), then [1,1'-Bis(diphenylphosphino)ferrocene]dichloropalladium(II) Pd(dppf)Cl₂ (81.4 mg, 0.11 mmol) was added and the mixture heated at 120 °C in a MW reactor for 30 minutes. The mixture was diluted with EtOAc, washed with water, the aqueous layer was extracted with EtOAc, the combined organic layers were additionally washed with brine, dried over Na₂SO₄, filtered and evaporated under reduced pressure. The crude was purified by NP Flash Chromatography (silica gel column, Sfar D, 25 g, 0-100% EtOAc in cyclohexane) to give a brown oil. The product was purified additionally by RP Flash Chromatography (Sfar C18, 12 g, eluent: CH₃CN (+0.1% HCOOH) in H₂O (+0.1% HCOOH)). The appropriate fractions were collected and lyophilized to give **E148** (20 mg, 0.050 mmol, yield= 10.4%) as an off-white solid.

Method 2: R*t*= 1.43 min, MS (ESI) m/z = 354.3 [M+H] ⁺.

¹H NMR (400 MHz, DMSO-*d*₆) δ ppm 8.59 (s, 1 H), 8.16 (s, 1 H), 7.73 (d, J=5.7 Hz, 1 H), 7.27 - 7.34 (m, 1 H), 7.12 - 7.22 (m, 3 H), 7.09 (br d, J=8.1 Hz, 1 H), 6.93 - 7.00 (m, 2 H), 6.87 (t, J=7.3 Hz, 1 H), 6.35 (br t, J=5.3 Hz, 1 H), 6.15 - 6.20 (m, 1 H), 6.13 (s, 1 H), 3.75 (s, 3 H), 3.31 - 3.39 (m, 2 H), 2.78 (br t, J=7.5 Hz, 2 H).

### Example 149: N²-(2-methoxyphenethyl)-N⁴-(3-methoxyphenyl)pyridine-2,4-diamine (E149)

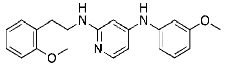

Prepared similarly as described for compound **E148** starting from **D41** (125 mg, 0.350 mmol) and 3-methoxyaniline (60 µL, 0.520 mmol). After work-up, the crude was purified by RP Flash Chromatography (silica C18 12 g, 0-50% CH₃CN (+0.1% HCOOH) in H₂O (+0.1% HCOOH)) to give **E149** (75 mg, 0.215 mmol, yield= 61.43%) as a pale yellow oil.

Method 2: R*t*= 1.32 min, MS (ESI) m/z =350.29 [M+H]⁺.

¹H NMR (400 MHz, DMSO-*d*₆) δ ppm 9.06 (br s, 1 H), 7.65 (d, J=6.6 Hz, 1 H), 7.28 (t, J=8.1 Hz, 1 H), 7.18 - 7.24 (m, 1 H), 7.16 (dd, J=7.3, 1.3 Hz, 1 H), 7.10 - 7.24 (m, 1 H), 6.95 (d, J=8.1 Hz, 1 H), 6.87 (t, J=7.3 Hz, 1 H), 6.73 - 6.80 (m, 2 H), 6.68 (dd, J=8.2, 2.0 Hz, 1 H), 6.28 (dd, J=6.5, 1.9 Hz, 1 H), 6.17 (d, J=1.9 Hz, 1 H), 3.75 (s, 3 H), 3.73 (s, 3 H), 3.31 - 3.39 (m, 2 H), 2.80 (t, J=7.4 Hz, 2 H)

**Example 150: *N*²-(2-methoxyphenethyl)-*N*⁴-(2-methoxyphenyl)pyridine-2,4-diamine (SCU-626, N1360-43-1) (E150)**

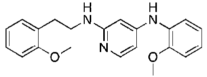

Prepared similarly as described for compound **E148** starting from **D41** (75 mg, 0.210 mmol), tert-butanol (106 µL) and 2-methoxyaniline (40 µL, 0.320 mmol). After work-up, the crude was purified by RP Flash Chromatography (silica C18 12 g, 0-50% CH₃CN (+0.1% HCOOH) in H₂O (+0.1% HCOOH)). The appropriate fractions were collected and evaporated to give **E150** (25 mg, 0.072 mmol, yield= 33.8%) as a pale yellow oil.

Method 2: R*t*= 1.31 min, MS (ESI) m/z =350.29 [M+H]⁺.

¹H NMR (400 MHz, DMSO-*d*₆) δ ppm 7.59 - 7.65 (m, 2 H), 7.24 (d, J=7.5 Hz, 1 H), 7.15 - 7.21 (m, 1 H), 7.13 (dd, J=7.3, 1.4 Hz, 1 H), 7.00 - 7.08 (m, 2 H), 6.94 (d, J=8.1 Hz, 1 H), 6.91 (td, J=5.3, 2.6 Hz, 1 H), 6.83 - 6.88 (m, 1 H), 6.13 (dd, J=5.8, 1.9 Hz, 1 H), 6.06 (br t, J=5.5 Hz, 1 H), 5.92 (d, J=1.8 Hz, 1 H), 3.79 (s, 3 H), 3.75 (s, 3 H), 3.23 - 3.31 (m, 2 H), 2.76 (t, J=7.5 Hz, 2 H)

### Example 151: 4-(3-chlorophenoxy)-N-(2-methoxyphenethyl)pyridin-2-amine (E151)

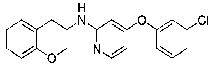

Prepared similarly as described for compound **E31** (step A) starting from 2-(2-methoxyphenyl)ethanamine (157.5 mg, 1.04 mmol) and 2-chloro-4-(3-chlorophenoxy)pyridine (ChemSpace, cat n°CSMB00011922705) (250 mg, 1.04 mmol). The reaction was heated under microwave irradiation at 150 °C in a closed vial for 4 h. After work-up, the residue was purified by RP Flash Chromatography (silica C18 12 g, 1-70% CH₃CN (+0.1% HCOOH) in H₂O (+0.1% HCOOH)). The appropriate fractions were collected and then evaporated to give **E151** (24.7 mg, 0.070 mmol, yield= 6.69%) as colorless oil

Method 2: R*t*= 1.56 min, MS (ESI) m/z =355.2 [M+H]⁺.

¹H NMR (400 MHz, DMSO-*d*₆) δ ppm 7.92 (d, J=5.8 Hz, 1 H), 7.43 - 7.53 (m, 1 H), 7.32 (ddd, J=8.0, 1.9, 0.8 Hz, 1 H), 7.25 (t, J=2.1 Hz, 1 H), 7.16 - 7.22 (m, 1 H), 7.05 - 7.15 (m, 2 H), 6.95 (d, J=7.9 Hz, 1 H), 6.86 (td, J=7.4, 0.9 Hz, 1 H), 6.63 (t, J=5.6 Hz, 1 H), 6.16 (dd, J=5.8, 2.2 Hz, 1 H), 5.93 (d, J=2.2 Hz, 1 H), 3.76 (s, 3 H), 3.33 - 3.42 (m, 2 H), 2.77 (t, J=7.5 Hz, 2 H)

### Example 152: N⁴-(3-chlorophenyl)-N²-(2-methoxybenzyl)pyridine-2,4-diamine (E152)

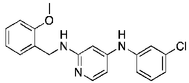

Prepared similarly as described for compound **E148** starting from **D42** and 3-chloroaniline (0.07 mL, 0.710 mmol). The reaction was heated at 120 °C in a MW reactor for 30 minutes. After work-up the crude was purified by NP Flash Chromatography (silica gel column, Sfar D, 25 g, 0-100% EtOAc in cyclohexane) to give an impure product. The product was purified additionally by RP Flash Chromatography (Sfar C18, 12 g, eluent: CH₃CN (+0.05% NH₃) in H₂O (+0.05% NH₃)). The appropriate fractions were collected and lyophilized to give **E152** (8 mg, 0.024 mmol, yield= 4%) as a white solid.

### Method 2: Rt= 1.38 min, MS (ESI) m/z = 340.2 [M+H]⁺.

¹H NMR (400 MHz, DMSO-*d*₆) δ ppm 8.58 (s, 1 H), 7.70 (d, J=6.4 Hz, 1 H), 7.24 - 7.30 (m, 1 H), 7.16 - 7.23 (m, 2 H), 7.10 (t, J=2.0 Hz, 1 H), 7.04 (dd, J=8.2, 1.4 Hz, 1 H), 6.92 - 6.99 (m, 2 H), 6.87 (t, J=7.2 Hz, 1 H), 6.63 (t, J=6.0 Hz, 1 H), 6.08 - 6.25 (m, 2 H), 4.37 (d, J=5.9 Hz, 2 H), 3.80 (s, 3 H).

### Example 153: 4-(6,6-difluoro-3-azabicyclo[3.1.0]hexan-3-yl)-N-(2-methoxyphenethyl)pyridin-2-amine (E153)

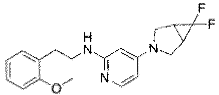

A test tube (1), which was equipped with a magnetic stir bar and fitted with a teflon septum, was charged with Palladium (II) Acetate (23.8 mg, 0.110 mmol) and 2,2'-Bis(diphenylphosphino)-1,1'-binaphthalene (rac-BINAP) (151.4 mg, 0.320 mmol). The vessel was evacuated and backfilled with nitrogen (3 x) and tert-butanol (400 µL) was added via syringe. Then, the solution was heated to 110 °C for 2 min.

A second test tube (2), which was equipped with a magnetic stir bar and fitted with a Teflon septum, was charged with potassium carbonate (117.1 mg, 0.850 mmol), **D41** (75 mg, 0.210 mmol), tert-butanol (400 µL), 6,6-difluoro-3-azabicyclo[3.1.0]hexane;hydrochloride (72.7 mg, 0.420 mmol). The vessel was evacuated and backfilled with nitrogen (this process was repeated 3 times).

Then, to solution 2, the activated catalyst (solution 1) was added and the reaction was heated at 110 °C for 12 h. Then, the reaction was cooled to RT, poured into water, and extracted with EtOAc (2x), the combined organic layers were washed with brine, dried over Na₂SO₄, filtered and evaporated under reduced pressure. The residue was purified by RP Flash Chromatography (silica C18 12 g, 1-50% CH₃CN (+0.1% HCOOH) in H₂O (+0.1% HCOOH)). The appropriate fractions were collected and evaporated to give **E153** (15 mg, 0.043 mmol, yield= 20.51%) as a pale yellow oil.

Method 2: Rt= 1.27 min, MS (ESI) m/z =346.3 [M+H]⁺.

¹H NMR (400 MHz, DMSO-*d*₆) δ ppm 7.62 (d, J=5.9 Hz, 1 H), 7.09 - 7.26 (m, 2 H), 6.96 (d, J=8.1 Hz, 1 H), 6.87 (t, J=7.3 Hz, 1 H), 6.16 (br s, 1 H), 5.79 - 5.90 (m, 1 H), 5.45 (d, J=1.5 Hz, 1 H), 3.79 (s, 3 H), 3.56 (s, 4 H), 3.29 - 3.36 (m, 2 H), 2.78 (t, J=7.5 Hz, 2 H), 2.68 (br d, J=11.6 Hz, 2 H)

### Example 154: 4-((6,6-difluoro-3-azabicyclo[3.1.0]hexan-3-yl)methyl)-N-(2-methoxyphenethyl)pyridin-2-amine; dihydrochloride (E154)

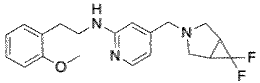

Prepared similarly as described for compound **E49** starting from **D94** (350 mg, 1.2445 mmol, 1.0 eq) and 2-(2-methoxyphenyl)ethan-1-amine (380 mg, 2.4891 mmol, 2.0 eq). The reaction was stirred at 180°C for 16 hours. After work-up, the crude was purified by RP preparative HPLC. The fractions containing the product of interest were concentrated. Few drops of conc HCl were added. Through lyophilization, **E154** (52.2 mg, 0.1488 mmol, yield= 12%) was obtained as off-white solid (2x HCl salt).

### Method 4: Rt= 1.67 min, MS (ESI) m/z = 360.3 [M+H] ⁺.

¹H NMR (400 MHz, DMSO-*d*₆) δ ppm 13.81 (s, 1H), 12.88 (s, 1H), 9.19 (s, 1H), 7.98 (s, 1H), 7.40-7.03 (m, 4H), 6.98 (dd, J = 8.3, 1.1 Hz, 1H), 6.90 (td, J = 7.4, 1.1 Hz, 1H), 4.85-4.07 (m, 4H), 3.78 (s, 3H), 3.58 (d, J = 6.6 Hz, 3H), 3.13 (s, 1H), 2.98-2.71 (m, 4H).

### Example 155: trans-6,6-difluoro-N-((2-((2-methoxyphenethyl)amino)pyridin-4-yl)methyl)bicyclo[3.1.0]hexane-3-carboxamide (E155)

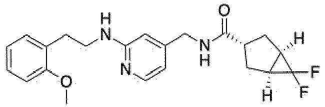

Prepared similarly as described for compound **E49** starting from **D84** (400 mg, 1.48 mmol, 1.0 eq.) and 2-(2-methoxyphenyl)ethan-1-amine (440 mg, 2.96 mmol, 2.0 eq.). The reaction was stirred at 170°C, ON. After work-up, the crude was purified using RP preparative HPLC. The appropriate fractions were concentrated and freeze-dried to yield **E155** (120 mg, 0.29 mmol, yield= 19%).

Method 4: R*t*= 2.15 min, MS (ESI) m/z = 402.2 [M+H]⁺.

¹H NMR (400 MHz, DMSO-*d*₆) δ ppm 8.43 (t, J = 6.0 Hz, 1H), 7.87 (d, J = 5.2 Hz, 1H), 7.24-7.12 (m, 2H), 6.96 (dd, J = 8.3, 1.1 Hz, 1H), 6.86 (td, J = 7.4, 1.1 Hz, 1H), 6.51 (t, J = 5.7 Hz, 1H), 6.31 (dd, J = 5.3, 1.4 Hz, 1H), 6.26 (d, J = 1.2 Hz, 1H), 4.10 (d, J = 6.0 Hz, 2H), 3.79 (s, 3H), 3.42-3.33 (m, 2H), 2.79 (dd, J = 8.5, 6.4 Hz, 2H), 2.71-2.58 (m, 1H), 2.21-2.09 (m, 6H).

### Example 156: 1-(6,6-difluoro-3-azabicyclo[3.1.0]hexan-3-yl)-2-(2-((2-methoxyphenethyl)amino)pyridin-4-yl)ethan-1-one (E156)

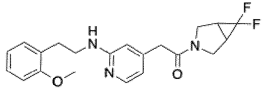

Prepared similarly as described for compound **E49** starting from **D85** (120 mg, 0.375 mmol, 1.0 eq) and 2-(2-methoxyphenyl)ethan-1-amine (113 mg, 0.749 mmol, 2.0 eq). The reaction was stirred at 180°C for 4h. After work-up, the crude was purified using RP preparative HPLC. The appropriate fractions were freeze-dried to yield **E156** (1.6 mg, 0.004 mmol, yield= 1%).

Method 9: Rt= 3.34 min, MS (ESI) m/z = 388.2 [M+H]⁺.

¹H NMR (400 MHz, DMSO-*d*₆) δ ppm 7.86 (d, J = 5.2 Hz, 1H), 7.24-7.10 (m, 2H), 6.96 (dd, J = 8.3, 1.1 Hz, 1H), 6.86 (td, J = 7.4, 1.1 Hz, 1H), 6.46 (t, J = 5.7 Hz, 1H), 6.33-6.29 (m, 1H), 6.28 (s, 1H), 3.87-3.69 (m, 6H), 3.62-3.53 (m, 1H), 3.46 (s, 2H), 3.41-3.34 (m, 2H), 2.79 (t, 2H), 2.71-2.52 (m, 2H).

### Example 157: trans-6,6-difluoro-N-(2-((2-methoxyphenethyl)amino)pyridin-4-yl)bicyclo[3.1.0]hexane-3-carboxamide (E157)

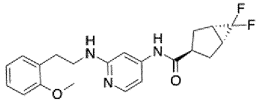

Prepared similarly as described for compound **E49** starting from **D95** (70 mg, 0.22 mmol, 1.0 eq.) and 2-(2-methoxyphenyl)ethan-1-amine (100 mg, 0.66 mmol, 3.0 eq.). The reaction was stirred at 180 °C, ON. After work-up, the crude was purified using RP preparative HPLC. The appropriate fractions were freeze-dried to yield **E157** (0.025 mg, 0.065mmol, yield= 27%) as white solid.

Method 4: Rt= 2.42 min, MS (ESI) m/z = 388.2 [M+H]⁺.

¹H NMR (400 MHz, DMSO-*d*₆) δ ppm 10.00 (s, 1H), 7.81 (d, J = 5.7 Hz, 1H), 7.25-7.11 (m, 2H), 6.95 (d, J = 8.2 Hz, 1H), 6.93-6.81 (m, 2H), 6.58 (dd, J = 5.7, 1.8 Hz, 1H), 6.50 (s, 1H), 3.79 (s, 3H), 3.35 (s, 2H), 2.78 (t, J = 7.5 Hz, 3H), 2.24-2.15 (m, 6H).

### Example 158: 2-((2-((2-(1H-indol-3-yl)ethyl)amino)pyrimidin-4-yl)amino)benzonitrile (E158)

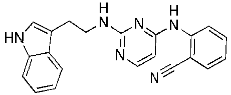

In a MW vial, a mixture of **D43** (100 mg, 0.430 mmol), 2-(1H-indol-3-yl)ethanamine (69.5 mg, 0.430 mmol) and DIPEA (110 µL, 0.650 mmol) in 1-Butanol (1 mL) was heated to 150 °C and stirred for 17 hours. The mixture was concentrated under reduced pressure. The oily residue was purified by NP Flash Chromatography (Sfar Amino D, 11 g, 25-95% EtOAc in cyclohexane). The appropriate fractions were collected and evaporated. The yellow sticky solid was triturated in DCM, filtered and dried under vacuum for two days to give **E158** (72 mg, 0.203 mmol, yield= 46.86%) as a yellow solid.

Method 2: R*t*= 1.18 min, MS (ESI) m/z = 355.3 [M+H]⁺.

¹H NMR (400 MHz, DMSO-*d*₆) δ ppm 10.76 (br s, 1 H), 9.25 (br s, 1 H), 7.94 (br s, 1 H), 7.80 (d, J=8.3 Hz, 1 H), 7.75 (d, J=7.5 Hz, 1 H), 7.36 - 7.68 (m, 2 H), 7.32 (d, J=8.1 Hz, 1 H), 7.22 (t, J=7.6 Hz, 1 H), 7.05 (t, J=7.6 Hz, 1 H), 7.08 (br s, 1 H), 6.93 (br t, J=7.1 Hz, 1 H), 6.79 (br s, 1 H), 6.09 (d, J=5.5 Hz, 1 H), 3.45 (br s, 2 H), 2.87 (br t, J=7.2 Hz, 2 H).

### Example 159: 2-((2-((2-methoxyphenethyl)amino)pyrimidin-4-yl)amino)benzonitrile (E159)

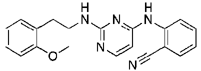

Prepared similarly as described for compound **E158** starting from **D43** (100 mg, 0.430 mmol) and 2-(2-methoxyphenyl)ethanamine (60 µL, 0.430 mmol). After work-up, the crude product was purified by RP Flash Chromatography (Sfar C18, 12 g, eluent: CH₃CN (+0.1% HCOOH) in H₂O (+0.1% HCOOH)). The appropriate fractions were collected and lyophilized to give **E159** (41.3 mg, 0.120 mmol, yield= 27.58%) as a white solid.

Method 2: Rt= 1.22 min, MS (ESI) m/z = 346.3 [M+H]⁺.

¹H NMR (400 MHz, DMSO-*d*₆) δ ppm 9.25 (br s, 1 H), 7.91 (br d, J=3.0 Hz, 1 H), 7.80 (br d, J=8.2 Hz, 1 H), 7.76 (dd, J=7.8, 1.2 Hz, 1 H), 7.60 (br t, J=7.6 Hz, 1 H), 7.25 (t, J=7.5 Hz, 1 H), 7.14 - 7.20 (m, 1 H), 6.92 (d, J=8.2 Hz, 1 H), 6.82 (br t, J=7.0 Hz, 1 H), 6.47 - 7.38 (m, 2 H), 6.07 (d, J=5.6 Hz, 1 H), 3.74 (br s, 3 H), 3.20 - 3.46 (m, 2 H), 2.73 (br s, 2 H).

### Example 160: 2-((2-((2-methoxybenzyl)amino)pyrimidin-4-yl)amino)-benzonitrile (E160)

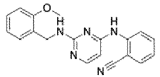

Prepared similarly as described for compound **E158** starting from **D43** (100 mg, 0.430 mmol) and (2-methoxyphenyl)methanamine (60 µL, 0.430 mmol). The crude was purified by RP Flash Chromatography (Sfar Amino D, 11 g, 25-95% EtOAc in cyclohexane). The appropriate fractions were concentrated to give impure product. A second purification by RP Flash Chromatography (Sfar C18, 12 g, eluent: CH₃CN (+0.1% HCOOH) in H₂O (+0.1% HCOOH)) was performed to yield **E160** (43 mg, 0.130 mmol, yield= 29.93%) as a white solid.

Method 2: R*t*= 1.11 min, MS (ESI) m/z = 332.3 [M+H]⁺.

¹H NMR (400 MHz, DMSO-*d*₆) δ ppm 9.24 (s, 1 H), 7.91 (br d, J=4.9 Hz, 1 H), 7.15 - 7.21 (m, 1 H), 6.99 - 8.00 (m, 6 H), 6.95 (d, J=8.2 Hz, 1 H), 6.83 (t, J=7.3 Hz, 1 H), 6.10 (d, J=5.8 Hz, 1 H), 4.36 (br s, 2 H), 3.78 (s, 3 H)

### Example 161: N⁴-(2-chlorobenzyl)-N²-(2-methoxyphenethyl)pyrimidine-2,4-diamine (E161)

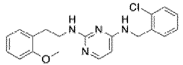

Prepared similarly as described for compound **E158** starting from **D44** (100 mg, 0.390 mmol) and 2-(2-methoxyphenyl)ethanamine (60 µL, 0.390 mmol).The crude was purified by NP Flash Chromatography (Sfar KP-Amino D, 11 g, 20-70% EtOAc in cyclohexane) to give an oil that was washed 3 times with DEE and then dried under vacuum to afford E161 (79 mg, 0.214 mmol, yield= 54.43%) as an off-white solid.

Method 2: Rt=1.49 min, MS (ESI) m/z = 369.3 [M+H]⁺.

¹H NMR (400 MHz, DMSO-*d*₆) δ ppm 7.67 (br d, J=5.2 Hz, 1 H), 7.39 - 7.46 (m, 1 H), 7.32 - 7.38 (m, 1 H), 7.31 - 7.49 (m, 1 H), 7.22 - 7.32 (m, 2 H), 7.12 - 7.20 (m, 1 H), 6.92 (d, J=8.2 Hz, 1 H), 6.87 - 7.10 (m, 1 H), 6.81 (br t, J=7.2 Hz, 1 H), 6.41 (br s, 1 H), 5.81 (br s, 1 H), 4.56 (br s, 2 H), 3.74 (s, 3 H), 3.25 - 3.39 (m, 2 H), 2.70 (br s, 2 H).

### Example 162: N²-(2-(1H-indol-3-yl)ethyl)-N⁴-(2-chlorobenzyl)pyrimidine-2,4-diamine (E162)

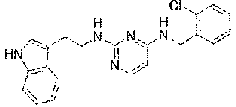

Prepared similarly as described for compound **E158** starting from **D44** (100 mg, 0.390 mmol) and 2-(1H-indol-3-yl)ethanamine (63.1 mg, 0.390 mmol). The reaction was stirred under microwave heating at 140°C for 24h. Then, 2-(1H-indol-3-yl)ethanamine (18.9 mg, 0.120 mmol) and DIPEA (20 µL, 0.120 mmol) were added and the mixture stirred at 140 °C for 6 hours. After work-up, the crude was purified by NP Flash Chromatography (Sfar Amino D, 11 g, 25-95% EtOAc in cyclohexane. The appropriate fractions were collected and evaporated to give **E162** (56 mg, 0.148 mmol, yield= 37.66%) as an off-white solid.

Method 1: R*t*= 0.81 min, MS (ESI) m/z = 378.3 [M+H]⁺.

¹H NMR (400 MHz, DMSO-*d*₆) δ ppm 10.75 (br s, 1 H), 7.69 (br s, 1 H), 7.49 (br s, 1 H), 7.33 - 7.44 (m, 3 H), 7.20 - 7.33 (m, 3 H), 7.07 (br s, 1 H), 6.99 - 7.08 (m, 1 H), 6.84 - 6.97 (m, 1 H), 6.46 (br s, 1 H), 5.81 (br s, 1 H), 4.57 (br s, 2 H), 3.43 (q, J=6.6 Hz, 2 H), 2.83 (br s, 2 H).

### Example 163: N⁴-(2-chlorobenzyl)-N²-(2-methoxybenzyl)pyrimidine-2,4-diamine (E163)

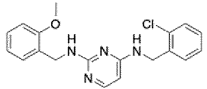

Prepared similarly as described for compound **E158** starting from **D46** (100 mg, 0.400 mmol) and (2-chlorophenyl)methanamine (50 µL, 0.400 mmol). The reaction mixture was stirred under microwave heating at 140°C for 24h. Then (2-chlorophenyl)methanamine (10 µL, 0.120 mmol) was added and the mixture was stirred at 140 °C for 14 hours. After work-up, the crude oily residue was purified by NP Flash Chromatography (Sfar D, 11 g, 25-65% EtOAc in cyclohexane). The appropriate fractions were collected and evaporated to give **E163** (85 mg, 0.240 mmol, yield= 59.82%) as an off-white solid.

### Method 1: Rt=0.80 min, MS (ESI) m/z = 355.3 [M+H]⁺.

¹H NMR (400 MHz, DMSO-*d*₆) δ ppm 7.64 (br d, J=5.6 Hz, 1 H), 7.36 - 7.46 (m, 2 H), 7.16 (br t, J=7.7 Hz, 1 H), 7.03 - 7.13 (m, 1 H), 7.02 - 7.47 (m, 3 H), 6.92 (d, J=8.1 Hz, 1 H), 6.74 - 6.86 (m, 1 H), 6.68 (br t, J=6.2 Hz, 1 H), 5.65 - 6.02 (m, 1 H), 4.50 (br s, 2 H), 4.36 (br d, J=6.2 Hz, 2 H), 3.78 (s, 3 H).

### Example 164: N4-(2-methoxybenzyl)-N2-(2-methoxyphenethyl)pyrimidine-2,4-diamine (E164)

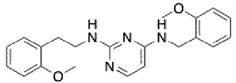

Prepared similarly as described for compound **E158** starting from **D45** (100 mg, 0.400 mmol) and 2-(2-methoxyphenyl)ethanamine (60 µL, 0.400 mmol). The reaction mixture was stirred under microwave heating at 150°C for 8h. After work-up, the crude was purified by NP Flash Chromatography (Sfar KP-Amino D, 11 g, 20-70% EtOAc in cyclohexane). The oily residue was stripped one time with cyclohexane and 3 times with DEE to afford **E164** (67 mg, 0.184 mmol, yield= 45.91%) as an off-white solid.

Method 2: Rt= 1.45 min, MS (ESI) m/z = 365.3 [M+H]⁺.

¹H NMR (400 MHz, DMSO-*d*₆) δ ppm 7.63 (br d, J=4.9 Hz, 1 H), 7.12 - 7.24 (m, 3 H), 6.99 - 7.10 (m, 1 H), 6.90 - 6.99 (m, 2 H), 6.77 - 7.27 (m, 1 H), 6.76 - 6.90 (m, 2 H), 6.36 (br s, 1 H), 5.62 - 5.91 (m, 1 H), 4.31 - 4.61 (m, 2 H), 3.80 (s, 3 H), 3.75 (s, 3 H), 3.24 - 3.39 (m, 2 H), 2.73 (br t, J=7.2 Hz, 2 H).

### Example 165: N²-(2-(1H-indol-3-yl)ethyl)-N⁴-(2-methoxybenzyl)pyrimidine-2,4-diamine (E165)

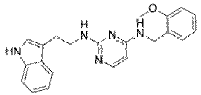

Prepared similarly as described for compound **E158** starting from **D45** (100 mg, 0.400 mmol) and 2-(1H-indol-3-yl)ethanamine (64.16 mg, 0.400 mmol). After work-up, the crude was purified by RP Flash Chromatography (Sfar Amino D, 11 g, 25-95% EtOAc in cyclohexane). The appropriate fractions were collected and evaporated to give **E165** (32 mg, 0.086 mmol, yield= 21.4%) as a white solid.

Method 1: R*t*= 0.81 min, MS (ESI) m/z = 374.4 [M+H]⁺.

¹H NMR (400 MHz, DMSO-*d₆*)δ ppm 10.76 (br s, 1 H), 7.57 - 7.76 (m, 1 H), 7.42 - 7.57 (m, 1 H), 7.31 (d, J=8.1 Hz, 1 H), 7.20 (d, J=7.4 Hz, 1 H), 7.17 - 7.26 (m, 1 H), 7.11 - 7.18 (m, 1 H), 7.06 - 7.11 (m, 1 H), 7.04 (t, J=7.4 Hz, 1 H), 6.96 (d, J=8.1 Hz, 1 H), 6.93 (br t, J=7.3 Hz, 1 H), 6.87 (br t, J=7.5 Hz, 1 H), 6.41 (br s, 1 H), 5.63 - 5.92 (m, 1 H), 4.19 - 4.71 (m, 2 H), 3.79 (s, 3 H), 3.37 - 3.53 (m, 2 H), 2.86 (br t, J=7.5 Hz, 2 H).

### Example 166: N²,N⁴-bis(2-methoxybenzyl)pyrimidine-2,4-diamine (E166)

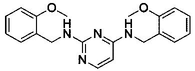

A mixture of **D45** (100 mg, 0.400 mmol) and (2-methoxyphenyl)methanamine (50 µL, 0.400 mmol) and potassium carbonate (166 mg, 1.2 mmol) in NMP (800 µL) was heated to 140 °C and stirred for 24 hours. Then, (2-methoxyphenyl)methanamine (20 µL, 0.120 mmol) was added and the mixture was stirred at 140 °C for 14 hours. The reaction was allowed to RT, HCl (1M) was added and the mixture was extracted with EtOAc (x2). The organic layer was washed with ss NaHCOs solution, water, then it was dried over Na₂SO₄, filtered and evaporated under reduced pressure. The oily residue was purified by NP Flash Chromatography (Sfar D, 11 g, 30-70% EtOAc in cyclohexane) to give **E166** (40 mg, 0.114 mmol, yield= 28.5%) as a yellow sticky solid.

Method 1: R*t*= 0.79 min, MS (ESI) m/z = 351.4 [M+H]⁺.

¹H NMR (400 MHz, DMSO-*d*₆) δ ppm 7.51 - 7.69 (m, 1 H), 7.07 - 7.26 (m, 5 H), 6.90 - 6.99 (m, 2 H), 6.74 - 6.88 (m, 2 H), 6.62 (br t, J=6.2 Hz, 1 H), 5.46 - 5.95 (m, 1 H), 4.27 - 4.52 (m, 4 H), 3.72 - 3.85 (m, 6 H).

### Example 167: N⁴-(2-chlorobenzyl)-N⁶-(2-methoxyphenethyl)pyrimidine-4,6-diamine (E167)

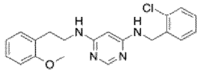

Prepared similarly as described for compound E158 starting from D47 (100 mg, 0.390 mmol) and 2-(2-methoxyphenyl)ethanamine (60 µL, 0.390 mmol). The reaction was stirred under microwave heating at 150°C for 17h. Then, 2-(2-methoxyphenyl)ethanamine (20 µL, 0.120 mmol) and DIPEA (2O µL, 0.120 mmol) were added and the mixture was stirred at 150°C for 4 hours. The crude was purified by NP Flash Chromatography (Sfar KP-Amino D, 11 g, 20-70% EtOAc in cyclohexane) to give **E167** (108 mg, 0.293 mmol, yield= 74.4%) as an off-white solid.

Method 2: R*t*=1.42 min, MS (ESI) m/z = 369.3 [M+H]⁺.

¹H NMR (400 MHz, DMSO-*d*₆) δ ppm 7.90 (s, 1 H), 7.39 - 7.50 (m, 1 H), 7.23 - 7.35 (m, 3 H), 7.15 - 7.23 (m, 1 H), 7.01 - 7.15 (m, 2 H), 6.95 (d, J=8.1 Hz, 1 H), 6.86 (t, J=7.5 Hz, 1 H), 6.65 (br s, 1 H), 5.40 (br s, 1 H), 4.46 (br d, J=5.7 Hz, 2 H), 3.78 (s, 3 H), 3.29 (br s, 2 H), 2.74 (br t, J=7.5 Hz, 2 H).

### Example 168: N⁴-(2-(1H-indol-3-yl)ethyl)-N⁶-(2-chlorobenzyl)pyrimidine-4,6-diamine (E168)

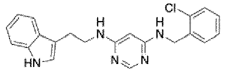

Prepared similarly as described for compound **E158** starting from **D47** (100 mg, 0.390 mmol) and 2-(1H-indol-3-yl)ethanamine (63 mg, 0.390 mmol). The reaction mixture was stirred under microwave heating at 140 °C for 17 hours. Then, 2-(1H-indol-3-yl)ethanamine (18.9 mg, 0.120 mmol) and DIPEA (20 µL, 0.120 mmol) were added and the mixture was stirred for 6 hours. The crude was purified by NP Flash Chromatography (Sfar Amino D, 11 g, 25-95% EtOAc in cyclohexane) to give **E168** (86 mg, 0.228 mmol, yield= 57.84%) as an off-white solid.

### Method 1: Rt= 0.77 min, MS (ESI) m/z = 378.3 [M+H]⁺.

¹H NMR (400 MHz, DMSO-*d*₆) δ ppm 10.80 (br s, 1 H), 7.93 (s, 1 H), 7.52 (d, J=7.7 Hz, 1 H), 7.39 - 7.46 (m, 1 H), 7.23 - 7.36 (m, 4 H), 7.15 (d, J=1.8 Hz, 1 H), 7.10 - 7.15 (m, 1 H), 7.02 - 7.09 (m, 1 H), 6.91 - 7.01 (m, 1 H), 6.69 (br s, 1 H), 5.43 (br s, 1 H), 4.46 (br d, J=5.7 Hz, 2 H), 3.34 - 3.49 (m, 2 H), 2.88 (br t, J=7.5 Hz, 2 H).

### Example 169: N⁴-(2-chlorobenzyl)-N⁶-(2-methoxybenzyl)pyrimidine-4,6-diamine (E169)

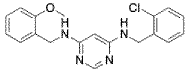

Prepared similarly as described for compound **E158** starting from **D99** (100 mg, 0.400 mmol) and (2-chlorophenyl)methanamine (50 µL, 0.400 mmol). The reaction mixture was stirred under microwave heating at 140 °C for 17h. Then, (2-chlorophenyl)methanamine (10 µL, 0.120 mmol) and DIPEA (30 µL, 0.200 mmol) were added and the mixture was stirred for 8 hours. Then, (2-chlorophenyl)methanamine (10 µL, 0.120 mmol) and DIPEA (30 µL, 0.200 mmol) were added, the temperature was raised to 150 °C and the mixture was stirred for 17 hours. The reaction was allowed to cool to RT and a white precipitate formed. DEE (1 mL) was added, the solid was filtered, washed with DEE and dried under vacuum to give **E169** ( 72 mg, 0.203 mmol, yield= 50.67%) as a white solid.

Method 1: R*t*= 0.76 min, MS (ESI) m/z = 355.3 [M+H]⁺.

¹H NMR (400 MHz, DMSO-*d*₆) δ ppm 7.89 (s, 1 H), 7.42 (d, J=6.7 Hz, 1 H), 7.23 - 7.32 (m, 3 H), 7.18 - 7.23 (m, 1 H), 7.09 - 7.17 (m, 2 H), 6.93 - 7.02 (m, 2 H), 6.86 (t, J = 7.5 Hz, 1 H), 5.25 - 5.54 (m, 1 H), 4.43 (br d, J = 4.9 Hz, 2 H), 4.32 (br s, 2 H), 3.80 (s, 3 H).

### Example 170: N⁴,N⁶-bis(2-methoxybenzyl)pyrimidine-4,6-diamine (E170)

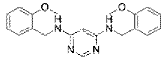

Prepared similarly as described for compound **E158** starting from **D99** (100 mg, 0.400 mmol) and (2-methoxyphenyl)methanamine (50 µL, 0.400 mmol). The reaction was stirred under microwave heating at 140 °C and for 17 hours. Then, (2-methoxyphenyl)methanamine (20 µL, 0.120 mmol) and DIPEA (30 µL, 0.200 mmol) were added and the mixture was stirred for 6 hours. After work-up, the crude was purified by NP Flash Chromatography (Sfar Amino D, 11 g, 20-65% EtOAc in cyclohexane) to give **E170** (35 mg, 0.100 mmol, yield= 24.94%) as a white solid.

Method 1: R*t*= 0.75 min, MS (ESI) m/z = 351.4 [M+H]⁺.

¹H NMR (400 MHz, DMSO-*d*₆) δ ppm 7.87 (s, 1 H), 7.17 - 7.24 (m, 2 H), 7.13 (d, J = 7.0 Hz, 2 H), 6.96 (d, J = 8.0 Hz, 2 H), 6.91 (br t, J = 6.0 Hz, 2 H), 6.86 (t, J = 7.3 Hz, 2 H), 5.25 - 5.52 (m, 1 H), 4.21 - 4.40 (m, 4 H), 3.80 (s, 6 H).

### Example 171: N⁴-(2-methoxybenzyl)-N⁶-(2-methoxyphenethyl)pyrimidine-4,6-diamine (E171)

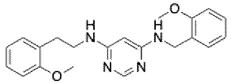

Prepared similarly as described for compound **E158** starting from **D99** (100 mg, 0.400 mmol) and 2-(2-methoxyphenyl)ethanamine (60 µL, 0.400 mmol). The reaction was stirred under microwave heating at150 °C and for 8 hours. Then DIPEA (30 µL, 0.200 mmol) and 2-(2-methoxyphenyl)ethanamine (10 µL, 0.080 mmol) were added and the mixture was stirred for 6 hours. The reaction was allowed to cool to RT and concentrated under reduced pressure. The oily residue was purified by NP Flash Chromatography (Sfar KP-Amino D, 11 g, 20-75% EtOAc in cyclohexane) to give **E171** (90 mg, 0.247 mmol, yield= 61.66%) as a white solid.

Method 4: Rt=1.35 min, MS (ESI) m/z = 365.3 [M+H]⁺.

¹H NMR (400 MHz, DMSO-*d*₆) δ ppm 7.88 (s, 1 H), 7.13 - 7.25 (m, 3 H), 7.10 (br d, J=7.0 Hz, 1 H), 6.93 - 7.00 (m, 2 H), 6.79 - 6.92 (m, 3 H), 6.58 (br d, J=3.8 Hz, 1 H), 5.23 - 5.48 (m, 1 H), 4.34 (br d, J=5.2 Hz, 2 H), 3.80 (s, 3 H), 3.77 (s, 3 H), 3.19 - 3.31 (m, 2 H), 2.74 (br t, J=7.3 Hz, 2 H).

### Example 172: N⁴-(2-(1H-indol-3-yl)ethyl)-N⁶-(2-methoxybenzyl)pyrimidine-4,6-diamine (E172)

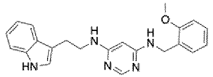

Prepared similarly as described for compound **E158** starting from **D99** (100 mg, 0.400 mmol) and 2-(1H-indol-3-yl)ethanamine (64.2 mg, 0.400 mmol). The reaction was stirred under microwave heating at 140 °C and for 17 hours. Then, 2-(1H-indol-3-yl)ethanamine (19.3 mg, 0.120 mmol) and DIPEA (20 µL, 0.120 mmol) were added and the mixture was stirred for 6 hours. After work-up, the crude was purified by NP Flash Chromatography (Sfar Amino D, 11 g, 25-95% EtOAc in cyclohexane) to give **E172** (88 mg, 0.236 mmol, yield= 58.84%) as a light brown solid.

Method 1: Rt= 0.76 min, MS (ESI) m/z = 374.4 [M+H]⁺.

¹H NMR (400 MHz, DMSO-*d*₆) δ ppm 10.80 (br s, 1 H), 7.91 (s, 1 H), 7.52 (d, J=7.8 Hz, 1 H), 7.33 (d, J=8.1 Hz, 1 H), 7.18 - 7.24 (m, 1 H), 7.12 - 7.18 (m, 2 H), 7.03 - 7.08 (m, 1 H), 6.94 - 6.99 (m, 2 H), 6.84 - 6.92 (m, 2 H), 6.62 (br s, 1 H), 5.38 (br s, 1 H), 4.34 (br d, J=3.4 Hz, 2 H), 3.80 (s, 3 H), 3.34 - 3.49 (m, 2 H), 2.87 (br t, J=7.5 Hz, 2 H).

### Example 173: 6-(3-chlorophenoxy)-N-(2-methoxyphenethyl)pyrimidin-4-amine (E173)

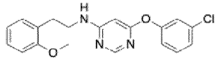

Prepared similarly as described for compound **E158** starting from **D100** (100 mg, 0.410 mmol) and 2-(2-methoxyphenyl)ethanamine (62.72 mg, 0.410 mmol) in Ethanol (3 mL). The reaction was stirred at 70 °C for 20 h. After work-up the crude was purified by RP Flash Chromatography (C-18 column, 30 g, 10-100% MeCN (+0.1% HCOOH) in water (+0.1% HCOOH)) to afford **E173** (30 mg, 0.084 mmol, yield= 20.33%) as white solid.r.t.

Method 2: R*t*= 2.25 min, MS (ESI) m/z = 356.09 [M+H]⁺.

¹H NMR (400 MHz, DMSO-*d*₆) δ ppm 7.89 - 8.41 (m, 1 H), 7.47 - 7.54 (m, 1 H), 7.42 - 7.47 (m, 1 H), 7.29 - 7.34 (m, 1 H), 7.27 - 7.29 (m, 1 H), 7.18 - 7.24 (m, 1 H), 7.07 - 7.17 (m, 2 H), 6.96 (d, J=8.1 Hz, 1 H), 6.87 (t, J=7.4 Hz, 1 H), 5.50 - 6.13 (m, 1 H), 3.78 (s, 3 H), 3.33 - 3.59 (m, 2 H), 2.79 (t, J=7.4 Hz, 2 H)

### Example 174: 1-(2-((2-methoxyphenethyl)amino)pyridin-4-yl)imidazolidin-2-one (E174)

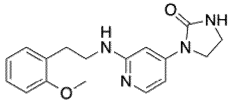

A flame-dried resealable tube was charged with Cul (6 mg, 0.031 mmol, 0.1 eq), **D41** (110 mg, 0.3106mmol, 1 eq), K₃PO₄ (138 mg, 0.652 mmol, 2.1 eq), 2-Imidazolidone (401 mg, 4.659 mmol, 15 eq), trans-N,N'-Dimethylcyclohexane-1,2-diamine (9 mg, 0.062 mmol, 0.2 eq) and anh DMF (5 mL, 0.06M). The Schlenk tube was fixed with a rubber septum, evacuated twice and back-filled with argon. The reaction tube was sealed and the contents were stirred with heating via an oil bath at 110°C for 18 hours. The reaction mixture was cooled to RT, diluted with 50 mL EtOAc, washed with brine, a few drops of water sol. NH₃, brine and dried over Na₂SO₄. Crude was purified by RP Preparative HPLC to provide **E174** (30.9 mg, 0.1mmol, yield= 32%) of the as a white solid after freeze drying.

Method 4: R*t*= 1.76 min, MS (ESI) m/z = 313.2 [M+H]⁺.

¹H (400 MHz, DMSO-*d*₆) δ ppm 7.80 (d, J= 5.9 Hz, 1 H), 7.24-7.09 (m, 3 H), 6.96 (dd, J=8.2, 1.1 Hz, 1 H), 6.87 (td, J= 7.4, 1.1 Hz, 1 H), 6.79 (dd, J= 5.9, 2.0 Hz, 1 H), 6.58 ( d, J= 2.0 Hz, 1 H), 6.33 (t, J= 5.6 Hz, 1 H), 3.79 (s, 3 H), 3.79-3.72 (m, 2 H), 3.44-3.33 (m, 4 H), 2.84-2.75 (m, 2 H).

### Example 175:1-(2-((2-methoxyphenethyl)amino)pyridin-4-yl)-3-methylimidazolidin-2-one (E175)

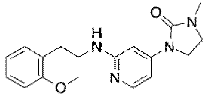

Prepared similarly as described for compound **E174** starting from **D41** (110 mg, 0.3106mmol, 1 eq), and 1-Methyl-2-imidazolidinone (62 mg, 0.621 mmol, 2 eq). After work-up, the crude was purified by RP Preparative HPLC and freeze-dried to yield **E175** (0,061 mg, 0.19mmol, yield= 61%) as a white solid.

Method 4: R*t*= 1.91 min, MS (ESI) m/z = 327.2 [M+H]⁺.

¹H (400 MHz, DMSO-*d*₆) δ ppm 7.81 (d, J= 5.9 Hz, 1 H), 7.24-7.13 (m, 2 H), 6.96 (dd, J= 8.3, 1.1 H, 1 H), 6.87 ( td, J= 7.4, 1.1Hz, 1 H), 6.82 (dd, J= 5.9, 2.0 Hz, 1 H), 6.56 (d,J= 1.9 Hz, 1 H), 6.34 ( t, J= 5.6 Hz, 1 H), 3.79 (s, 3 H), 3.75-3.67 (m, 2 H), 3.48-3.40 (m, 2 H), 3.40-3.34 (m, 2 H), 2.80 (t, J= 7.9Hz, 2 H), 2.76 (s, 3 H).

### Example 176: 1-(2-((2-methoxyphenethyl)amino)pyridin-4-yl)-1,3-dihydro-2H-benzo[d]imidazol-2-one (E176)

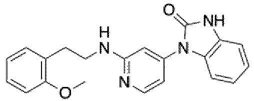

Prepared similarly as described for compound **E174** starting from **D41** (100 mg, 0.282 mmol, 1.0 eq) and 2-Hydroxybenzimidazole (568 mg, 4.235 mmol, 15.0 eq). After work-up, the crude was purified by RP Preparative HPLC and freeze-dried to yield **E176** (305 mg, 0.097 mmol, yield= 34%) as a white powder.

Method 4: R*t*= 2.04 min, MS (ESI) m/z = 361.2 [M+H]⁺.

1H NMR (400 MHz, DMSO-*d*₆) δ ppm 11.23 (s, 1H), 8.11 (d, J = 5.5 Hz, 1H), 7.24-7.14 (m, 3H), 7.13-7.01 (m, 3H), 6.96 (d, J = 8.1 Hz, 1H), 6.92-6.81 (m, 2H), 6.71 (dd, J = 7.0, 1.5 Hz, 2H), 3.76 (s, 3H), 3.45 (dt, J = 8.3, 6.1 Hz, 2H), 2.84 (dd, J = 8.6, 6.4 Hz, 2H).

### Example 177: 1-(2-((2-methoxyphenethyl)amino)pyridin-4-yl)-3,3-dimethylindolin-2-one (E177)

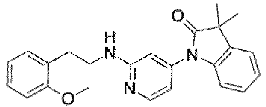

Prepared similarly as described for compound **E174** starting from **D41** (130 mg, 0.367 mmol, 1 eq) and 3,3-Dimethylindolin-2-one (71 mg, 0.44 mmol, 1.2 eq). After work-up, the crude was purified by RP Preparative HPLC and freeze-dried to yield **E177** (109 mg, 0.281mmol, yield= 72%).

Method 4: R*t*= 2.5 min, MS (ESI) m/z = 388.3 [M+H]⁺.

1H NMR (400 MHz, DMSO-*d*₆) δ ppm 8.16-8.10 (m, 1H), 7.46 (dd, J = 7.3, 1.3 Hz, 1H), 7.26 (td, J = 7.8, 1.4 Hz, 1H), 7.23-7.16 (m, 2H), 7.13 (td, J = 7.5, 1.0 Hz, 1H), 6.99-6.93 (m, 2H), 6.88 (td, J = 7.4, 1.1 Hz, 1H), 6.82 (t, J = 5.6 Hz, 1H), 6.61-6.55 (m, 2H), 3.76 (s, 3H), 3.44 (q, J = 6.8 Hz, 2H), 2.84 (t, J = 8.0, 2H), 1.39 (s, 6H).

### Example 178: 2-(2-((2-methoxyphenethyl)amino)pyridin-4-yl)isoindolin-1-one (E178)

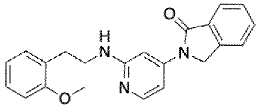

Prepared similarly as described for compound **E174** starting from **D41** (100 mg, 0.282 mmol, 1.0 eq) and 2,3-dihydroisoindol-1-one (190 mg, 1.4117 mmol, 5.0 eq). After work-up, the crude was purified by RP Preparative HPLC and freeze-dried to yield **E178** as white solid (62 mg, 0.172mmol, yield= 61%).

Method 4: R*t*= 2.25 min, MS (ESI) m/z = 360.2 [M+H]⁺.

¹H NMR (400 MHz, DMSO-*d*₆) δ ppm 7.97 (d, J = 5.8 Hz, 1H), 7.79 (dt, J = 7.6, 1.0 Hz, 1H), 7.74-7.65 (m, 2H), 7.55 (td, J = 7.2, 6.7, 1.6 Hz, 1H), 7.24-7.15 (m, 2H), 7.07 (d, J = 1.9 Hz, 1H), 7.04 (dd, J = 5.8, 2.0 Hz, 1H), 6.97 (d, J = 8.0 Hz, 1H), 6.88 (td, J = 7.4, 1.1 Hz, 1H), 6.60 (t, J = 5.6 Hz, 1H), 4.95 (s, 2H), 3.81 (s, 3H), 3.41 (dt, J = 7.9, 6.0 Hz, 2H), 2.84 (dd, J = 8.5, 6.4 Hz, 2H).

### Example 179: 3-(2-((2-methoxyphenethyl)amino)pyridin-4-yl) benzo[d]oxazol-2(3H)-one (E179)

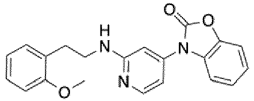

Prepared similarly as described for compound **E174** starting from **D41** (130 mg, 0.367 mmol, 1 eq) and 2-Benzoxazolinone (60 mg, 0.44 mmol, 1.2 eq). After work-up, the crude was purified by RP Preparative HPLC and freeze-dried to yield **E179** (30 mg, 0.083mmol, yield= 23%).

Method 4: R*t*= 2.38 min, MS (ESI) m/z = 362.2 [M+H]⁺.

¹H NMR (400 MHz, DMSO-*d*₆) δ ppm 8.16 (dd, J = 5.5, 0.7 Hz, 1H), 7.50-7.42 (m, 1H), 7.34-7.15 (m, 5H), 7.01-6.93 (m, 2H), 6.88 (td, J = 7.4, 1.1 Hz, 1H), 6.80-6.72 (m, 2H), 3.77 (s, 3H), 3.46 (q, J = 6.9 Hz, 2H), 2.85 (t, J = 7.0 Hz, 2H).

### Example 180: 1-(2-((2-methoxybenzyl)amino)pyridin-4-yl)-1,3-dihydro-2H-benzo[d]imidazol-2-one (E180)

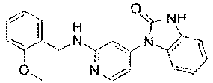

Prepared similarly as described for compound **E174** starting from **D42** (680 mg, 0.153 mmol, 1.0 eq) and 2-Hydroxybenzimidazole (307 mg, 2.292 mmol, 15.0 eq). After work-up, the crude was purified by RP Preparative HPLC (eluent: CH₃CN (+0.05% NH₃) in H₂O (+0.05% NH₃), basic conditions) and freeze-dried to yield **E180** (20 mg, 0.058 mmol, yield= 38%) as beige powder.

Method 4: R*t*= 1.99 min, MS (ESI) m/z = 347.2 [M+H]⁺.

¹H NMR (400 MHz, DMSO-*d*₆) δ ppm 11.23 (s, 1H), 8.08 (d, J = 6.0 Hz, 1H), 7.31-7.21 (m, 2H), 7.19-7.06 (m, 4H), 7.04-6.98 (m, 2H), 6.91 (td, J = 7.4, 1.1 Hz, 1H), 6.74 (d, J = 4.8 Hz, 2H), 4.48 (d, J = 5.9 Hz, 2H), 3.82 (s, 3H).

### Example 181: 3-(2-((2-methoxybenzyl)amino)pyridin-4-yl)pyrrolidin-2-one (E181)

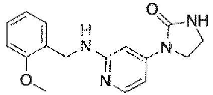

Prepared similarly as described for compound **E174** starting from **D42** (68 mg, 0.153 mmol, 1.0 eq) and 2-lmidazolidone (197 mg, 2.292 mmol, 15.0 eq). After work-up, the crude was purified by RP Preparative HPLC (eluent: CH₃CN (+0.05% NH₃) in H₂O (+0.05% NH₃), basic conditions) and freeze-dried to yield **E181** (10 mg, 0.033 mmol, yield= 22%) as white powder.

Method 4: R*t*= 1.62 min, MS (ESI) m/z = 299.2 [M+H]⁺.

¹H NMR (400 MHz, DMSO-*d*₆) δ ppm 7.76 (d, J = 5.9 Hz, 1H), 7.20 (ddd, J = 7.2, 4.3, 2.6 Hz, 2H), 7.13 (s, 1H), 6.96 (dd, J = 8.7, 1.1 Hz, 1H), 6.91-6.79 (m, 2H), 6.74-6.64 (m, 2H), 4.41 (d, J = 6.1 Hz, 2H), 3.82 (s, 3H), 3.76 (dd, J = 9.2, 6.8 Hz, 2H), 3.47-3.37 (m, 2H).

### Example 182: 1-(2-((2-(benzo[d][1,3]dioxol-4-yl)ethyl)amino)pyridin-4-yl)-1,3-dihydro-2H-benzo[d]imidazol-2-one (E182)

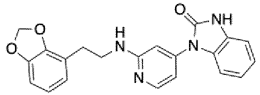

Prepared similarly as described for compound **E174** starting from **D96** (160 mg, 0.4346 mmol, 1 eq) and 2-Hydroxybenzimidazole (874 mg, 6.519 mmol, 15 eq). After work-up, the crude was purified by RP Preparative HPLC (eluent: CH₃CN (+0.1% HCOOH) in H₂O (+0.1% HCOOH)) and freeze-dried to yield **E182** (52 mg, 0.139mmol, yield=32%) as white solid.

Method 4: R*t*= 2.00 min, MS (ESI) m/z = 375.3 [M+H]⁺.

¹H NMR (400 MHz, DMSO-*d*₆) δ ppm 11.23 (s, 1H), 8.12 (d, J = 5.5 Hz, 1H), 7.19 (dt, J = 7.6, 1.1 Hz, 1H), 7.15-7.00 (m, 3H), 6.88 (t, J = 5.7 Hz, 1H), 6.82-6.73 (m, 4H), 6.76-6.68 (m, 2H), 5.98 (s, 2H), 3.53 (dt, J = 7.9, 6.0 Hz, 2H), 2.83 (t, J = 7.7 Hz, 2H).

### Example 183: 1-(2-((2-(cyclopentyloxy)phenethyl)amino)pyridin-4-yl) imidazolidin-2-one (E183)

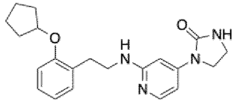

Prepared similarly as described for compound **E174** starting from **D97** (150 mg, 0.349 mmol, 1 eq) and 2-lmidazolidone (451 mg, 5.235 mmol, 15 eq). After work-up, the crude was purified by RP Preparative HPLC (eluent: CH₃CN (+0.1% HCOOH) in H₂O (+0.1% HCOOH)) and freeze-dried. The resulting compound was dissolved in DCM and passed through the cartridge with NH₂-bonded silica. DCM was evaporated and the residual was freeze-dried to yield **E183** (24 mg, 0.065mmol, yield= 19%).

Method 4: R*t*= 2.23 min, MS (ESI) m/z = 367.1 [M+H]⁺.

¹H NMR (400 MHz, DMSO-*d*₆) δ ppm 7.80 (d, J = 5.9 Hz, 1H), 7.20-7.07 (m, 3H), 6.97-6.90 (m, 1H), 6.83 (td, J = 7.4, 1.1 Hz, 1H), 6.78 (dd, J = 5.9, 2.0 Hz, 1H), 6.57 (d, J = 2.0 Hz, 1H), 6.32 (t, J = 5.6 Hz, 1H), 4.88-4.82 (m, 1H), 3.77 (dd, J = 9.3, 6.7 Hz, 2H), 3.43-3.36 (m, 4H), 2.76 (t, J = 7.4 Hz, 2H), 1.94-1.82 (m, 2H), 1.80-1.68 (m, 4H), 1.64-1.55 (m, 2H).

### Example 184: 1-(2-((2-(benzo[d][1,3]dioxol-4-yl)ethyl)amino)pyridin-4-yl) imidazolidin-2-one (E184)

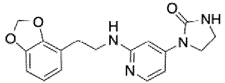

Prepared similarly as described for compound **E174** starting from **D96** (160 mg, 0.4346mmol, 1 eq) and 2-lmidazolidone (561 mg, 6.519 mmol, 15 eq). After work-up, the crude was purified by RP Preparative HPLC (eluent: CH₃CN (+0.1% HCOOH) in H₂O (+0.1% HCOOH)) and freeze-dried to yield **E184** (20 mg, 0.061mmol, yield = 14%).

Method 4: R*t*= 1.69 min, MS (ESI) m/z = 327.0 [M+H]⁺.

¹H NMR (400 MHz, DMSO-*d*₆) δ ppm 7.81 (d, J = 5.9 Hz, 1H), 7.13 (s, 1H), 6.80 (dd, J = 5.9, 2.0 Hz, 1H), 6.79-6.70 (m, 3H), 6.58 (d, J = 2.0 Hz, 1H), 6.41 (t, J = 5.8 Hz, 1H), 5.99 (s, 2H), 3.82-3.73 (m, 2H), 3.47-3.37 (m, 4H), 2.77 (t, J = 7.3 Hz, 2H).

### Example 185: 1-(2-((2-(cyclopentyloxy)phenethyl)amino)pyridin-4-yl)-1,3-dihydro-2H-benzo[d]imidazol-2-one (E185)

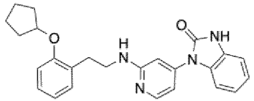

Prepared similarly as described for compound **E174** starting from **D97** (150 mg, 0.367 mmol, 1 eq) and 2-Hydroxybenzimidazole (702 mg, 5.235 mmol, 15 eq). After work-up, the crude was purified by RP Preparative HPLC (eluent: CH₃CN (+0.1% HCOOH) in H₂O (+0.1% HCOOH)) and freeze-dried to yield **E185** (83 mg, 0.2mmol, yield= 55%).

Method 4: R*t*= 2.48 min, MS (ESI) m/z = 415.3 [M+H]⁺.

¹H NMR (300 MHz, DMSO-*d*₆) δ ppm 11.22 (br. s, 1H), 8.11 (d, J = 5.4 Hz, 1H), 7.22-6.97 (m, 6H), 6.97-6.89 (m, 1H), 6.89-6.77 (m, 2H), 6.75-6.65 (m, 2H), 4.84 (tt, J = 5.5, 2.3 Hz, 1H), 3.50-3.38 (m, 2H), 2.81 (dd, J = 8.6, 6.3 Hz, 2H), 1.93-1.80 (m, 2H), 1.77-1.64 (m, 4H), 1.63-1.51 (m, 2H).

### Example 186: 1-(2-(((1r,3r)-3-(2-methoxyphenyl)cyclobutyl)amino)pyridin-4-yl)-1,3-dihydro-2H-benzo[d]imidazol-2-one (E186)

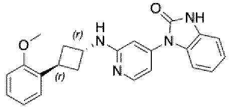

Prepared similarly as described for compound **E174** starting from **D98** (55 mg, 0.056 mmol, 1.0 eq) and 2-Hydroxybenzimidazole, 98% (112 mg, 0.839 mmol, 15.0 eq). After work-up, the crude was purified by RP Preparative HPLC (eluent: CH₃CN (+0.1% HCOOH) in H₂O (+0.1% HCOOH)) and freeze-dried to yield **E186** (21 mg, 0.054 mmol, yield= 97%) as a white powder.

Method 4: Rt= 2.27 min, MS (ESI) m/z = 387.2 [M+H]⁺.

¹H NMR (400 MHz, DMSO-*d*₆) δ ppm 8.09 (d, J = 5.4 Hz, 1H), 7.36 (dd, J = 7.6, 1.7 Hz, 1H), 7.28-7.22 (m, 1H), 7.22-7.18 (m, 2H), 7.12-7.01 (m, 3H), 6.96 (t, J = 7.5 Hz, 2H), 6.73 (dd, J = 5.6, 1.8 Hz, 1H), 6.68 (d, J = 1.9 Hz, 1H), 4.34 (t, J = 6.7 Hz, 1H), 3.80 (d, J = 8.0 Hz, 1H), 3.77 (s, 3H), 2.53 (d, J = 2.6 Hz, 1H), 2.43 (dt, J = 11.3, 7.1 Hz, 2H), 2.33 (ddd, J = 11.8, 9.1, 5.5 Hz, 2H).

### Example 187: 2-(2-((2-methoxyphenethyl)amino)pyridin-4-yl)-2-azabicyclo[2.2.1]heptan-3-one (E187)

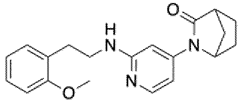

Prepared similarly as described for compound **E174** starting from **D41** (100 mg, 0.27 mmol, 1.0 eq.) and 2-Azabicyclo[2.2.1]heptan-3-one (90 mg, 0.80 mmol, 3.0 eq.). After work-up, the crude was purified by RP Preparative HPLC (eluent: CH₃CN (+0.1% HCOOH) in H₂O (+0.1% HCOOH)) and freeze-dried to yield **E187** as white sticky solid (51 mg, 0,15 mmol, yield= 56%).

Method 4: R*t*= 2.08 min, MS (ESI) m/z = 338.3 [M+H]⁺.

¹H NMR (400 MHz, DMSO-*d*₆) δ ppm 7.86 (d, J = 5.8 Hz, 1H), 7.22-7.13 (m, 2H), 6.95 (dd, J = 8.3, 1.1 Hz, 1H), 6.87 (td, J = 7.4, 1.1 Hz, 1H), 6.71 (d, J = 1.9 Hz, 1H), 6.65 (dd, J = 5.8, 2.0 Hz, 1H), 6.47 (t, J = 5.6 Hz, 1H), 4.55 (s, 1H), 3.79 (s, 3H), 3.36 (td, J = 8.8, 8.1, 4.2 Hz, 2H), 2.83-2.74 (m, 3H), 1.99-1.84 (m, 3H), 1.63 (t, J = 9.2 Hz, 1H), 1.52 (dt, J = 9.7, 1.4 Hz, 2H).

### Example 188: 1-(2-((2-methoxyphenethyl)amino)pyridin-4-yl)-5,5-dimethylpyrrolidin-2-one (E188)

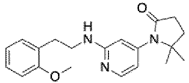

Prepared similarly as described for compound **E174** starting from **D41** (150 mg, 0.381 mmol, 1.0 eq) and 5,5-Dimethylpyrrolidin-2-one (431 mg, 3.812 mmol, 10 eq). After work-up, the crude was purified by RP Preparative HPLC (C18 column-prep, eluent: CH₃CN (+0.05% NH₃) in H₂O (+0.05% NH₃), basic conditions) and freeze-dried to yield **E188** (27 mg, 0.078 mmol, yield= 20%) as straw colour solid.

Method 4: R*t*= 1.99 min, MS (ESI) m/z = 340.11 [M+H]⁺.

¹H NMR (400 MHz, DMSO-*d*₆) δ ppm 7.98 (d, J = 5.5 Hz, 1H), 7.31-7.06 (m, 2H), 6.97 (dd, J = 8.3, 1.1 Hz, 1H), 6.88 (td, J = 7.3, 1.1 Hz, 1H), 6.65 (t, J = 5.7 Hz, 1H), 6.41-6.18 (m, 2H), 3.79 (s, 3H), 3.40 (dt, J = 7.9, 6.0 Hz, 2H), 2.81 (dd, J = 8.6, 6.4 Hz, 2H), 2.45 (t, J = 7.9 Hz, 2H), 1.96 (t, J = 7.9 Hz, 2H), 1.25 (s, 6H).

### Example 189: 1-(2-((2-methoxyphenethyl)amino)pyridin-4-yl)-4,4-dimethylpyrrolidin-2-one (E189)

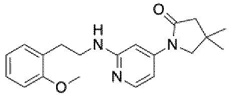

Prepared similarly as described for compound **E174** starting from **D41**(175 mg, 0.445 mmol, 1.0 eq) and 4,4-Dimethyl-2-pyrrolidinone (503 mg, 4.447 mmol, 10.0 eq). After work-up, the crude was purified by RP Preparative HPLC and freeze-dried to yield E189 (70 mg, 0.206 mmol, yield= 46%) as a white powder.

Method 4: R*t*= 2.22 min, MS (ESI) m/z = 340.39 [M+H]⁺.

¹H NMR (400 MHz, DMSO-*d*₆) δ ppm 7.88 (d, J = 5.7 Hz, 1H), 7.22-7.17 (m, 1H), 7.16 (dd, J = 7.5, 1.6 Hz, 1H), 6.98-6.94 (m, 1H), 6.87 (td, J = 7.4, 1.2 Hz, 1H), 6.78 (dd, J = 5.8, 1.9 Hz, 1H), 6.76 (d, J = 1.9 Hz, 1H), 6.49 (t, J = 5.6 Hz, 1H), 3.79 (d, J = 1.4 Hz, 3H), 3.49 (s, 2H), 3.40-3.34 (m, 2H), 2.80 (t, J = 7.5 Hz, 2H), 2.37 (d, J = 1.5 Hz, 2H), 1.14 (s, 6H).

### Example 190: tert-butyl 5-(2-((2-methoxyphenethyl)amino)pyridin-4-yl)-1,2,5-thiadiazolidine-2-carboxylate 1,1-dioxide (E190)

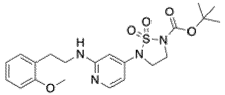

Prepared similarly as described for compound **E174** starting from **D41** (230 mg, 0.6169 mmol, 1 eq) and tert-Butyl 1,2,5-thiadiazolidine-2-carboxylate 1,1-dioxide (151 mg, 0.679 mmol, 1.1 eq). After work-up, the crude was purified by RP Preparative HPLC and freeze-dried to yield **E190** (25.4 mg, 0.06mmol, yield= 9%) as a white solid.

Method 4: R*t*= 2.48 min, MS (ESI) m/z =449.25[M+H]⁺.

¹H NMR (400 MHz, DMSO-*d*₆) δ ppm 7.94 (d, J = 5.8 Hz, 1H), 7.25-7.13 (m, 2H), 6.97 (d, J = 8.2 Hz, 1H), 6.88 (td, J = 7.4, 1.1 Hz, 1H), 6.75 (t, J = 5.6 Hz, 1H), 6.42 (dd, J = 5.8, 2.1 Hz, 1H), 6.28 (d, J = 2.1 Hz, 1H), 3.94 (t, J = 6.4 Hz, 2H), 3.84 (t, J = 6.4 Hz, 2H), 3.79 (s, 3H), 3.40 (q, J = 6.6 Hz, 2H), 2.81 (t, J = 7.4 Hz, 2H), 1.50 (s, 9H).

### Example 191: 2-(2-((2-methoxyphenethyl)amino)pyridin-4-yl)-1,2,5-thiadiazolidine 1,1-dioxide (E191)

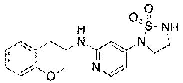

To a solution of **E190** (16 mg, 0.036 mmol, 1.0 eq) and anh DCM (~1 mL) in vial, trifluoroacetic acid (14 µL, 0.178 mmol, 5.0 eq) was added. Mixture was stirred for 0.5 h. Then, ss NaHCO₃ was added and mixed to adjust pH~9. Mixture was extracted with DCM (3x5mL). The organic phase was separated through separator pad and concentrate to yield **E191** (10 mg, 0.029mmol, yield= 81%)

Method 4: R*t*= 1.91 min, MS (ESI) m/z =349.03[M+H]⁺.

¹H NMR (400 MHz, DMSO-*d*₆) δ 7.90-7.76 (m, 2H), 7.24-7.12 (m, 2H), 6.96 (d, J = 7.8 Hz, 1H), 6.87 (td, J = 7.4, 0.9 Hz, 1H), 6.56 (t, J = 5.5 Hz, 1H), 6.29 (dd, J = 5.8, 2.1 Hz, 1H), 6.10 (d, J = 1.9 Hz, 1H), 3.83-3.76 (m, 5H), 3.55-3.46 (m, 2H), 3.41-3.36 (m, 2H), 2.85-2.75 (m, 2H).

### Example 192: N-(2-methoxyphenethyl)-4-(5-phenylthiazol-2-yl)pyridin-2-amine (E192)

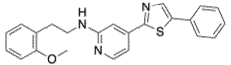

Prepared similarly as described for compound **E49** starting from **D102** (50 mg, 0.088 mmol, 1.0 eq) and 2-(2-methoxyphenyl)ethan-1-amine (16 mg, 0.105 mmol, 1.2 eq). After work-up, the crude was purified by RP Preparative HPLC (C18 column-prep, eluent: CH₃CN (+0.05% NH₃) in H₂O (+0.05% NH₃), basic conditions) and freeze-dried to yield E192 (17 mg, 0.044 mmol, yield = 50%).

Method 4: Rt= 2.74 min, MS (ESI) m/z =388.24 [M+H]⁺.

¹H NMR (400 MHz, DMSO-*d*₆) δ 8.40 (s, 1H), 8.11 (dd, J = 5.4, 0.7 Hz, 1H), 7.86-7.62 (m, 2H), 7.54-7.45 (m, 2H), 7.46-7.36 (m, 1H), 7.20 (ddd, J = 15.9, 7.9, 1.6 Hz, 2H), 7.06 (s, 1H), 7.03-6.94 (m, 3H), 6.89 (td, J = 7.3, 1.1 Hz, 1H), 3.83 (s, 3H), 3.54-3.41 (m, 2H), 2.85 (dd, J = 8.5, 6.4 Hz, 2H).

### Example 193: N-(2-methoxyphenethyl)-4-(5-phenyloxazol-2-yl)pyridin-2-amine (E193)

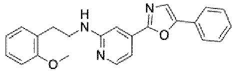

Prepared similarly as described for compound **E49** starting from **D103** (40 mg, 0.16 mmol, 1.0 eq) and 2-(2-methoxyphenyl)ethan-1-amine (29 mg, 0.192 mmol, 1.2 eq). After work-up, the crude was purified by RP Preparative HPLC (C18 column-prep, eluent: CH₃CN (+0.05% NH₃) in H₂O (+0.05% NH₃), basic conditions) and freeze-dried to yield **E193** (8 mg, 0.021 mmol, yield = 13%) as a tan colour powder.

Method 4: Rt= 2.64 min, MS (ESI) m/z =371.9 [M+H]⁺.

¹H NMR (400 MHz, DMSO-*d*₆) δ 8.15 (d, J = 5.3 Hz, 1H), 7.90 (s, 1H), 7.87-7.81 (m, 2H), 7.53 (dd, J = 8.4, 7.0 Hz, 2H), 7.48-7.38 (m, 1H), 7.20 (ddd, J = 15.2, 7.6, 1.7 Hz, 2H), 7.13 (t, J = 1.1 Hz, 1H), 7.08 (dd, J = 5.2, 1.5 Hz, 1H), 7.01-6.83 (m, 3H), 3.82 (s, 3H), 3.48 (q, J = 6.8 Hz, 2H), 2.85 (t, J = 7.4 Hz, 2H).

### Example 194: 1-(2-((2-methoxyphenethyl)amino)pyridin-4-yl)-4-phenylimidazolidin-2-one (E194)

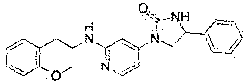

Prepared similarly as described for compound **E174** starting from **D41** (230 mg, 0.6169 mmol, 1 eq) and 4-phenylimidazolidin-2-one (236 mg, 1.457 mmol, 2.0 eq). After work-up, the crude was purified by RP Preparative HPLC (C18 column-prep; eluent: CH₃CN (+0.05% NH₃) in H₂O (+0.05% NH₃), basic conditions) and freeze-dried to yield **E194** (9 mg, 0.023 mmol, yield= 3%) as a white powder.

Method 4: R*t*= 2.25 min, MS (ESI) m/z = 389.2 [M+H]⁺.

¹H NMR (400 MHz, DMSO-*d*₆) δ 7.88-7.72 (m, 2H), 7.41 (d, J = 3.4 Hz, 4H), 7.37-7.29 (m, 1H), 7.18 (ddd, J = 14.4, 7.3, 1.7 Hz, 2H), 6.95 (dd, J = 8.2, 1.1 Hz, 1H), 6.86 (td, J = 7.4, 1.1 Hz, 1H), 6.79 (dd, J = 5.9, 2.0 Hz, 1H), 6.61 (d, J = 2.0 Hz, 1H), 6.32 (t, J = 5.7 Hz, 1H), 4.95-4.80 (m, 1H), 4.24 (t, J = 9.4 Hz, 1H), 3.78 (s, 3H), 3.50 (dd, J = 9.5, 6.7 Hz, 1H), 3.35 (d, J = 9.0 Hz, 2H), 2.79 (dd, J = 8.5, 6.4 Hz, 2H).

### Example 195: 1-(2-((2-methoxy-6-(2-methoxyethoxy)phenethyl)-amino)pyridin-4-yl)-1,3-dihydro-2H-benzo[d]imidazol-2-one (E195)

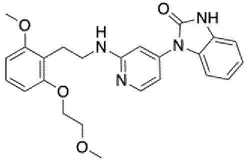

Prepared similarly as described for compound **E174** starting from **D41** (230 mg, 0.6169 mmol, 1 eq) and 2-Hydroxybenzimidazole (2.09 g, 15.583 mmol, 15 eq). After work-up, the crude was purified by NP Flash Chromatography (silica gel column, 0-20% MeOH in DCM) to provide 333 mg of impure product (75%) as an off-white solid. 54 mg of the latter was purified by RP Preparative HPLC and freeze-dried to provide **E195** (36 mg, 0.086 mmol) as a white powder.

Method 4: R*t*= 2.14 min, MS (ESI) m/z = 421.13 [M+H]⁺.

¹H NMR (600 MHz, DMSO-*d*₆) δ 11.23 (s, 1H), 8.12-8.08 (m, 1H), 7.18 (d, J = 7.8 Hz, 1H), 7.13 (t, J = 8.4 Hz, 1H), 7.10-7.06 (m, 2H), 7.04 (tt, J = 8.2, 5.6 Hz, 1H), 6.83 (t, J = 5.7 Hz, 1H), 6.73-6.68 (m, 3H), 6.66 (d, J = 8.3 Hz, 1H), 5.14 (s, 2H), 3.71 (s, 3H), 3.34 (s, 3H), 3.33-3.28 (m, 2H), 2.92-2.86 (m, 2H).

### Example 196: 1-(2-((2-hydroxy-6-methoxyphenethyl)amino)pyridin-4-yl)-1,3-dihydro-2H-benzo[d]imidazol-2-one (E196)

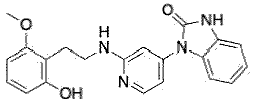

Prepared similarly as described for compound **E47** starting from **E195** (279 mg, 0.6503 mmol, 1 eq). After work-up , the crude was purified by RP Preparative HPLC. The fractions containing the product of interest were concentrated to dryness. Resulting solid was sonicated in mixture H₂O/MeOH (25:1), filtered and dried on filter under vacuum to provide **E196** (140 mg, 0,373mmol, yield = 57%).

Method 4: R*t*= 1.94 min, MS (ESI) m/z = 377.18 [M+H]⁺.

¹H NMR (400 MHz, DMSO-*d*₆) δ 11.24 (s, 1H), 9.51 (s, 1H), 8.13-8.07 (m, 1H), 7.20 (dt, J = 7.7, 1.0 Hz, 1H), 7.14-7.02 (m, 3H), 6.98 (t, J = 8.2 Hz, 1H), 6.82 (t, J = 5.5 Hz, 1H), 6.72 (dt, J = 3.0, 1.6 Hz, 2H), 6.45 (ddd, J = 14.9, 8.3, 1.0 Hz, 2H), 3.68 (s, 3H), 3.28 (dd, J = 9.8, 5.4 Hz, 2H), 2.88-2.79 (m, 2H).

### Example 197: 3-(2-((2-methoxyphenethyl)amino)pyridin-4-yl)-1,3-dihydro-2H-imidazo[4,5-c]pyridin-2-one (E197)

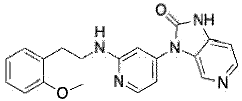

### Example 198: 1-(2-((2-methoxyphenethyl)amino)pyridin-4-yl)-1,3-dihydro-2H-imidazo[4,5-c]pyridin-2-one (E198)

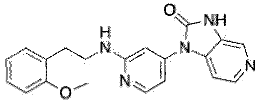

Prepared similarly as described for compound **E174** starting from **D41** (230 mg, 0.6169 mmol, 1 eq) and 3H-Imidazo[4,5-c]pyridin-2-ol (255 mg, 1.885 mmol, 5.0 eq). After work-up, the crude was purified by RP Preparative HPLC (C18 column-prep; eluent: CH₃CN (+0.05% NH₃) in H₂O (+0.05% NH₃), basic conditions) to isolate two products, subsequently freeze-dried:
**E197** (12 mg, 0.033mmol, yield = 9%).

Method 4: R*t*= 1.297 min, MS (ESI) m/z = 362.06 [M+H]⁺.

¹H NMR (400 MHz, DMSO-*d*₆) δ 11.66 (s, 1H), 8.42 (s, 1H), 8.26 (d, J = 5.2 Hz, 1H), 8.12 (d, J = 5.6 Hz, 1H), 7.24-7.16 (m, 2H), 7.14 (dd, J = 5.2, 0.9 Hz, 1H), 7.00-6.93 (m, 1H), 6.92-6.83 (m, 2H), 6.79 (dd, J = 5.5, 1.9 Hz, 1H), 6.75 (d, J = 1.8 Hz, 1H), 3.77 (s, 3H), 3.46 (q, J = 6.8 Hz, 2H), 2.85 (dd, J = 8.5, 6.4 Hz, 2H).

**E198** (14 mg, 0.038mmol, yield = 10%).

Method 4: R*t*= 1.307 min, MS (ESI) m/z = 362.34 [M+H]⁺.

¹H NMR (400 MHz, DMSO-*d*₆) δ 11.52 (s, 1H), 8.31 (s, 1H), 8.23 (d, J = 5.4 Hz, 1H), 8.14 (d, J = 5.5 Hz, 1H), 7.20 (ddt, J = 9.2, 7.5, 1.7 Hz, 3H), 6.96 (dd, J = 8.1, 1.1 Hz, 1H), 6.92-6.84 (m, 2H), 6.73-6.66 (m, 2H), 3.76 (s, 3H), 3.45 (dt, J = 8.2, 6.1 Hz, 2H), 2.84 (dd, J = 8.5, 6.4 Hz, 2H).

### Example 199: 1-(2-((2-methoxy-6-(methoxymethoxy)phenethyl)amino)-pyridin-4-yl)imidazolidin-2-one (E199)

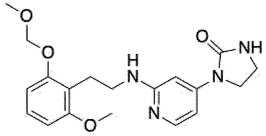

Prepared similarly as described for compound **E174** starting from **D41** (230 mg, 0.617 mmol, 1 eq) and 2-lmidazolidone (1.3 g, 15.58 mmol, 15 eq). After work-up, the crude was purified by RP Flash chromatography (5-95% CH₃CN in H₂O) and freeze-dried to provide 144 mg (yield = 35%) of the desired impure product as a white solid. The latter was repurified by RP Preparative HPLC and freeze-dried to provide **E199** (28 mg, 0.075 mmol) as a white powder.

Method 4: R*t*= 2.49 min, MS (ESI) m/z = 372.82 [M+H]⁺.

¹H NMR (400 MHz, DMSO-*d*₆) δ 7.78 (d, J = 5.8 Hz, 1H), 7.18-7.09 (m, 2H), 6.82 (dd, J = 5.9, 1.9 Hz, 1H), 6.72 (dd, J = 8.5, 0.9 Hz, 1H), 6.67 (dd, J = 8.4, 0.9 Hz, 1H), 6.53 (d, J = 1.9 Hz, 1H), 6.24 (t, J = 5.4 Hz, 1H), 5.19 (s, 2H), 3.82-3.74 (m, 5H), 3.43-3.38 (m, 5H), 3.28-3.19 (m, 2H), 2.91-2.82 (m, 2H).

### Example 200: 1-(2-((2-hydroxy-6-methoxyphenethyl)amino)pyridin-4-yl)imidazolidin-2-one (E200)

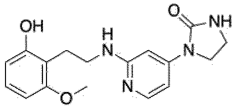

Prepared similarly as described for compound **E47** starting from **E199** (100 mg, 0.2551 mmol, 1 eq). After work-up , the crude was purified by RP Preparative HPLC. The appropriate fractions were concentrated and freeze-dried to provide **E200** (25 mg, 0.077mmol, yield = 30%).

Method 4: R*t*= 1.66 min, MS (ESI) m/z = 329.04 [M+H]⁺.

¹H NMR (400 MHz, DMSO-*d*₆) δ 9.85 (s, 1H), 7.78 (d, J = 5.9 Hz, 1H), 7.16 (s, 1H), 6.98 (t, J = 8.2 Hz, 1H), 6.91 (dd, J = 6.0, 2.0 Hz, 1H), 6.53 (d, J = 2.0 Hz, 1H), 6.50-6.39 (m, 3H), 3.80 (dd, J = 9.3, 6.7 Hz, 2H), 3.74 (s, 3H), 3.41 (dd, J = 9.3, 6.7 Hz, 2H), 3.23-3.13 (m, 2H), 2.84-2.76 (m, 2H).

### Example 201: 1-(2-((2-methoxyphenethyl)amino)pyridin-4-yl)-1,3-dihydro-2H-imidazo[4,5-b]pyrazin-2-one (E201)

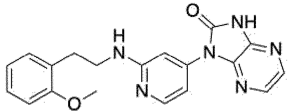

Prepared similarly as described for compound **E174** starting from **D41** (0.05 g, 0.126 mmol, 1.0 eq) and 1,3-Dihydro-2H-imidazo[4,5-b]pyrazin-2-one (0.821 g, 6.031 mmol, 10.0 eq). The reaction mixture was stirred for 24 hours at 110 °C. After work-up, the crude was purified by RP Preparative HPLC (C18 column-prep; eluent: CH₃CN (+0.05% NH₃) in H₂O (+0.05% NH₃), basic conditions) to yield **E201** (0.02 g, 0.054 mmol, 9%), tan colour powder.

Method 4: R*t*= 1.89 min, MS (ESI) m/z = 363.06 [M+H]⁺.

¹H NMR (400 MHz, DMSO-*d*₆) δ 12.33 (s, 1H), 8.23-8.02 (m, 1H), 8.00 (d, J = 3.3 Hz, 1H), 7.96 (d, J = 3.3 Hz, 1H), 7.24-7.13 (m, 2H), 7.01 (d, J = 4.8 Hz, 2H), 6.97 (d, J = 8.0 Hz, 1H), 6.92-6.81 (m, 2H), 3.78 (s, 3H), 3.44 (dt, J = 7.9, 5.9 Hz, 2H), 2.85 (dd, J = 8.5, 6.4 Hz, 2H).

### Example 237: N-(2-((2-methoxyphenethyl)amino)pyridin-4-yl)pyrrolidine-1-carboxamide (E237)

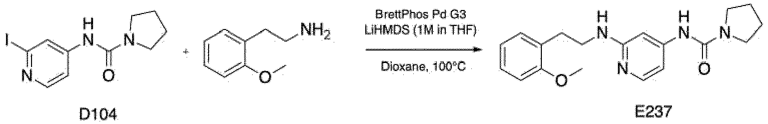

2-(2-methoxyphenyl)ethan-1-amine (172 mg, 1.137 mmol, 1.3 eq), BrettPhos Pd G3 (80 mg, 0.087 mmol, 0.1 eq) and **D104** (292 mg, 0.899 mmol, 1.0 eq) were suspended in anh Dioxane (0.1M, 9 mL). Argon was bubbled through the suspension for 5-10 minutes and then LiHMDS (1M in THF, 2.3 mL, 2.274 mmol, 2.6 eq) was added. The reaction vial was capped and heated to 100°C with rapid stirring for 1 hour. The reaction mixture was diluted with -150 mL of EtOAc. The organic layer was washed with a mixture of distilled water/brine (1:1), brine, dried over anh Na₂SO₄, filtered and evaporated to give a crude product. The crude was purified by RP Preparative HPLC and freeze-dried to provide **E237** ( 87 mg, 0.25mmol, yield= 29%) as a white solid.

Method 4: R*t*= 2.03 min, MS (ESI) m/z = 341.13 [M+H]⁺.

¹H NMR (400 MHz, DMSO-*d*₆) δ 8.14 (s, 1H), 7.73 (d, J = 5.7 Hz, 1H), 7.26-7.12 (m, 2H), 6.96 (dd, J = 8.2, 1.1 Hz, 1H), 6.87 (td, J = 7.4, 1.2 Hz, 1H), 6.78 (d, J = 1.8 Hz, 1H), 6.66 (dd, J = 5.7, 1.8 Hz, 1H), 6.22 (t, J = 5.6 Hz, 1H), 3.80 (s, 3H), 3.41-3.36 (m, 4H), 3.32-3.26 (m, 2H), 2.80 (t, J = 7.7 Hz, 2H), 1.92-1.74 (m, 4H).

### Example 238: 1-(2-(((1r,3r)-3-(2-methoxyphenyl)cyclobutyl)amino)pyridin-4-yl)imidazolidin-2-one (E238)

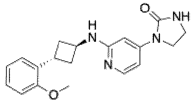

Prepared similarly as described for compound **E174** starting from **D98** (0.035 g, 0.035 mmol, 1.0 eq) and 2-lmidazolidone (0.046 g, 0.531 mmol, 15.0 eq). The reaction mixture was stirred for 24 hours at 110 °C. After work-up, the crude was purified by RP Preparative HPLC (C18 column-prep; eluent: CH₃CN (+0.05% NH₃) in H₂O (+0.05% NH₃), basic conditions) to yield **E238** (0.004 g, 0.012 mmol, yield= 34%) as a white powder.

Method 4: R*t*= 1.99 min, MS (ESI) m/z = 339.41 [M+H]⁺.

¹H NMR (400 MHz, DMSO-*d*₆) δ 7.78 (d, J = 5.9 Hz, 1H), 7.39-7.29 (m, 1H), 7.20 (td, J = 7.7, 1.7 Hz, 1H), 7.12 (s, 1H), 6.95 (t, J = 7.7 Hz, 2H), 6.81-6.73 (m, 2H), 6.58 (d, J = 2.0 Hz, 1H), 4.25 (q, J = 6.7 Hz, 1H), 3.82-3.77 (m, 2H), 3.76 (s, 3H), 3.72 (d, J = 7.5 Hz, 1H), 3.44-3.37 (m, 2H), 2.37 (dt, J = 13.5, 7.1 Hz, 2H), 2.32-2.21 (m, 2H).

**Table 2: Compounds were prepared as referenced:**

| EXA MPL E | STRUCTURE | IUPAC NAME | CALCULATED DATA | EXPERIMENTAL DATA |
|---|---|---|---|---|
| E202 | 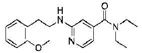 | *N,N*-diethyl-2-((2-methoxyphenethyl)amino)isonicotinamide | Chemical Formula: C₁₉H₂₅N₃O₂ | Method 10: R*t*= 0.97min, |
| | | | | MS (ESI) m/z = 328.2[M+H]⁺. |
| | | | Exact Mass: 327,19 | |
| E203 | 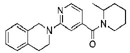 | (2-(3,4-dihydroisoquinolin-2(1*H*)-yl)pyridin-4-yl)(2-methylpiperidin-1-yl)methanone | Chemical Formula: C₂₁H₂₅N₃O | Method 10: R*t*= 1.04min, |
| | | | | MS (ESI) m/z = 336.2[M+H]⁺. |
| | | | Exact Mass: 335,20 | |
| E204 | 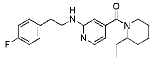 | (2-ethylpiperidin-1-yl)(2-((4-fluorophenethyl)amino)pyridin-4-yl)methanone | Chemical Formula: C₂₁H₂₆FN₃O | Method 10: R*t*= 0.95min, |
| | | | | MS (ESI) m/z = 356.2[M+H]⁺. |
| | | | Exact Mass: 355,21 | |
| E205 | 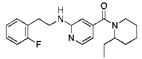 | (2-ethylpiperidin-1-yl)(2-((2-fluorophenethyl)amino)pyridin-4-yl)methanone | Chemical Formula: C₂₁H₂₆FN₃O | |
| | | | Exact Mass: 355,21 | |
| E206 | 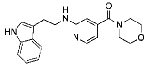 | (2-((2-(1*H*-indol-3-yl)ethyl)amino)pyridin-4-yl)(morpholino)methano | Chemical Formula: C₂₀H₂₂N₄O₂ | Method 10: R*t*= 0.83min, |
| | | | | MS (ESI) m/z = 351.1[M+H]⁺. |
| | | | Exact Mass: 350,17 | |
| E207 | 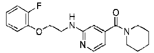 | (2-((2-(2-fluorophenoxy)ethyl)amino)pyridin-4-yl)(piperidin-1-yl)methanone | Chemical Formula: C₁₉H₂₂FN₃O₂ | Method 10: R*t*= 0.72min, |
| | | | | MS (ESI) m/z = 344.0[M+H]⁺. |
| | | | Exact Mass: 343,17 | |
| E208 | 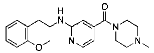 | (2-((2-methoxyphenethyl)amino)pyridin-4-yl)(4-methylpiperazin-1-yl)methanone | Chemical Formula: C₂₀H₂₆N₄O₂ | Method 10: R*t*= 0.38min, |
| | | | | MS (ESI) m/z = 355.2[M+H]⁺. |
| | | | Exact Mass: 354,21 | |
| E209 | 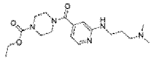 | ethyl 4-(2-((3-(dimethylamino)propyl)amino)isonicotinoy l)piperazine-1-carboxylate | Chemical Formula: C₁₈H₂₉N₅O₃ | Method 10: R*t*= 0.68min, |
| | | | | MS (ESI) m/z = 364.2[M+H]⁺. |
| | | | Exact Mass: 363,23 | |
| E210 | 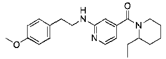 | (2-ethylpiperidin-1-yl)(2-((4-methoxyphenethyl)amino)pyridin-4-yl)methano | Chemical Formula: C₂₂H₂₉N₃O₂ | Method 10: R*t*= 0.92min, |
| | | | | MS (ESI) m/z = 368.2[M+H]⁺. |
| | | | Exact Mass: 367,23 | |
| E211 | 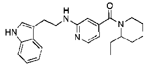 | (2-((2-(1*H*-indol-3-yl)ethyl)amino)pyridin-4-yl)(2-ethylpiperidin-1-yl)methanone | Chemical Formula: C₂₃H₂₈N₄O | Method 10: R*t*= 1.11 min, |
| | | | | MS (ESI) m/z = 377.2[M+H]⁺. |
| | | | Exact Mass: 376,23 | |
| E212 | 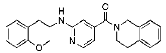 | (3,4-dihydroisoquinolin-2(1*H*)-yl)(2-((2-methoxyphenethyl)amino)pyridin-4-yl)methanone | Chemical Formula: C₂₄H₂₅N₃O₂ | Method 10: R*t*= 0.97min, |
| | | | | MS (ESI) m/z = 388.2[M+H]⁺. |
| | | | Exact Mass: 387,19 | |
| E213 | 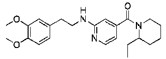 | (2-((3,4-dimethoxyphenethyl)amino)pyridin-4-yl)(2-ethylpiperidin-1-yl)methanone | Chemical Formula: C₂₃H₃₁N₃O₃ | Method 10: R*t*= 1.08min, |
| | | | | MS (ESI) m/z = 398.0[M+H]⁺. |
| | | | Exact Mass: 397,24 | |
| E214 | 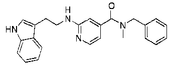 | 2-((2-(1*H*-indol-3-yl)ethyl)amino)-*N-*benzyl-*N*-methylisonicotinamide | Chemical Formula: C₂₄H₂₄N₄O | Method 10: R*t*= 1.04min, |
| | | | | MS (ESI) m/z = 385.2[M+H]⁺. |
| | | | Exact Mass: 384,20 | |
| E215 | 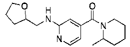 | (2-ethylpiperidin-1-yl)(2-(((tetrahydrofuran-2-yl)methyl)amino)pyridin-4-yl)methanone | Chemical Formula: C₁₈H₂₇N₃O₂ | Method 10: R*t*= 0.92min, |
| | | | | MS (ESI) m/z = 318.2[M+H]⁺. |
| | | | Exact Mass: 317,21 | |
| E216 | 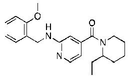 | (2-ethylpiperidin-1-yl)(2-((2-methoxybenzyl)amino)pyridin-4-yl)methanone | Chemical Formula: C₂₁H₂₇N₃O₂ | Method 10: R*t*= 1.12min, |
| | | | | MS (ESI) m/z = 354.2[M+H]⁺. |
| | | | Exact Mass: 353,21 | |
| E217 | 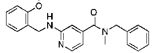 | *N*-benzyl-2-((2-methoxybenzyl)amino)-*N-*methylisonicotinamide | Chemical Formula: C₂₂H₂₃N₃O₂ | Method 10: R*t*= 1.1min, |
| | | | | MS (ESI) m/z = 362.0[M+H]⁺. |
| | | | Exact Mass: 361,18 | |
| E218 | 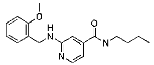 | *N*-butyl-2-((2-methoxybenzyl)amino)-*N-*methylisonicotinamide, trifluoroacetate | Chemical Formula: C₁₉H₂₅N₃O₂ | Method 10: R*t*= 0.94min, |
| | | | | MS (ESI) m/z = 328.2[M+H]⁺. |
| | | | Exact Mass: 327,19 | |
| E219 | 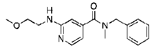 | *N*-benzyl-2-((2-methoxyethyl)amino)-N-methylisonicotinamide | Chemical Formula: C₁₇H₂₁N₃O₂ | Method 10: R*t*= 0.85min, |
| | | | | MS (ESI) m/z = 300.2[M+H]⁺. |
| | | | Exact Mass: 299,16 | |
| E220 | 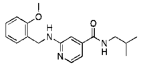 | *N*-isobutyl-2-((2-methoxybenzyl)amino)isonicotinamide | *N*-isobutyl-2-((2-methoxybenzyl)amino)iso nicotinamide | Method 10: R*t*= 0.88min, |
| | | | | MS (ESI) m/z = 314.0[M+H]⁺. |
| E221 | 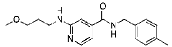 | 2-((3-methoxypropyl)amino)-N-(4-methylbenzyl)isonicotinamide | Chemical Formula: C₁₈H₂₃N₃O₂ | Method 10: R*t*= 0.80 min, |
| | | | | MS (ESI) m/z = 314.22[M+H]⁺. |
| | | | Exact Mass: 313,18 | |
| E222 | 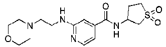 | *N*-(1,1-dioxidotetrahydrothiophen-3-yl)-2-((2-morpholinoethyl)amino)isonicotinamide | Chemical Formula: C₁₆H₂₄N₄O₄S | Method 10: R*t*= 1.66min, |
| | | | | MS (ESI) m/z = 369.2[M+H]⁺. |
| | | | Exact Mass: 368,15 | |
| E223 | 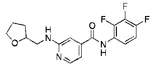 | 2-(((tetrahydrofuran-2-yl)methyl)amino)-*N*-(2,3,4-trifluorophenyl)isonicotinamide | Chemical Formula: C₁₇H₁₆F₃N₃O₂ | Method 10: R*t*= 0.77min, |
| | | | | MS (ESI) m/z = 352.2[M+H]⁺. |
| | | | Exact Mass: 351,12 | |
| E224 | 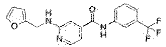 | 2-((furan-2-ylmethyl)amino)-*N*-(3-(trifluoromethyl)phenyl)isonicotinamide | Chemical Formula: C₁₈H₁₄F₃N₃O₂ | Method 10: R*t*= 1.23min, |
| | | | | MS (ESI) m/z = 362.0[M+H]⁺. |
| | | | Exact Mass: 361,10 | |
| E225 | 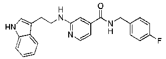 | 2-((2-(1*H*-indol-3-yl)ethyl)amino)-*N*-(4-fluorobenzyl)isonicotinamide | Chemical Formula: C₂₃H₂₁FN₄O | Method 10: R*t*= 1.10 min, |
| | | | | MS (ESI) m/z = 389.0[M+H]⁺. |
| | | | Exact Mass: 388,17 | |
| E226 | 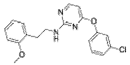 | 4-(3-chlorophenoxy)-N-(2-methoxyphenethyl)pyrimidin-2-amine | Chemical Formula: C₁₉H₁₈ClN₃O₂ | Method 2: R*t*= 1.94min, MS (ESI) m/z =356.2 [M+H]⁺. |
| | | | Exact Mass: 355.108755 | |
| E227 | 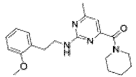 | (2-((2-methoxyphenethyl)amino)-6-methylpyrimidin-4-yl)(piperidin-1-yl)methanone | Chemical Formula: C₂₀H₂₆N₄O₂ | Method 10: R*t*= 1.35min, |
| | | | | MS (ESI) m/z = 355.2[M+H]⁺. |
| | | | Exact Mass:354.205576 | |
| E228 | 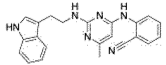 | 2-((2-((2-(1H-indol-3-yl)ethyl)amino)-6-methylpyrimidin-4-yl)amino)benzonitrile | Chemical Formula: C₂₂H₂₆N₆ | |
| | | | Exact Mass:368.174945 | |
| E229 | 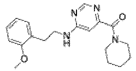 | (6-((2-methoxyphenethyl)amino)pyrimidin-4-yl)(piperidin-1-yl)methanone | Chemical Formula: C₁₉H₂₄N₄O₂ | Method 10: R*t*= 1.05min, |
| | | | | MS (ESI) m/z = 341.0[M+H]⁺. |
| | | | Exact Mass:340.1899 | |
| E230 | 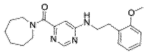 | azepan-1-yl(6-((2-methoxyphenethyl)amino)pyrimidin-4-yl)methanone | Chemical Formula: C₂₀H₂₆N₄O₂ | Method 10: R*t*= 1.20 min, |
| | | | | MS (ESI) m/z = 355.2[M+H]⁺. |
| | | | Exact Mass:354.205576 | |
| E231 | 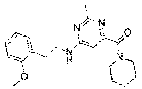 | (6-((2-methoxyphenethyl)amino)-2-methylpyrimidin-4-yl)(piperidin-1-yl)methanone | Chemical Formula: C₂₀H₂₆N₄O₂ | Method 10: R*t*= 0.99min, |
| | | | | MS (ESI) m/z = 355.2[M+H]⁺. |
| | | | Exact Mass:354.205576 | |
| E232 | 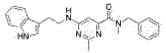 | 6-((2-(1H-indol-3-yl)ethyl)amino)-N-benzyl-N,2-dimethylpyrimidine-4-carboxamide | Chemical Formula: C₂₄H₂₅N₅O | |
| | | | Exact Mass:399.20591 | |
| E233 | 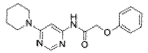 | 2-phenoxy-N-(6-(piperidin-1-yl)pyrimidin-4-yl)acetamide | Chemical Formula: C₁₇H₂₀N₄O₂ | Method 10: R*t*= 1.17min, |
| | | | | MS (ESI) m/z = 313.2[M+H]⁺. |
| | | | Exact Mass:312.158626 | |
| E234 | 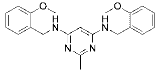 | N⁴,N⁶-bis(2-methoxybenzyl)-2-methylpyrimidine-4,6-diamine | Chemical Formula: C₂₁H₂₄N₄O₂ | |
| | | | Exact Mass:364.189926 | |
| E235 | 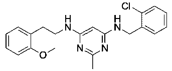 | N⁴-(2-chlorobenzyl)-N⁶-(2-methoxyphenethyl)-2-methylpyrimidine-4,6-diamine | Chemical Formula: C₂₁H₂₃ClN₄O | |
| | | | Exact Mass:382.156039 | |
| E236 | 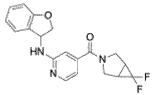 | (6,6-difluoro-3-azabicyclo[3.1.0]hexan-3-yl)(2-((2,3-dihydrobenzofuran-3-yl)amino)pyridin-4-yl)methanone | Chemical Formula: C₁₉H₁₇F₂N₃O₂ | Method 2:R*t*= 0.62min, |
| | | | | MS (ESI) m/z = 358.09[M+H]⁺. |
| | | | Exact Mass: 357,13 | |

### Biological assay

Molecules were screened in cultured cells in high throughput through the evaluation of cell growth by HOECHST and DRAQ7 staining. Western blot analysis was used to quantify the effect of the molecules on the level of Cyclin D1 protein. As cell models, we used the hepatocellular carcinoma cell line Huh7 highly dependent on cyclin D1 expression for proliferation.

### Cell seeding

Huh7 cells (CLS, Cat. 300156, and Sigma Aldrich, Cod. EP-CL-0120) were routinely cultured in DMEM High Glucose GlutaMAX-supplement (ThermoFisher Scientific, Cat. 61965-026) with the addition of 10% Fetal Bovine Serum (FBS, ThermoFisher Scientific, Cat. 10500064), 1% Non-Essential Amino Acids (ThermoFisher Scientific, Cat. 11140050) and 1% Pen/Strep (ThermoFisher Scientific, Cat. 15140122). Alternatively, cells were cultured in DMEM, high glucose, no Glutamine (ThermoFisher Scientific, Cat. 11960044) with the addition of 10% FBS (ThermoFisher Scientific, Cat. A5256801), 1% Non-Essential Amino Acids (Euroclone, Cat. ECB3054D) and 1% L-Glutamine Pen/Strep (Euroclone, Cat. ECB3003D). All cell lines were cultured in a humidified incubator with 5% CO₂ at 37° C.

For the analysis of cell proliferation, cells were seeded in 384-well plates (Greiner, Cat. 781092) at 1800 cells/well in a final volume of 40 µl cell solution/well. Alternatively, cells were seeded in 96-well plates (Revity, Cat. 6055300) at 5000 cells/well in a final volume of 100 µl cell solution/well. For each experiment, either a sentinel plate or few sentinel wells were seeded to count cells at the moment of the treatment (t = 0, 24h after seeding) to determine the initial cell number.

For western blotting analysis, cells were plated in 24-well plates (Euroclone CC 3524 24 well) at 0.8 × 10⁵ cells per well in a final volume of 1 mL cell solution/well.

### Compound plate preparation and treatment

For the analysis of cell proliferation, each compound was tested in a 10-points dilution curve spanning from 10 µM to 0.5 nM (final concentration). As controls, we included DMSO (vehicle control, 0.1%) and either Palbociclib (10 or 5 µM), Nocodazole (66 nM) a previously identified internal compound with demonstrated activity from earlier screenings. To treat 384-well plates, dilutions were prepared in a 384 LDV Echo plate (Beckman, Cat. 001-14555). Conversely, to treat 96-well plates, dilutions were performed in 96-deep well plates (Sial, Cat. DW222) with Tecan Fluent 780. Treated cells were then incubated for 48h.

For western blotting analysis, stock solutions of the compounds were prepared at 3, 10, and 30 mM (1000X). To treat cells, 1 µL aliquot of each stock solution was added to a well of a 24-well plate containing 1 mL cell medium (corresponding to final concentrations of 3, 10, and 30 µM). Vehicle controls were obtained by adding equivalent volumes of DMSO. The treatment was performed either for 5h or 8h . After treatment, cells were collected in lysis buffer (Tris 10 mM, pH 7.4, 0.5% NP-40, 0.5% TX-100, 150 mM NaCl plus complete EDTA-free Protease Inhibitor Cocktail Tablet purchased from Merck, Cat. 11697498001) and cell lysates frozen at -80°C.

### Cell staining and imaging

Concerning 384-well plates, staining solution containing HOECHST 33342 (ThermoFisher Scientific, Cat. H3570) and DRAQ7 (ThermoFisher Scientific, Cat. D15106) was prepared fresh for every cell plate, right before cell staining. The staining solution was added to each well using Integra 16 channel VIAFLO Lightweight electronic pipette with a final dye dilution of 2 µg/mL HOECHST and 0.3 µM DRAQ7. Cells were incubated with staining solution for 15 min at room temperature in the dark. Afterwards, cells were washed and 20 µl HBSS solution for imaging (ThermoFisher Scientific, Cat. 14025-050) were added to each well and the plate was loaded in Operetta CLS High Content Analysis System (PerkinElmer) for automated image acquisition.

Concerning 96-well plates, staining solution containing HOECHST 33342 and Propidium-lodide (ThermoFisher Scientific, Cat. P1304MP) was prepared fresh for every cell plate, right before cell staining. The staining solution was added to each well using ErgoOne E-Multi-Channel electronic pipette (Starlab, Cod. G9012-0100) with a final dye dilution of 4 µg/mL HOECHST and 1 µg/mL Propidium-lodide. Cells were incubated with staining solution for 30 min in the incubator at 37°C. Afterwards, cells were imaged with Agilent BioTek Cytation5 (AHSI, Cod. BKCYT5FW-SN) for automated image acquisition.

### Image analysis

Concerning 384-well plates, image analysis was performed using a building block analysis designed using Harmony High-Content Imaging and Analysis Software (Ver. 5.1, PerkinElmer). In this analysis, cell nuclei were identified and counted using the HOECHST signal, while dead cells were detected measuring the DRAQ7.

Concerning 96-well plates, image analysis was performed using Gen5 Software (Ver. 3.15, BioTek). In this analysis, cell nuclei were identified and counted using the HOECHST signal, while dead cells were detected measuring Propidium-lodide signal.

Cell number is measured at the moment of treatment with compound (t=0) on intended sentinel wells and 48h after the treatment with compound (t = 48) on intended test wells. Cell proliferation is evaluated by normalizing, for every cell line, cell number at t = 0 with cell number at t = 48 and setting DMSO-treated wells as 100% proliferation capacity.

In 384-well plate analysis, toxic compounds are identified by comparing DRAQ7+ cells in compound-treated wells with DRAQ7+ cells in Nocodazole-treated wells. For each test plate , the average % of dead cells measured in Nocodazole-treated wells is used as a threshold to discard toxic compounds. Conversely, in 96-well plate analysis, toxicity is defined as growth <0, meaning that viable cells at t = 48 resulted to be less than cells at time of treatment (t = 0).

### Western Blot

Protein quantification of cell lysates was performed using the BCA kit (Thermo Fisher #23225). 30 µg of total proteins were diluted 2:1 in 4X Laemmli sample buffer (Bio-rad #1610747) containing 100 mM Dithiothreitol, boiled for 8 min at 95 °C and loaded on SDS-PAGE gels and then transferred to hydrophilic polyvinylidene fluoride (PVDF) membranes. After blocking the membranes in 5% (w/v) non-fat dry milk in Tris-buffered saline containing 0.01% Tween-20 (TBS-T), blots were probed with anti-cyclin D1 antibody (1:10000) (Abcam #ab134175) in 5% (w/v) non-fat dry milk in TBS-T overnight at 4 °C, washed 3 times with TBS-T 10 min each, then probed with a 1:10000 dilution of horseradish conjugated goat anti-rabbit for 1 h at RT (Jackson ImmunoResearch Cat. 111-035-045). After 2 washes with TBS-T and one with Milli-Q water, signals were revealed using the ECL^{™} Select Western Blotting Detection Reagent (GEHRPN2235, GE Healthcare) or ECL Prime (GEHRPN2232) and visualized with a ChemiDoc XRS Touch Imaging System (Bio-rad).

### Table 3: Proliferation assay in Huh7 cell line

Results are in Table 3 below for exemplary compounds.

The symbol "++++" indicates inhibition of proliferation higher than 50% at concentration lower or equal than 1 µM.

The symbol "+++" indicates inhibition of proliferation higher than 50% at concentration higher than 1 and lower or equal than 10 µM.

The symbol "++" indicates an inhibition of proliferation higher than 50% at concentration higher than 10 and lower or equal than 30 µM.

The symbol "+" indicates an inhibition of proliferation higher than 50% at concentration higher than 30 µM.

**Table 3: Proliferation assay in Huh7 cell line:**

| Exam ple | IUPAC | Structure | % Proliferation inhibition range in Huh7 cells |
|---|---|---|---|
| E1 | (4,4-difluoropiperidin-1-yl)(2-((2-methoxy-phenethyl)amino) pyridin-4-yl)methanone; hydrochloride | 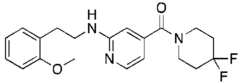 | +++ |
| E2 | (R)-(2-((2-methoxyphenethyl)ami no)pyridin-4-yl)(2-(trifluoromethyl)piperidi n-1-yl)methanone; hydrochloride | 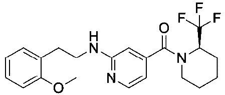 | +++ |
| E3 | (3,3-difluoropyrrolidin-1-yl)(2-((2-methoxy-phenethyl)amino)pyridi n-4-yl)methanone | 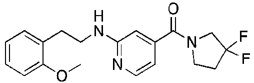 | +++ |
| E4 | azepan-1-yl(2-((2-methoxyphenethyl)ami no)pyridin-4-yl)methanone | 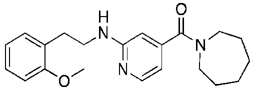 | +++ |
| E5 | (6,6-dimethyl-3-azabicyclo[3.1.0]hexan-3-yl)(2-((2-methoxy-phenethyl)amino)pyridi n-4-yl)methanone; hydrochloride | 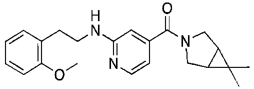 | ++ |
| E6 | (6,6-difluoro-3-azabicyclo[3.1.0]hexan-3-yl)(2-((2-methoxy-phenethyl)amino)pyridi n-4-yl)methanone; hydrochloride | 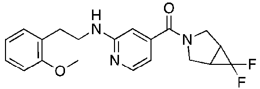 | ++++ |
| E7 | (8-oxa-3-azabicyclo[3.2.1]octan-3-yl)(2-((2-methoxy-phenethyl)amino)pyridi n-4-yl)methanon; hydrochloride | 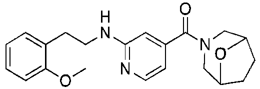 | + |
| E8 | (1-(hydroxymethyl)-3-azabicyclo[3.1.0]hexan-3-yl)(2-((2-methoxy-phenethyl)amino)pyridi n-4-yl)methanone; hydrochloride | 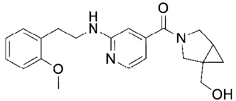 | + |
| E9 | (1-ethyl-3-azabicyclo-[3.1.0]hexan-3-yl)(2-((2-methoxyphenethyl)amino)pyridin-4-yl)methanone; hydrochloride | 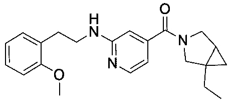 | ++ |
| E10 | (2-((2-methoxy-phenethyl)amino)pyridi n-4-yl)(piperidin-1-yl)methanone;hydrochl oride | 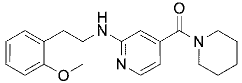 | +++ |
| E11 | 2-((2-methoxyphen-ethyl)amino)-*N*,*N-*dimethylisonicotinamid e; hydrochloride | 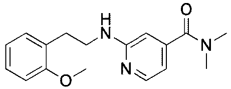 | + |
| E12 | (*S*)-(2-((2-methoxy-phenethyl)amino)pyridi n-4-yl)(2-(trifluoromethyl)-piperidin-1-yl)methanone; hydrochloride | 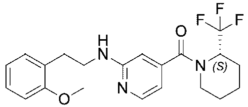 | ++ |
| E13 | (2-((2-methoxyphen-ethyl)amino)pyridin-4-yl)(4-azaspiro[2.5]-octan-4-yl)methanone | 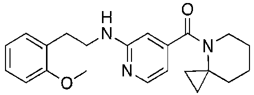 | ++ |
| E14 | *rac*-((3a*R*,6a*S*)-4,4-difluorohexahydrocyclo penta[*c*]pyrrol-2(1*H*)-yl)(2-((2-methoxy-phenethyl)amino)pyridi n-4-yl)methanone; hydrochloride | 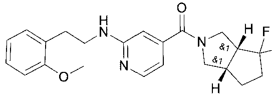 | ++ |
| E15 | (*S*)-(2-ethylpiperidin-1-yl)(2-((2-methoxy-phenethyl)amino)pyridi n-4-yl)methanone; hydrochloride | 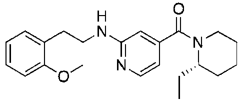 | ++++ |
| E16 | ((3a*R*,6a*S*)-hexahydrocyclopenta[c] pyrrol-2(1*H*)-yl)(2-((2-methoxyphenethyl)ami no)pyridin-4-yl)methanone | 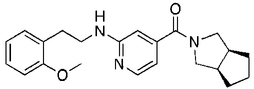 | +++ |
| E17 | (*R*)-(2-ethylpiperidin-1-yl)(2-((2-methoxy-phenethyl)amino)pyridi n-4-yl)methanone; hydrochloride | 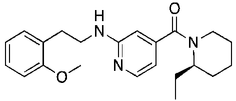 | ++ |
| E18 | (2-azabicyclo[4.1.0]-heptan-2-yl)(2-((2-methoxyphenethyl)ami no)pyridin-4-yl)methanone; hydrochloride | 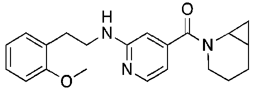 | +++ |
| E19 | (4,4-difluoroazepan-1-yl)(2-((2-methoxy-phenethyl)amino)pyridi n-4-yl)methanone; hydrochloride | 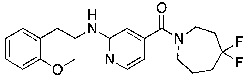 | ++ |
| E20 | (hexahydrocyclopenta[*c* ]pyrrol-2(1*H*)-yl)(2-((2-methoxyphenethyl)ami no)pyridin-4-yl)methanone | 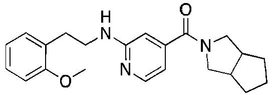 | ++ |
| E21 | (3-azabicyclo[3.1.0]-hexan-3-yl)(2-((2-methoxyphenethyl)ami no)pyridin-4-yl)methanone | 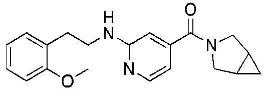 | +++ |
| E22 | (2-ethylpiperidin-1-yl)(2-((2-methoxy-phenethyl)amino)pyridi n-4-yl)methanone | 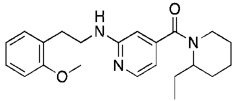 | +++ |
| E23 | (3,3-difluoroazetidin-1-yl)(2-((2-methoxy-phenethyl)amino)pyridi n-4-yl)methanone | 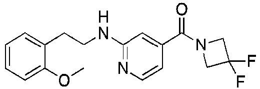 | ++++ |
| E24 | (2-((2-methoxyphen-ethyl)amino)pyridin-4-yl)(1-methyl-3-azabicyclo[3.1.0]hexan-3-yl)methanone | 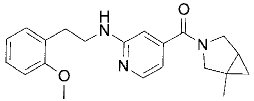 | +++ |
| E25 | azetidin-1-yl(2-((2-methoxyphenethyl)ami no)pyridin-4-yl)methanone | 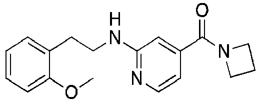 | +++ |
| E26 | (2-((2-methoxyphenethyl)amino)pyridin-4-yl)(2-azaspiro[3.3]-heptan-2-yl)methanone | 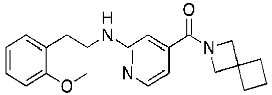 | +++ |
| E27 | (*R*)-(2-((2-methoxyphenethyl)ami no)pyridin-4-yl)(2-methylindolin-1-yl)methanone | 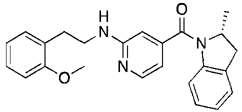 | ++++ |
| E28 | (6,6-difluoro-2-azaspiro[3.3]heptan-2-yl)(2-((2-methoxyphenethyl)ami no)pyridin-4-yl)methanone | 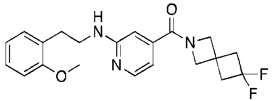 | + |
| E29 | (2-((3-chlorophenethyl)-amino)pyridin-4-yl)(2-ethylpiperidin-1-yl)methanone;hydrochl oride | 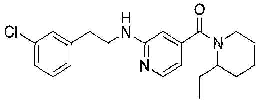 | +++ |
| E30 | (2-ethylpiperidin-1-yl)(2-((2-(pyridin-2-yl)ethyl)amino)pyridin-4-yl)methanone; hydrochloride | 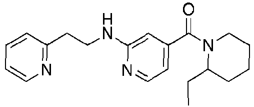 | + |
| E31 | (2-((2-chlorophenethyl)amino) pyridin-4-yl)(2-ethylpiperidin-1-yl)methanone | 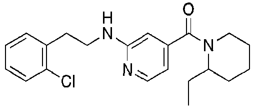 | +++ |
| E32 | 2-ethylpiperidin-1-yl)(2-((2-(trifluoromethyl)pheneth yl)amino)pyridin-4-yl)methanone | 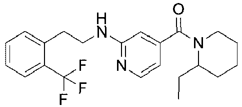 | +++ |
| E33 | (2-ethylpiperidin-1-yl)(2-(((1-(2-methoxyphenyl)cyclopr opyl)methyl)amino)pyri din-4-yl)methanone | 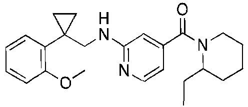 | ++ |
| E34 | (2-ethylpiperidin-1-yl)(2-((2-(pyridin-4-yl)ethyl)amino)pyridin-4-yl)methanone; hydrochloride | 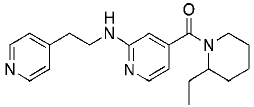 | + |
| E35 | (2-ethylpiperidin-1-yl)(2-((2-(pyridin-3-yl)ethyl)amino)pyridin-4-yl)methanone, hydrochloride | 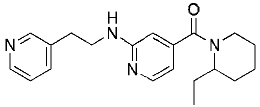 | + |
| E36 | (2-((2-methoxyphenethyl)ami no)pyridin-4-yl)(2-phenylazetidin-1-yl)methanone | 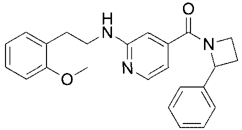 | ++ |
| E37 | 2-((2-(3*H*-indol-7-yl)ethyl)amino)pyridin-4-yl)(2-ethylpiperidin-1-yl)methanone | 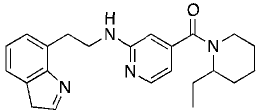 | +++ |
| E38 | 2-((2-(difluoromethoxy)phene thyl)amino)pyridin-4-yl)(2-ethylpiperidin-1-yl)methanone | 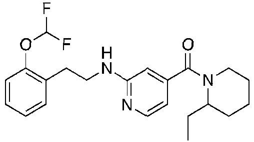 | +++ |
| E39 | (2-ethylpiperidin-1-yl)(2-((2-(piperidin-1-yl)ethyl)amino)pyridin-4-yl)methanone | 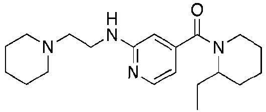 | + |
| E40 | (2-((2-methoxyphenethyl)ami no)pyridin-4-yl)(2-methylindolin-1-yl)methanone; hydrochloride | 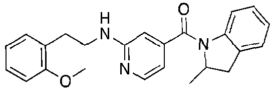 | ++++ |
| E41 | (*S*)-(2-((2-methoxyphenethyl)ami no)pyridin-4-yl)(2-methylindolin-1-yl)methanone;hydrochl oride | 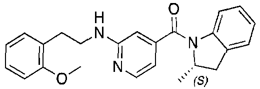 | +++ |
| E42 | (*S*)-(2-ethylpiperidin-1-yl)(2-((2-(trifluoromethoxy)phene thyl)amino)pyridin-4-yl)methanone | 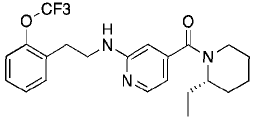 | ++++ |
| E43 | (*S*)-(2-ethylpiperidin-1-yl)(2-((2-(4-methoxypyridin-3-yl)ethyl)amino)pyridin-4-yl)methanone | 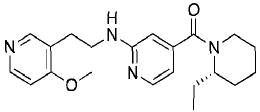 | + |
| E44 | (6,6-difluoro-3-azabicyclo[3.1.0]hexan-3-yl)(2-((2-(trifluoromethoxy)phene thyl)amino)pyridin-4-yl)methanone | 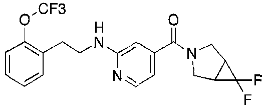 | ++++ |
| E45 | (2-((2-(1*H*-indol-3-yl)ethyl)amino)pyridin-4-yl)(6,6-difluoro-3-azabicyclo[3.1.0]hexan-3-yl)methanone | 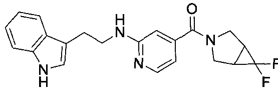 | +++ |
| E46 | (6,6-difluoro-3-azabicyclo[3.1.0]hexan-3-yl)(2-((2-methoxy-6-(methoxymethoxy)phen ethyl)amino)pyridin-4-yl)methanone | 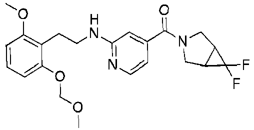 | ++++ |
| E47 | (6,6-difluoro-3-azabicyclo[3.1.0]hexan-3-yl)(2-((2-hydroxy-6-methoxyphenethyl)ami no)pyridin-4-yl)methanone | 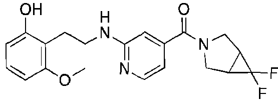 | +++ |
| E48 | *rac*-(6,6-difluoro-3-azabicyclo[3.1.0]hexan-3-yl)(2-((2-(2-methoxyphenyl) propyl) amino)pyridin-4-yl)methanone | 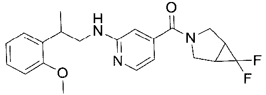 | +++ |
| E49 | (6,6-difluoro-3-azabicyclo[3.1.0]hexan-3-yl)(2-((3-methoxyphenethyl)ami no)pyridin-4-yl)methanone; hydrochloride | 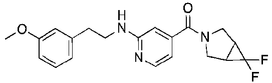 | ++ |
| E50 | (2-((2-(benzofuran-7-yl)ethyl)amino)pyridin-4-yl)(6,6-difluoro-3-azabicyclo[3.1.0]hexan-3-yl)methanone; hydrochloride | 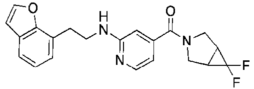 | +++ |
| E51 | (6,6-difluoro-3-azabicyclo[3.1.0]hexan-3-yl)(2-((2-(2,3-dihydrobenzofuran-7-yl)ethyl)amino)pyridin-4-yl)methanone | 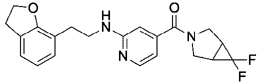 | ++++ |
| E52 | (2-((2-cyclopropoxyphenethyl) amino)pyridin-4-yl)(6,6-difluoro-3-azabicyclo[3.1.0]hexan-3-yl)methanone | 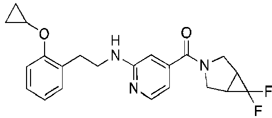 | ++++ |
| E53 | (2-((3-methoxyphenethyl)ami no)pyridin-4-yl)(piperidin-1-yl)methanone | 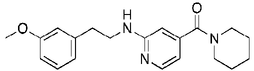 | ++ |
| E54 | (2-ethylpiperidin-1-yl)(2-((3-methoxyphenethyl)ami no)pyridin-4-yl)methanone; hydrochloride | 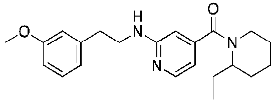 | +++ |
| E55 | (6,6-difluoro-3-azabicyclo[3.1.0]hexan-3-yl)(2-((2-(2-methylthiazol-5-yl)ethyl)amino)pyridin-4-yl)methanone | 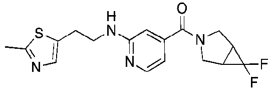 | + |
| E56 | (6,6-difluoro-3-azabicyclo[3.1.0]hexan-3-yl)(2-((2-fluoro-6-methoxyphenethyl)ami no)pyridin-4-yl)methanone | 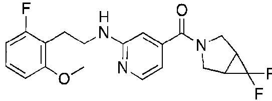 | +++ |
| E57 | (2-((3-chloro-2-fluoro-6-methoxyphenethyl)ami no)pyridin-4-yl)(6, 6-difluoro-3-azabicyclo[3.1.0]hexan-3-yl)methanone | 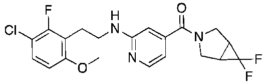 | +++ |
| E58 | (6,6-difluoro-3-azabicyclo[3.1.0]hexan-3-yl)(2-((2-methoxy-5-(trifluoromethoxy)phene thyl)amino)pyridin-4-yl)methanone | 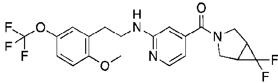 | ++++ |
| E59 | (2-((4,5-difluoro-2-methoxyphenethyl)ami no)pyridin-4-yl)(6,6-difluoro-3-azabicyclo[3.1.0]hexan-3-yl)methanone | 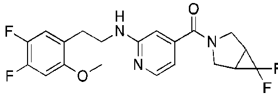 | +++ |
| E60 | (6,6-difluoro-3-azabicyclo[3.1.0]hexan-3-yl)(2-((2-(2,2-difluorobenzo[d][1,3]dio xol-4-yl)ethyl)amino)pyridin-4-yl)methanone | 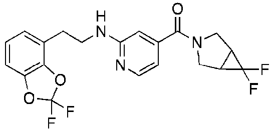 | ++++ |
| E61 | (6,6-difluoro-3-azabicyclo[3.1.0]hexan-3-yl)(2-((2-(3-methoxythiophen-2-yl)ethyl)amino)pyridin-4-yl)methanone | 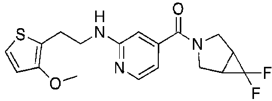 | +++ |
| E62 | (6,6-difluoro-3-azabicyclo[3.1.0]hexan-3-yl)(2-((4-fluoro-2-methoxyphenethyl)ami no)pyridin-4-yl)methanone | 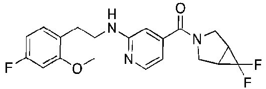 | +++ |
| E63 | (6,6-difluoro-3-azabicyclo[3.1.0]hexan-3-yl)(2-((6-fluoro-2,3-dimethoxyphenethyl)a mino)pyridin-4-yl)methanone | 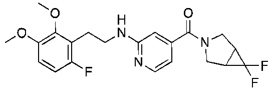 | ++ |
| E64 | (6,6-difluoro-3-azabicyclo[3.1.0]hexan-3-yl)(2-((4-methoxy-2-(trifluoromethoxy)phene thyl)amino)pyridin-4-yl)methanone | 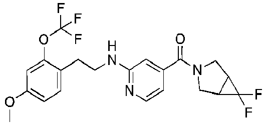 | +++ |
| E65 | (2-((2-(benzo[d][1,3]dioxol-4-yl)ethyl)amino)pyridin-4-yl)(6,6-difluoro-3-azabicyclo[3.1.0]hexan-3-yl)methanone | 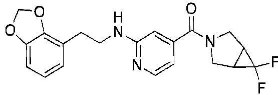 | ++++ |
| E66 | (6,6-difluoro-3-azabicyclo[3.1.0]hexan-3-yl)(2-((2,3-dimethoxyphenethyl)a mino)pyridin-4-yl)methanone | 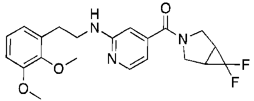 | +++ |
| E67 | (2-((5-chloro-2-methoxyphenethyl)ami no)pyridin-4-yl)(6, 6-difluoro-3-azabicyclo[3.1.0]hexan-3-yl)methanone | 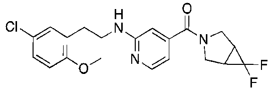 | ++++ |
| E68 | (2-((2-chloro-6-methoxyphenethyl)ami no)pyridin-4-yl)(6,6-difluoro-3-azabicyclo[3.1.0]hexan-3-yl)methanone | 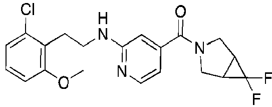 | +++ |
| E69 | (2-((5-bromo-2-methoxyphenethyl)ami no)pyridin-4-yl)(6,6-difluoro-3-azabicyclo[3.1.0]hexan-3-yl)methanone | 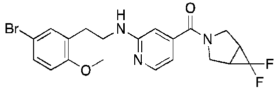 | ++++ |
| E70 | (2-ethylpiperidin-1-yl)(2-((2-methoxyphenethyl)ami no)-6-methylpyridin-4-yl)methanone | 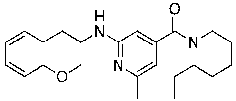 | +++ |
| E71 | (2-chloro-6-((2-methoxyphenethyl)ami no)pyridin-4-yl)(2-ethylpiperidin-1-yl)methanone | 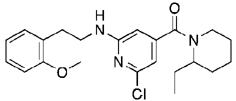 | ++ |
| E72 | (6,6-difluoro-3-azabicyclo[3.1.0]hexan-3-yl)(2-((2-methoxyphenethyl)ami no)-6-(pyrrolidin-1-yl)pyridin-4-yl)methanone | 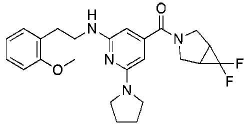 | + |
| E73 | (6,6-difluoro-3-azabicyclo[3.1.0]hexan-3-yl)(2-((2-methoxyphenethyl)ami no)-3-methylpyridin-4-yl)methanone | 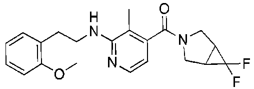 | + |
| E74 | (2-ethylpiperidin-1-yl)(2-hydroxy-6-((2-methoxyphenethyl)ami no)pyridin-4-yl)methanone | 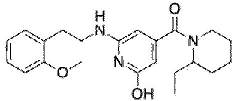 | ++ |
| E75 | *trans-* (6,6-difluoro-3-azabicyclo[3.1.0]hexan-3-yl)(2-(((1*r*,3*r*)-3-(2-methoxyphenyl)cyclobu tyl)amino)pyridin-4-yl)methanone | 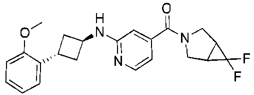 | +++ |
| E76 | (6,6-difluoro-3-azabicyclo[3.1.0]hexan-3-yl)(2-((1-(2-methoxyphenyl)azetidin -3-yl)amino)pyridin-4-yl)methanone | 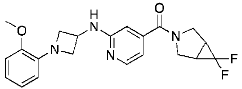 | + |
| E77 | *trans-* (6,6-difluoro-3-azabicyclo[3.1.0]hexan-3-yl)(2-((2-(2-methoxyphenyl)cyclopr opyl)amino)pyridin-4-yl)methanone | 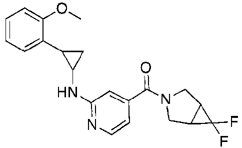 | ++++ |
| E78 | (*S*)-(2-((2-methoxybenzyl)amino) pyridin-4-yl)(2-methylindolin-1-yl)methanone | 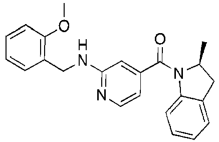 | +++ |
| E79 | (*S*)-(2-((3-methoxybenzyl)amino) pyridin-4-yl)(2-methylindolin-1-yl)methanone | 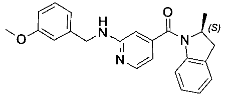 | ++ |
| E80 | (6,6-difluoro-3-azabicyclo[3.1.0]hexan-3-yl)(2-((2-methoxybenzyl)amino) pyridin-4-yl)methanone | 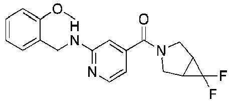 | +++ |
| E81 | (6,6-difluoro-3-azabicyclo[3.1.0]hexan-3-yl)(2-((3-methoxybenzyl)amino) pyridin-4-yl)methanone | 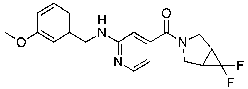 | ++ |
| E82 | (4,4-difluoropiperidin-1-yl)(2-((2-methoxybenzyl)amino) pyridin-4-yl)methanone | 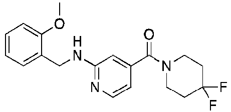 | + |
| E83 | (*R*)-(2-((2-methoxybenzyl)amino) pyridin-4-yl)(2-methylindolin-1-yl)methanone | 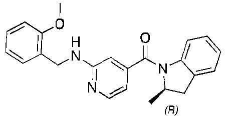 | +++ |
| E84 | (6,6-difluoro-3-azabicyclo[3.1.0]hexan-3-yl)(2-((2-(trifluoromethoxy)benzy l)amino)pyridin-4-yl)methanone | 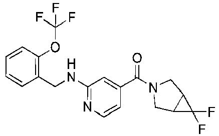 | +++ |
| E85 | (6,6-difluoro-3-azabicyclo[3.1.0]hexan-3-yl)(2-(((2-methylthiazol-5-yl)methyl)amino)pyridin -4-yl)methanone | 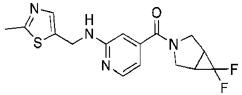 | + |
| E86 | *N*-(2-fluorobenzyl)-2-((2-methoxyphenethyl)ami no)isonicotinamide ( | 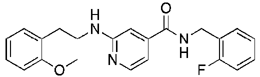 | + |
| E87 | *N*-(3-fluorobenzyl)-2-((2-methoxyphenethyl)ami no)isonicotinamide | 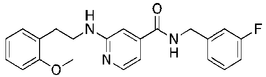 | + |
| E88 | (*S*-*N*-(1-(4-fluorophenyl)-2-hydroxyethyl)-2-((2-methoxyphenethyl)ami no)isonicotinamide | 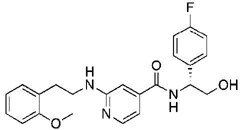 | + |
| E89 | *N*-(cuban-1-yl)-2-{[2-(2-methoxyphenyl)ethyl]a mino}pyridine-4-carboxamide | 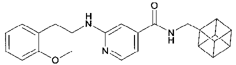 | + |
| E90 | (*S*)-*N*-(1-(4-fluorophenyl)ethyl)-2-((2-methoxyphenethyl)amino)isonicotinamide | 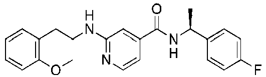 | ++ |
| E91 | *N*-(1-(4-fluorophenyl)cyclopropy l)-2-((2-methoxyphenethyl)ami no)isonicotinamide | 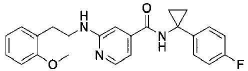 | + |
| E92 | *N*-(2-(4-fluorophenyl)propan-2-yl)-2-((2-methoxyphenethyl)ami no)isonicotinamide | 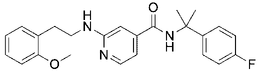 | + |
| E93 | (*S*)-*N*-(5-fluoro-2,3-dihydro-1*H*-inden-1-yl)-2-((2-methoxyphenethyl)ami no)isonicotinamide | 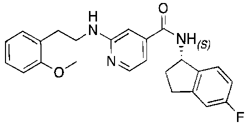 | ++ |
| E94 | *N*-(4-fluorobenzyl)-2-((2-methoxyphenethyl)ami no)isonicotinamide | 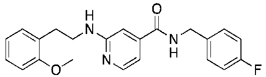 | + |
| E95 | *N*-(4-fluorocuban-1-yl)-2-((2-methoxyphenethyl)ami no)isonicotinamide | 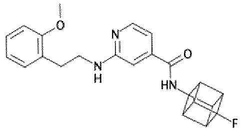 | ++ |
| E96 | (*R*)-*N*-(1-(4-fluorophenyl)ethyl)-2-((2-methoxyphenethyl)ami no)isonicotinamide | 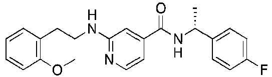 | ++ |
| E97 | *N*-(cuban-1-yl)-2-{[2-(2-methoxyphenyl)ethyl]a mino}pyridine-4-carboxamide | 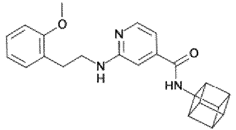 | + |
| E98 | (4-(benzo[*d*]thiazol-2-yl)piperidin-1-yl)(2-((2-methoxyphenethyl)ami no)pyridin-4-yl)methanone | 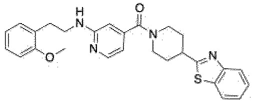 | +++ |
| E99 | (6,6-difluoro-3-azabicyclo[3.1.0]hexan-3-yl)(2-((2-hydroxyphenethyl)amin o)pyridin-4-yl)methanone | 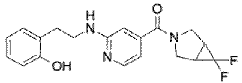 | +++ |
| E100 | (2-((2-methoxyphenethyl)ami no)pyridin-4-yl)(pyrrolidin-1-yl)methanone | 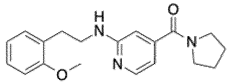 | ++++ |
| E101 | (*S*)-(3-hydroxypyrrolidin-1-yl)(2-((2-methoxyphenethyl)ami no)pyridin-4-yl)methanone | 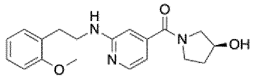 | ++ |
| E102 | (*R*)-(3-hydroxypyrrolidin-1-yl)(2-((2-methoxyphenethyl)ami no)pyridin-4-yl)methanone | 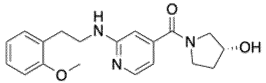 | +++ |
| E103 | (4-(benzo[d]thiazol-2-yl)piperidin-1-yl)(2-((2-methoxybenzyl)amino) pyridin-4-yl)methanone | 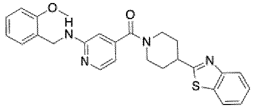 | + |
| E104 | (2-((2-(cyclopentyloxy)phenet hyl)amino)pyridin-4-yl)(6,6-difluoro-3-azabicyclo[3.1.0]hexan-3-yl)methanone | 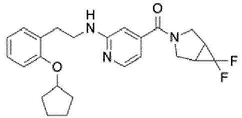 | ++++ |
| E105 | (2-ethylpiperidin-1-yl)(2-((2-methoxyphenethyl)ami no)-6-morpholinopyridin-4-yl)methanone | 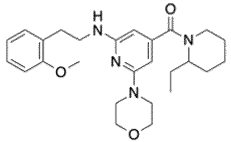 | ++ |
| E106 | (2-(3-azabicyclo[3.1.0]hexan-3-yl)-6-((2-methoxyphenethyl)ami no)pyridin-4-yl)(2-ethylpiperidin-1-yl)methanone | 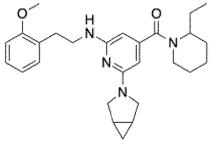 | ++ |
| E107 | (6,6-difluoro-3-azabicyclo[3.1.0]hexan-3-yl)(2-((3-fluoro-2-methoxyphenethyl)ami no)pyridin-4-yl)methanone | 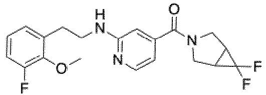 | +++ |
| E108 | (6,6-difluoro-3-azabicyclo[3.1.0]hexan-3-yl)(2-((5-fluoro-2-methoxyphenethyl)ami no)pyridin-4-yl)methanone | 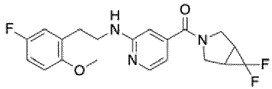 | +++ |
| E109 | (2-((3,6-difluoro-2-methoxyphenethyl)ami no)pyridin-4-yl)(6, 6-difluoro-3-azabicyclo[3.1.0]hexan-3-yl)methanone | 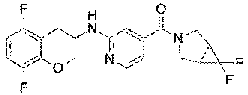 | +++ |
| E110 | (6,6-difluoro-3-azabicyclo[3.1.0]hexan-3-yl)(2-((2-methoxy-4-(methoxymethoxy)phen ethyl)amino)pyridin-4-yl)methanone | 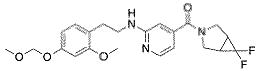 | + |
| E111 | (6,6-difluoro-3-azabicyclo[3.1.0]hexan-3-yl)(2-((4-hydroxy-2-methoxyphenethyl)ami no)pyridin-4-yl)methanone | 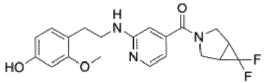 | + |
| E112 | (6,6-difluoro-3-azabicyclo[3.1.0]hexan-3-yl)(2-((2,4-dimethoxyphenethyl)a mino)pyridin-4-yl)methanone | 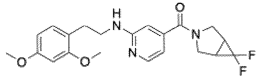 | +++ |
| E113 | (6,6-difluoro-3-azabicyclo[3.1.0]hexan-3-yl)(2-((2,5-dimethoxyphenethyl)a mino)pyridin-4-yl)methanone | 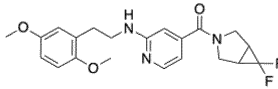 | ++++ |
| E114 | (6,6-difluoro-3-azabicyclo[3.1.0]hexan-3-yl)(2-((2,4-dimethoxy-3-methylphenethyl)amino )pyridin-4-yl)methanone | 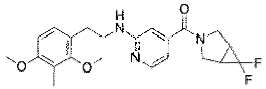 | +++ |
| E115 | (6,6-difluoro-3-azabicyclo[3.1.0]hexan-3-yl)(2-((2-(4-methoxy-1*H*-indol-3-yl)ethyl)amino)pyridin-4-yl)methanone | 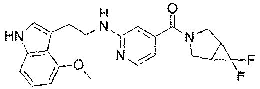 | +++ |
| E116 | (1,1-difluoro-5-azaspiro[2.4]heptan-5-yl)(2-((2-methoxyphenethyl)ami no)pyridin-4-yl)methanone | 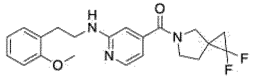 | +++ |
| E117 | (2-((2-methoxyphenethyl)ami no)pyridin-4-yl)(3-(oxetan-3-yl)pyrrolidin-1-yl)methanone | 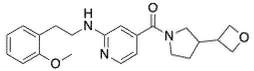 | + |
| E118 | (6,6-difluoro-3-azabicyclo[3.1.0]hexan-3-yl)(2-(phenethylamino)pyridi n-4-yl)methanone | 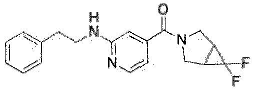 | + |
| E119 | (2-((2-methoxyphenethyl)ami no)pyridin-4-yl)(piperidin-1-yl-*d*₁₀)methanone | 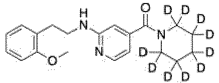 | +++ |
| E120 | (6,6-difluoro-1-azaspiro[3.3]heptan-1-yl)(2-((2-methoxyphenethyl)ami no)pyridin-4-yl)methanone | 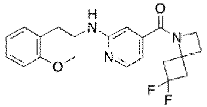 | + |
| E121 | (2,3-dihydro-1*H-*pyrrolo[3,2-b]pyridin-1-yl)(2-((2-methoxyphenethyl)ami no)pyridin-4-yl)methanone | 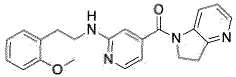 | + |
| E122 | (6,6-difluoro-3-azabicyclo[3.1.0]hexan-3-yl)(2-((2-methoxyphenethyl)ami no)-3-nitropyridin-4-yl)methanone | 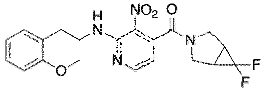 | + |
| E123 | (3-amino-2-((2-methoxyphenethyl)ami no)pyridin-4-yl)(6,6-difluoro-3-azabicyclo[3.1.0]hexan-3-yl)methanone | 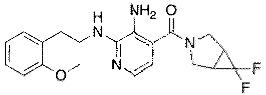 | + |
| E124 | *N*-(4-(6,6-difluoro-3-azabicyclo[3.1.0]hexan e-3-carbonyl)-2-((2-methoxyphenethyl)ami no)pyridin-3-yl)acetamide | 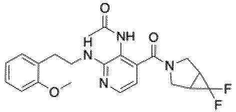 | + |
| E125 | (2-(benzo[*d*]thiazol-2-ylamino)pyridin-4-yl)(6,6-difluoro-3-azabicyclo[3.1.0]hexan-3-yl)methanone | 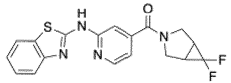 | +++ |
| E126 | (2-((benzo[*d*]thiazol-2-ylmethyl) amino) pyridin-4-yl)(6,6-difluoro-3-azabicyclo[3.1.0]hexan-3-yl)methanone | 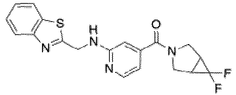 | + |
| E127 | (2-((2-(benzo[*d*]thiazol-2-yl)ethyl)amino)pyridin-4-yl)(6,6-difluoro-3-azabicyclo[3.1.0]hexan-3-yl)methanone | 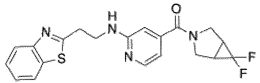 | + |
| E128 | (6,6-difluoro-3-azabicyclo[3.1.0]hexan-3-yl)(2-((2-(2-methoxy-[1,1'-biphenyl]-3-yl)ethyl)amino)pyridin-4-yl)methanone | 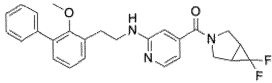 | +++ |
| E129 | (6,6-difluoro-3-azabicyclo[3.1.0]hexan-3-yl)(2-((2-(3-methoxy-[1,1'-biphenyl]-4-yl)ethyl)amino) pyridin-4-yl)methanone | 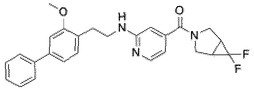 | ++++ |
| E130 | (6,6-difluoro-3-azabicyclo[3.1.0]hexan-3-yl)(2-((2-(4-methoxy-[1,1'-biphenyl]-3-yl)ethyl)amino)pyridin-4-yl)methanone | 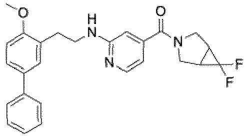 | +++ |
| E131 | (2-(chroman-3-ylamino)pyridin-4-yl)(6,6-difluoro-3-azabicyclo[3.1.0]hexan-3-yl)methanone | 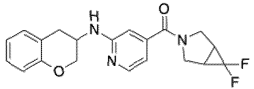 | + |
| E132 | (6,6-difluoro-3-azabicyclo[3.1.0]hexan-3-yl)(2-((2-methoxyphenethyl)ami no)pyridin-4-yl)methanethione | 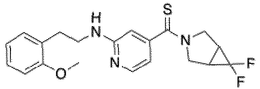 | +++ |
| E133 | *N*-(2-methoxyphenethyl)-4-(4-phenyloxazol-2-yl)pyridin-2-amine | 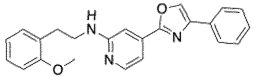 | + |
| E134 | *N*-(2-methoxyphenethyl)-4-(4-phenylthiazol-2-yl)pyridin-2-amine | 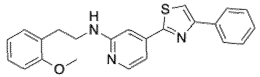 | + |
| E135 | *N*-(2-methoxyphenethyl)-4-(5-phenyl-1*H*-imidazol-2-yl)pyridin-2-amine | 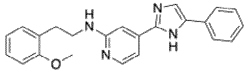 | +++ |
| E136 | (*S*)-(2-((2-methoxyphenethyl)ami no)pyridin-4-yl)(2-methylpyrrolidin-1-yl)methanone | 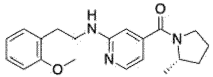 | +++ |
| E137 | (*R*)-(2-((2-methoxyphenethyl)ami no)pyridin-4-yl)(2-methylpyrrolidin-1-yl)methanone | 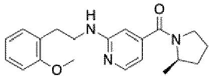 | ++++ |
| E138 | 1-(2-((2-methoxyphenethyl)ami no)isonicotinoyl)pyrrolid in-2-one | 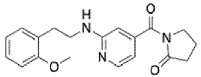 | + |
| E139 | (1,1-dioxidothiomorpholino)( 2-((2-methoxyphenethyl)ami no)pyridin-4-yl)methanone | 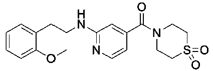 | ++++ |
| E140 | ((1*S*,4*S*)-2,2-dioxido-2-thia-5-azabicyclo[2.2.1]heptan -5-yl)(2-((2-methoxyphenethyl)ami no)pyridin-4-yl)methanone | 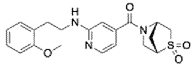 | + |
| E141 | (3,3-dioxido-3-thia-8-azabicyclo[3.2.1]octan-8-yl)(2-((2-methoxyphenethyl)ami no)pyridin-4-yl)methanone | 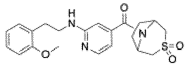 | + |
| E142 | (3,3-dioxido-3-thia-6-azabicyclo[3.1.1]heptan -6-yl)(2-((2-methoxyphenethyl)ami no)pyridin-4-yl)methanone | 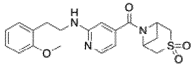 | + |
| E143 | ((1*R*,4*R*)-2,2-dioxido-2-thia-5-azabicyclo[2.2.1]heptan -5-yl)(2-((2-methoxyphenethyl)ami no)pyridin-4-yl)methanone | 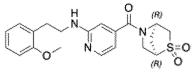 | + |
| E144 | 1-(2-methoxyphenyl)-3-(4-(piperidine-1-carbonyl)pyridin-2-yl)urea | 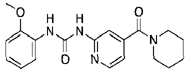 | + |
| E145 | 1-(4-(azetidine-1-carbonyl)pyridin-2-yl)-3-(2-methoxyphenyl)urea | 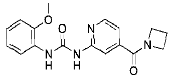 | + |
| E146 | 1-(4-(2-ethylpiperidine-1-carbonyl) pyridin-2-yl)-3-(2-methoxyphenyl)urea | 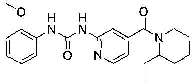 | + |
| E147 | *N*-(4-(2-ethylpiperidine-1-carbonyl)pyridin-2-yl)-2-(2-methoxyphenyl)acetami de | 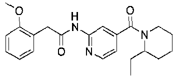 | + |
| E148 | *N*⁴-(3-chlorophenyl)-*N*²-(2-methoxyphenethyl)pyri dine-2,4-diamine; formate | 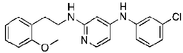 | ++++ |
| E149 | *N*²-(2-methoxyphenethyl)-*N*⁴-(3-methoxyphenyl)pyridine -2,4-diamine | 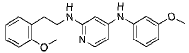 | +++ |
| E150 | *N*²-(2-methoxyphenethyl)-*N*⁴-(2-methoxyphenyl)pyridine -2,4-diamine | 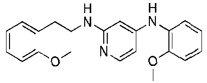 | ++++ |
| E151 | 4-(3-chlorophenoxy)-*N-*(2-methoxyphenethyl)pyri din-2-amine | 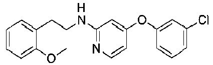 | + |
| E152 | *N*⁴-(3-chlorophenyl)-*N*²-(2-methoxybenzyl)pyridine -2,4-diamine | 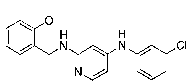 | + |
| E153 | 4-(6,6-difluoro-3-azabicyclo[3.1.0]hexan-3-yl)-*N*-(2-methoxyphenethyl)pyri din-2-amine e | 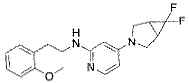 | +++ |
| E154 | 4-((6,6-difluoro-3-azabicyclo[3.1.0]hexan-3-yl)methyl)-*N*-(2-methoxyphenethyl)pyri din-2-amine dihydrochloride | 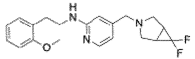 | ++ |
| E155 | trans-6,6-difluoro-N-((2-((2-methoxyphenethyl)ami no)pyridin-4-yl)methyl)bicyclo[3.1.0] hexane-3-carboxamide | 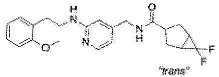 | + |
| E156 | 1-(6,6-difluoro-3-azabicyclo[3.1.0]hexan-3-yl)-2-(2-((2-methoxyphenethyl)ami no)pyridin-4-yl)ethan-1-one | 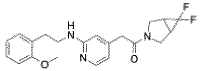 | ++ |
| E157 | *trans*-6,6-difluoro-*N*-(2-((2-methoxyphenethyl)ami no)pyridin-4-yl)bicyclo[3.1.0]hexane-3-carboxamide | 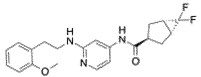 | ++ |
| E158 | 2-((2-((2-(1*H*-indol-3-yl)ethyl)amino)pyrimidin -4-yl)amino)benzonitrile | 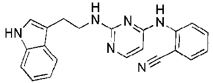 | +++ |
| E159 | 2-((2-((2-methoxyphenethyl)ami no)pyrimidin-4-yl)amino)benzonitrile | 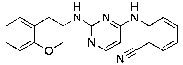 | ++ |
| E160 | 2-((2-((2-methoxybenzyl)amino) pyrimidin-4-yl)amino)benzonitrile | 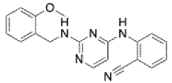 | +++ |
| E161 | *N*⁴-(2-chlorobenzyl)-*N*²-(2-methoxyphenethyl)pyri midine-2,4-diamine | 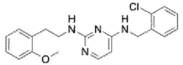 | ++++ |
| E162 | *N*²-(2-(1*H*-indol-3-yl)ethyl)-*N*⁴-(2-chlorobenzyl)pyrimidine -2,4-diamine | 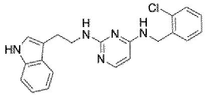 | +++ |
| E163 | *N*⁴-(2-chlorobenzyl)-*N*²-(2-methoxybenzyl)pyrimidi ne-2,4-diamine | 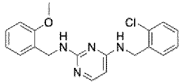 | ++++ |
| E164 | *N*4-(2-methoxybenzyl)-*N*2-(2-methoxyphenethyl)pyri midine-2,4-diamine | 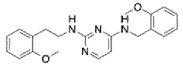 | ++++ |
| E165 | *N*²-(2-(1*H*-indol-3-yl)ethyl)-*N*⁴-(2-methoxybenzyl)pyrimidi ne-2,4-diamine | 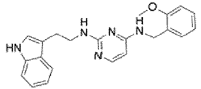 | +++ |
| E166 | *N*²,*N*⁴-bis(2-methoxybenzyl)pyrimidi ne-2,4-diamine | 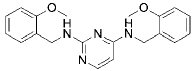 | ++++ |
| E167 | *N*⁴-(2-chlorobenzyl)-*N*⁶-(2-methoxyphenethyl)pyri midine-4,6-diamine | 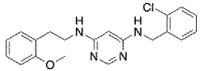 | +++ |
| E168 | *N*⁴-(2-(1*H*-indol-3-yl)ethyl)-*N*⁶-(2-chlorobenzyl)pyrimidine -4,6-diamine | 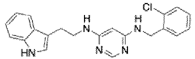 | +++ |
| E169 | *N*⁴-(2-chlorobenzyl)-*N*⁶-(2-methoxybenzyl)pyrimidi ne-4,6-diamine | 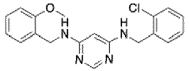 | + |
| E170 | *N*⁴,*N*⁶-bis(2-methoxybenzyl)pyrimidi ne-4,6-diamine | 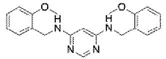 | + |
| E171 | *N*⁴-(2-methoxybenzyl)-*N*⁶-(2-methoxyphenethyl)pyri midine-4,6-diamine | 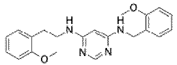 | +++ |
| E172 | *N*⁴-(2-methoxybenzyl)-*N*⁶-(2-methoxyphenethyl)pyri midine-4,6-diamine | 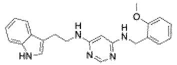 | +++ |
| E173 | 6-(3-chlorophenoxy)-*N-*(2-methoxyphenethyl)pyri midin-4-amine | 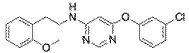 | + |
| E174 | 1-(2-((2-methoxyphenethyl)ami no)pyridin-4-yl)imidazolidin-2-one | 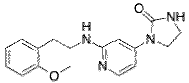 | +++ |
| E175 | 1-(2-((2-methoxyphenethyl)ami no)pyridin-4-yl)-3-methylimidazolidin-2-one | 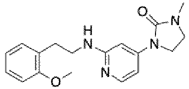 | +++ |
| E176 | 1-(2-((2-methoxyphenethyl)ami no)pyridin-4-yl)-1,3-dihydro-2*H-*benzo[*d*]imidazol-2-one | 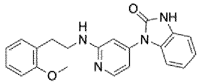 | ++++ |
| E177 | 1-(2-((2-methoxyphenethyl)ami no)pyridin-4-yl)-3,3-dimethylindolin-2-one | 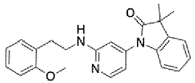 | ++++ |
| E178 | 2-(2-((2-methoxyphenethyl)ami no)pyridin-4-yl)isoindolin-1-one | 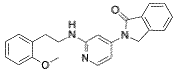 | +++ |
| E179 | 3-(2-((2-methoxyphenethyl)ami no)pyridin-4-yl)benzo[*d*]oxazol-2(3*H*)-one | 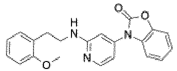 | +++ |
| E180 | 1-(2-((2-methoxybenzyl)amino) pyridin-4-yl)-1,3-dihyd1967 ro-2*H-*benzo[*d*]imidazol-2-one | 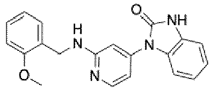 | +++ |
| E181 | 3-(2-((2-methoxybenzyl)amino) pyridin-4-yl)pyrrolidin-2-one | 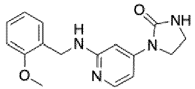 | ++++ |
| E182 | 1-(2-((2-(benzo[*d*][1,3]dioxol-4-yl)ethyl)amino)pyridin-4-yl)-1,3-dihydro-2*H-*benzo[*d*]imidazol-2-one | 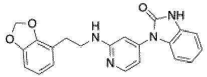 | +++ |
| E183 | 1-(2-((2-(cyclopentyloxy)phenet hyl)amino)pyridin-4-yl)imidazolidin-2-one | 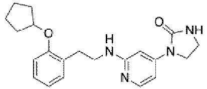 | ++++ |
| E184 | 1-(2-((2-(benzo[*d*][1,3]dioxol-4-yl)ethyl)amino)pyridin-4-yl)imidazolidin-2-one | 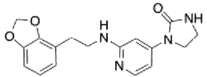 | ++++ |
| E185 | 1-(2-((2-(cyclopentyloxy)phenet hyl)amino)pyridin-4-yl)-1,3-dihydro-2*H-*benzo[*d*]imidazol-2-one | 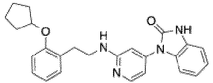 | ++++ |
| E186 | 1-(2-(((1*r*,3*r*)-3-(2-methoxyphenyl)cyclobu tyl)amino)pyridin-4-yl)-1,3-dihydro-2*H-*benzo[*d*]imidazol-2-one | 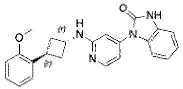 | +++ |
| E187 | 2-(2-((2-methoxyphenethyl)ami no)pyridin-4-yl)-2-azabicyclo[2.2.1]heptan-3-one | 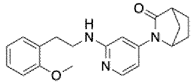 | +++ |
| E189 | 1-(2-((2-methoxyphenethyl)ami no)pyridin-4-yl)-4,4-dimethylpyrrolidin-2-one | 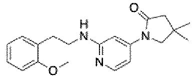 | ++++ |
| E190 | *tert*-butyl 5-(2-((2-methoxyphenethyl)ami no)pyridin-4-yl)-1,2,5-thiadiazolidine-2-carboxylate 1,1-dioxide | 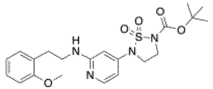 | + |
| E194 | 1-(2-((2-methoxyphenethyl)ami no)pyridin-4-yl)-4-phenylimidazolidin-2-one | 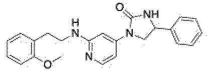 | +++ |
| E195 | 1-(2-((2-methoxy-6-(methoxymethoxy)phen ethyl)amino)pyridin-4-yl)-1,3-dihydro-2H-benzo[d]imidazol-2-one | 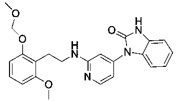 | ++++ |
| E196 | Example 196: 1-(2-((2-hydroxy-6-methoxyphenethyl)ami no)pyridin-4-yl)-1,3-dihydro-2*H-*benzo[*d*]imidazol-2-one | 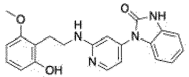 | +++ |
| E197 | 3-(2-((2-methoxyphenethyl)ami no)pyridin-4-yl)-1,3-dihydro-2*H-*imidazo[4,5-c]pyridin-2-one | 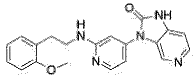 | +++ |
| E198 | 1-(2-((2-methoxyphenethyl)ami no)pyridin-4-yl)-1,3-dihydro-2*H-*imidazo[4,5-c]pyridin-2-one | 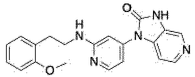 | +++ |
| E199 | 1-(2-((2-methoxy-6-(methoxymethoxy)phen ethyl)amino)pyridin-4-yl)imidazolidin-2-one | 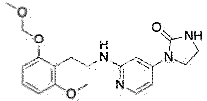 | ++++ |
| E200 | 1-(2-((2-hydroxy-6-methoxyphenethyl)ami no)pyridin-4-yl)imidazolidin-2-one | 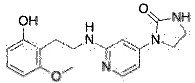 | +++ |
| E226 | 4-(3-chlorophenoxy)-N-(2-methoxyphenethyl)pyri midin-2-amine | 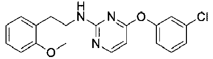 | + |
| E236 | (6,6-difluoro-3-azabicyclo[3.1.0]hexan-3-yl)(2-((2,3-dihydrobenzofuran-3-yl)amino)pyridin-4-yl)methanone | 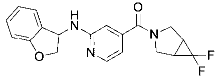 | + |
| E237 | *N*-(2-((2-methoxyphenethyl)ami no)pyridin-4-yl)pyrrolidine-1-carboxamide | 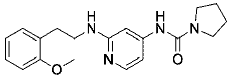 | ++++ |
| E238 | 1-(2-(((1r,3r)-3-(2-methoxyphenyl)cyclobu tyl)amino)pyridin-4-yl)imidazolidin-2-one | 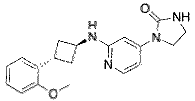 | ++++ |
| E202 | *N,N*-diethyl-2-((2-methoxyphenethyl)ami no)isonicotinamide | 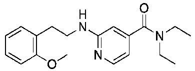 | ++ |
| E203 | (2-(3,4-dihydroisoquinolin-2(1*H*)-yl)pyridin-4-yl)(2-methylpiperidin-1-yl)methanone | 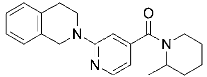 | ++ |
| E204 | (2-ethylpiperidin-1-yl)(2-((4-fluorophenethyl)amino) pyridin-4-yl)methanone | 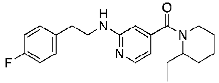 | ++ |
| E205 | (2-ethylpiperidin-1-yl)(2-((2-fluorophenethyl)amino) pyridin-4-yl)methanone | 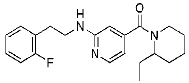 | ++ |
| E206 | (2-((2-(1*H*-indol-3-yl)ethyl)amino)pyridin-4-yl)(morpholino)methan o | 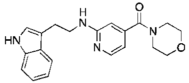 | + |
| E207 | (2-((2-(2-fluorophenoxy)ethyl)am ino)pyridin-4-yl)(piperidin-1-yl)methanone | 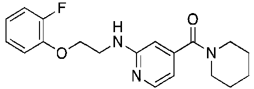 | + |
| E210 | (2-ethylpiperidin-1-yl)(2-((4-methoxyphenethyl)ami no)pyridin-4-yl)methano | 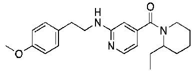 | ++ |
| E211 | (2-((2-(1*H*-indol-3-yl)ethyl)amino)pyridin-4-yl)(2-ethylpiperidin-1-yl)methanone | 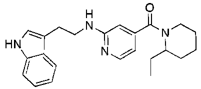 | ++ |
| E212 | (3,4-dihydroisoquinolin-2(1*H*-yl)(2-((2-methoxyphenethyl)ami no)pyridin-4-yl)methanone | 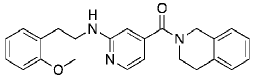 | ++ |
| E213 | (2-((3,4-dimethoxyphenethyl)a mino)pyridin-4-yl)(2-ethylpiperidin-1-yl)methanone | 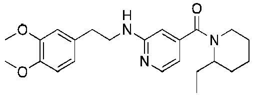 | ++ |
| E214 | 2-((2-(1*H*-indol-3-yl)ethyl)amino)-*N-*benzyl-*N-*methylisonicotinamide | 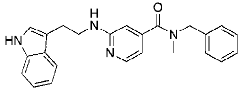 | ++ |
| E216 | (2-ethylpiperidin-1-yl)(2-((2-methoxybenzyl)amino) pyridin-4-yl)methanone | 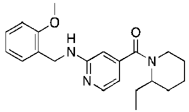 | ++ |
| E217 | *N*-benzyl-2-((2-methoxybenzyl)amino)-*N-*methylisonicotinamide | 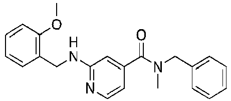 | ++ |
| E220 | *N*-isobutyl-2-((2-methoxybenzyl)amino)i sonicotinamide | 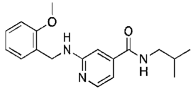 | + |
| E224 | 2-((furan-2-ylmethyl)amino)-*N*-(3-(trifluoromethyl)phenyl)i sonicotinamide | 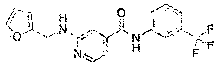 | + |
| E225 | 2-((2-(1*H*-indol-3-yl)ethyl)amino)-N-(4-fluorobenzyl)isonicotina mide | 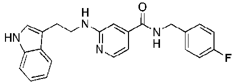 | ++ |
| E227 | (2-((2-methoxyphenethyl)ami no)-6-methylpyrimidin-4-yl)(piperidin-1-yl)methanone | 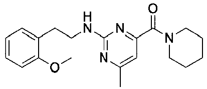 | + |
| E228 | 2-((2-((2-(1H-indol-3-yl)ethyl)amino)-6-methylpyrimidin-4-yl)amino)benzonitrile | 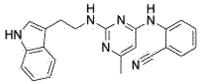 | +++ |
| E230 | azepan-1-yl(6-((2-methoxyphenethyl)ami no)pyrimidin-4-yl)methanone | 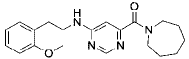 | + |
| E232 | 6-((2-(1H-indol-3-yl)ethyl)amino)-*N-*benzyl-N,2-dimethylpyrimidine-4-carboxamide | 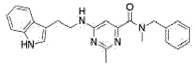 | ++ |
| E233 | 2-phenoxy-*N*-(6-(piperidin-1-yl)pyrimidin-4-yl)acetamide | 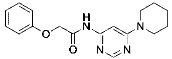 | ++ |
| E234 | *N*4,*N*6-bis(2-methoxybenzyl)-2-methylpyrimidine-4,6-diamine | 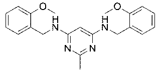 | +++ |
| E235 | *N*4-(2-chlorobenzyl)-*N*6-(2-methoxyphenethyl)-2-methylpyrimidine-4,6-diamine | 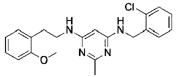 | +++ |

**Table 4: CCND1 protein detection in Huh7 by Western blot: The symbol "A" indicates more than 50% degradation at the indicated compound concentration; the symbol "B" indicates less than 50% degradation at the indicated concentration, but still a detectable and significant degradation.**

| Example | Structure | % CCND1 degradation at | |
|---|---|---|---|
| | | 3µM | 10µM |
| E70 | 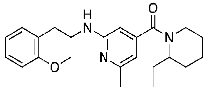 | A | A |
| E22 | 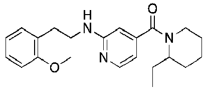 | B | A |
| E42 | 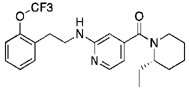 | A | A |
| E15 | 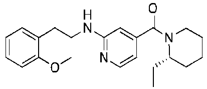 | A | A |
| E59 | 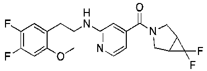 | B | A |
| E67 | 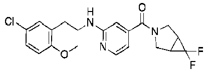 | A | A |
| E58 | 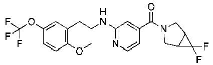 | A | A |
| E68 | 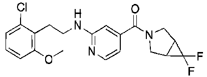 | A | A |
| E149 | 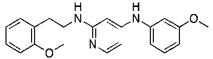 | A | A |
| E47 | 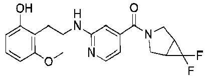 | A | A |
| E6 | 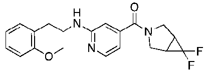 | A | A |
| E21 | 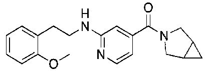 | A | A |
| E3 | 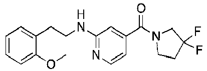 | B | A |
| E18 | 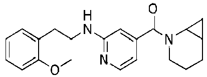 | B | A |
| E4 | 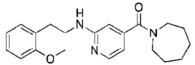 | B | A |
| E2 | 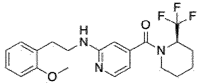 | A | A |
| E40 | 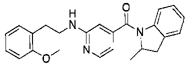 | A | A |
| E153 | 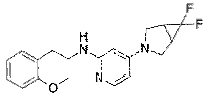 | A | A |
| E148 | 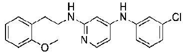 | A | A |
| E150 | 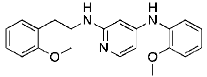 | A | A |
| E60 | 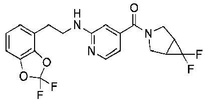 | A | A |
| E51 | 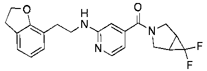 | A | A |
| E50 | 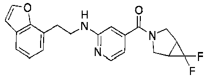 | A | A |
| E44 | 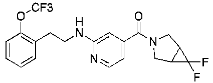 | A | A |
| E52 | 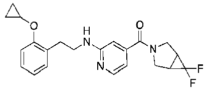 | A | A |
| E104 | 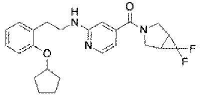 | A | A |
| E84 | 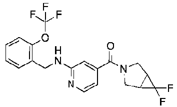 | A | A |
| E235 | 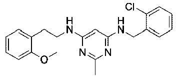 | B | A |
| E161 | 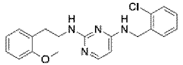 | A | A |
| E164 | 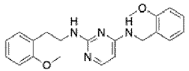 | A | A |
| E234 | 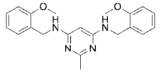 | B | B |
| E166 | 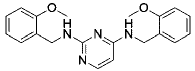 | A | A |
| E163 | 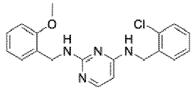 | A | A |
| E228 | 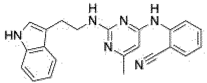 | B | A |
| E232 | 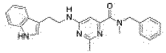 | | B |
| E168 | 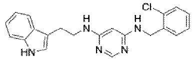 | B | B |
| E162 | 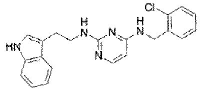 | A | A |
| E189 | 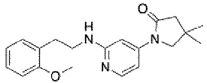 | A | A |
| E139 | 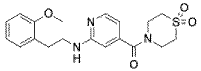 | A | A |
| E182 | 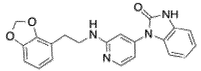 | A | A |
| E238 | 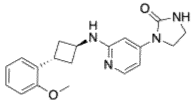 | A | A |
| E48 | 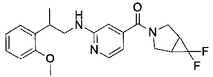 | A | |
| E115 | 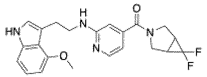 | A | |
| E56 | 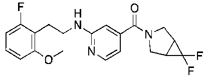 | A | |
| E46 | 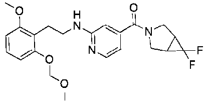 | A | |

## Claims

1. A compound of formula (I): 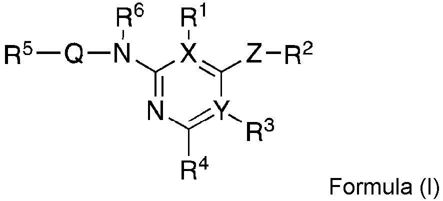 wherein, independently for each occurrence:
- X and Y are selected from C or N atom
- Z is a direct bond, -C(=O)-, -C(=S)-, or -(CH2)n- , wherein n represents an integer from 1 to 2;
- R¹ is selected from the group consisting of -H, halogen, and -CH3 with the proviso that if X is a nitrogen atom, then R¹ is null
- R² is selected from the group consisting of:
a) -NHR⁷
b) -NR⁷R⁸
c) -NHC(=O)R⁷
d) -NHC(=O)NR⁷R⁸
e) -C(=O)H
f) -OR⁷
g) 5-membered heteroaromatic ring
- R³ is selected from the group consisting of -H, -F, -CH3, -CF3, and -CHF2 with the proviso that if Y is a nitrogen atom, then R³ is null
- R⁴ is selected from the group consisting of -H, -CH3, -NH2, halogen, hydroxy, methoxy, secondary and tertiary amine, cycloalkylamine;
- Q is a direct bond, C1-4 alkanediyl, substituted C1-4 alkanediyl, cycloalkanediyl, substituted cycloalkanediyl, azetidine, piperidine, -CH2C(=O)-, -NHC(=O)-, -OCH2C(=O)- ;
- R⁵ is selected from the group consisting of -(C1-6)-alkyl, -(C1-6)-alkyloxy, a monocyclic or bicyclic 5- to 14-membered aryl, wherein aryl is unsubstituted or mono-, di- or tri-substituted halogen, alkylhalogen, of-(C1-6)-alkyl, -(C1-6)-alkyloxy, -(C1-6)-haloalkyloxy, aryl or heteroaryl;
- R⁶ is selected from the group consisting of -H, -CH3
- R⁷ and R⁸ can be the same or different and independently represent a hydrogen atom, -(C1-6)-alkyl, -(C1-6)- cycloalkyl, phenyl or benzyl, wherein those can be unsubstituted or mono-, di or trisubstituted independently of one another by R⁹
or
R⁷ and R⁸ can form together with the nitrogen atom to which they are attached a 4- to 8-term ring, substituted or unsubstituted condensed bicycles or spiro cycles,
or
R⁷ and R⁸ can form together with the nitrogen atom to which they are attached a substituted or unsubstituted cyclic amine, a substituted or unsubstituted cyclic urea, substituted or unsubstituted cyclic carbamate, substituted or unsubstituted lactam, - R⁹ is selected from the group consisting of halogen, -OH, -CN, -OCH3, - OCF3;

2. The compound according to claim 1, wherein said compound is selected from the group consisting of:

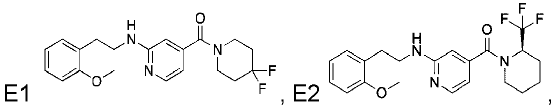

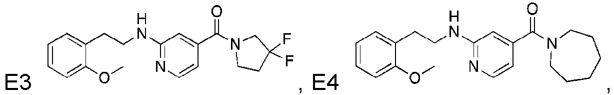

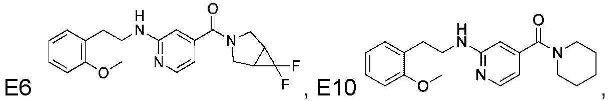

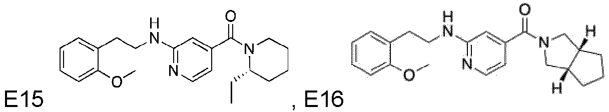

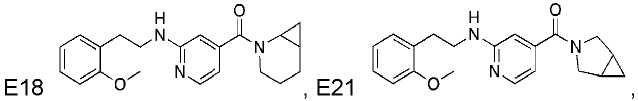

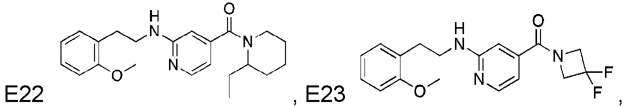

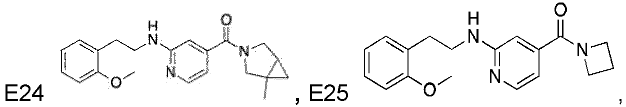

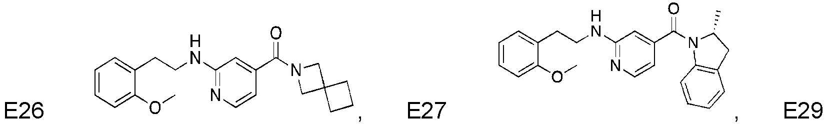

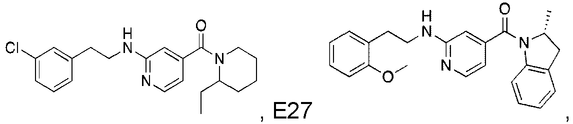

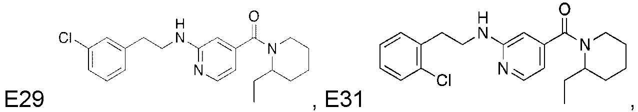

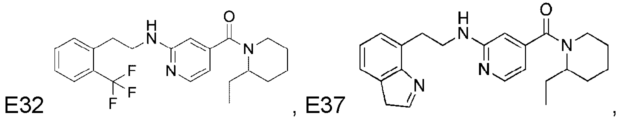

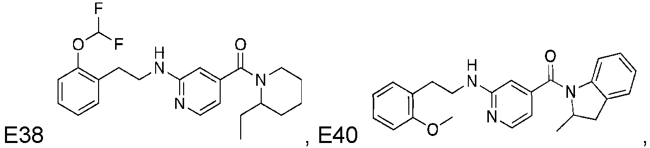

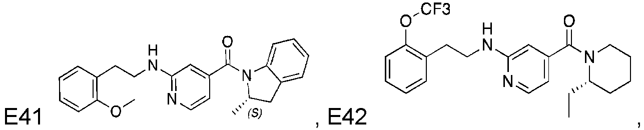

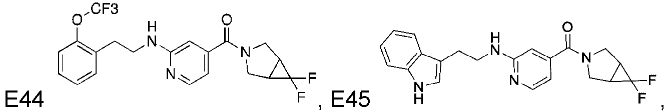

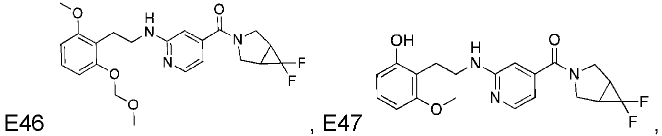

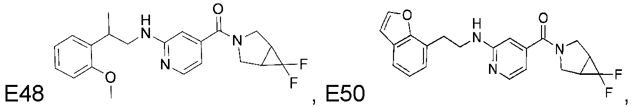

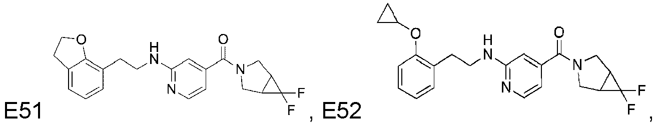

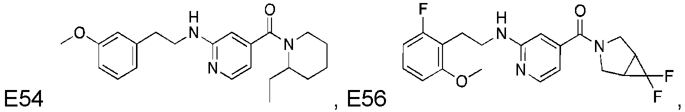

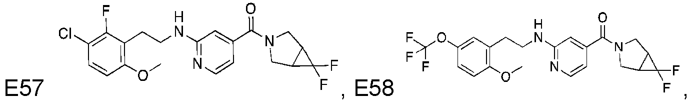

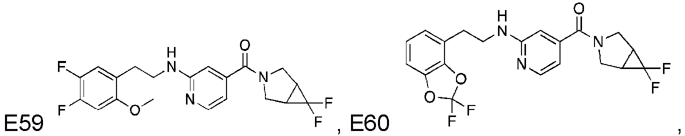

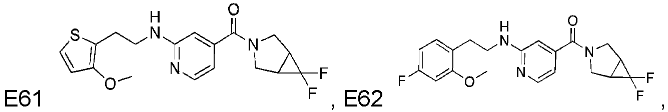

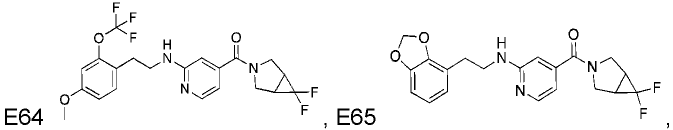

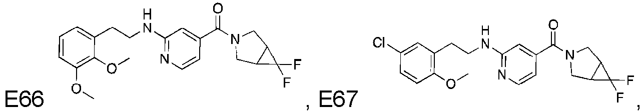

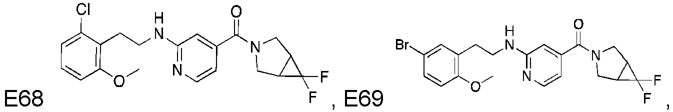

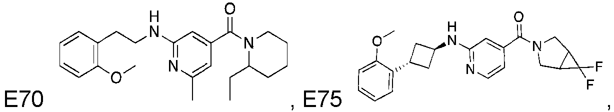

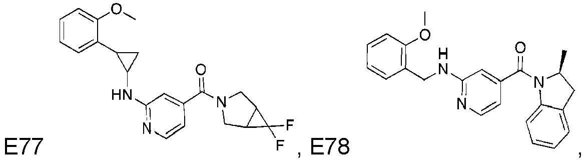

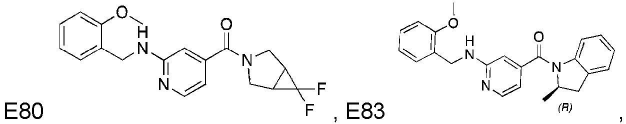

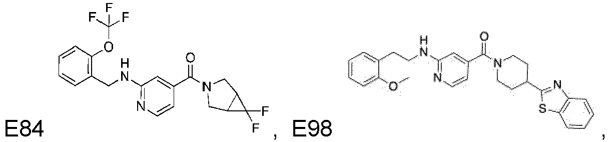

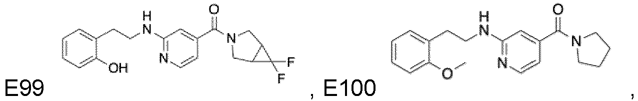

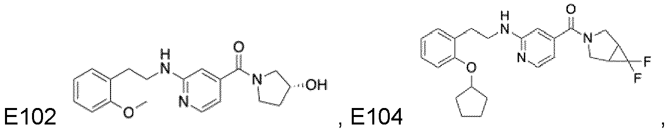

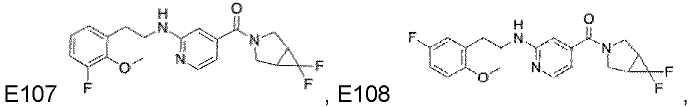

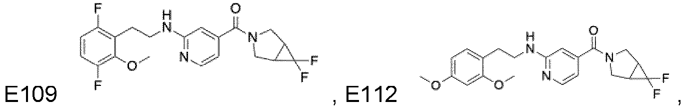

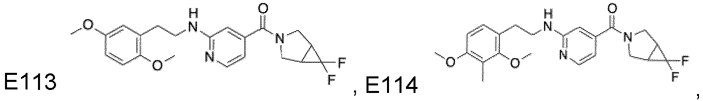

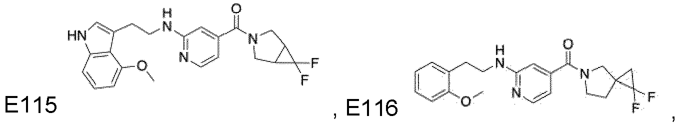

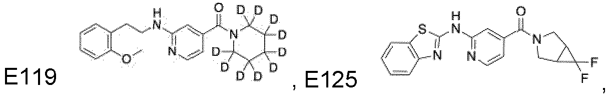

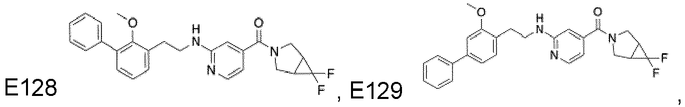

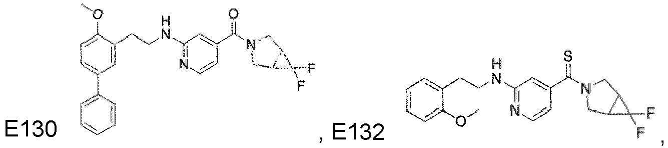

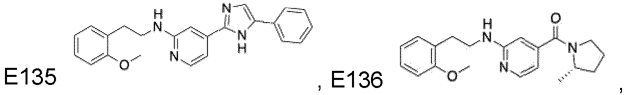

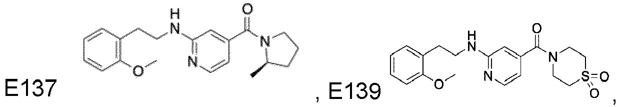

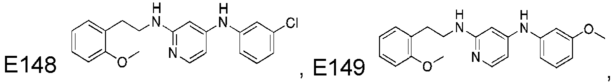

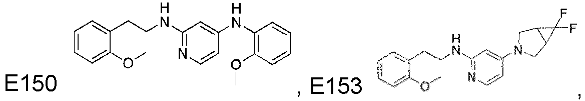

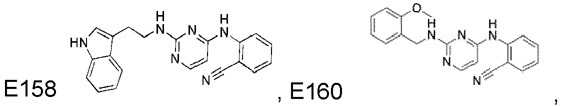

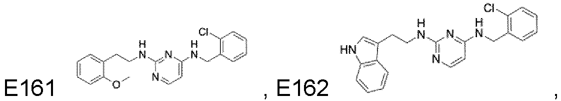

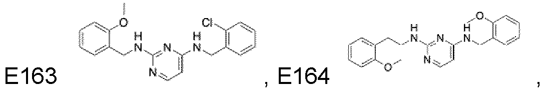

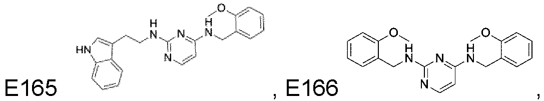

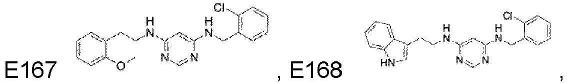

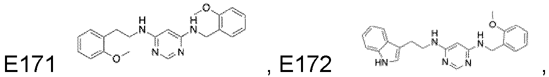

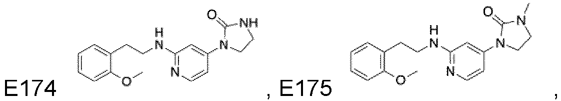

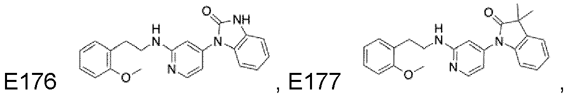

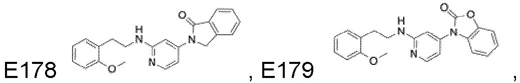

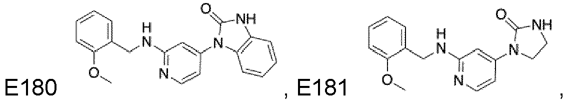

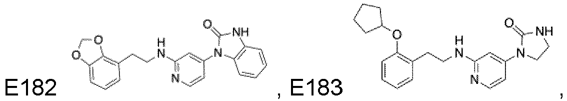

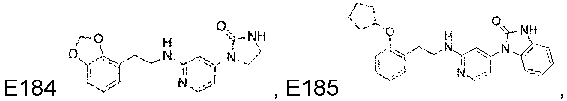

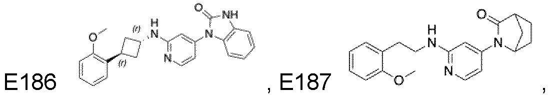

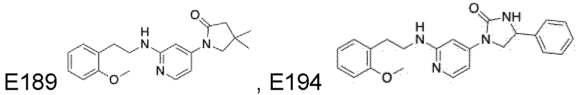

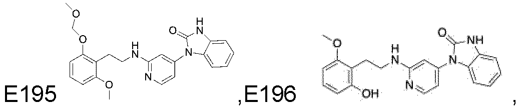

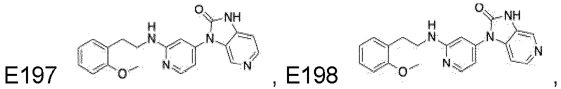

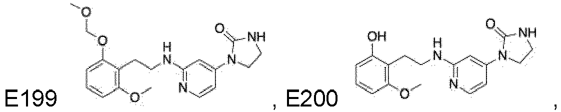

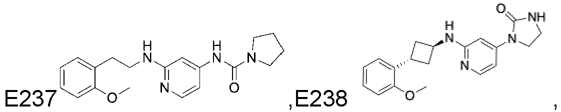

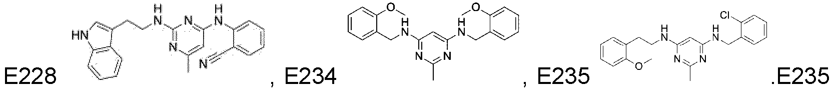

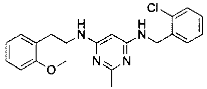

3. The compound according to any one of the preceding claims, wherein the compound is selected from the group consisting of E1, E2, E3, E4, E6, E10, E15, E16, E18, E21, E22, E23, E24, E25, E27, E29, E31, E32, E37, E38, E40, E41, E42, E44, E45, E46, E47, E48, E50, E51, E52, E54, E56, E57, E58, E59, E60, E61, E62, E64, E65, E66, E67, E68, E69, E70, E75, E77, E78, E80, E84, E99, E98, E100, E102, E104, E107, E115, E116, E119, E125, E128, E129, E130, E132, E135, E136, E137, E148, E149, E150, E153, E158, E160, E161, E162, E163, E164, E165, E166, E167, E168, E171, E172, E174, E175, E176, E177, E178 E179, E180, E181, E182, E183, E184, E185, E186, E187, E189, E194, E195, E196, E199, E237, E238, E228, E234, E235.

4. The compound according to any one of the preceding claims, wherein the compound is selected from the group consisting of: E6, E15, E22, E27, E40, E42, E44, E46, E51, E58, E60, E69, E77, E100, E104, E113, E129, E137, E139, E148, E150, E161, E162, E163, E164, E166, E174, E176, E177, E181, E182, E183, E184, E185, E189, E199, E237, E238.

5. The compound according to any one of the preceding claims, for use in the treatment or prevention of cancer.

6. The compound for use according to claim 5, wherein the cancer is selected from the group consisting of head and neck squamous cell carcinoma, nasopharyngeal carcinoma (NPC), small cell lung cancer, non-small-cell lung cancer (NSCLC), endometrial cancer, melanoma, pancreatic cancer, gastric cancer, breast cancer, colorectal cancer, mantle cell lymphoma, multiple myeloma, prostate cancer, and papillary thyroid carcinoma (PTC).

7. The compound for use according to claim 5 or 6, wherein said cancer is breast cancer or liver cancer.

8. A pharmaceutical composition comprising at least one compound according to any one of the claims 1-4 or its salt or solvate and one or more pharmaceutically acceptable excipients.

9. The pharmaceutical composition according to claim 8, for use in the treatment or prevention of cancer.

10. The pharmaceutical composition for use according to claim 9, wherein the cancer is selected from the group consisting of head and neck squamous cell carcinoma, nasopharyngeal carcinoma (NPC), small cell lung cancer, non-small-cell lung cancer (NSCLC), endometrial cancer, melanoma, pancreatic cancer, gastric cancer, breast cancer, colorectal cancer, mantle cell lymphoma, multiple myeloma, prostate cancer, and papillary thyroid carcinoma (PTC).

11. The pharmaceutical composition for use according to claim 9 or 10, wherein the cancer is breast cancer or liver cancer.
